(19) Europäisches Patentamt
European Patent Office
Office européen des brevets

(11) EP 4 721 767 A1

(12) **EUROPEAN PATENT APPLICATION**
published in accordance with Art. 153(4) EPC

(43) Date of publication:
**08.04.2026 Bulletin 2026/15**

(21) Application number: **24818681.9**

(22) Date of filing: **05.06.2024**

(51) International Patent Classification (IPC):
***A61K 47/68*** *(2017.01)* ***C07D 491/22*** *(2006.01)*
***A61P 35/00*** *(2006.01)*

(52) Cooperative Patent Classification (CPC):
**A61K 31/4745; A61K 47/68; A61P 35/00; C07D 491/22; C07K 16/28**

(86) International application number:
**PCT/CN2024/097511**

(87) International publication number:
**WO 2024/251149 (12.12.2024 Gazette 2024/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **05.06.2023 CN 202310655433**

(71) Applicants:
- **Jiangsu Mabwell Health Pharmaceutical R&D Co., Ltd.**
  **China Medical City**
  **Taizhou, Jiangsu 225300 (CN)**
- **Mabwell (Shanghai) Bioscience Co., Ltd.**
  **Shanghai 201210 (CN)**

(72) Inventors:
- **TAN, Xiaoding**
  **Taizhou, Jiangsu 225300 (CN)**
- **MEI, Fei**
  **Taizhou, Jiangsu 225300 (CN)**
- **FANG, Peng**
  **Taizhou, Jiangsu 225300 (CN)**
- **YOU, Meng**
  **Taizhou, Jiangsu 225300 (CN)**
- **YU, Dongan**
  **Taizhou, Jiangsu 225300 (CN)**
- **YIN, Long**
  **Taizhou, Jiangsu 225300 (CN)**
- **SUN, Xiaowei**
  **Taizhou, Jiangsu 225300 (CN)**
- **XU, Hui**
  **Shanghai 201210 (CN)**
- **ZHU, Huikai**
  **Shanghai 201210 (CN)**
- **WANG, Zhenzhen**
  **Shanghai 201210 (CN)**
- **ZHOU, Wei**
  **Shanghai 201210 (CN)**
- **LIU, Datao**
  **Shanghai 201210 (CN)**

(74) Representative: **Schiweck Weinzierl Koch Patentanwälte Partnerschaft mbB**
**Ganghoferstraße 68 B**
**80339 München (DE)**

Remarks:
The complete document including Reference Table(s) and the Sequence Listing(s) can be downloaded from the EPO website

# (54) ANTI-B7-H3 ANTIBODY OR FRAGMENT THEREOF, AND ANTIBODY DRUG CONJUGATE TARGETING B7-H3

(57) A series of novel anti-B7-H3 antibodies. The antibodies have high affinity to B7-H3 and have stable structures. Also provided is an antibody drug conjugate prepared by using said antibody. The antibody drug conjugate uses a camptothecin compound having a specific structure as a toxic molecule, has significant tumor inhibition activity, and has a bystander killing effect.



Processed by Luminess, 75001 PARIS (FR)

## Description

### CROSS-REFERENCE TO RELATED APPLICATION(S)

The present patent application claims the benefit of priority to Chinese Patent Application No. CN202310655433.X filed on 5 June 2023, which is incorporated herein by reference in its entirety.

### TECHNICAL FIELD

The invention belongs to the field of biotechnology, and specifically relates to an antibody-drug conjugate targeting B7-H3 and its application in tumor treatment.

### BACKGROUND OF THE INVENTION

B7-H3, also known as B7H3 or CD276, is a type I transmembrane protein belonging to the B7 family of proteins, which includes co-stimulatory and co-inhibitory members. Encoded by chromosome 15q24, B7-H3 consists of an extracellular region, a transmembrane region, and a short intracellular region. Extensive immunohistochemical (IHC) analysis of normal tissues has shown that B7-H3 is expressed at low levels in only a few tissues or is even undetectable. However, B7-H3 has been found to be overexpressed in various solid tumors, such as bladder cancer, prostate cancer, and melanoma. Furthermore, as research progresses, increasing evidence indicates that B7-H3 primarily exerts a co-inhibitory function in immune cells, which facilitates tumor cells' evasion of immune surveillance. Consequently, the overexpression of B7-H3 is associated with poor prognosis in patients suffering from tumors and correlates with tumor invasion and metastatic potential in vitro models.

Currently, more than 20 clinical trials have been or are being conducted to evaluate the safety and efficacy of B7-H3-targeting monoclonal antibody immunotherapy strategies. Additionally, multiple B7-H3-targeting antibody-drug conjugate (ADC) agents have entered clinical trial stages globally.

The B7-H3-targeting ADC agent DS-7300, developed by Daiichi Sankyo Co Ltd., is prepared by conjugating the Linker-payload MC-GGFG-Dxd to the anti-B7-H3 antibody hM30 via stochastic conjugation, resulting in a B7-H3-targeting ADC with a drug-to-antibody ratio (DAR) of 4. Its structure is shown below:

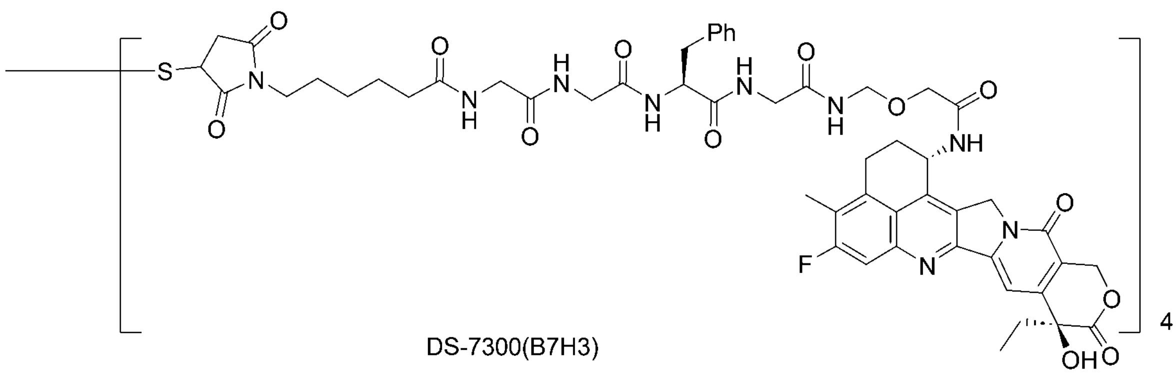

DS-7300(B7H3)

The B7-H3-targeting ADC agent MGC018, developed by MacroGenics, Inc., is composed of an anti-B7-H3 humanized IgG1 monoclonal antibody conjugated to Duocarmycin (DUBA) via a cleavable linker, with an average DAR of 2.7. Its structure is shown below:

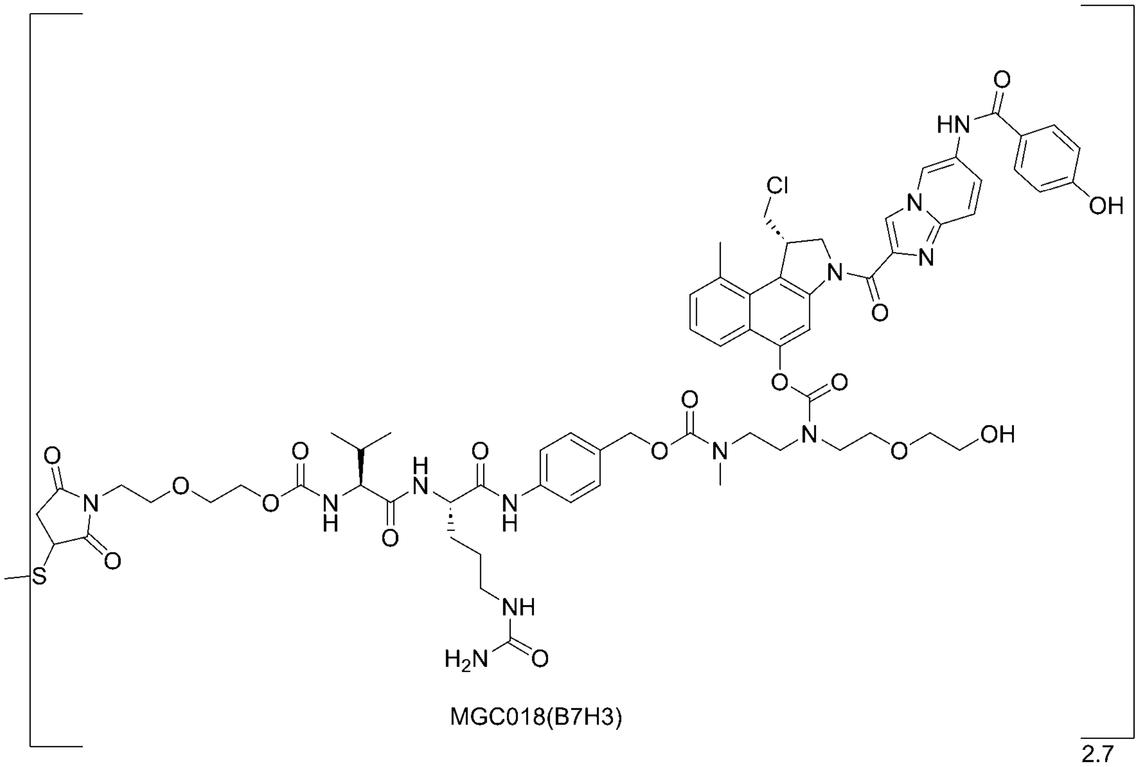

MGC018(B7H3)

The B7-H3-targeting ADC agent BAT8009, developed by Bio-Thera Solutions, Ltd., is composed of a recombinant anti-B7-H3 humanized antibody conjugated to a DNA topoisomerase inhibitor via a cleavable linker.

The B7-H3-targeting ADC agent ABBV-155, developed by AbbVie Inc., is composed of an anti-B7-H3 antibody conjugated to an apoptosis-promoting BCL-XL inhibitor via a linker, and is intended for the treatment of advanced solid tumors. Its structure is shown below:

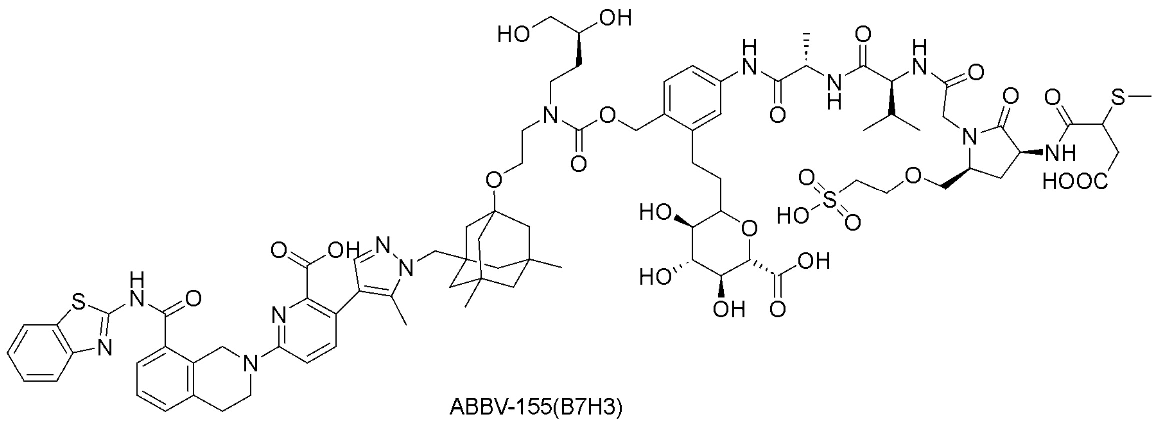

ABBV-155(B7H3)

The aforementioned ADC agents have all demonstrated certain therapeutic efficacy, though with limitations. For instance, in 2021, MacroGenics reported Phase I clinical trial results (cohort expansion, NCT03729596) of MGC018 for treating advanced solid tumors. Regarding safety, 43 patients (50%) experienced Grade 3 and higher treatment-related adverse events (TRAEs), with common severe TRAEs including neutropenia (22.1%) and thrombocytopenia (7%). In contrast, DS-7300 developed by Daiichi Sankyo, employs a stochastic conjugation method, which results in significant heterogeneity in the ADC product.

Currently, there is still a limited selection of B7-H3-targeting ADCs incorporating camptothecins. Furthermore, in light of the aforementioned limitations associated with existing ADCs of this class, there remains a need in the art to provide B7-H3-targeting ADCs incorporating camptothecins that demonstrate improved targeting, reduced product heterogeneity, minimized side effects, and consequently, enhanced therapeutic efficacy.

The Chinese patent application CN202111459992.0 (Publication CN114573695A), filed on 2 December, 2021 and entitled "ANTI-HUMAN B7-H3 ANTIBODY AND APPLICATION THEREOF", discloses anti-human B7-H3 murine and

humanized monoclonal antibodies. In this application, the recombinant extracellular domain of human B7-H3 was used as an immunogen to generate anti-human B7-H3 murine monoclonal antibodies capable of binding the B7-H3 extracellular domain through hybridoma technology; subsequently, human-mouse chimeric antibodies capable of specifically binding B7-H3 on the cell surface were constructed based on the murine antibodies; furthermore, humanized antibodies were developed through CDR grafting and CDR mutagenesis, and these humanized antibodies retained the ability to specifically bind both the human B7-H3 extracellular domain and cell membrane-bound B7-H3, and could undergo internalization mediated by cell membrane-bound B7-H3. However, although the murine and humanized antibodies described in this application exhibit favorable functional properties, there remains room for further improvement.

## SUMMARY OF THE INVENTION

The inventors of the present invention have found that although an anti-B7-H3 humanized antibody disclosed in CN114573695A exhibits high affinity for human B7-H3, it is prone to fragmentation. In practical applications of an antibody, such fragmentation of the antibody will lead to increased heterogeneity of a product containing the antibody, thereby elevating immunogenicity, reducing efficacy, and potentially causing toxic side effects. Therefore, this humanized antibody presents certain limitations for both direct use and further product development.

Therefore, based on this known anti-B7-H3 humanized antibody, the inventors of the present invention employed site-directed mutagenesis in the variable regions to screen for novel anti-B7-H3 antibodies capable of overcoming the aforementioned limitations. Furthermore, they conjugated the mutated antibodies with camptothecins of specific structures to generate antibody-drug conjugates (ADCs), aiming to identify ADCs with improved B7-H3 targeting and enhanced cytotoxicity against B7-H3-expressing cells.

Accordingly, an object of the present disclosure is to provide an antibody or fragment thereof that specifically binds to B7-H3. Another object of the present disclosure is to provide an antibody-drug conjugate targeting B7-H3 or a salt thereof, prepared using the antibody or fragment thereof.

In the context of the present disclosure, halogen refers to fluorine (F), chlorine (Cl), bromine (Br) or iodine (I).

In the context of the present disclosure, the term "linker" and the term "linker compound" are used interchangeably.

In the context of the present disclosure, the term "linker-payload" refers to a compound resulting from covalent bonding of a drug (e.g., a small molecule drug, such as a compound of camptothecins) to a linker directly or indirectly.

The present disclosure provides technical solutions as follows.

### First Aspect

The present disclosure provides an anti-B7-H3 antibody or fragment thereof comprising a heavy chain and a light chain, wherein the heavy chain and the light chain comprise three heavy chain complementarity determining regions, i.e., CDR-H1, CDR-H2, and CDR-H3, and three light chain complementarity determining regions, i.e., CDR-L1, CDR-L2, and CDR-L3, derived from the following combinations of amino acid sequences:

(i) the amino acid sequence shown in SEQ ID NO: 1; and, the amino acid sequence shown in SEQ ID NO: 3;
(ii) the amino acid sequence shown in SEQ ID NO: 1; and, the amino acid sequence shown in SEQ ID NO: 4; or
(iii) the amino acid sequence shown in SEQ ID NO: 1; and, the amino acid sequence shown in SEQ ID NO: 5.

In the context of the present disclosure, the term "fragment" encompasses various functional fragments of the anti-B7-H3 antibody that retain the binding capacity to the antigen and the corresponding biological activity of the antibody. It is commonly known in the art that the antigen-binding capacity and corresponding biological activity of an antibody can be achieved by fragments of the full-length antibody. Such fragments can be obtained using conventional techniques known to a person skilled in the art and screened for functionality in the same manner as the full-length antibody. For example, antigen-binding fragments of an antibody may be produced by recombinant DNA techniques, or by enzymatic or chemical cleavage of the intact antibody. Illustrative fragments may be, Fab, $F(ab')_2$, scFv fragments, or the like.

The amino acid sequences described above correspond to the amino acid sequences of the heavy chain variable regions and the light chain variable regions of the specific anti-B7-H3 antibodies provided by the present disclosure; refer to the **"DETAILED DESCRIPTION OF PREFERRED EMBODIMENTS"** section of the present disclosure. By applying commonly known or conventional definition tools for complementarity determining regions (CDRs) in antibody heavy or light chain variable regions (such as the Kabat definition, AbM definition, Chothia definition, Contact definition, IMGT definition, or enhanced Chothia/AbM definition), a person skilled in the art can readily determine the heavy chain CDRs and light chain CDRs contained when these variable regions combine to form an antibody. For example, in Example 1 of the present disclosure, the positions of amino acid sequences of CDRs within the full-length variable regions are specified when defined according to the Kabat definition. Combinations of the heavy chain CDRs and light chain CDRs can be

derived based on these commonly known or conventional definition tools in the art, and antibodies or fragments thereof, each comprising any combination of these heavy chain CDRs and light chain CDRs, fall within the scope of the invention.

Further, the anti-B7-H3 antibody or fragment thereof provided in the present disclosure may comprise a combination of heavy chain CDRs and light chain CDRs (CDR-H1, CDR-H2, and CDR-H3; and CDR-L1, CDR-L2, and CDR-L3) selected from the group consisting of:

(i) The CDR-H1, CDR-H2, and CDR-H3 respectively comprise amino acid sequences having at least 75% identity to the amino acid sequences shown in SEQ ID NO: 8, SEQ ID NO: 9, and SEQ ID NO: 10; and the CDR-L1, CDR-L2, and CDR-L3 respectively comprise amino acid sequences having at least 75% identity to the amino acid sequences shown in SEQ ID NO: 11, SEQ ID NO: 12, and SEQ ID NO: 14;
(ii) The CDR-H1, CDR-H2, and CDR-H3 respectively comprise amino acid sequences having at least 75% identity to the amino acid sequences shown in SEQ ID NO: 8, SEQ ID NO: 9, and SEQ ID NO: 10; and the CDR-L1, CDR-L2, and CDR-L3 respectively comprise amino acid sequences having at least 75% identity to the amino acid sequences shown in SEQ ID NO: 11, SEQ ID NO: 12, and SEQ ID NO: 15; and
(iii) The CDR-H1, CDR-H2, and CDR-H3 respectively comprise amino acid sequences having at least 75% identity to the amino acid sequences shown in SEQ ID NO: 8, SEQ ID NO: 9, and SEQ ID NO: 10; and the CDR-L1, CDR-L2, and CDR-L3 respectively comprise amino acid sequences having at least 75% identity to the amino acid sequences shown in SEQ ID NO: 11, SEQ ID NO: 12, and SEQ ID NO: 16.

In the context of the present disclosure, the term "at least 75% identity" encompasses any percentage identity from at least 75% identity to 100% identity, for example, 75%, 80%, 85%, 90%, and even 91%, 92%, 93%, 94%, 95%, 96%, 97%, 98%, 99%, or even 100% identity. Depending on the context in which the term is used, the "at least 75% identity" permits up to 25% variance in the amino acid sequence, which may be located within the heavy or light chain CDRs, or within any region outside the heavy or light chain CDRs. Such regions include, but are not limited to, the framework regions of the heavy chain variable region and light chain variable region of the antibody or fragment thereof provided by the present disclosure, or the heavy chain constant region and light chain constant region of the antibody or fragment thereof provided by the present disclosure. The variance may result from deletion, addition, or substitution of amino acids at any position, wherein the substitution may be a conservative substitution or a non-conservative substitution.

According to specific embodiments of the invention, the anti-B7-H3 antibody or fragment thereof provided by the present disclosure may comprise a combination of heavy chain CDRs and light chain CDRs (CDR-H1, CDR-H2, and CDR-H3; and CDR-L1, CDR-L2, and CDR-L3) selected from the group consisting of:

(i) The CDR-H1, CDR-H2, and CDR-H3 respectively comprise the amino acid sequences shown in SEQ ID NO: 8, SEQ ID NO: 9, and SEQ ID NO: 10; and the CDR-L1, CDR-L2, and CDR-L3 respectively comprise the amino acid sequences shown in SEQ ID NO: 11, SEQ ID NO: 12, and SEQ ID NO: 14;
(ii) The CDR-H1, CDR-H2, and CDR-H3 respectively comprise the amino acid sequences shown in SEQ ID NO: 8, SEQ ID NO: 9, and SEQ ID NO: 10; and the CDR-L1, CDR-L2, and CDR-L3 respectively comprise the amino acid sequences shown in SEQ ID NO: 11, SEQ ID NO: 12, and SEQ ID NO: 15; and
(iii) The CDR-H1, CDR-H2, and CDR-H3 respectively comprise the amino acid sequences shown in SEQ ID NO: 8, SEQ ID NO: 9, and SEQ ID NO: 10; and the CDR-L1, CDR-L2, and CDR-L3 respectively comprise the amino acid sequences shown in SEQ ID NO: 11, SEQ ID NO: 12, and SEQ ID NO: 16.

The antibody or fragment thereof provided by the present disclosure may be an antibody or fragment thereof that binds to B7-H3, particularly human B7-H3. The amino acid sequence of human B7-H3 is exemplarily provided in UniProtKB-Q5ZPR3.

The anti-B7-H3 antibody or fragment thereof provided by the present disclosure may comprise at least a heavy chain variable region (VH) and a light chain variable region (VL). Both regions include the aforementioned CDRs as well as framework regions (FRs) interspersed therebetween, arranged in the order of FR1-CDR1-FR2-CDR2-FR3-CDR3-FR4. Furthermore, in the anti-B7-H3 antibody or fragment thereof provided by the present disclosure, the heavy chain variable region may comprise the amino acid sequence shown in SEQ ID NO: 1, or an amino acid sequence having at least 75% identity to the amino acid sequence shown in SEQ ID NO: 1; and/or the light chain variable region may comprise the amino acid sequence shown in any one of SEQ ID NOs: 3-5, or an amino acid sequence having at least 75% identity to the amino acid sequence shown in any one of SEQ ID NOs: 3-5.

Preferably, in the antibody or fragment thereof provided by the present disclosure, the heavy chain variable region and the light chain variable region may comprise a sequence combination selected from the group consisting of:

(i) The heavy chain variable region comprises an amino acid sequence having at least 75% identity to the amino acid sequence shown in SEQ ID NO: 1; and the light chain variable region comprises an amino acid sequence having at

least 75% identity to the amino acid sequence shown in SEQ ID NO: 3;
(ii) The heavy chain variable region comprises an amino acid sequence having at least 75% identity to the amino acid sequence shown in SEQ ID NO: 1; and the light chain variable region comprises an amino acid sequence having at least 75% identity to the amino acid sequence shown in SEQ ID NO: 4; and
(iii) The heavy chain variable region comprises an amino acid sequence having at least 75% identity to the amino acid sequence shown in SEQ ID NO: 1; and the light chain variable region comprises an amino acid sequence having at least 75% identity to the amino acid sequence shown in SEQ ID NO: 5.

According to specific embodiments of the invention, the antibody or fragment thereof provided by the present disclosure may comprise a combination of the heavy chain variable region and the light chain variable region selected from the group consisting of:

(i) The heavy chain variable region comprises the amino acid sequence shown in SEQ ID NO: 1; and the light chain variable region comprises the amino acid sequence shown in SEQ ID NO: 3;
(ii) The heavy chain variable region comprises the amino acid sequence shown in SEQ ID NO: 1; and the light chain variable region comprises the amino acid sequence shown in SEQ ID NO: 4; and
(iii) The heavy chain variable region comprises the amino acid sequence shown in SEQ ID NO: 1; and the light chain variable region comprises the amino acid sequence shown in SEQ ID NO: 5.

The anti-B7-H3 antibody or fragment thereof provided by the present disclosure may be in any form comprising the defined domains described above, for example, a monoclonal antibody, a chimeric antibody, a partially or fully humanized antibody, or the like. The antibody or fragment thereof provided by the present disclosure may also be in the form of scFv, BsFv, dsFv, $(dsFv)_2$, Fab, Fab', $F(ab')_2$, Fv or the like.

In addition to the variable regions, the anti-B7-H3 antibody or fragment thereof provided by the present disclosure may further comprise a constant region, such as a heavy chain constant region (CH) and/or a light chain constant region (CL), or any one or more domains of the constant regions, for example, any one or more of CH1, CH2, CH3, CH4, and the like. Alternatively, the anti-B7-H3 antibody or fragment thereof provided by the present disclosure may comprise a heavy chain and/or a light chain, wherein for example, the heavy chain may comprise a heavy chain constant region of IgG, IgA, IgM, IgD, or IgE, and the light chain may comprise a light chain constant region of kappa or lambda type.

Preferably, the present disclosure provides an anti-B7-H3 humanized antibody or fragment thereof. More preferably, the present disclosure provides an anti-B7-H3 humanized monoclonal antibody or fragment thereof.

The present disclosure provides an isolated and structurally characterized humanized monoclonal antibody that specifically binds to B7-H3. According to specific embodiments of the invention, the anti-B7-H3 antibodies provided by the present disclosure are hz10B4m1, hz10B4m2, or hz10B4m3, as detailed in the **"DETAILED DESCRIPTION OF PREFERRED EMBODIMENTS"** section of the present disclosure.

**Second Aspect**

The present disclosure further provides a nucleic acid molecule comprising a nucleotide sequence encoding the heavy chain CDRs, the light chain CDRs, the heavy chain variable region, the light chain variable region, the heavy chain, and/or the light chain comprised by the anti-B7-H3 antibody or fragment thereof according to the invention.

**Third Aspect**

The nucleic acid molecule provided by the present disclosure may be cloned into a vector which in turn transforms or transfects a host cell. Therefore, in the third aspect, the present disclosure provides a vector comprising the nucleic acid molecule according to the invention. The vector may be a eukaryotic expression vector, a prokaryotic expression vector, an artificial chromosome, a phage vector and the like.

**Fourth Aspect**

The nucleic acid molecule or vector provided by the present disclosure may be used to transform or transfect a host cell, for the purpose of antibody preservation or expression, etc. Thus, in the fourth aspect, the present disclosure provides a host cell comprising the nucleic acid molecule and/or the vector according to the invention, or transformed or transfected with the nucleic acid molecule and/or the vector according to the invention. The host cell may be any prokaryotic or eukaryotic cell, such as a bacterial or insect, fungus, plant or animal cell.

The anti-B7-H3 antibody or fragment thereof provided by the present disclosure may be obtained using any method known in the art. For example, the host cell provided by the present disclosure is cultured under conditions that

allow the host cell to express the heavy chain and the light chain of the antibody. Optionally, the method may further comprise a step of recovering the produced antibody. As described above, for example, a fragment of the antibody may be produced by recombinant DNA techniques, or by enzymatic or chemical cleavage of the intact antibody. The anti-B7-H3 antibody or fragment thereof provided by the present disclosure may be obtained using any method known in the art. For example, the host cell provided by the present disclosure is cultured under conditions that allow the host cell to express the heavy chain and/or the light chain of the antibody for assembling them into an antibody. Optionally, the method may further comprise a step of recovering the produced antibody.

**Fifth Aspect**

The anti-B7-H3 antibody or fragment thereof, the nucleic acid molecule, the vector, and/or the host cell provided by the present disclosure may be contained in a pharmaceutical composition, more particularly a pharmaceutical preparation, to be used for various purposes as actually needed. Thus, in the fifth aspect, the present disclosure also provides a composition comprising the anti-B7-H3 antibody or fragment thereof, the nucleic acid molecule, the vector, and/or the host cell according to the invention. Preferably, the composition is a pharmaceutical composition, optionally comprising a pharmaceutically acceptable carrier, adjuvant, or excipient.

**Sixth Aspect**

The present disclosure provides use of the anti-B7-H3 antibody or fragment thereof, the nucleic acid molecule, the vector, the host cell, and/or the composition in the manufacture of a medicament for the treatment of a disease associated with B7-H3 expression (including overexpression). Preferably, the disease is a tumor or cancer. More preferably, the disease is a solid tumor, such as gastric cancer, colorectal cancer, liver cancer, pancreatic cancer, esophageal cancer, non-small cell lung cancer, prostate cancer, ovarian cancer, neuroblastoma, rhabdomyosarcoma, osteosarcoma, Ewing sarcoma, Wilms tumor, and desmoplastic small round cell tumor.

**Seventh Aspect**

The present disclosure further provides use of the anti-B7-H3 antibody or fragment thereof, the nucleic acid molecule, the vector, the host cell, and/or the composition in the manufacture of an agent for the diagnosis of a disease associated with B7-H3 expression (including overexpression). Preferably, the disease is a tumor or cancer. More preferably, the disease is a solid tumor, such as gastric cancer, colorectal cancer, liver cancer, pancreatic cancer, esophageal cancer, non-small cell lung cancer, prostate cancer, ovarian cancer, neuroblastoma, rhabdomyosarcoma, osteosarcoma, Ewing sarcoma, Wilms tumor, and desmoplastic small round cell tumor.

**Eighth Aspect**

The present disclosure also provides a method for treating a disease associated with B7-H3 expression (including overexpression), comprising administering to a subject in need thereof the anti-B7-H3 antibody or fragment thereof, the nucleic acid molecule, the vector, the host cell, and/or the composition according to the invention, optionally along with any one of other drugs or means. The other drugs or means refer to other drugs or means that can be co-administered with the antibody or fragment thereof, the nucleic acid molecule, the vector, the host cell, and/or the composition according to the invention, such as small molecule chemical drugs, targeted drugs, recombinant protein drugs like antibodies, vaccines, ADCs, oncolytic viruses, gene and nucleic acid therapeutics, and radiotherapies. The co-administration of the two may be carried out in any form, for example, simultaneously, sequentially, or at set intervals.

Preferably, the disease is a tumor or cancer. More preferably, the disease is a solid tumor, such as gastric cancer, colorectal cancer, liver cancer, pancreatic cancer, esophageal cancer, non-small cell lung cancer, prostate cancer, ovarian cancer, neuroblastoma, rhabdomyosarcoma, osteosarcoma, Ewing sarcoma, Wilms tumor, and desmoplastic small round cell tumor.

Preferably, the subject is a mammal, preferably a primate, more preferably a human.

When applied, the method for treating a disease described above provided by the present disclosure depends on multiple factors, including the specific active ingredient administered, the age, body weight, gender, physical and medical condition of the patient, the severity of the condition to be treated, the route of administration, and so forth.

**Ninth Aspect**

The present disclosure also provides a method for diagnosing a disease associated with B7-H3 expression (including overexpression), comprising contacting the anti-B7-H3 antibody or fragment thereof, the nucleic acid molecule,

the vector, the host cell, and/or the composition according to the invention to a sample from a subject. Preferably, the disease is a tumor or cancer. More preferably, the disease is a solid tumor, such as gastric cancer, colorectal cancer, liver cancer, pancreatic cancer, esophageal cancer, non-small cell lung cancer, prostate cancer, ovarian cancer, neuroblastoma, rhabdomyosarcoma, osteosarcoma, Ewing sarcoma, Wilms tumor, and desmoplastic small round cell tumor.

Preferably, the subject is a mammal, preferably a primate, more preferably a human.

**Tenth Aspect**

The present disclosure further provides a kit comprising the anti-B7-H3 antibody or fragment thereof, the nucleic acid molecule, the vector, the host cell, and/or the composition according to the invention. The kit may be intended for the therapeutic or diagnostic purposes mentioned above. Optionally, the kit may also include instructions for use.

**Eleventh Aspect**

As described above, another object of the present disclosure is to provide an antibody-drug conjugate targeting B7-H3 or a salt thereof. Preferably, the antibody-drug conjugate targeting B7-H3 or a salt thereof is prepared using the anti-B7-H3 antibody or fragment thereof provided by the present disclosure. Therefore, in the eleventh aspect, the present disclosure provides an antibody-drug conjugate or a salt thereof having a structure represented by structural formulae Ia and/or Ib:

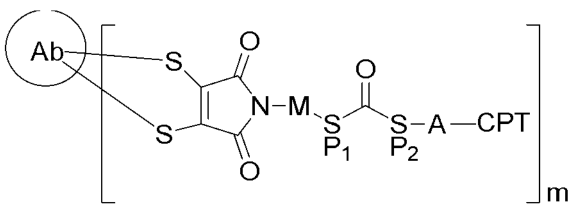

Ia

and/or

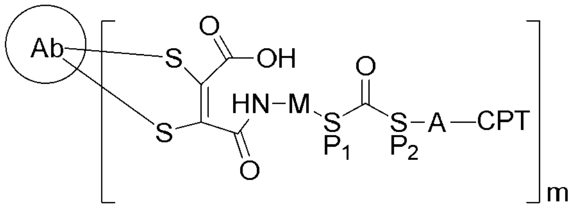

Ib,

wherein in structural formulae Ia and/or Ib, Ab is the anti-B7-H3 antibody or fragment thereof. Preferably, the antibody or fragment thereof is the anti-B7-H3 antibody or fragment thereof defined in the first aspect of the present disclosure above.

In structural formulae Ia and/or Ib, and if not otherwise stated in the context of the present disclosure, group M is phenylene or phenylene substituted with one or more substituents, or a chemical bond; and in the substituted phenylene, the phenylene is substituted with substituent(s) selected from the group consisting of alkyl (e.g., C1-6 alkyl, preferably C1-4 alkyl), haloalkyl (e.g., C1-6 haloalkyl, preferably C1-4 haloalkyl, e.g., trifluoromethyl), alkoxy (e.g., C1-6 alkoxy, preferably C1-4 alkoxy, preferably methoxy), halogen, ester, amide, and cyano; and preferably, group M is halogen-substituted phenylene.

In structural formulae Ia and/or Ib, and if not otherwise stated in the context of the present disclosure, group $SP_1$ is selected from the group consisting of C1-8 alkylene, C1-8 cycloalkylene, and C1-21 (preferably C1-16, more preferably C1-11, more preferably C5-9) linear heteroalkylene comprising 1-11 (preferably 1-6, more preferably 3-5) heteroatoms selected from the group consisting of N, O, and S, in which the C1-8 alkylene, C1-8 cycloalkylene, and C1-21 linear heteroalkylene independently of one another are optionally substituted with one or more substituents selected from the group consisting of hydroxyl, amino, sulfonic acid group, and cyano.

In structural formulae Ia and/or Ib, and if not otherwise stated in the context of the present disclosure, group $SP_2$ is selected from the group consisting of $-NH(CH_2CH_2O)_aCH_2CH_2CO-$, $-NH(CH_2CH_2O)_aCH_2CO-$, $-S(CH_2)_aCO-$, and a chemical bond, in which a is an integer in the range of 1 to 20, preferably an integer in the range of 1 to 10, more preferably

an integer in the range of 1 to 6.

In structural formulae Ia and/or Ib, and if not otherwise stated in the context of the present disclosure, group A means a peptide group of 2 to 4 amino acids. When group A means a peptide group of 2 amino acids, it may be *NH*-Phe-Lys-CO, *NH*-Val-Ala-CO, *NH*-Val-Lys-*CO*, *NH*-Ala-Lys-*CO*, *NH*-Val-Cit-*CO*, *NH*-Phe-Cit-*CO, NH-Leu-Cit-CO, NH-Phe-Arg-CO,* or *NH*-Gly-Val-*CO*, preferably *NH*-Phe-Lys-*CO*, *NH*-Val-Ala-*CO*, or *NH*-Val-Cit-*CO*. When group A means a peptide group of 3 amino acids, it may be *NH*-Glu-Val-Ala-*CO, NH*-Glu-Val-Cit-*CO,* or *NH*-Ala-Ala-Ala-*CO*, preferably *NH*-Glu-Val-Ala-*CO* or *NH*-Ala-Ala-Ala-*CO*. When group A means a peptide group of 4 amino acids, it may be *NH*-Gly-Gly-Phe-Gly-*CO* or *NH*-Gly-Phe-Gly-Gly-*CO*, preferably *NH*-Gly-Gly-Phe-Gly-*CO*. Preferably, group A is *NH*-Val-Ala-*CO, NH*-Gly-Gly-Phe-Gly-*CO* or *NH*-Ala-Ala-Ala-*CO*. In structural formulae Ia and/or Ib, group A is bonded to group $SP_2$ via the amino at the amino terminus of its short peptide structure.

In structural formulae Ia and/or Ib, group M is preferably halogen-substituted phenylene, particularly fluorine-substituted phenylene.

In structural formulae Ia and/or Ib, group $SP_1$ is preferably C1-11, preferably C5-9, more preferably C7 linear heteroalkylene containing 1-6, preferably 3-5, more preferably 4 heteroatoms selected from the group consisting of N, O, and S.

In structural formulae Ia and/or Ib, group $SP_2$ is preferably a chemical bond.

In structural formulae Ia and/or Ib, and if not otherwise stated in the context of the present disclosure, m is in the range of 1 to 10, preferably of 1 to 8 (e.g., 1 to 5), more preferably of 3 to 8. m may be an integer or a non-integer.

Further, the antibody-drug conjugate or a salt thereof provided by the present disclosure may have a structure represented by structural formulae Ic and/or Id:

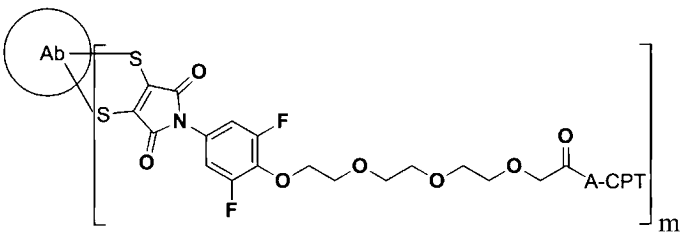

Ic

and/or

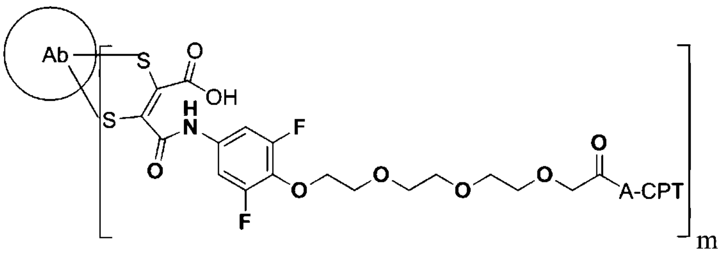

Id,

wherein in structural formulae Ic and/or Id, Ab, group A and m have the same meaning as defined above for Ab, group A and m in structural formulae Ia and/or Ib.

In any one of structural formulae Ia, Ib, Ic and Id, and if not otherwise stated in the context of the present disclosure, group CPT refers to a compound of camptothecins.

Further, in any one of structural formulae Ia, Ib, Ic and Id, group CPT may have a structure represented by structural formula I:

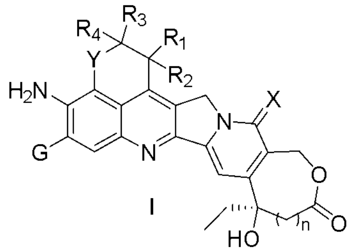

structural formula I,

wherein structural formula I is bonded to the carboxyl of group A in any one of structural formulae Ia, Ib, Ic and Id via an amide bond; and preferably, the amino adjacent to group G in structural formula I is bonded to the carboxyl of group A in any one of structural formulae Ia, Ib, Ic, and Id via an amide bond.

In structural formula I, and if not otherwise stated in the context of the present disclosure, groups $R_1$, $R_2$, $R_3$, and $R_4$ independently of one another are selected from the group consisting of hydrogen, halogen, hydroxyl, C1-6 alkoxy, amino or substituted amino, and C1-7 alkyl or substituted C1-7 alkyl, or any two of groups $R_1$, $R_2$, $R_3$, and $R_4$, together with the carbon atoms to which they are bonded, form a C3-6 (preferably C3-5) cycloalkyl. When groups $R_1$, $R_2$, $R_3$, and $R_4$ independently of one another are C1-6 alkoxy, the C1-6 alkoxy includes linear or branched C1-6 alkoxy, preferably linear or branched C1-3 alkoxy, more preferably methoxy. When groups $R_1$, $R_2$, $R_3$, and $R_4$ independently of one another are substituted amino, the substituted amino is an amino substituted with one or more substituents selected from the group consisting of methyl and ethyl. When groups $R_1$, $R_2$, $R_3$, and $R_4$ independently of one another are C1-7 alkyl or substituted C1-7 alkyl, the C1-7 alkyl or substituted C1-7 alkyl includes linear or branched C1-7 (preferably C3-5, more preferably C4) alkyl or substituted C1-7 (preferably C3-5, more preferably C4) alkyl, and the substituted C1-7 alkyl is a C1-7 alkyl substituted with one or more substituents selected from the group consisting of cyclopropyl and cyclobutyl; or, the linear or branched C1-7 alkyl or substituted C1-7 alkyl is preferably C1-3 alkyl or substituted C1-3 alkyl, e.g., methyl, and halomethyl (preferably trifluoromethyl).

In structural formula I, and if not otherwise stated in the context of the present disclosure, group G is hydrogen, halogen, methyl or methoxy. Preferably, group G is hydrogen, fluorine or chlorine.

In structural formula I, and if not otherwise stated in the context of the present disclosure, group Y is oxygen, sulfur, sulfone, sulfoxide, methylene, or substituted methylene. In the substituted methylene, either one hydrogen of methylene may be substituted, or both hydrogens of methylene may be substituted, with a substituent which can be benzyl or alkyl; and when the substituent is alkyl, the alkyl and $R_3$ and/or $R_4$, together with the carbon atoms to which they are bonded, may form a C3-6 fused or spiro ring structure; or when both hydrogens of methylene are substituted with alkyl, the two alkyl together with group Y may form a C3-6 spiro ring structure. When group Y is a substituted methylene, the substituted methylene is preferably a methylene substituted with alkyl, more preferably a linear or branched C1-4 alkyl.

Preferably, group Y is oxygen, sulfur, sulfone, sulfoxide, or methylene; or, preferably, group Y is oxygen, sulfur or methylene.

In structural formula I, and if not otherwise stated in the context of the present disclosure, group X is oxygen or sulfur.

In structural formula I, and if not otherwise stated in the context of the present disclosure, n = 0 or 1.

In structural formula I, in the case groups $R_1$, $R_2$, $R_3$, and $R_4$ are hydrogen simultaneously, group X is oxygen, and n = 0, group G cannot be hydrogen or fluorine when group Y is methylene; and group G cannot be hydrogen when group Y is oxygen or sulfur.

Preferably, groups $R_1$, $R_2$, $R_3$, and $R_4$ independently of one another are hydrogen, halogen (e.g., fluorine), C1-7 alkyl or substituted C1-7 alkyl, or any two of groups $R_1$, $R_2$, $R_3$, and $R_4$, together with the carbon atoms to which they are bonded, form a C3-6 cycloalkyl (e.g., C3-5 cycloalkyl). Further, groups $R_1$ and $R_2$ may be the same; and/or, groups $R_3$ and $R_4$ may be the same.

Preferably, group Y is a methylene substituted with alkyl, and the alkyl and $R_3$ and/or $R_4$, together with the carbon atoms to which they are bonded, may form a C3-6 fused or spiro ring structure.

Preferably, group X may be oxygen.

Preferably, group X is oxygen, group G is hydrogen, halogen (e.g. fluorine or chlorine), methyl or methoxy, and group Y and groups $R_1$, $R_2$, $R_3$, and $R_4$ are defined as above.

Preferably, group X is oxygen, group G is hydrogen, group Y is methylene or substituted methylene, oxygen, sulfur, sulfoxide, or sulfone, and groups $R_1$, $R_2$, $R_3$, and $R_4$ are defined as above.

Preferably, group X is oxygen, group G is fluorine, group Y is methylene or substituted methylene, oxygen or sulfur, and groups $R_1$, $R_2$, $R_3$, and $R_4$ are defined as above.

Preferably, group X is oxygen, group G is chlorine, group Y is methylene or substituted methylene, oxygen or

sulfur, and groups $R_1$, $R_2$, $R_3$, and $R_4$ are defined as above.

Preferably, group X is oxygen, group G is methyl, group Y is methylene or substituted methylene, oxygen or sulfur, and groups $R_1$, $R_2$, $R_3$, and $R_4$ are defined as above.

Preferably, group X is oxygen, group G is methoxy, group Y is methylene or substituted methylene, oxygen or sulfur, and groups $R_1$, $R_2$, $R_3$, and $R_4$ are defined as above.

Preferably, group X is oxygen, group G is hydrogen, group Y is methylene, sulfoxide, sulfone, oxygen, or sulfur, groups $R_1$ and $R_2$ independently of one another are hydrogen, fluorine, or methyl, and groups $R_3$ and $R_4$ independently of one another are hydrogen.

Preferably, group X is oxygen, group G is fluorine, group Y is methylene, sulfoxide, sulfone, oxygen, or sulfur, groups $R_1$ and $R_2$ independently of one another are hydrogen, fluorine, or methyl, and groups $R_3$ and $R_4$ independently of one another are hydrogen.

Preferably, n = 0.

According to specific embodiments of the invention, in structural I:

(1) group G is hydrogen, group Y is methylene, both groups $R_1$ and $R_2$ are methyl, both groups $R_3$ and $R_4$ are hydrogen, group X is oxygen, and n = 0;
(2) group G is hydrogen, group Y is methylene, both groups $R_1$ and $R_2$ are fluorine, both groups $R_3$ and $R_4$ are hydrogen, group X is oxygen, and n = 0;
(3) group G is hydrogen, group Y is methylene, one of groups $R_1$ and $R_2$ and one of groups $R_3$ and $R_4$ together with the carbon atoms to which they are bonded form a C3 cycloalkyl, the other of groups $R_1$ and $R_2$ and the other of groups $R_3$ and $R_4$ are hydrogen, group X is oxygen, and n = 0;
(4) group G is hydrogen, group Y is sulfur, both groups $R_1$ and $R_2$ are hydrogen, both groups $R_3$ and $R_4$ are hydrogen, group X is oxygen, and n = 0;
(5) group G is hydrogen, group Y is sulfoxide, both groups $R_1$ and $R_2$ are hydrogen, both groups $R_3$ and $R_4$ are hydrogen, group X is oxygen, and n = 0;
(6) group G is hydrogen, group Y is sulfur, both groups $R_1$ and $R_2$ are fluorine, both groups $R_3$ and $R_4$ are hydrogen, group X is oxygen, and n = 0;
(7) group G is hydrogen, group Y is sulfone, both groups $R_1$ and $R_2$ are hydrogen, both groups $R_3$ and $R_4$ are hydrogen, group X is oxygen, and n = 0;
(8) group G is hydrogen, group Y is methylene, both groups $R_1$ and $R_2$ are hydrogen, both groups $R_3$ and $R_4$ are hydrogen, group X is oxygen, and n = 1;
(9) group G is fluorine, group Y is oxygen, both groups $R_1$ and $R_2$ are hydrogen, both groups $R_3$ and $R_4$ are hydrogen, group X is oxygen, and n = 0;
(10) group G is fluorine, group Y is sulfur, both groups $R_1$ and $R_2$ are hydrogen, both groups $R_3$ and $R_4$ are hydrogen, group X is oxygen, and n = 0;
(11) group G is fluorine, group Y is oxygen, both groups $R_1$ and $R_2$ are fluorine, both groups $R_3$ and $R_4$ are hydrogen, group X is oxygen, and n = 0;
(12) group G is fluorine, group Y is methylene, both groups $R_1$ and $R_2$ are fluorine, both groups $R_3$ and $R_4$ are hydrogen, group X is oxygen, and n = 0;
(13) group G is hydrogen, group Y is oxygen, both groups $R_1$ and $R_2$ are fluorine, both groups $R_3$ and $R_4$ are hydrogen, group X is oxygen, and n = 0; or
(14) group G is fluorine, group Y is sulfur, both groups $R_1$ and $R_2$ are fluorine, both groups $R_3$ and $R_4$ are hydrogen, group X is oxygen, and n = 0.

Preferably, in any one of structural formulae Ia, Ib, Ic and Id, group CPT may have a structure represented by structural formula IA:

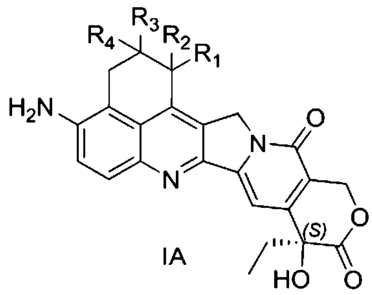

structural formula IA,

wherein structural formula IA is bonded to the carboxyl of group A in any one of structural formulae Ia, Ib, Ic and Id via an amide bond; and preferably, the amino on the left-side benzene ring in structural formula IA is bonded to the carboxyl of group A in any one of structural formulae Ia, Ib, Ic, and Id via an amide bond.

In structural formula IA, groups $R_1$, $R_2$, $R_3$, and $R_4$ have the same meaning as defined above for the groups $R_1$, $R_2$, $R_3$, and $R_4$ in structural formula I, but groups $R_1$, $R_2$, $R_3$, and $R_4$ are not hydrogen simultaneously.

Alternatively, in any one of structural formulae Ia, Ib, Ic and Id, group CPT may have a structure represented by structural formula II:

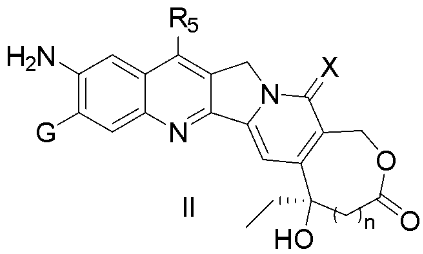

structural formula II,

wherein structural formula II is bonded to the carboxyl of group A in any one of structural formulae Ia, Ib, Ic and Id via an amide bond; and preferably, the amino adjacent to group G in structural formula II is bonded to the carboxyl of group A in any one of structural formulae Ia, Ib, Ic, and Id via an amide bond.

In structural formula II, and if not otherwise stated in the context of the present disclosure, group $R_5$ is C1-5 alkyl or C1-5 alkyl substituted with one or more substituents, C3-6 cycloalkyl or C3-6 cycloalkyl substituted with one or more substituents, phenyl or substituted phenyl. When group $R_5$ is C1-5 alkyl or substituted C1-5 alkyl, the C1-5 alkyl includes linear or branched C1-5 alkyl. Further, group $R_5$ is C1-4 linear alkyl. When group $R_5$ is substituted C1-5 alkyl or substituted C3-6 cycloalkyl, the substituted C1-5 alkyl or substituted C3-6 cycloalkyl is C1-5 alkyl or C3-6 cycloalkyl substituted with substituent(s) selected from the group consisting of halogen, hydroxyl, methoxy, trifluoromethyl, amino or substituted amino, methanesulfonyl and C3-6 cycloalkyl; and among the substituent(s), the substituted amino is an amino substituted with one or more substituents selected from the group consisting of methyl and ethyl. When group $R_5$ is substituted phenyl, the substituted phenyl is a phenyl substituted with substituent(s) selected from the group consisting of alkyl (e.g., C1-6 alkyl, preferably C1-3 alkyl) and halogen.

In structural formula II, and if not otherwise stated in the context of the present disclosure, group G is hydrogen, halogen (e.g., fluorine), methyl, or methoxy. Preferably, group G is hydrogen, fluorine or chlorine.

In structural formula II, group X is oxygen or sulfur.

In structural formula II, n = 0 or 1.

In structural formula II, when group X is oxygen, group G is hydrogen, and n = 0, group $R_5$ cannot be n-butyl.

Preferably, in any one of structural formulae Ia, Ib, Ic and Id, group CPT may have a structure represented by structural formula IIA:

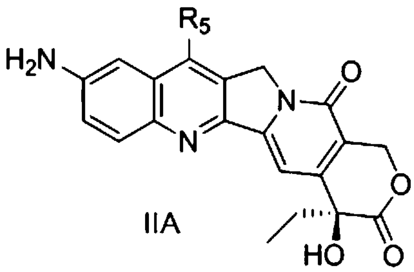

IIA

structural formula IIA,

wherein structural formula IIA is bonded to the carboxyl of group A in any one of structural formulae Ia, Ib, Ic and Id via an amide bond; and preferably, the amino on the left-side benzene ring in structural formula IIA is bonded to the carboxyl of group A in any one of structural formulae Ia, Ib, Ic, and Id via an amide bond.

In structural formula IIA, group $R_5$ has the same meaning as defined above for the group $R_5$ in structural formula II, but group $R_5$ cannot be n-butyl.

According to specific embodiments of the invention, in any one of structural formulae Ia, Ib, Ic and Id, group CPT may have a structure shown below:

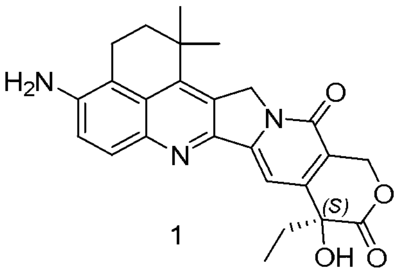

1

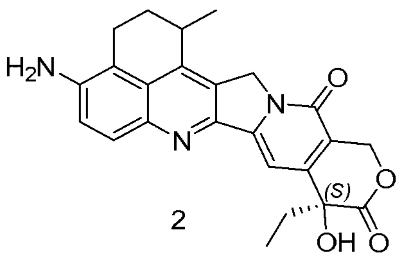

2

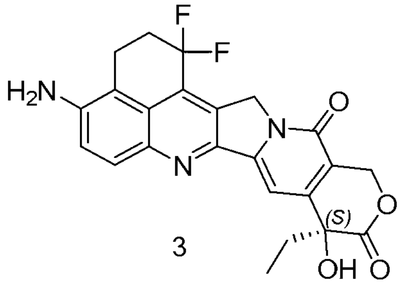

3

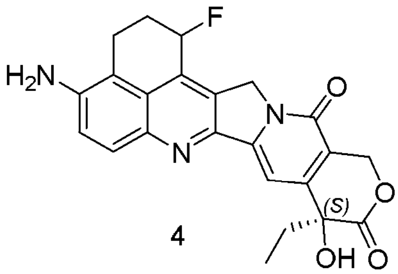

4

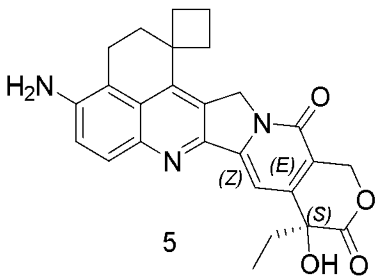

5

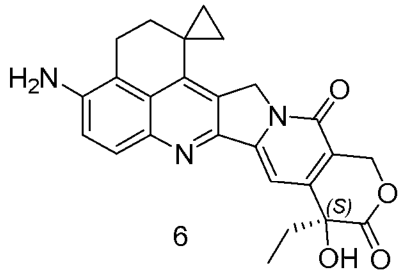

6

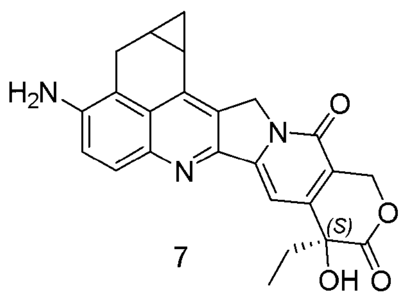

7

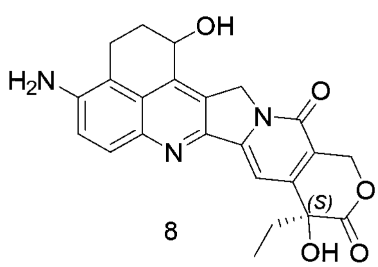

8

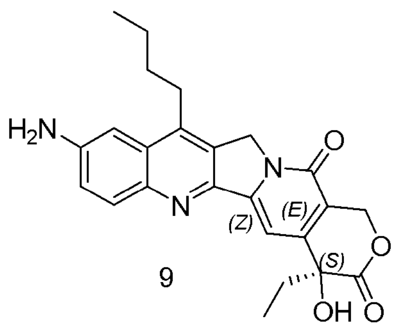

9

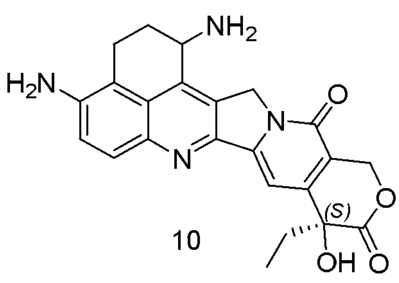
10

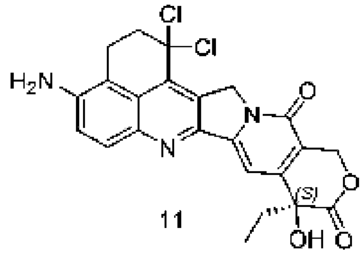
11

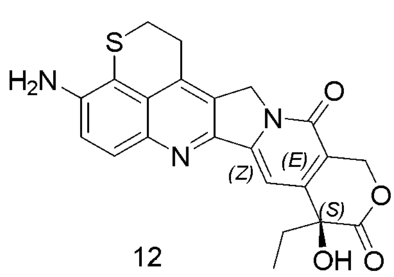
12

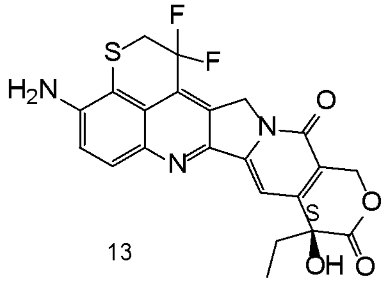
13

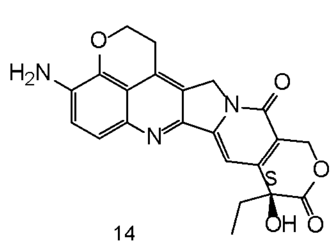
14

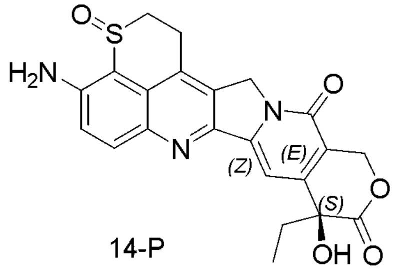
14-P

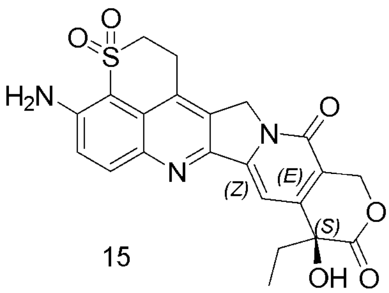
15

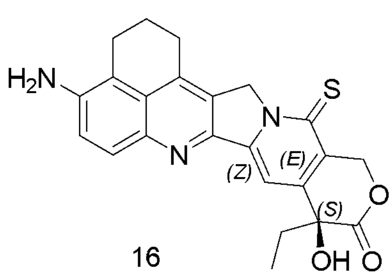
16

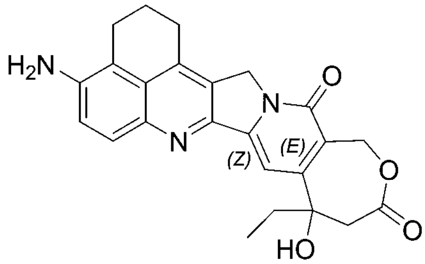
17

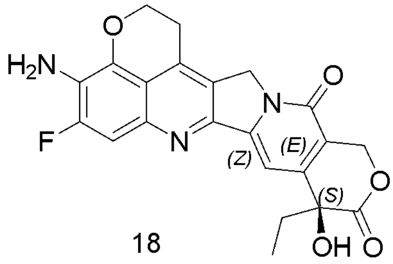
18

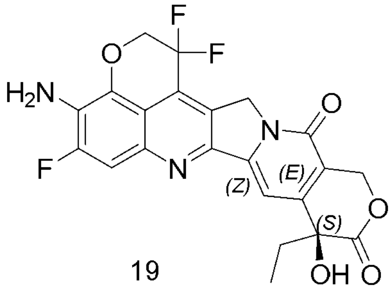
19

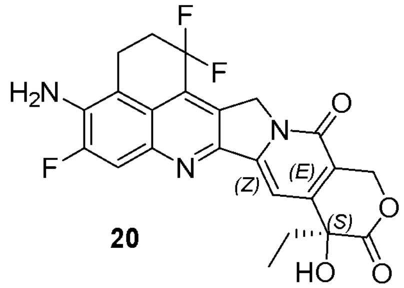
**20**

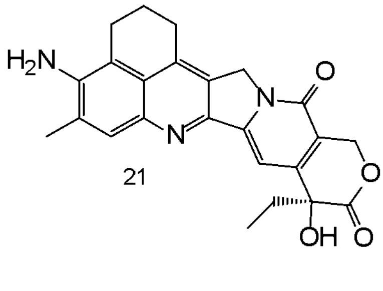
21

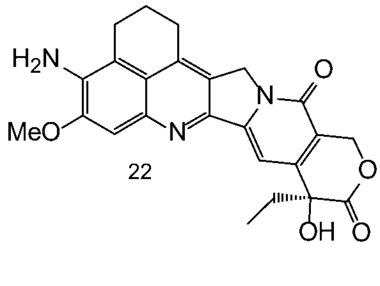
22

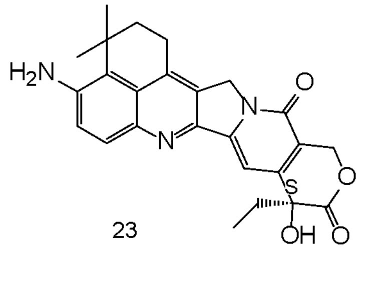
23

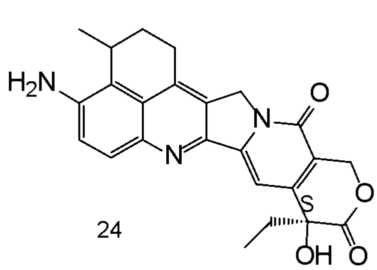
24

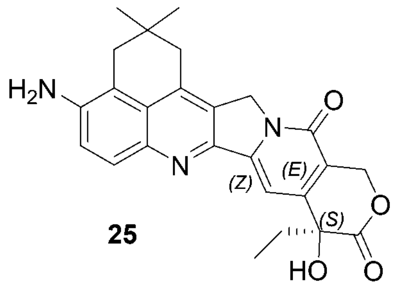
**25**

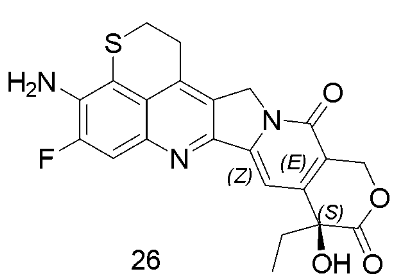
26

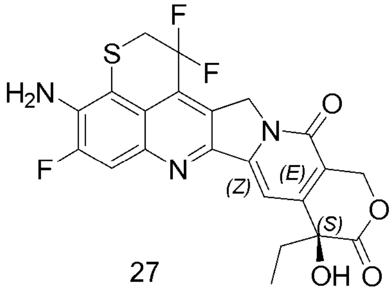
27

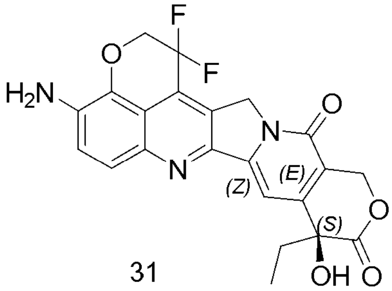
31

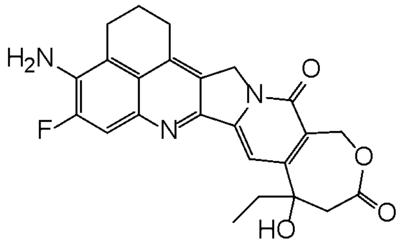
32

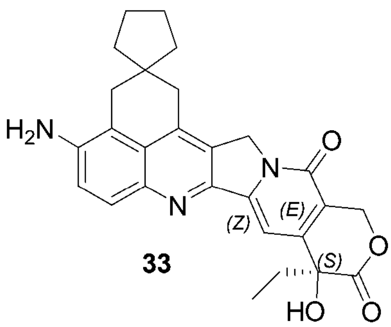
**33**

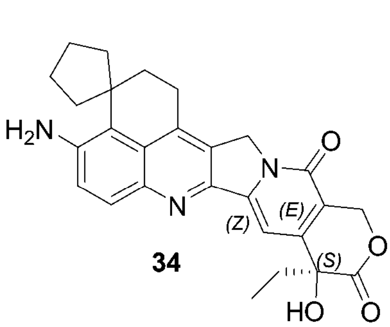
**34**

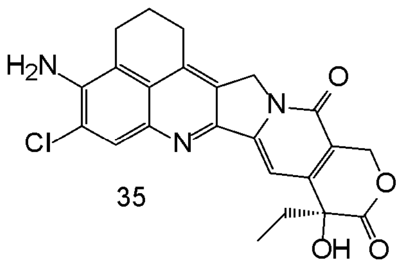
35

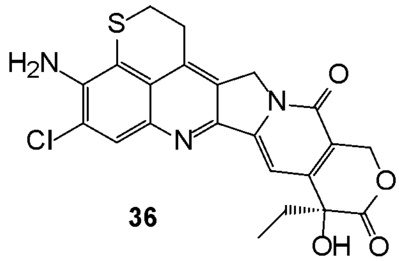
**36**

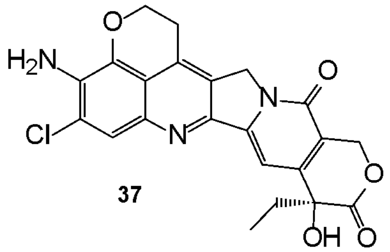
**37**

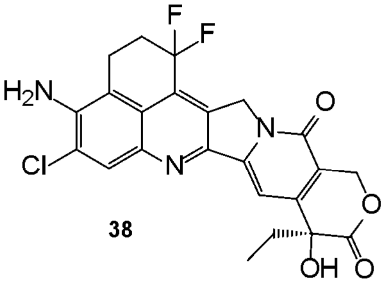
**38**

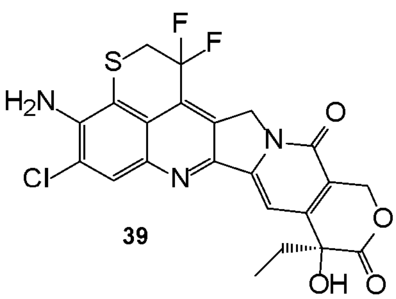
**39**

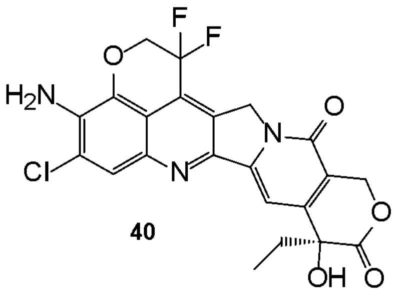

40

,

wherein each structure is bonded to the carboxyl of group A in any one of structural formulae Ia, Ib, Ic and Id via an amide bond; and preferably, the amino on the left-side benzene ring in each structure is bonded to the carboxyl of group A in any one of structural formulae Ia, Ib, Ic, and Id via an amide bond.

Further, in any one of structural formulae Ia, Ib, Ic and Id, group CPT may have a structure represented by structural formula IV:

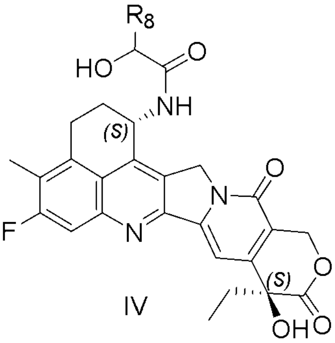

IV

structural formula IV,

wherein the hydroxyl bonded to the same carbon as group R8 in structural formula IV is bonded to the carboxyl of group A in any one of structural formulae Ia, Ib, Ic and Id via a self-immolative structure. The self-immolative structure is, e.g.

( 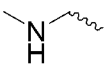 or 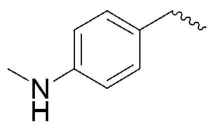 ),

in which the solid line "-" means bonding to the carboxyl of group A in any one of structural formulae Ia, Ib, Ic, and Id, and the wavy line "〰" means bonding to the hydroxyl in the structural formula IV.

In structural formula IV, and if not otherwise stated in the context of the present disclosure, group $R_8$ is hydrogen, trifluoromethyl, C1-5 alkyl or C1-5 alkyl substituted with one or more substituents, C3-6 cycloalkyl, or C3-6 cycloalkyl substituted with one or more substituents, or halogen.

When group $R_8$ is substituted C1-5 alkyl or substituted C3-6 cycloalkyl, the C1-5 alkyl or C3-6 cycloalkyl is substituted with substituent(s) selected from the group consisting of halogen, hydroxyl, methoxy, trifluoromethyl, amino or substituted amino, methanesulfonyl and C3-6 cycloalkyl; and among the substituents, the substituted amino is an amino substituted with one or more substituents selected from the group consisting of methyl and ethyl.

**Twelfth Aspect**

The present disclosure provides an antibody-drug conjugate or a salt thereof, having a structure represented by general formula

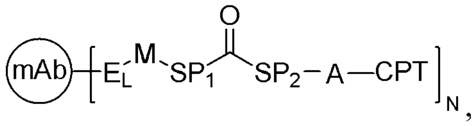

wherein mAb is an anti-B7-H3 antibody or fragment thereof. Preferably, mAb is the anti-B7-H3 antibody or fragment thereof defined in the first aspect of the present disclosure above.

In that general formula, groups M, $SP_1$, $SP_2$, A, and CPT have the same meaning as defined in the eleventh aspect above for the groups M, $SP_1$, $SP_2$, A, and CPT; and N is in the range of 1 to 10, preferable of 1 to 8 (e.g., 1 to 5), more preferably of 3 to 8.

In that general formula, $E_L$ is selected from the group consisting of following groups:

$E_L$-1a and/or $E_L$-1b:

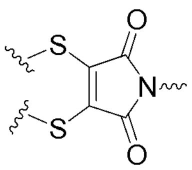 and/or 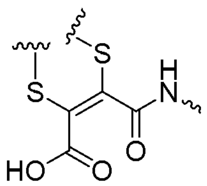 ; $E_L$-2: 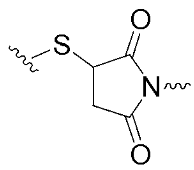 ; $E_L$-3:

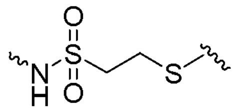 ; $E_L$-4: 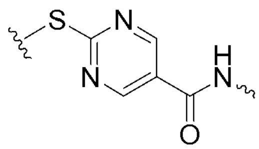 ; $E_L$-5: 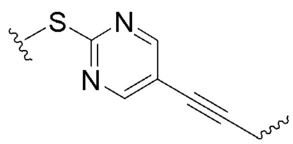 ; $E_L$-6:

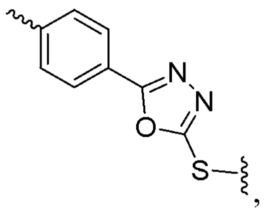

wherein,

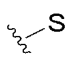

means bonding to the cysteine in mAb, and the wavy line "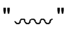" means bonding to group M.

**Thirteenth Aspect**

The present disclosure provides a linker-payload having a structure represented by general formula "L-A-CPT", in which group L means a linker used in an antibody-drug conjugate (ADC), group A means a peptide group of one or more amino acids, and group CPT is a compound of camptothecins.

In the thirteenth aspect of the present disclosure, the linker-payload having the structure represented by the general formula "L-A-CPT" is a compound represented by structural formula III or a pharmaceutically acceptable salt, stereoisomer, solvate or prodrug thereof:

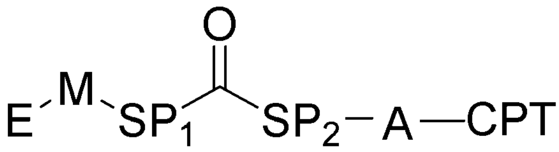

III

structural formula III.

In structural formula III, and if not otherwise stated in the context of the present disclosure, E is selected from the group consisting of the following groups:

E-1: 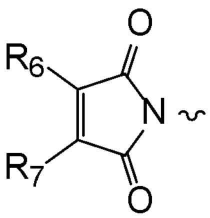 ; E-2: 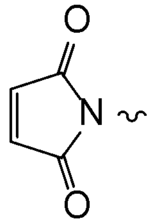 ; E-3: 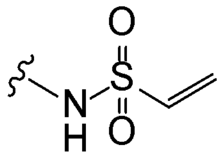 ;

E-4: 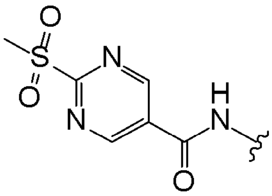 ; E-5: 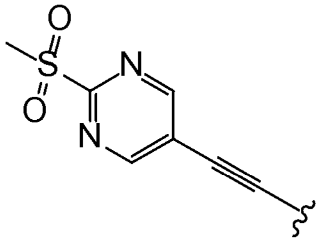 ; and E-6:

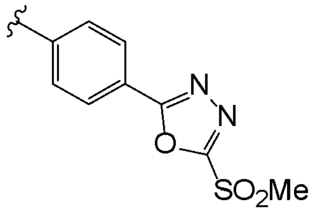 ,

wherein the wavy line

“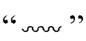”

means bonding to group M.

In structural formula III, and if not otherwise stated in the context of the present disclosure, group M is phenylene or phenylene substituted with one or more substituents, or a chemical bond; and in the substituted phenylene, the phenylene is substituted with substituent(s) selected from the group consisting of alkyl (e.g., C1-6 alkyl, preferably C1-4 alkyl), haloalkyl (e.g., C1-6 haloalkyl, preferably C1-4 haloalkyl, e.g., trifluoromethyl), alkoxy (e.g., C1-6 alkoxy, preferably C1-4 alkoxy, preferably methoxy), halogen, ester, amide, and cyano; and preferably, group M is halogen-substituted phenylene.

In structural formula III, and if not otherwise stated in the context of the present disclosure, group $SP_1$ is selected from the group consisting of C1-8 alkylene, C1-8 cycloalkylene, and C1-21 (preferably C1-16, more preferably C1-11, more preferably C5-9) linear heteroalkylene comprising 1-11 (preferably 1-6, more preferably 3-5) heteroatoms selected from the group consisting of N, O, and S, in which the C1-8 alkylene, C1-8 cycloalkylene, and C1-21 linear heteroalkylene independently of one another are optionally substituted with one or more substituents selected from the group consisting of hydroxyl, amino, sulfonic acid group, and cyano.

In structural formula III, and if not otherwise stated in the context of the present disclosure, group $SP_2$ is selected from the group consisting of -NH(CH2CH2O)aCH2CH2CO-, -NH(CH2CH2O)aCH2CO-, -S(CH2)aCO-, and a chemical bond, in which a is an integer in the range of 1 to 20, preferably an integer in the range of 1 to 10, more preferably an integer in the range of 1 to 6.

In structural formula III, and if not otherwise stated in the context of the present disclosure, group A means a peptide group of 2 to 4 amino acids. When group A means a peptide group of 2 amino acids, it may be *NH*-Phe-Lys-*CO*, *NH*-Val-Ala-*CO*, *NH*-Val-Lys-*CO*, *NH*-Ala-Lys-*CO*, *NH*-Val-Cit-*CO*, *NH*-Phe-Cit-*CO, NH*-Leu-Cit-*CO, NH*-Phe-Arg-*CO,* or *NH*-Gly-Val-*CO*, preferably *NH*-Phe-Lys-*CO*, *NH*-Val-Ala-*CO*, or *NH*-Val-Cit-*CO*. When group A means a peptide group of 3 amino acids, it may be *NH*-Glu-Val-Ala-*CO, NH*-Glu-Val-Cit-*CO,* or *NH*-Ala-Ala-Ala-*CO*, preferably *NH*-Glu-Val-Ala-*CO* or *NH*-Ala-Ala-Ala-*CO*. When group A means a peptide group of 4 amino acids, it may be *NH*-Gly-Gly-Phe-Gly-*CO* or *NH*-Gly-Phe-Gly-Gly-*CO*, preferably *NH*-Gly-Gly-Phe-Gly-*CO*. Preferably, group A is *NH*-Val-Ala-*CO, NH*-Gly-Gly-Phe-Gly-*CO* or *NH*-Ala-Ala-Ala-*CO*. In structural formula III, group A is bonded to group $SP_2$ via the amino at the amino terminus of its short peptide structure.

In structural formula III, group M is preferably halogen-substituted phenylene, particularly fluorine-substituted phenylene.

In structural formula III, group $SP_1$ is preferably C1-11, preferably C5-9, more preferably C7 linear heteroalkylene containing 1-6, preferably 3-5, more preferably 4 heteroatoms selected from the group consisting of N, O, and S.

In structural formula III, group $SP_2$ is preferably a chemical bond.

In structural formula III, and if not otherwise stated in the context of the present disclosure, group CPT is a compound of camptothecins.

When group E is E1, structural formula III provided by the present disclosure further may be structural formula IIIA:

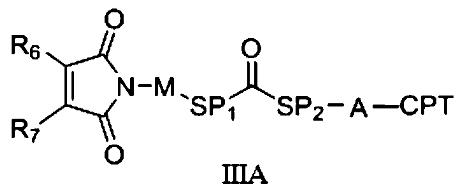

IIIA

structural formula IIIA.

In structural formula IIIA, and if not otherwise stated in the context of the present disclosure, groups $R_6$ and $R_7$ independently of one another are hydrogen, halogen or Ar'S in which Ar' is phenyl or phenyl substituted with one or more substituents; and in the substituted phenyl, the phenyl is substituted with substituent(s) selected from the group consisting of alkyl (e.g., C1-6 alkyl, preferably C1-4 alkyl), alkoxy (e.g., C1-6 alkoxy, preferably C1-4 alkoxy, preferably methoxy), halogen, ester, amide, and cyano. Preferably, Ar' is phenyl, phenyl
substituted with 4-formylmethylamine

( 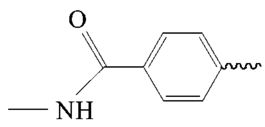 )

or phenyl substituted with 4-formylmorpholine

( 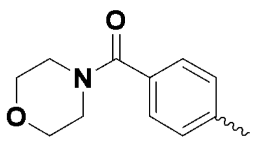 ).

For example, in structural formula III and structural formula IIIA, group CPT is a compound represented by structural formula I or structural formula IA or a pharmaceutically acceptable salt, stereoisomer, solvate or prodrug thereof, as well as one of the corresponding specific compounds, all of which are provided in the eleventh aspect of the present disclosure above.

When group CPT is a compound represented by structural formula I or a pharmaceutically acceptable salt,

stereoisomer, solvate or prodrug thereof which is provided in the eleventh aspect of the present disclosure above:

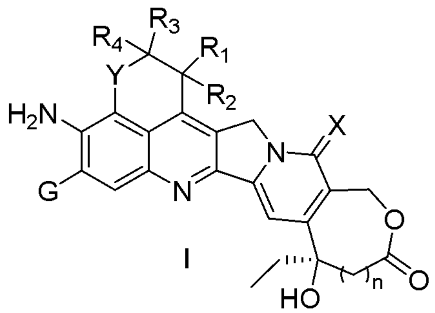

structural formula I,

groups G, X, Y, $R_1$, $R_2$, $R_3$, $R_4$, and n in structural formula I have the same meaning as defined in the eleventh aspect above for the groups G, X, Y, $R_1$, $R_2$, $R_3$, $R_4$, and n in structural formula I. And in structural formula III or structural formula IIIA, structural formula I is bonded to the carboxyl of group A via an amide bond; and preferably, the amino adjacent to group G in structural formula I is bonded to the carboxyl of group A via an amide bond. Namely an amide bond is formed between the amino of the compound represented by structural formula I and the carboxyl of group A in structural formula III or IIIA.

When group CPT is a compound represented by structural formula IA or a pharmaceutically acceptable salt, stereoisomer, solvate or prodrug thereof which is provided in the eleventh aspect of the present disclosure above:

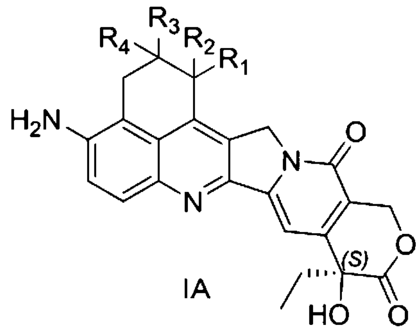

structural formula IA,

groups $R_1$, $R_2$, $R_3$, and $R_4$ in structural formula IA have the same meaning as defined in the eleventh aspect above for the groups $R_1$, $R_2$, $R_3$, and $R_4$ in structural formula IA, but $R_1$, $R_2$, $R_3$, and $R_4$ can be hydrogen simultaneously. And in structural formula III or structural formula IIIA, structural formula IA is bonded to the carboxyl of group A via an amide bond; and preferably, the amino on the left-side benzene ring in structural formula IA is bonded to the carboxyl of group A via an amide bond. Namely an amide bond is formed between the amino of the compound represented by structural formula IA and the carboxyl of group A in structural formula III or IIIA.

In particular, when group "CPT" in the compound represented by structural formula IIIA is a compound represented by structural formula IA or a pharmaceutically acceptable salt, stereoisomer, solvate or prodrug thereof, groups $R_6$ and $R_7$ may or may not be hydrogen simultaneously. Similarly, groups $R_1$, $R_2$, $R_3$, and $R_4$ in structural formula IA may or may not be hydrogen simultaneously. According to specific embodiments of the invention, in the case groups $R_6$ and $R_7$ are hydrogen simultaneously, groups $R_1$, $R_2$, $R_3$, and $R_4$ in structural formula IA cannot be hydrogen simultaneously; and in the case groups $R_6$ and $R_7$ are not hydrogen simultaneously, groups $R_1$, $R_2$, $R_3$, and $R_4$ in structural formula IA may or may not be hydrogen simultaneously.

For another example, in structural formula III and structural formula IIIA, group CPT is a compound represented by structural formula II or structural formula IIA or a pharmaceutically acceptable salt, stereoisomer, solvate or prodrug thereof, as well as one of the corresponding specific compounds, all of which are provided in the eleventh aspect of the present disclosure above.

When group CPT is a compound represented by structural formula II or a pharmaceutically acceptable salt, stereoisomer, solvate or prodrug thereof which is provided in the eleventh aspect of the present disclosure above:

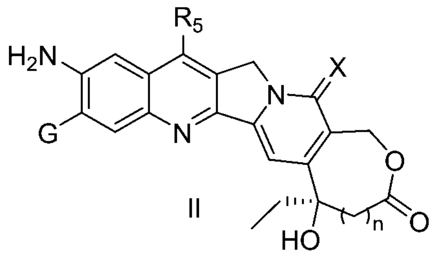

structural formula II,

groups G, $R_5$, X, and n in structural formula II have the same meaning as defined in the eleventh aspect above for the groups G, $R_5$, X, and n in structural formula II. And in structural formula III or structural formula IIIA, structural formula II is bonded to the carboxyl of group A via an amide bond; and preferably, the amino adjacent to group G in structural formula II is bonded to the carboxyl of group A via an amide bond. Namely an amide bond is formed between the amino of the compound represented by structural formula II and the carboxyl of group A in structural formula III or IIIA.

When CPT is a compound represented by structural formula IIA or a pharmaceutically acceptable salt, stereoisomer, solvate or prodrug thereof which is provided in the eleventh aspect of the present disclosure above:

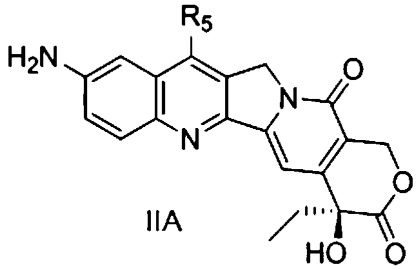

structural formula IIA,

group $R_5$ in structural formula IIA has the same meaning as defined in the eleventh aspect above for the group $R_5$ in structural formula IIA, but group $R_5$ can be n-butyl. Then in structural formula III or structural formula IIIA, structural formula IIA is bonded to the carboxyl of group A via an amide bond; and preferably, the amino on the left-side benzene ring in structural formula IIA is bonded to the carboxyl of group A via an amide bond. Namely an amide bond is formed between the amino of the compound represented by structural formula IIA and the carboxyl of group A in structural formula III or IIIA.

Each of the compounds 1, 2, 3, 4, 5, 6, 7, 8, 9, 10, 11, 12, 13, 14, 14-P, 15, 16, 17, 18, 19, 20, 21, 22, 23, 24, 25, 26, 27, 31, 32, 33, 34, 35, 36, 37, 38, 39, 40 or a pharmaceutically acceptable salt, stereoisomer, solvate or prodrug thereof provided in the eleventh aspect above can be used as the group "CPT" in structural formula III or IIIA and is bonded to, via its amino, the carboxyl of group A in structural formula III or IIIA through an amide bond.

For another example, in structural formula III and structural formula IIIA, group CPT can be an Exatecan derivative represented by structural formula IV or a pharmaceutically acceptable salt, stereoisomer, solvate or prodrug thereof:

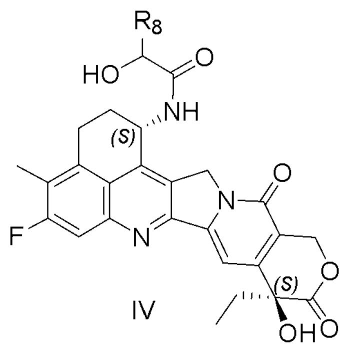

structural formula IV.

In structural formula IV, and if not otherwise stated in the context of the present disclosure, group $R_8$ is hydrogen,

trifluoromethyl, C1-5 alkyl or C1-5 alkyl substituted with one or more substituents, C3-6 cycloalkyl, or C3-6 cycloalkyl substituted with one or more substituents, or halogen. And in the structural formula III and structural formula IIIA, the hydroxyl bonded to the same carbon as group R8 in structural formula IV is bonded to the carboxyl of group A via a self-immolative structure. The self-immolative structure is, e.g.

( 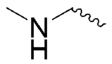 or

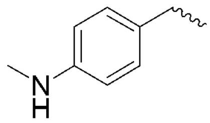 ),

in which the solid line "-" means bonding to the carboxyl of group A in

structural formula III or structural formula IIIA, and the wavy line "~" means bonding to the hydroxyl in the structural formula IV.

When group $R_8$ is substituted C1-5 alkyl or substituted C3-6 cycloalkyl, the C1-5 alkyl or C3-6 cycloalkyl is substituted with substituent(s) selected from the group consisting of halogen, hydroxyl, methoxy, trifluoromethyl, amino or substituted amino, methanesulfonyl and C3-6 cycloalkyl; and among the substituents, the substituted amino is an amino substituted with one or more substituents selected from the group consisting of methyl and ethyl.

In particular, when group "CPT" in the compound represented by structural formula IIIA is a compound represented by structural formula IV or a pharmaceutically acceptable salt, stereoisomer, solvate or prodrug thereof, groups $R_6$ and $R_7$ may or may not be hydrogen simultaneously. Similarly, group $R_8$ in structural formula IV may or may not be hydrogen. According to specific embodiments of the invention, in the case groups $R_6$ and $R_7$ are hydrogen simultaneously, group $R_8$ may or may not be hydrogen.

In particular, regarding the compound represented by structural formula IIIA, when groups $R_6$ and $R_7$ are Ar'S, group M is preferably phenylene or substituted phenylene, and at the meantime, group $SP_1$ is C1-21 (preferably C1-16, more preferably C1-11, more preferably C5-9) linear heteroalkylene containing 1-11 (preferably 1-6, more preferably 3-5) heteroatoms selected from the group consisting of N, O, and S.

Preferably, the compound represented by structural formula III or structural formula IIIA provided by the present disclosure is further a compound represented by structural formula V:

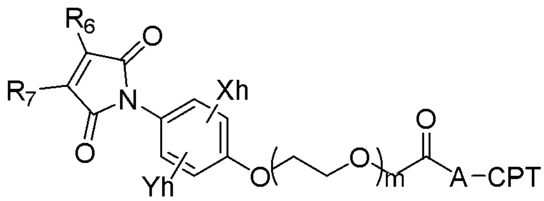

V

structural formula V,

in structural formula V, groups $R_6$ and $R_7$ independently of one another are Ar'S in which Ar' is phenyl or phenyl substituted with one or more substituents; and in the substituted phenyl, the phenyl is substituted with substituent(s) selected from the group consisting of alkyl (e.g., C1-6 alkyl, preferably C1-4 alkyl), alkoxy (e.g., C1-6 alkoxy, preferably C1-4 alkoxy, preferably methoxy), halogen, ester, amide, and cyano. Preferably, Ar' is phenyl, phenyl substituted with 4-formylmethylamine

( 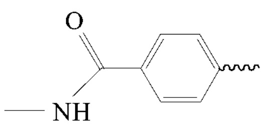 )

or phenyl substituted with 4-formylmorpholine

( 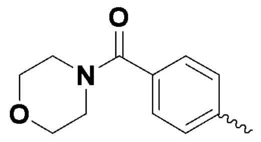 ).

In structural formula V, and if not otherwise stated in the context of the present disclosure, groups Xh and Yh independently of one another are hydrogen, halogen, haloalkyl (e.g., C1-6 haloalkyl, preferably C1-4 haloalkyl, e.g., trifluoromethyl), or alkoxy (e.g., C1-6 alkoxy, preferably C1-4 alkoxy, e.g., methoxy).

In structural formula V, and if not otherwise stated in the context of the present disclosure, m is any integer in the range of 1 to 10, preferably 1 to 5, more preferably 3 to 5.

In structural formula V, group A means a peptide group of 2-4 amino acids, as defined above.

In structural formula V, the meaning of group CPT and the bonding relationship thereof in structural formula V are the same as those of group CPT in structural formula III and structural formula IIIA as defined above.

Preferably, the compound represented by structural formula V provided by the present disclosure is further a compound represented by structural formula V-A:

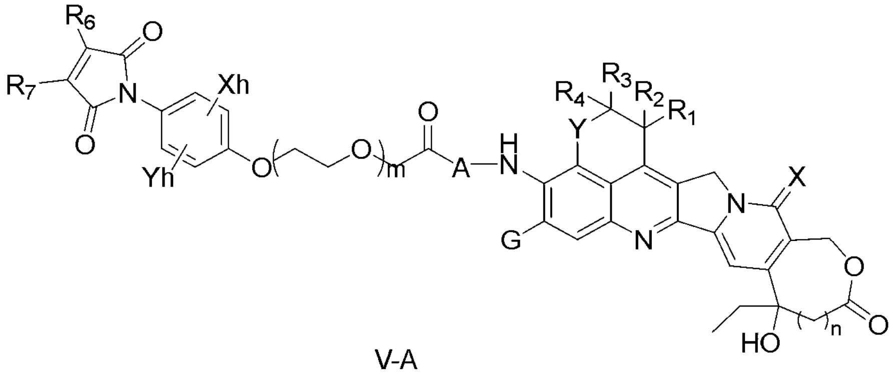

V-A

structural formula V-A,

in structural formula V-A, groups A, G, Y, $R_1$, $R_2$, $R_3$, $R_4$, X, and n have the same meaning as defined above for the groups A, G, Y, $R_1$, $R_2$, $R_3$, $R_4$, X, and n in structural formula III or structural formula IIIA.

Preferably, in structural formula V-A, group G is hydrogen, fluorine or chlorine.

Preferably, in structural formula V-A, group Y is methylene, sulfur or oxygen.

In structural formula V-A, and if not otherwise stated in the context of the present disclosure, "-A-NH-" means an amide bond is formed between the amino and the carboxyl of group A.

Preferably, the compound represented by structural formula V-A provided by the present disclosure is further a compound represented by structural formula V-A-1:

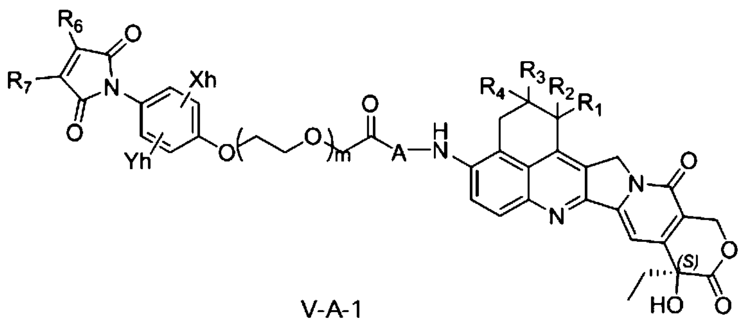

V-A-1

structural formula V-A-1.

Alternatively, the compound represented by structural formula V provided by the present disclosure is further a compound represented by structural formula V-B:

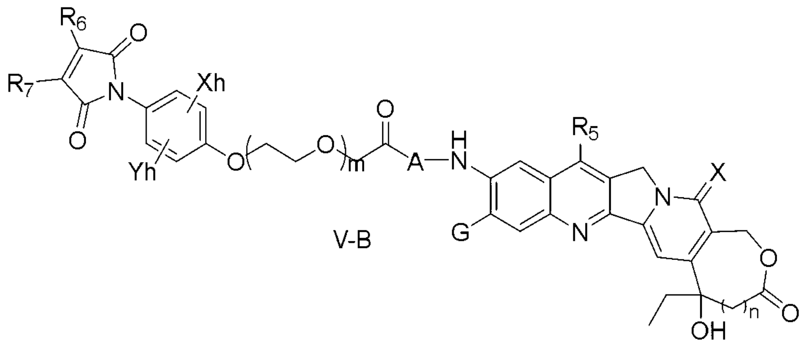

V-B

structural formula V-B,

in structural formula V-B, groups A, G, $R_5$, X, and n have the same meaning as defined above for the groups A, G, $R_5$, X, and n in structural formula III or structural formula IIIA.

In structural formula V-B, and if not otherwise stated in the context of the present disclosure, "-A-NH-" means an amide bond is formed between the amino and the carboxyl of A.

Preferably, the compound represented by structural formula V-B provided by the present disclosure is further a compound represented by structural formula V-B-1:

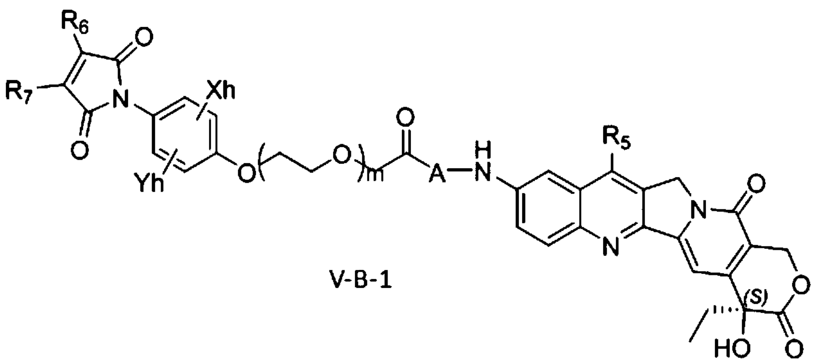

V-B-1

structural formula V-B-1.

Alternatively, the compound represented by structural formula V provided by the present disclosure is further a compound represented by structural formula V-C:

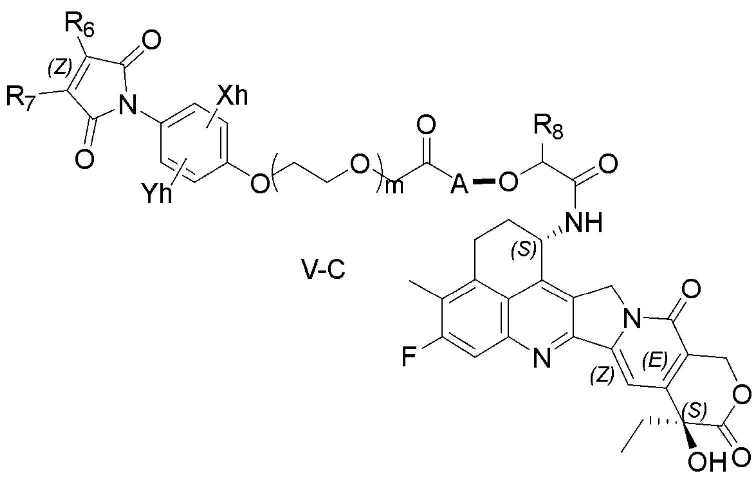

V-C

structural formula V-C,

in structural formula V-C, groups A, and $R_8$ have the same meaning as defined above for the groups A, and $R_8$ in structural formula III or structural formula IIIV.

In structural formula V-C, and if not otherwise stated in the context of the present disclosure, "-A-NH-" means an amide bond is formed between the amino and the carboxyl of group A.

In structural formula V-C, and if not otherwise stated in the context of the present disclosure, "-" is the same as the self-immolative structure above when group CPT in structure formula III or structure formula IIIV is represented by the structure formula IV.

According to specific embodiments of the invention, the compound represented by structural formula V provided by the present disclosure is further a compound represented by structural formula VI:

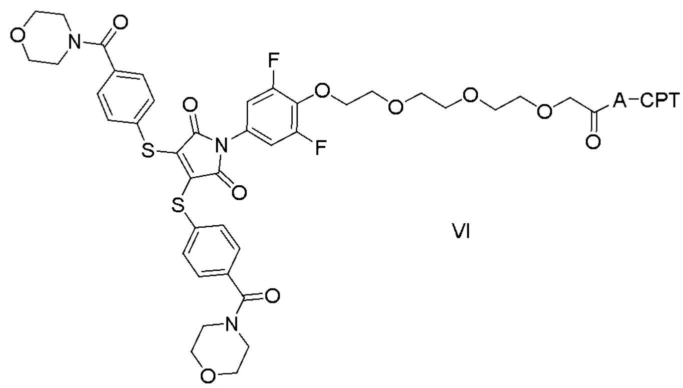

VI

structural formula VI.

In structural formula VI, group A means a peptide group of 2-4 amino acids, as defined above.

In structural formula VI, group CPT is a compound of camptothecins. The meaning of group CPT and the bonding relationship thereof in structural formula VI are the same as those of group CPT in structural formula III and structural formula IIIA defined above.

Preferably, the compound represented by structural formula VI provided by the present disclosure is further a compound represented by structural formula VI-A:

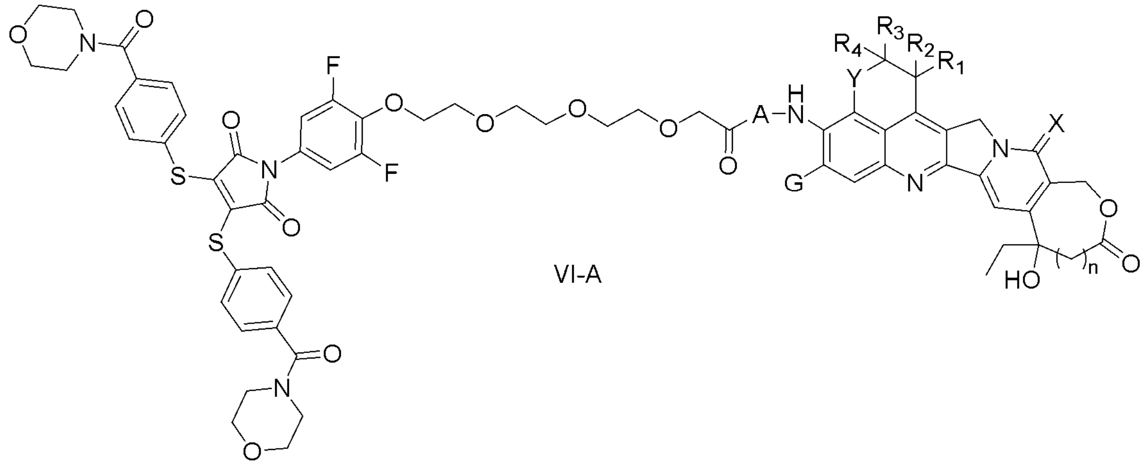

VI-A

structural formula VI-A,

in structural formula VI-A, groups A, G, Y, $R_1$, $R_2$, $R_3$, $R_4$, X, and n have the same meaning as defined above for the groups A, G, Y, $R_1$, $R_2$, $R_3$, $R_4$, X, and n in structural formula III or structural formula IIIA.

Preferably, in structural formula VI-A, group G is hydrogen, fluorine or chlorine.

Preferably, in structural formula VI-A, group Y is methylene, sulfur or oxygen.

In structural formula VI-A, and if not otherwise stated in the context of the present disclosure, "-A-NH-" means an amide bond is formed between the amino and the carboxyl of group A.

Preferably, the compound represented by structural formula VI-A provided by the present disclosure is further a compound represented by structural formula VI-A-1:

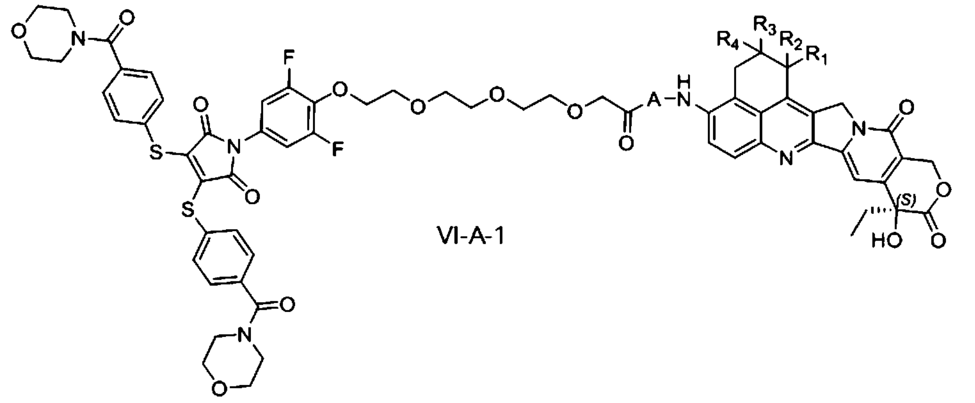

structural formula VI-A-1.

Alternatively, the compound represented by structural formula VI is further a compound represented by structural formula VI-B:

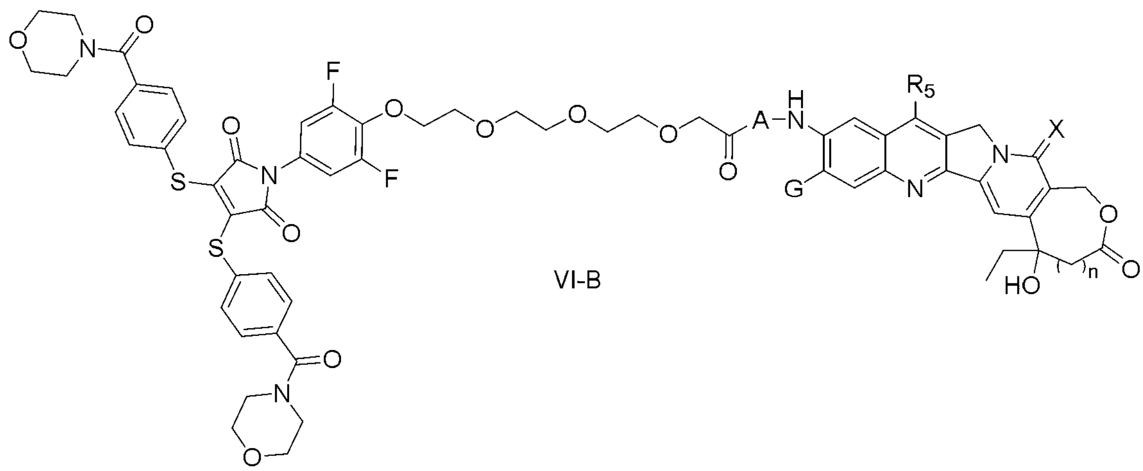

structural formula VI-B,

in structural formula VI-B, groups A, G, $R_5$, X, and n have the same meaning as defined above for the groups A, G, $R_5$, X, and n in structural formula III or structural formula IIIA.

In structural formula VI-B, and if not otherwise stated in the context of the present disclosure, "-A-NH-" means an amide bond is formed between the amino of group CPT shown and the carboxyl of group A.

More preferably, the compound represented by structural formula VI-B is further a compound represented by structural formula VI-B-1:

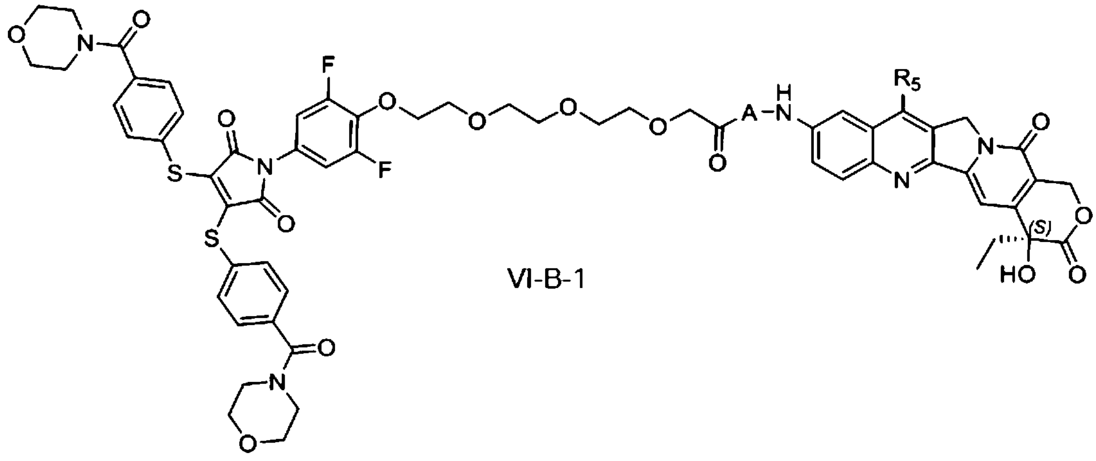

structural formula VI-B-1.

Alternatively, the compound represented by structural formula VI provided by the present disclosure is further a compound represented by structural formula VI-C:

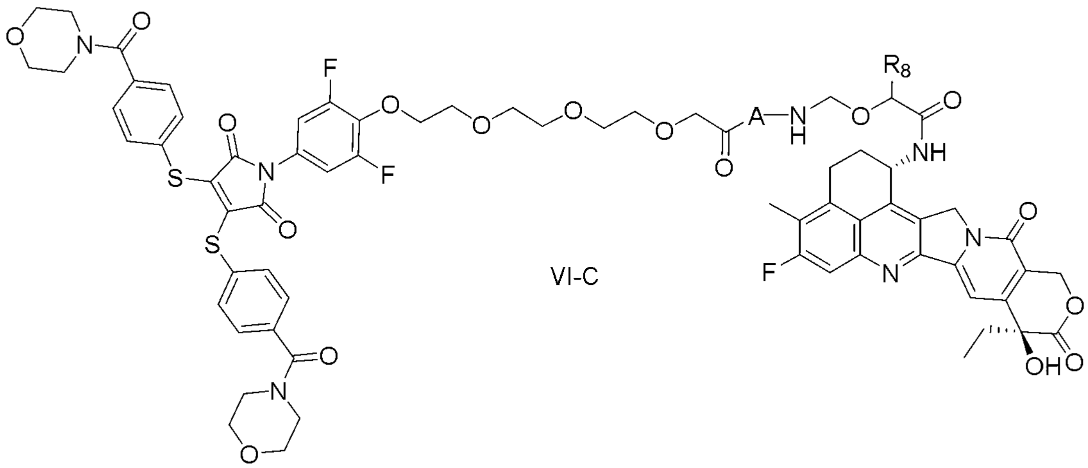

structural formula VI-C,

in structural formula VI-C, groups A, and $R_8$ have the same meaning as defined above for the groups A, and $R_8$ in structural formula III or structural formula IIIA.

In structural formula VI-C, and if not otherwise stated in the context of the present disclosure, "-A-NH-" means an amide bond is formed between the amino and the carboxyl of group A.

When the self-immolative structure in structural formula V-C is

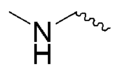

,

the structural formula V-C may be further structural formula VI-C.

According to specific embodiments of the invention, in the thirteenth aspect of the present disclosure, the compound has one of the following structures:

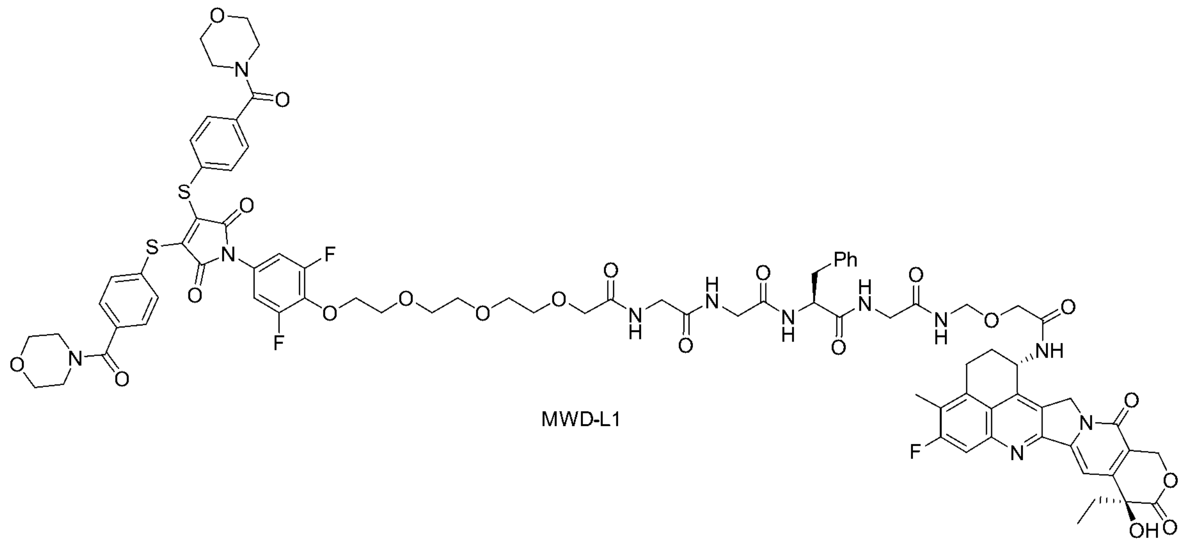

MWD-L1

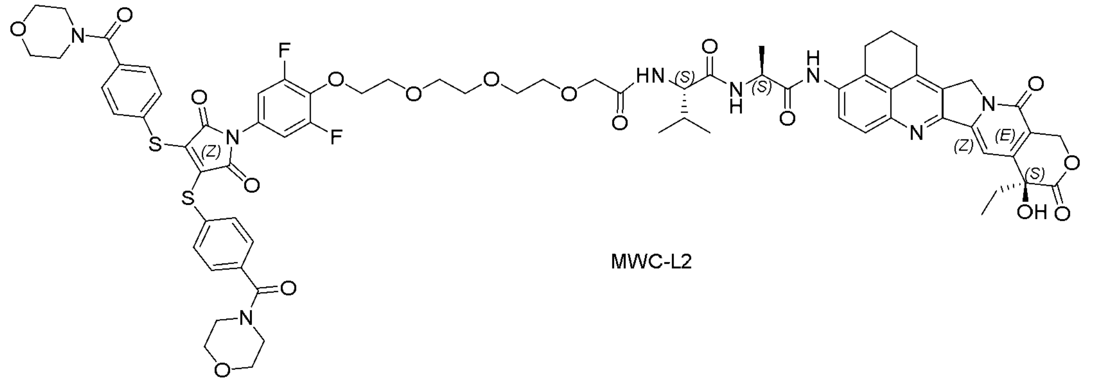

MWC-L2

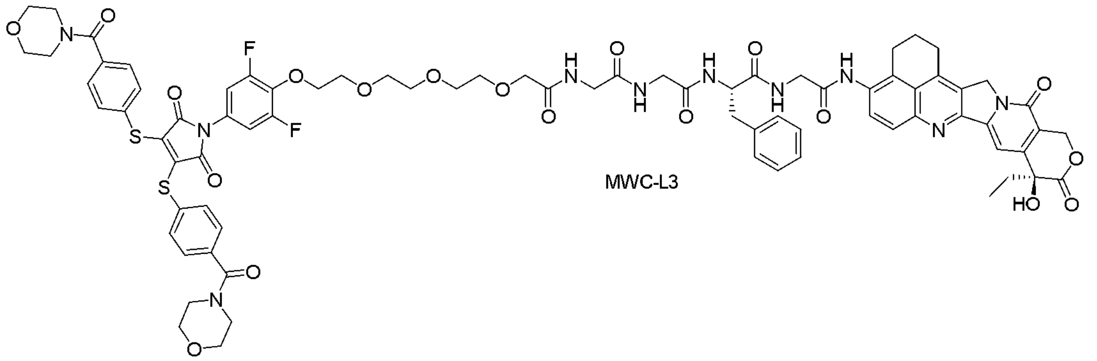

MWC-L3

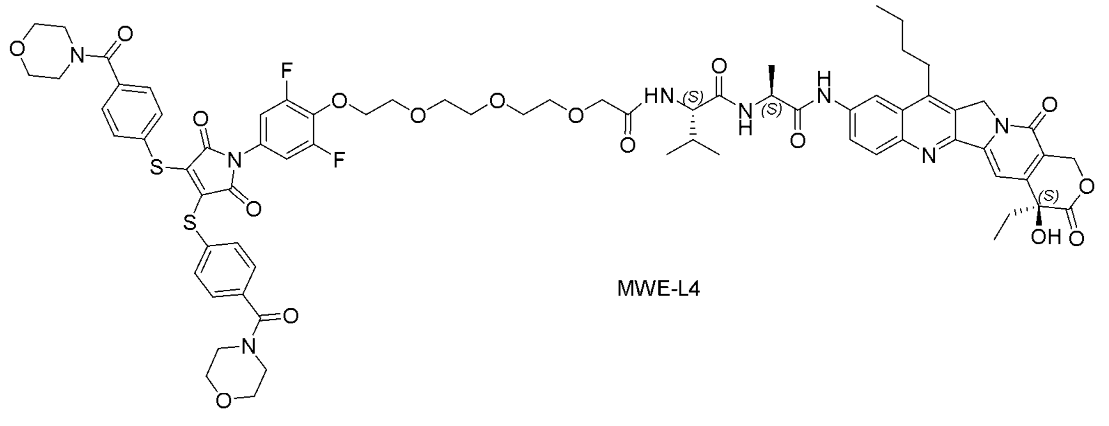

MWE-L4

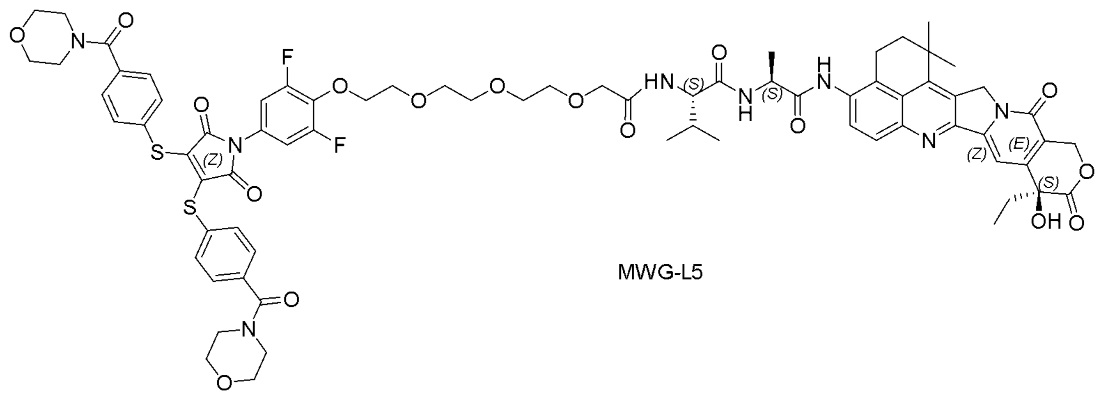

MWG-L5

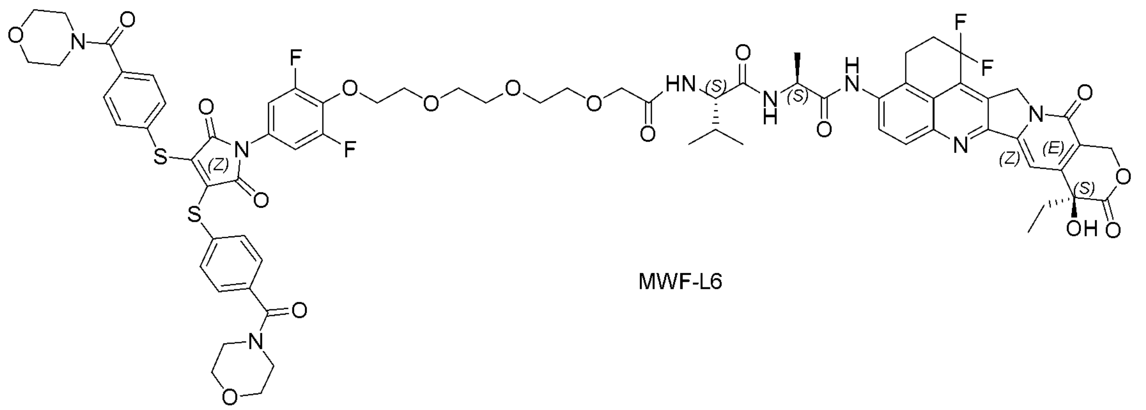

MWF-L6

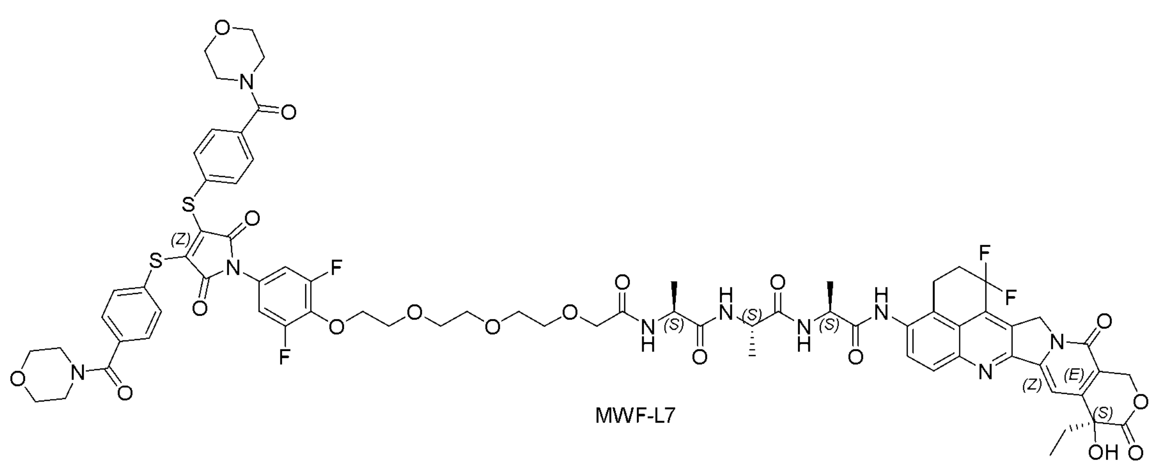

MWF-L7

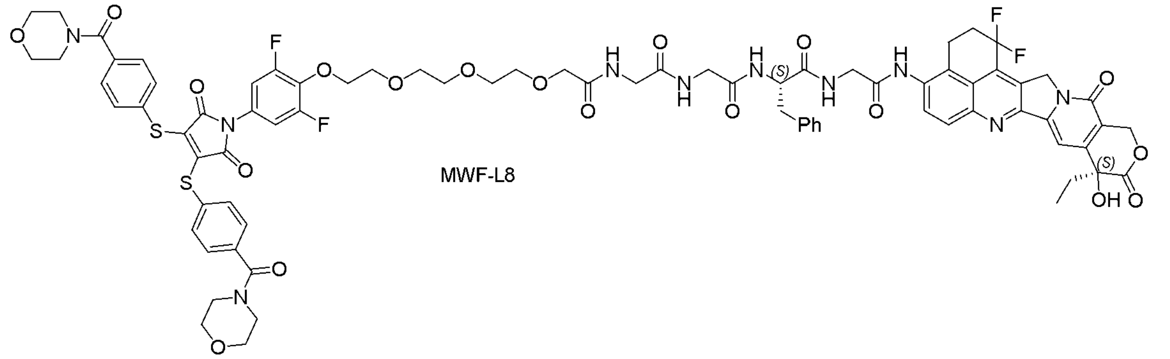

MWF-L8

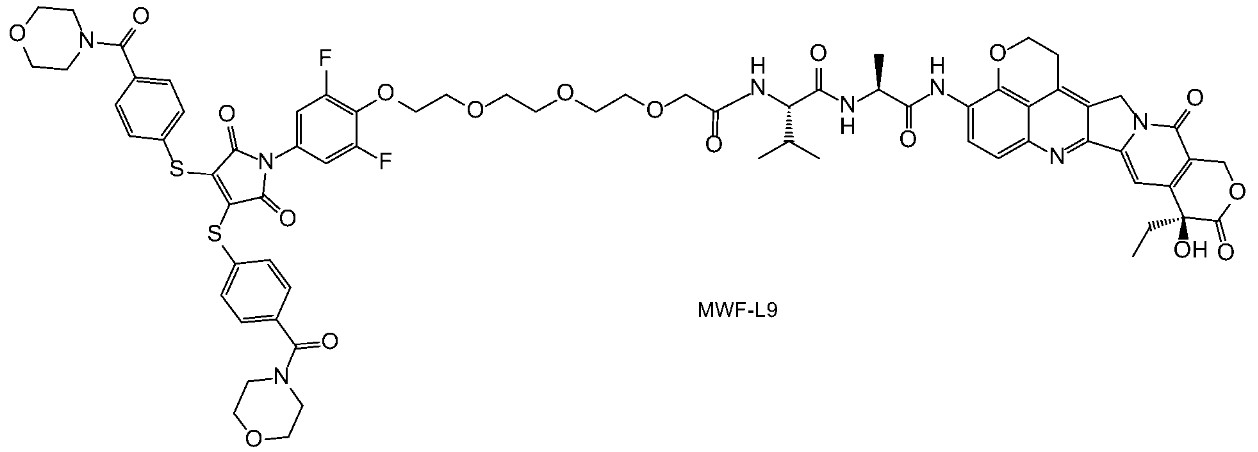

MWF-L9

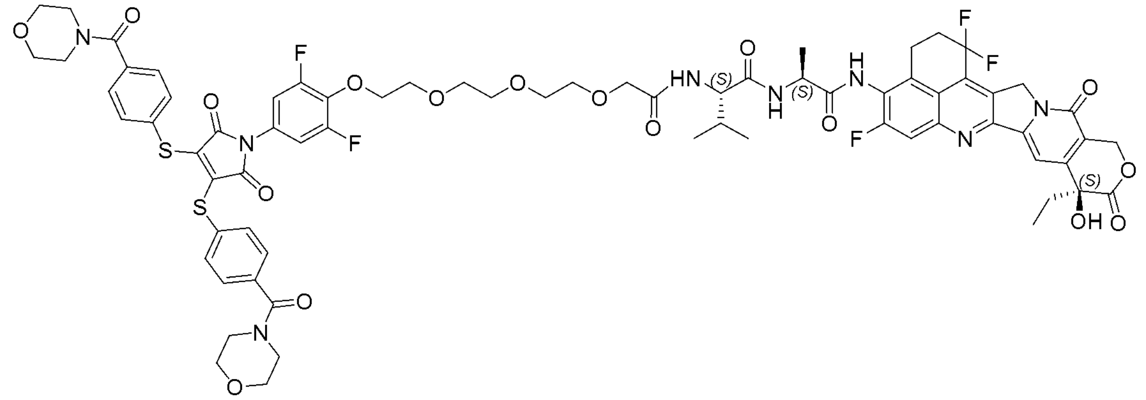

MWF-L10

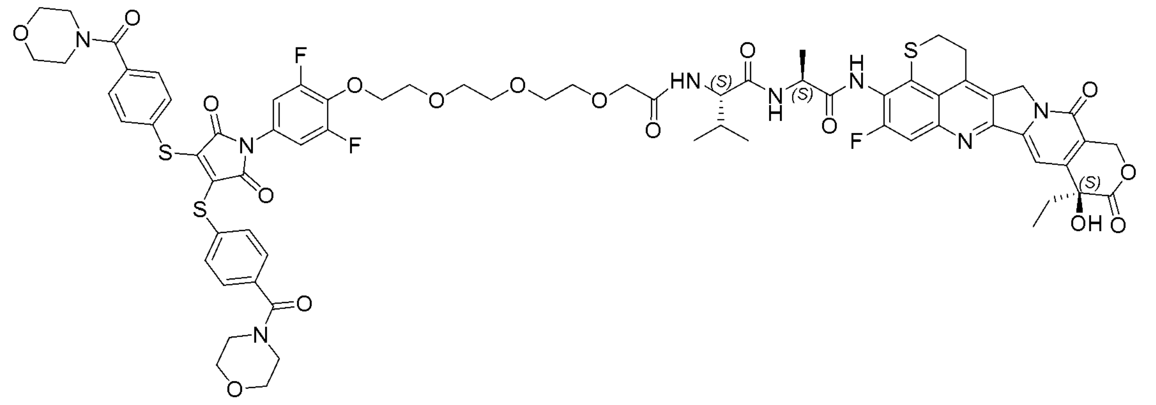

MWF-L11

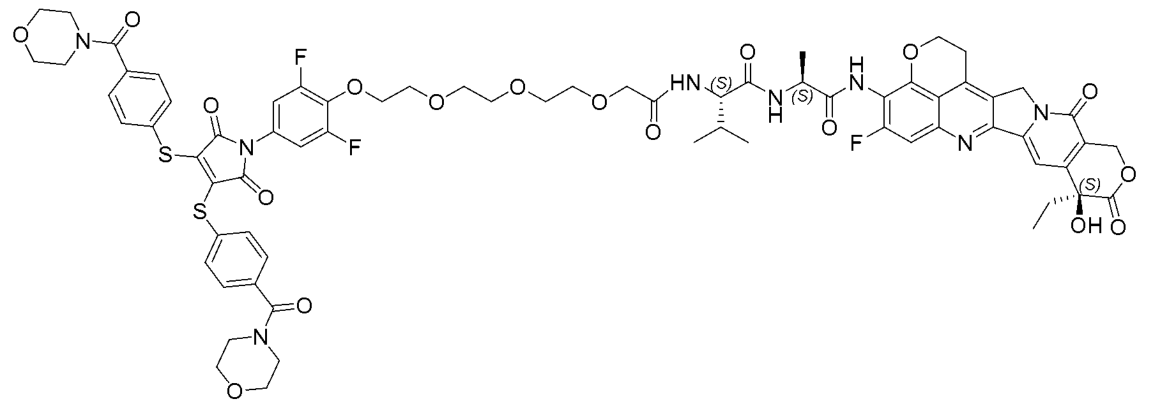

MWF-L12

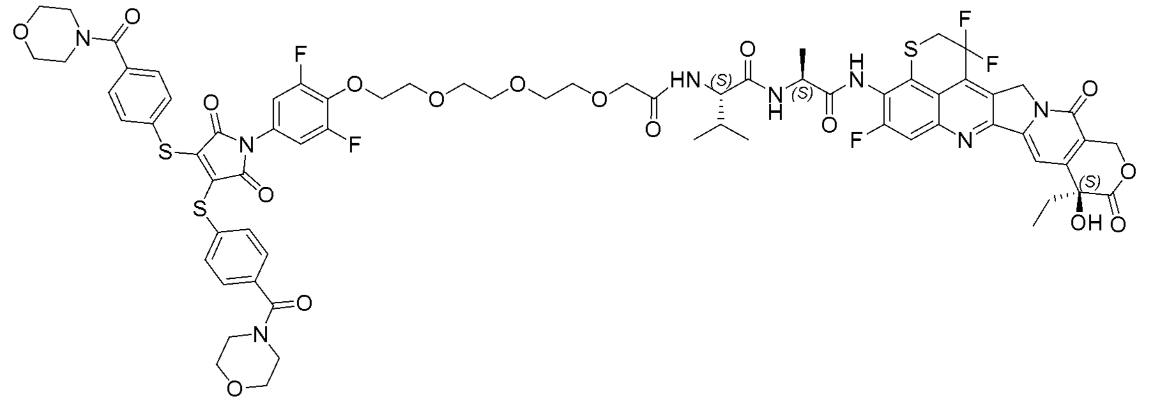

MWF-L13

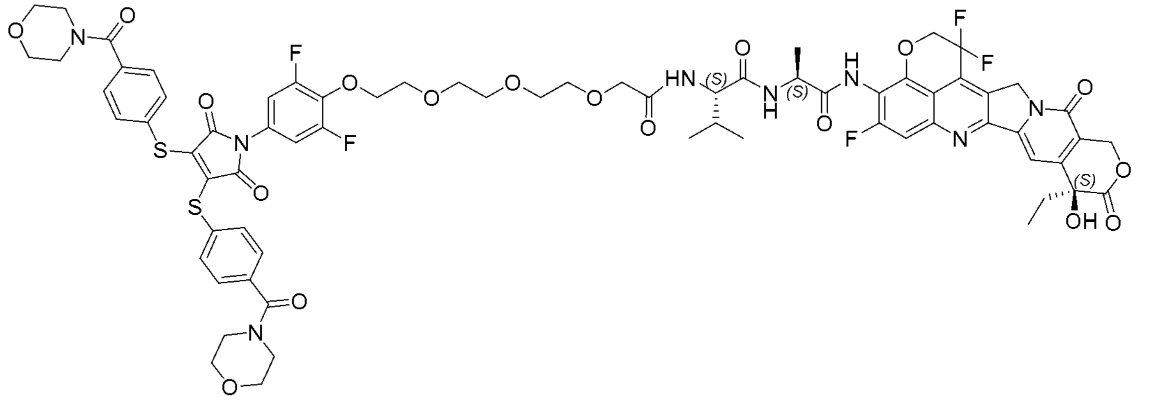

MWF-L14

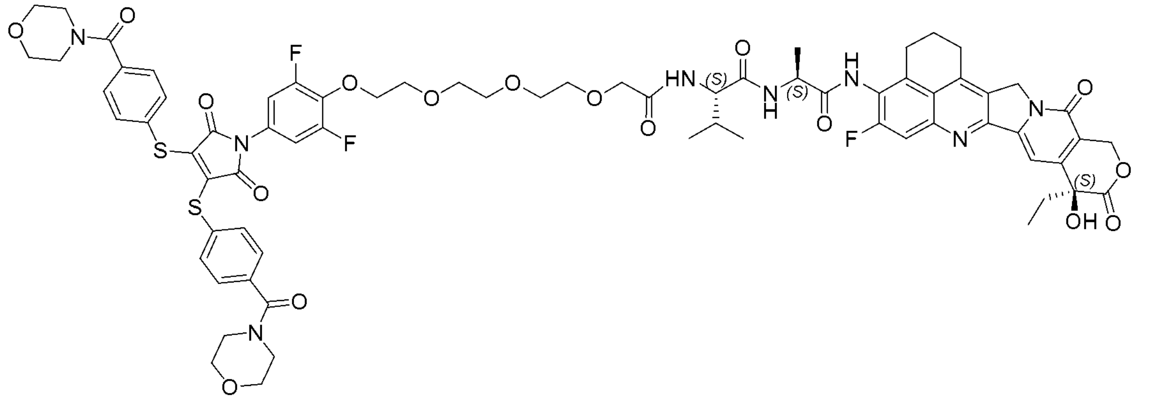

MWF-L15

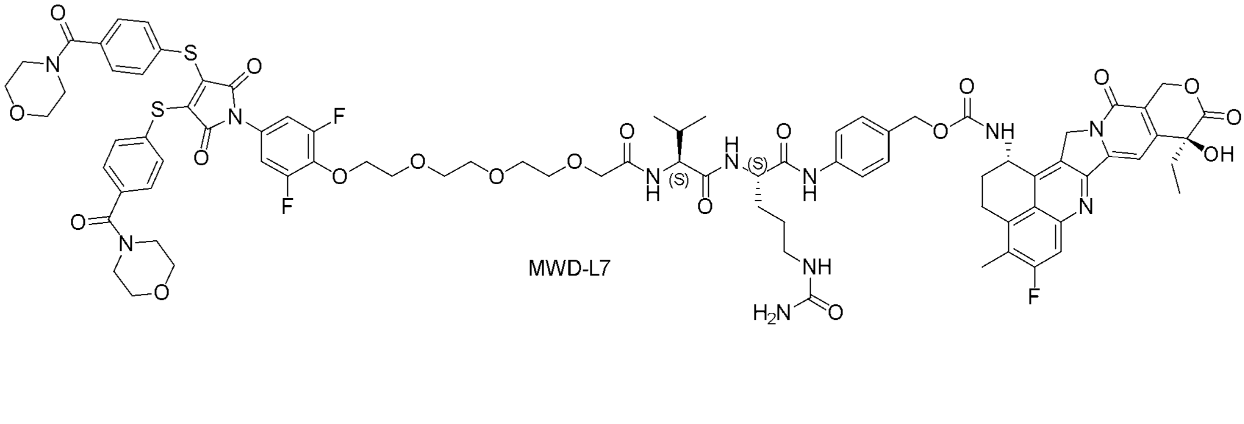

MWD-L7

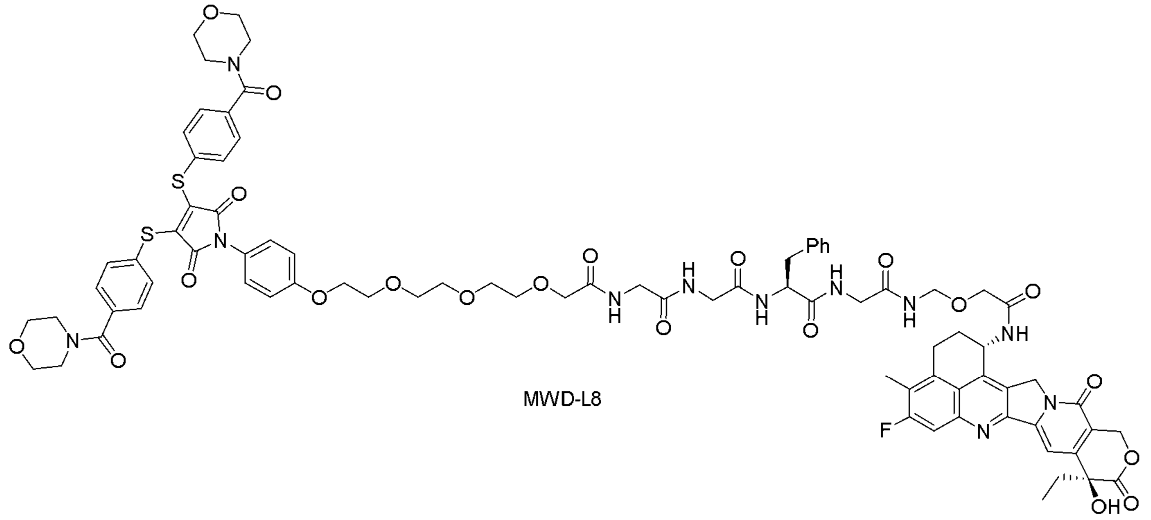

MWD-L8

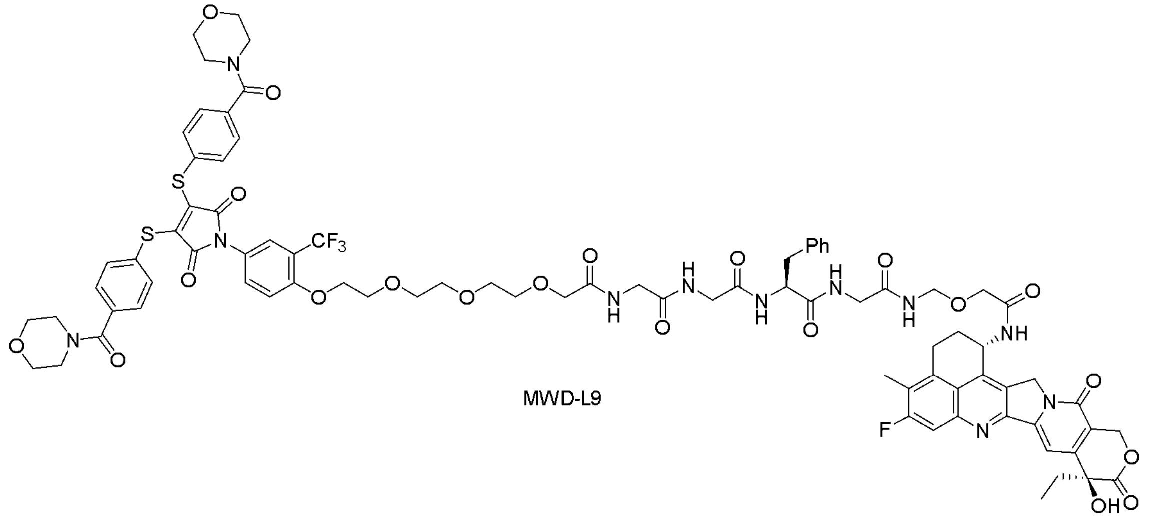

MWD-L9

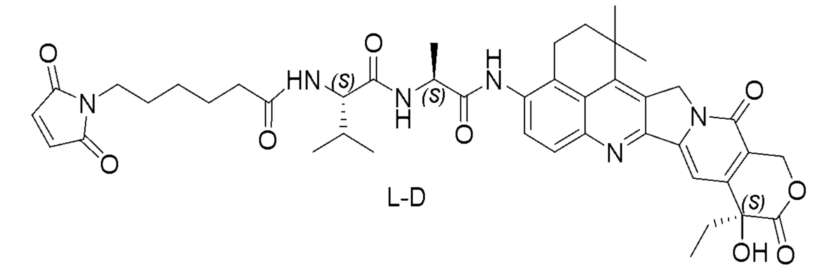

L-D

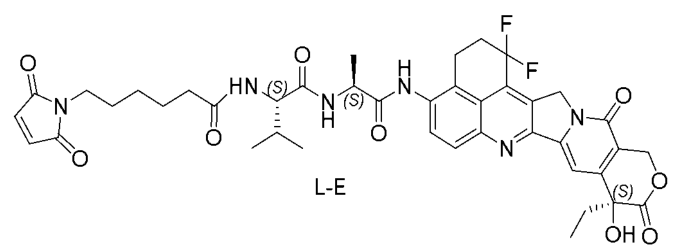

L-E

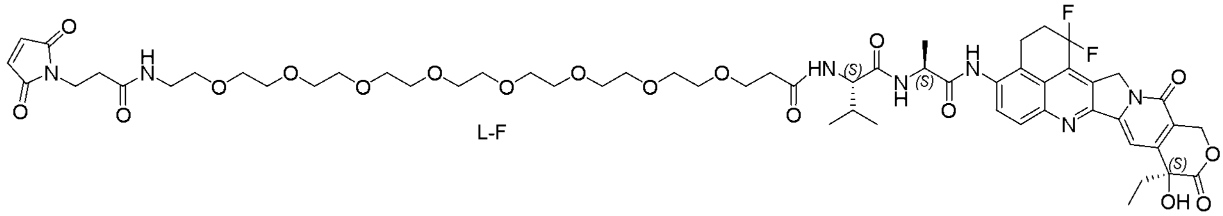

L-F

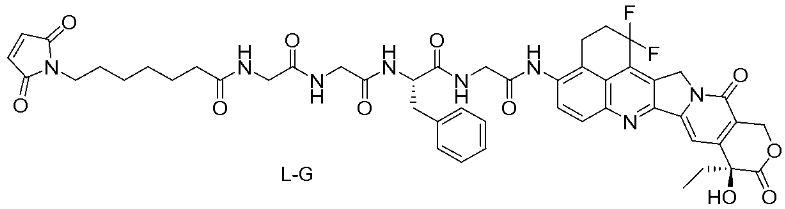

L-G

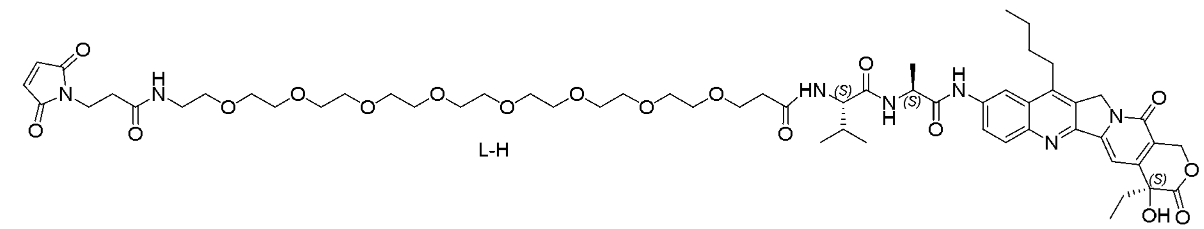

L-H

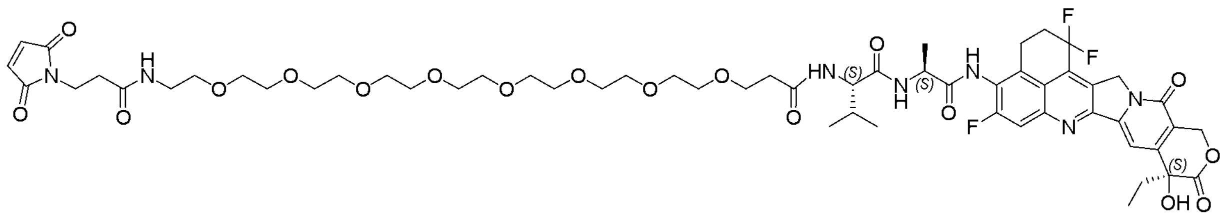

L-I

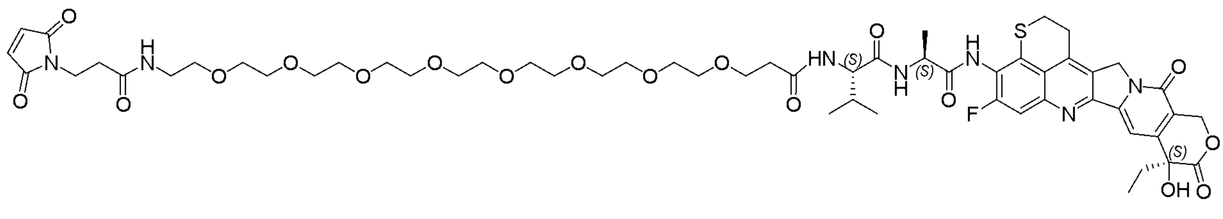

L-J

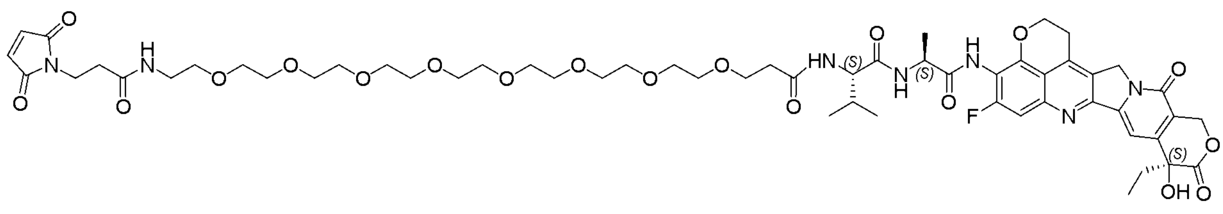

L-K

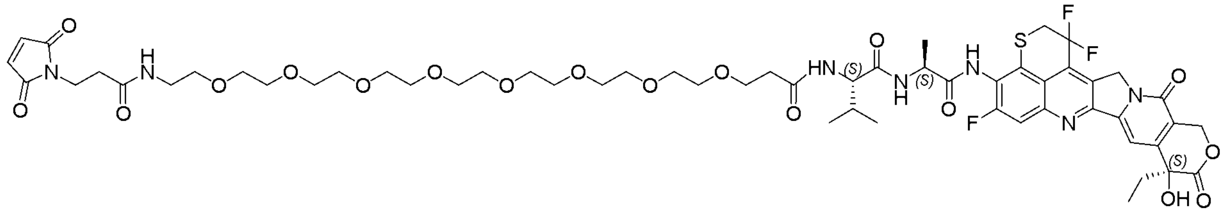

L-L

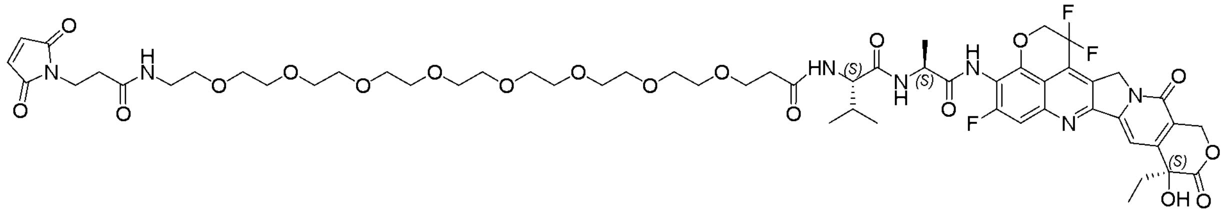

L-M

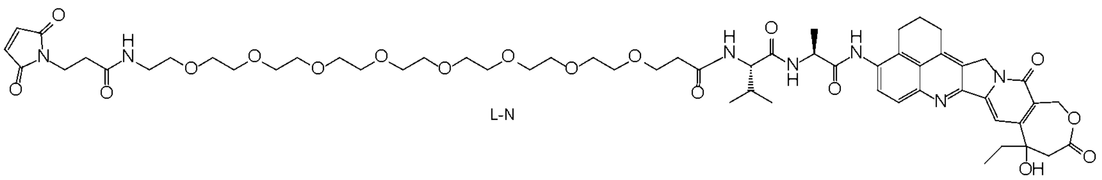

L-N

L-N

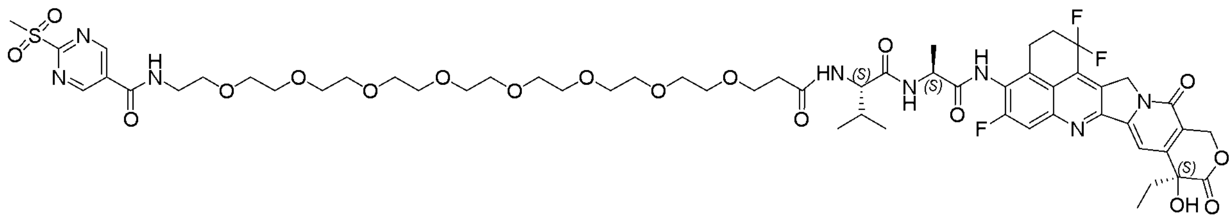

MWS-L1

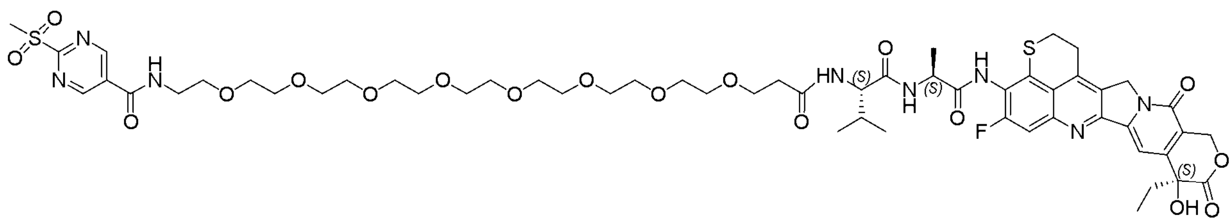

MWS-L2

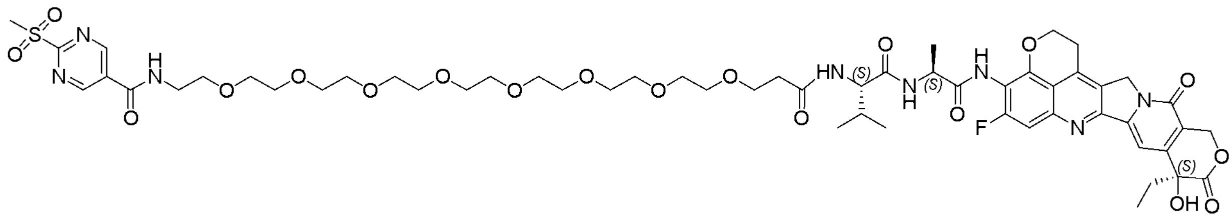

MWS-L3

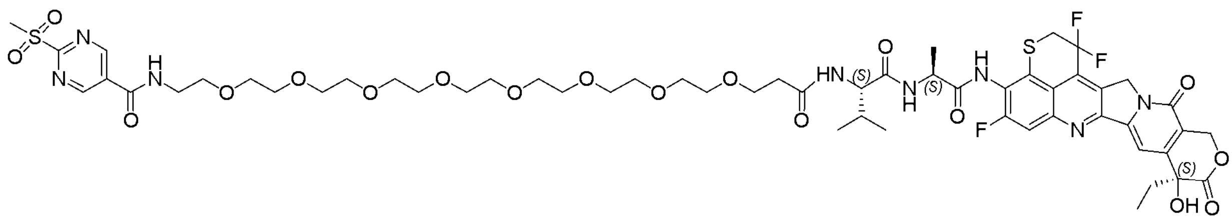

MWS-L4

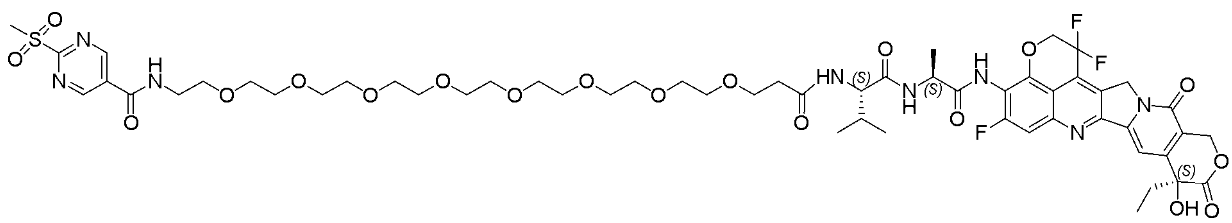

MWS-L5

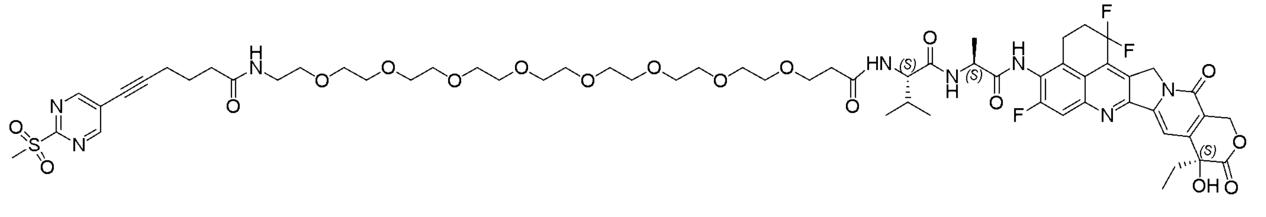

MWS-L6

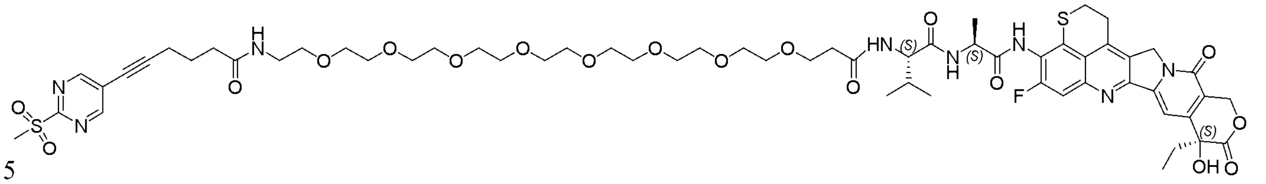

5

MWS-L7

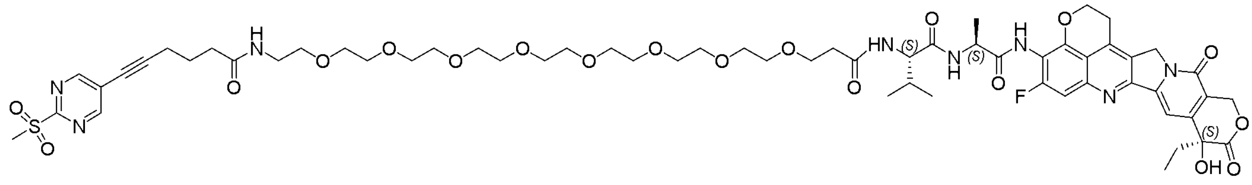

MWS-L8

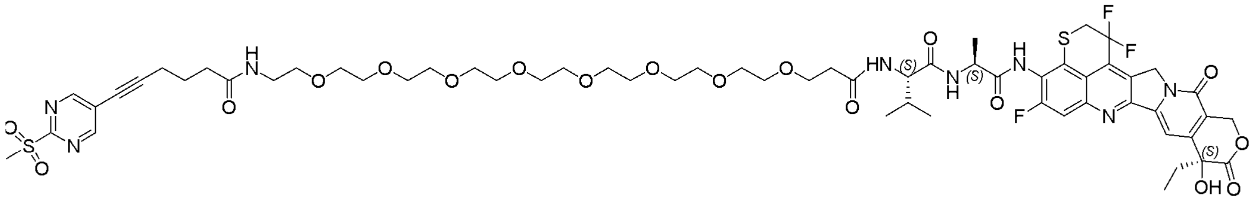

MWS-L9

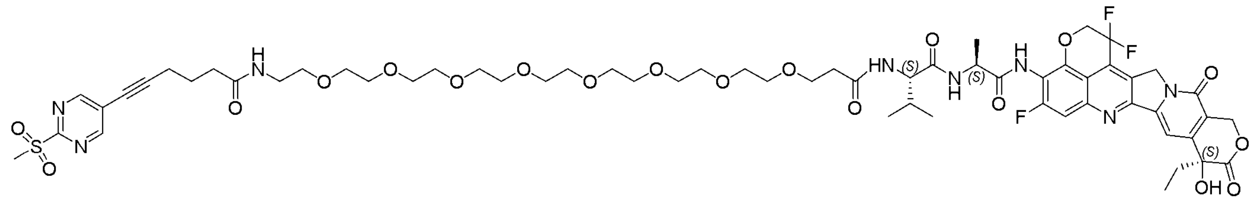

MWS-L10

**Fourteenth Aspect**

The present disclosure provides a method for preparing an antibody-drug conjugate or a salt thereof, comprising the following steps:

a. Antibody reduction: adding a reducing agent to a phosphate buffer containing an antibody in a concentration of 5-30 mg/mL at an equivalent molar ratio of ≥ 5.5: 1 (the reducing agent: the antibody), and reacting the reducing agent and the antibody for 1.5-2 hours, wherein the reducing agent is one or more selected from the group consisting of TCEP, DTT, 2-MEA, and DTBA;
b. Antibody conjugation and hydrolysis: displacing the reduced antibody obtained in step a into a phosphate buffer at pH 6.5-7.8, thereby diluting the antibody to a concentration of 3.5-15 mg/mL in the buffer to obtain a diluted antibody solution; adding a linker-payload dissolved in an organic co-solvent to the diluted antibody solution at an equivalent molar ratio of 4.5-6.5: 1 (the linker-payload: the antibody), and then reacting the reaction system under stirring at 15-35 °C for ≥ 0.5 hours, wherein the organic co-solvent is one or more selected from the group consisting of DMA, DMSO, DMF, and ACN; and then, displacing the antibody conjugation product into a phosphate buffer at pH 7.4-9.0, and heating the buffer at 35±10 °C for 2-24 hours, to obtain a hydrolysis product;
c. Hydrophobic chromatography: purifying the antibody conjugation product obtained through hydrophobic chromatography using hydrophobic filler.

In step a, the antibody is an antibody against tumor-associated antigen which is, e.g., HER2, B7-H3, HER3, CD19, CD20, CD22, CD30, CD33, CD37, CD45, CD56, CD66e, CD70, CD74, CD73, CD79b, CD138, CD147, CD223, EpCAM, Mucin 1, STEAP1, GPNMB, FGF2, FOLR1, EGFR, EGFRvIII, Tissue factor, c-MET, FGFR, Nectin 4, AGS-16, Guanylyl cyclase C, Mesothelin, SLC44A4, PSMA, EphA2, AGS-5, GPC-3, c-KIT, RoR1, PD-L1, CD27L, 5T4, Mucin16, NaPi2b, STEAP, SLITRK6, ETBR, BCMA, Trop-2, CEACAM5, SC-16, SLC39A6, Delta-like protein 3, Claudin 18.2, or the like.
Preferably, the antibody is an anti-B7-H3 antibody. More preferably, the antibody is the anti-B7-H3 antibody provided in the first aspect of the present disclosure above. For example, the anti-B7-H3 antibody may be the anti-B7-H3

antibody hz10B4, hz10B4m1, hz10B4m2, or hz10B4m3 provided in the Examples of the present disclosure.

Preferably, in step b, the linker-payload is a compound defined in the thirteenth aspect of the present disclosure above.

## Fifteenth Aspect

The antibody-drug conjugate or a salt thereof provided by the present disclosure may be contained in a composition, more particularly contained in a pharmaceutical composition, for example, a pharmaceutical preparation, to be used for various purposes as actually needed. Thus, in the fifteenth aspect, the present disclosure also provides a composition comprising the antibody-drug conjugate or a salt thereof according to the invention. Preferably, the composition is a pharmaceutical composition, optionally comprising a pharmaceutically acceptable carrier, adjuvant, or excipient. The pharmaceutical composition provided by the present disclosure can be formulated into various dosage forms known in the medical or pharmaceutical field and administered via appropriate routes of administration.

## Sixteenth Aspect

The present disclosure also provides use of the antibody-drug conjugate or a salt thereof and/or the composition comprising the same in the manufacture of a medicament for the treatment of a tumor.

Preferably, the tumor is associated with the positive or high expression of a tumor-associated antigen, which is, e.g., HER2, B7-H3, HER3, CD19, CD20, CD22, CD30, CD33, CD37, CD45, CD56, CD66e, CD70, CD74, CD73, CD79b, CD138, CD147, CD223, EpCAM, Mucin 1, STEAP1, GPNMB, FGF2, FOLR1, EGFR, EGFRvIII, Tissue factor, c-MET, FGFR, Nectin 4, AGS-16, Guanylyl cyclase C, Mesothelin, SLC44A4, PSMA, EphA2, AGS-5, GPC-3, c-KIT, RoR1, PD-L1, CD27L, 5T4, Mucin16, NaPi2b, STEAP, SLITRK6, ETBR, BCMA, Trop-2, CEACAM5, SC-16, SLC39A6, Delta-like protein 3, Claudin 18.2, or the like.

Preferably, the tumor is associated with B7-H3 expression. More preferably, the tumor is a solid tumor, such as gastric cancer, colorectal cancer, liver cancer, pancreatic cancer, esophageal cancer, non-small cell lung cancer, prostate cancer, ovarian cancer, neuroblastoma, rhabdomyosarcoma, osteosarcoma, Ewing sarcoma, Wilms tumor, and desmoplastic small round cell tumor.

## Seventeenth Aspect

Accordingly, the present disclosure also provides a method for treating a tumor, comprising administering to a subject in need thereof the antibody-drug conjugate or a salt thereof and/or the composition comprising the same according to the invention.

Preferably, the tumor is associated with the positive or high expression of a tumor-associated antigen, which is, e.g., HER2, B7-H3, HER3, CD19, CD20, CD22, CD30, CD33, CD37, CD45, CD56, CD66e, CD70, CD74, CD73, CD79b, CD138, CD147, CD223, EpCAM, Mucin 1, STEAP1, GPNMB, FGF2, FOLR1, EGFR, EGFRvIII, Tissue factor, c-MET, FGFR, Nectin 4, AGS-16, Guanylyl cyclase C, Mesothelin, SLC44A4, PSMA, EphA2, AGS-5, GPC-3, c-KIT, RoR1, PD-L1, CD27L, 5T4, Mucin16, NaPi2b, STEAP, SLITRK6, ETBR, BCMA, Trop-2, CEACAM5, SC-16, SLC39A6, Delta-like protein 3, Claudin 18.2, or the like.

Preferably, the tumor is associated with B7-H3 expression (including overexpression). More preferably, the tumor is a solid tumor, such as gastric cancer, colorectal cancer, liver cancer, pancreatic cancer, esophageal cancer, non-small cell lung cancer, prostate cancer, ovarian cancer, neuroblastoma, rhabdomyosarcoma, osteosarcoma, Ewing sarcoma, Wilms tumor, and desmoplastic small round cell tumor.

Preferably, the subject is a mammal, preferably a primate, more preferably a human.

When applied, the method for treating a disease described above provided by the present disclosure depends on multiple factors, including the specific active ingredient administered, the age, body weight, gender, physical and medical condition of the patient, the severity of the condition to be treated, the route of administration, and so forth.

Based on a known anti-B7-H3 humanized antibody, the inventors of the present invention employed site-directed mutagenesis in the variable region, and screened and obtained a series of novel anti-B7-H3 humanized antibodies. Compared to the parent antibody, these antibodies retain the high affinity of the parent antibody for B7-H3 while exhibiting significantly higher purity, thereby overcoming the parent antibody's susceptibility to fragmentation and reducing toxic side effects. Furthermore, the inventors of the present invention conjugated this series of novel humanized antibodies with camptothecins of specific structures, and generated antibody-drug conjugates. By leveraging the stable and robust structures and high targeting capability toward B7-H3-expressing cells of these antibodies and the potent cytotoxic effects of the specific camptothecins, the resulting ADCs achieve superior tumor-killing efficacy.

## BRIEF DESCRIPTION OF THE DRAWINGS

Embodiments of the invention are described in detail below with reference to the attached figures, in which:

**Figure 1** shows the binding activity of the anti-B7-H3 antibodies provided by the present disclosure to B7-H3 expressed on the surface of cancer cells.

**Figure 2** shows the results of drug potency of the antibody-drug conjugate targeting B7-H3 provided by the present disclosure in a mouse model of lung cancer.

**Figure 3** shows the results of drug potency of the antibody-drug conjugate targeting B7-H3 and the anti-B7-H3 antibody provided by the present disclosure in a mouse model of pancreatic cancer.

**Figure 4** shows the results of apoptosis induction in cancer cells by the antibody-drug conjugate targeting B7-H3 and the anti-B7-H3 antibody provided by the present disclosure.

**Figure 5** shows the detection results of bystander effect (mean ± SD, n = 2) of the antibody-drug conjugate targeting B7-H3 provided by the present disclosure.

**Figure 6** shows the detection results of the ability of the antibody-drug conjugate targeting B7-H3 provided by the present disclosure to induce cell cycle arrest in B7H3-positive cells (mean ± SD, n=2); in which the results for the G0/G1, G2/M, and S phases are arranged from top to bottom respectively.

## DETAILED DESCRIPTION OF PREFERRED EMBODIMENTS

The invention is illustrated below with reference to specific examples. It will be understood by those skilled in the art that these examples are merely illustrative of the invention and do not limit the scope of the invention in any way.
Experimental procedures in the following examples are all conventional ones, unless otherwise specified. Raw materials and reagents used in the following examples are all commercially available products, unless otherwise specified.
Human B7-H3: UniProtKB-Q5ZPR3;
Control antibody hum30: an anti-B7-H3 antibody, prepared using the method described in the patent publication US20160368990A1.

### Group 1 of Examples Screening and characterization of novel anti-B7-H3 humanized antibodies

### Example 1 Preparation of anti-B7-H3 antibodies

Antibody hz10B4 was prepared according to the method provided in patent application CN202111459992.0 (CN114573695A). The amino acid sequences of the heavy chain variable region and light chain variable region of antibody hz10B4 are shown below (i.e., SEQ ID NO: 20 and SEQ ID NO: 22 in CN114573695A), with CDRs underlined (using the Kabat definition).

> hz10B4-VH (VH/CDR-H1/CDR-H2/CDR-H3: SEQ ID NOs: 1/8/9/10)

EVQLVQSGAEVKKPGASVKVSCKASGYTFT**EYIMH**WVRQAPGQGLEWMG**GINPASGGTTYNQKFKG**RVTMTRDTSISTAYMELSRLRSEDTAVYYCAR**KGKDYFDWYFDV**WGQGTTVTVSS

> hz10B4-VL (VL/CDR-L1/CDR-L2/CDR-L3:SEQ ID NOs: 2/11/12/13)

DIQLTQSPSSLSASVGDRVTMTC**SASSSVSYMH**WYQQKPGKAPKLLIY**DTSKLAS**GVPSRFSGSGSGTDFTLTISSLQPEDFATYYC**QQWSSNPLT**FGGGTKVEIK

Five point mutations were designed for the LCDR3 of hz10B4. The resulting VL variants were each combined with hz10B4-VH, and a series of anti-B7-H3 antibodies were obtained by referring to the method described in Example 9 of

CN114573695A (see Table 1).

**Table 1.** Point mutations in LCDR3 of hz10B4

| Anti-B7-H3 antibody | VH×VL | Point mutation in LCDR3 |
|---|---|---|
| hz10B4 | hz10B4-VH×hz10B4-VL (SEQ ID NOs: 1×2) | / |
| hz10B4m1 | hz10B4-VH×hz10B4-Lm1-VL (SEQ ID NOs: 1×3) | SSN→SSA |
| hz10B4m2 | hz10B4-VH×hz10B4-Lm2-VL (SEQ ID NOs: 1×4) | SSN→SAS |
| hz10B4m3 | hz10B4-VH×hz10B4-Lm3-VL (SEQ ID NOs: 1×5) | SSN→SSQ |
| hz10B4m4 | hz10B4-VH×hz10B4-Lm4-VL (SEQ ID NOs: 1×6) | SSN→SSG |
| hz10B4m5 | hz10B4-VH×hz10B4-Lm5-VL (SEQ ID NOs: 1×7) | SSN→SGS |

> hz10B4-Lm1-VL (VL/CDR-L1/CDR-L2/CDR-L3: SEQ ID NOs: 3/11/12/14)

DIQLTQSPSSLSASVGDRVTMTC**SASSSVSYMH**WYQQKPGKAPKLLIY**DTSKLAS**GVPS RFSGSGSGTDFTLTISSLQPEDFATYYC**QQW*SSA*PLT**FGGGTKVEIK

> hz10B4-Lm2-VL (VL/CDR-L1/CDR-L2/CDR-L3: SEQ ID NOs: 4/11/12/15)

DIQLTQSPSSLSASVGDRVTMTC**SASSSVSYMH**WYQQKPGKAPKLLIY**DTSKLAS**GVPS RFSGSGSGTDFTLTISSLQPEDFATYYC**QQW*SAS*PLT**FGGGTKVEIK

> hz10B4-Lm3-VL (VL/CDR-L1/CDR-L2/CDR-L3: SEQ ID NOs: 5/11/12/16)

DIQLTQSPSSLSASVGDRVTMTC**SASSSVSYMH**WYQQKPGKAPKLLIY**DTSKLAS**GVPS

RFSGSGSGTDFTLTISSLQPEDFATYYC**QQW*SSQ*PLT**FGGGTKVEIK

> hz10B4-Lm4-VL (VL/CDR-L1/CDR-L2/CDR-L3: SEQ ID NOs: 6/11/12/17)

DIQLTQSPSSLSASVGDRVTMTC**SASSSVSYMH**WYQQKPGKAPKLLIY**DTSKLAS**GVPS RFSGSGSGTDFTLTISSLQPEDFATYYC**QQW*SSG*PLT**FGGGTKVEIK

> hz10B4-Lm5-VL (VL/CDR-L1/CDR-L2/CDR-L3: SEQ ID NOs: 7/11/12/18)

DIQLTQSPSSLSASVGDRVTMTC**SASSSVSYMH**WYQQKPGKAPKLLIY**DTSKLAS**GVPS RFSGSGSGTDFTLTISSLQPEDFATYYC**QQW*SGS*PLT**FGGGTKVEIK

**Example 2 Functional evaluation of the anti-B7-H3 antibodies**

**2.1** Study on the binding affinity of antibodies to human B7-H3 ECD-His

The binding affinity of hz10B4, hz10B4m1 to hz10B4m5, and the control antibody hum30 to human B7-H3 was determined using ForteBio Octet® QKe.

hz10B4, hz10B4m1 to hz10B4m5, and the control antibody hum30 were diluted to 4 μg/mL in PBS buffer and flowed over the surface of AHC probes (Cat: 18-0015, PALL) for 120 seconds. Recombinant human B7-H3 extracellular domain protein (Accession No.: UniProtKB-Q5ZPR3, amino acids 1-461, C-His tag) (60 nM) was used as the mobile phase. The association time was 300 seconds, and the dissociation time was 300 seconds. After the experiment, the data were fitted using a 1:1 Langmuir binding model with the analysis software to calculate the kinetic constants of the antigen-antibody binding.

The affinity detection results for hz10B4 (SEQ ID NOs: 1+2), hz10B4m1 (SEQ ID NOs: 1+3), hz10B4m2 (SEQ ID NOs: 1+4), hz10B4m3 (SEQ ID NOs: 1+5), hz10B4m4 (SEQ ID NOs: 1+6), hz10B4m5 (SEQ ID NOs: 1+7), and hum30 binding to the recombinant human B7-H3 extracellular domain protein are shown in Table 2. The results demonstrated that the affinity of hz10B4m1, hz10B4m2, and hz10B4m3 for the recombinant human B7-H3 ECD protein was similar to that of the parent hz10B4, with a slight decrease in Koff. Among them, the affinity of hz10B4m1 was slightly stronger than that of hz10B4m2 and hz10B4m3.

**Table 2.** Detection results of the affinity of the antibodies for the recombinant human B7-H3 ECD protein

| Anti-B7-H3 antibody | ka (1/Ms) | kd (1/s) | KD (nM) |
|---|---|---|---|
| hz10B4 | 6.65E+05 | 1.43E-04 | 2.16E-10 |
| hz10B4m1 | 6.39E+05 | 2.25E-04 | 3.52E-10 |
| hz10B4m2 | 7.55E+05 | 2.87E-04 | 3.81E-10 |
| hz10B4m3 | 6.66E+05 | 2.81E-04 | 4.22E-10 |
| hz10B4m4 | 5.05E+05 | 4.20E-04 | 8.31E-10 |
| hz10B4m5 | 5.92E+05 | 3.36E-04 | 5.68E-10 |
| hum30 | 9.41E+05 | 3.24E-04 | 3.44E-10 |
| NC | NA | NA | NA |

**2.2** Study on the binding activity of the antibodies to the surface antigen of SKOV3 cells

The binding activity of hz10B4 and hz10B4m1 to hz10B4m5 to the antigen on the surface of SKOV3 cells naturally express human B7-H3 used as target cells, was detected by FACS.

SKOV3 cells naturally expressing human B7-H3 and grown to the logarithmic phase were collected by centrifugation. The cell density was adjusted to $2\times10^5$ cells/mL using complete medium (F-12K medium containing 10% FBS and 250 μg/mL G418). 1 mL of the cell suspension (containing $2\times10^5$ cells) was aliquoted into each of several EP tubes, then centrifuged, and the supernatant was discarded. The cells were washed twice with PBS. 200 μL of each antibody solution (hz10B4 and hz10B4m1 to hz10B4m5, each at a starting concentration of 198 nM, serially diluted 3-fold across 12 concentrations) was added to the cell pellets in each tubes. The mixtures were incubated at 37 °C for 30 min. The following controls were included: (1) Positive Control (PC): control antibody hum30; (2) Negative Control (NC-huIgG1): an isotype control antibody. After incubation, the cells were washed three times with PBS. A 1:200 dilution of Goat anti-human IgG-FITC (Cat: F9512, Sigma) was added and incubated for 30 min. The cells were washed three times with PBS, and the Mean Fluorescence Intensity (MFI) was detected using a flow cytometer (Model B49007AD, SNAW31211, BECKMAN COULTER) to assess the binding capability of the test antibodies to B7-H3 on the surface of SKOV3 cells.

The results of the binding activity of hz10B4, hz10B4m1, hz10B4m2, hz10B4m3, hz10B4m4, hz10B4m5, and hum30 to the antigen on the surface of human SKOV3 cells are shown in Table 3 and Figure 1. The results demonstrated that the binding activities of hz10B4m1, hz10B4m2, and hz10B4m3 to the surface antigen of SKOV3 cells were similar to that of the parent hz10B4.

Table 3. Binding activity of the antibodies to the surface antigen of SKVO3 cells

| Anti-B7-H3 antibody | EC50 (nM) |
|---|---|
| hz10B4m1 | 1.893 |
| hz10B4m2 | 1.449 |
| hz10B4m3 | 1.589 |
| hz10B4m4 | 16.9 |
| hz10B4m5 | 2.247 |
| hum30 | 1.218 |
| hz10B4 | 1.585 |

**Example 3 Purity analysis of the anti-B7-H3 antibodies**

The purity of the anti-B7-H3 antibodies was determined by capillary electrophoresis-sodium dodecyl sulfate (CE-SDS) under both reduced and non-reduced conditions. The purity of the antibody products was assessed according to the method "Monoclonal antibodies - Determination of molecular size variants (CE-SDS)" described in Part IV, General rule 3127 of Chinese Pharmacopoeia. Specific results are shown in Table 4.

**Table 4.** Results of purity analysis of the anti-B7-H3 antibodies

| Antibody | NR-CE-SDS (%) | | R-CE-SDS (%) | | |
|---|---|---|---|---|---|
| | Fragment peak | Purity | Non-glycosylated heavy chain | Aggregates | Purity (LC+HC) |
| hz10B4m1 | 1.8 | 98.2 | 0.3 | 0.9 | 98.8 |
| hz10B4m2 | 1.6 | 98.4 | 0.3 | 0.8 | 98.7 |
| hz10B4m3 | 1.6 | 98.4 | 0.3 | 0.8 | 98.8 |
| hz10B4 | 9.5 | 90.5 | NA | NA | NA |

The results from both NR-CE-SDS and R-CE-SDS analyses demonstrated that the purity of hz10B4m1, hz10B4m2, and hz10B4m3 all exceeded 98%, and was superior to that of the parent antibody hz10B4.

**Group 2 of Examples Synthesis of camptothecins**

Universal method for synthesizing camptothecins:

The compound of camptothecins according to the invention can be obtained by Friedlander reaction with a compound represented by formula A and a tricyclic compound (cyclic compound CDE), and the general reaction is shown as follows:

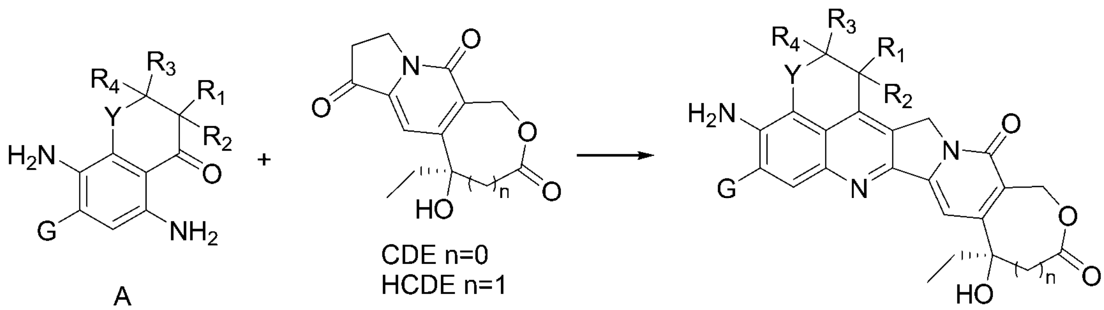

In the reaction, the tricyclic compound CDE can be purchased from MCE (MedChemExpress):

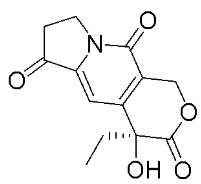

CDE .

The tricyclic compound HCDE can be obtained according to the method described in Bioorganic & Medicinal Chemistry, 2010, vol. 18, # 9, p. 3140-3146:

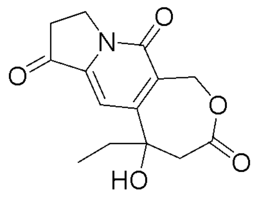

HCDE

.

**1 Synthesis of the compound represented by formula A**

**Example 4 Synthesis of Compound A1**

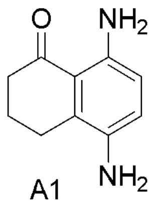

A1

Method 1: Compound A1 was synthesized according to the method described in the patent publication WO2020200880A1, and synthetic route is shown as follows:

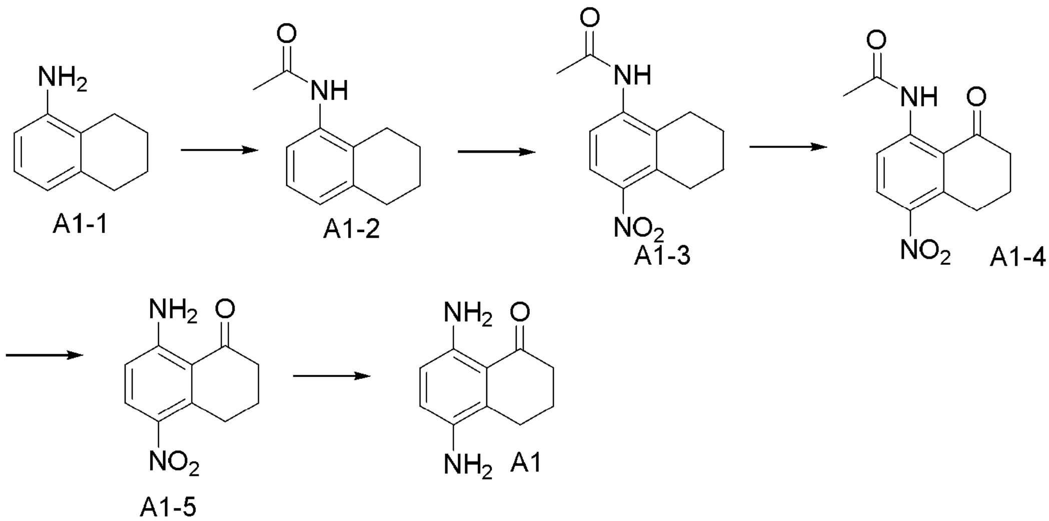

A1-1 A1-2 A1-3 A1-4 A1-5 A1

Method 2: Compound A1 was synthesized by a palladium-catalyzed coupling reaction with a zinc reagent, and synthetic route is shown as follows:

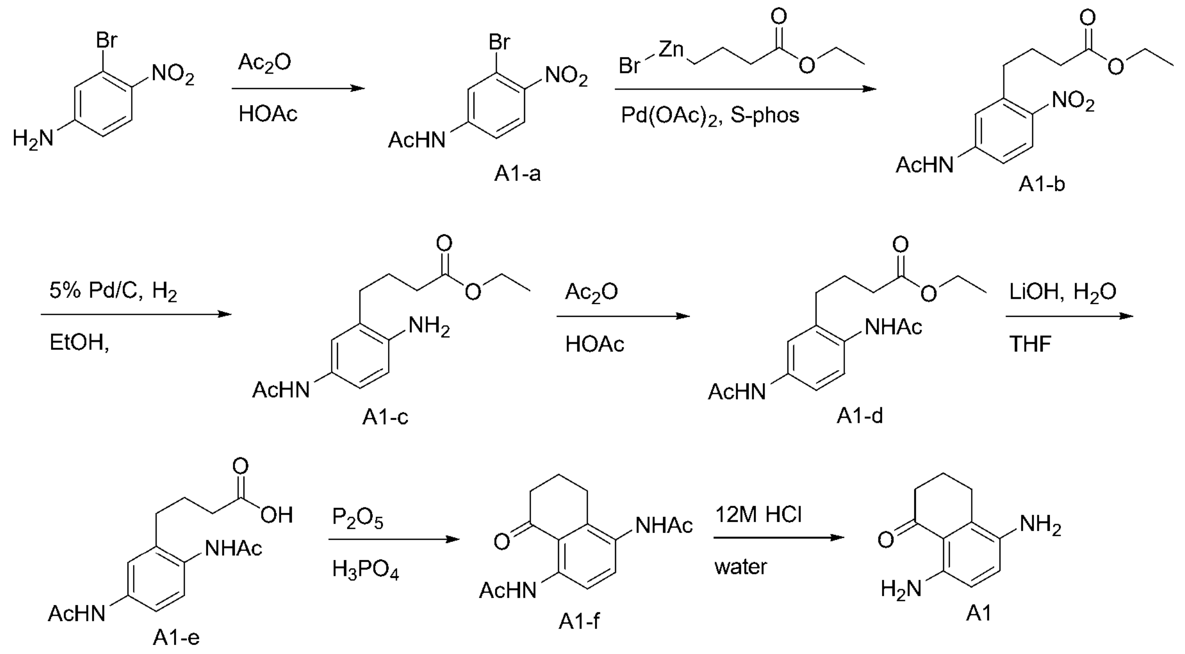


(1) Acetylation:

3-bromo-4-nitroaniline (25.00 g, 0.12 mol) and 200 mL of acetic acid were added into a 500 mL flask, into which 50 mL of acetic anhydride was then slowly added dropwise. Those starting compounds reacted at room temperature for 16 hours. After the reaction completed as confirmed with TLC detection, reaction liquid was filtered to obtain filtrate, which was in turn concentrated under reduced pressure to remove acetic acid. Filter cakes obtained were pooled and slurried with 200 mL of MTBE, and then the slurry was filtered, and 27.6 g of dried yellow solid (intermediate A1-a) was obtained with a yield of 92%. LC-MS (ESI): m/z 259 (M+H)+.

(2) Preparation of 4-ethoxy-4-oxobutylzinc bromide

Activated zinc powder (19.0 g, 0.29 mol, 2.00 eq) was added into a 250 mL three-neck flask (equipped with a thermometer, reflux condenser tubes and rubber plugs). Then air in the flask was replaced with nitrogen, anhydrous DMF (145 mL) was added, and then the air in the flask was replaced with nitrogen again. Iodine (1.86 g, 0.015mol, 0.1 eq) was added at room temperature, and the color of the solution was observed to change from colorless to brownish red, to light yellow gradually, and to colorless finally (in 2-3 min). Next, ethyl 4-bromobutyrate (28.6 g, 0.15 mol, and 1.00 eq) was added into the flask which was then heated to 80 °C (inner temperature) for reaction for 4-5 hours. When the reaction completed as confirmed with TLC detection, reaction liquid was allowed to stand and cool to room temperature for later use. The supernatant obtained was light yellow and had a concentration of 1 mol/L.

(3) Coupling with the Zinc reagent

Anhydrous DMF (120 mL) and the intermediate A1-a (25.0 g, 1.00 eq) were added into a 500 mL reaction flask. Then air in the flask was replaced with nitrogen, palladium acetate (433 mg, 0.02 eq) was added, and then the air in the flask was replaced with nitrogen again. The mixture in the flask was stirred at room temperature for 10 min, then S-PHOS (1.6 g, 0.04 eq) was added, and then the air in the flask was replaced with nitrogen again. Next, the mixture in the flask was stirred for 20 min, 4-ethoxy-4-oxobutylzinc bromide (145 mL, 1.50 eq) prepared in the above step was added dropwise at room temperature (25-30 °C), and then the reaction was maintained at 25-30 °C for 16 hours. When the starting compounds reacted completely as confirmed with TLC detection (DCM: EA =5: 1), reaction liquid was cooled to room temperature, and ammonium chloride solution (15 mL) was added to the reaction liquid to quench the reaction. Then the reaction liquid was poured into 1 L of water, and 400 mL of ethyl acetate was added, to obtain separated phases. The phases were filtered through a buchner funnel, and then aqueous phase obtained was extracted with ethyl acetate (400 mL × 2); and organic phases then obtained were washed with water (500 mL) twice and with saturated sodium chloride solution (500 mL) once, then dried over anhydrous sodium sulfate, and concentrated under reduced pressure, and 37.0 g of reddish brown oily liquid (the intermediate A1-b) was obtained with a yield of 130%. The crude product was used in the next reaction without further purification. LC-MS (ESI): [M+1]+ = 295.

(4) Synthesis of the intermediate A1-c

The intermediate A1-b (37.0 g, 1.0 eq) and ethanol (1.5 L) were added into a 3 L three-neck reaction flask and the starting material was completely dissolved in the ethanol. The reaction system was cooled to 5 °C, 5% Pd/C (5.7 g, 1.0 eq) was added, and the temperature was raised to 25 °C in a hydrogen atmosphere (atmospheric pressure) for reaction for 16 hours. When the reaction completed as confirmed with TLC detection (DCM: EA =5: 1), the reaction system was filtered through celite, and organic phase obtained was concentrated to obtain 33.0 g of a crude product (the intermediate A1-c) with a yield of 130%. The crude product was used directly in the next reaction without further purification. LC-MS (ESI): [M+1]+ = 265.

(5) Synthesis of the intermediate A1-d

The intermediate A1-c (33.0 g, 1.00 eq) and 260 mL of acetic acid were added into a 1 L flask, into which 46 mL of acetic anhydride was then slowly added dropwise. The starting material compounds reacted at room temperature for 2 hours. When the starting compounds reacted completely as confirmed with TLC detection (DCM: MeOH =10: 1), reaction liquid was filtered to obtain filtrate, which was in turn concentrated under reduced pressure to remove acetic acid. 250 mL of water was added, and then aqueous phase obtained was extracted with ethyl acetate (400 mL × 2); and organic phases then obtained were washed with water (500 mL) twice and with saturated sodium chloride solution (500 mL) once, then dried over anhydrous sodium sulfate, and concentrated under reduced pressure, and 32 g of a crude product was obtained. The crude product was slurried with 400 mL of MTBE, then filtered, and 27.0 g of dried light yellow solid (the intermediate Al-d) was obtained with a yield of 91%. The total yield of the four steps was found to be 83.7%.

LC-MS (ESI): [M+1]+ =307.

1H NMR (400 MHz, DMSO) δ 9.87 (s, 1H), 9.19 (s, 1H), 7.41 (s, 1H), 7.37 (d, J = 8.7 Hz, 1H), 7.20 (d, J = 8.5 Hz, 1H), 4.06 (q, J = 7.0 Hz, 2H), 2.52 (d, J = 8.5 Hz, 2H), 2.29 (t, J = 7.3 Hz, 2H), 2.02 (s, 6H), 1.79 - 1.64 (m, 2H), 1.18 (t, J = 7.1 Hz, 3H).

(6) Synthesis of the intermediate A1-e

The intermediate A1-d (27.0 g, 88 mmol, 1.0 eq), water (100 mL) and tetrahydrofuran (200 mL) were added into a 500 mL three-neck reaction flask and the starting material was completely dissolved. Lithium hydroxide monohydrate (18.5 g, 441 mmol, 5.0 eq) was added at room temperature and the reaction system was maintained at room temperature for reaction for 3 hours. When the reaction completed as confirmed with TLC detection, a majority of tetrahydrofuran was distilled off under reduced pressure, and 300 mL of water were added to residue obtained. Aqueous phase obtained was extracted with EA (2 × 100 mL), and then organic phases obtained were discarded and aqueous phases obtained were pooled and placed in an ice bath and adjusted to pH 4 with the addition of 6 N hydrochloric acid. Solid precipitated and was filtered, and 19.1 g of white solid (the intermediate A1-e) was obtained with a yield of 78%.

1H NMR (400 MHz, DMSO) δ 12.09 (s, 1H), 9.86 (s, 1H), 9.18 (s, 1H), 7.38 (d, J = 15.5 Hz, 2H), 7.23 (d, J = 8.5 Hz, 1H), 2.51 (d, J = 8.1 Hz, 2H), 2.23 (t, J = 7.1 Hz, 2H), 2.02 (s, 6H), 1.76 - 1.61 (m, 2H)

(7) Synthesis of Compound A1

40 mL of polyphosphoric acid was added to a 250 mL reaction flask and heated to 90 °C, and then the intermediate A1-e (5.0 g, 17.97 mmol) was added in several parts. The internal temperature was maintained at 95-100 °C for reaction for 5 hours. When the reaction completed as confirmed with TLC detection, heating source was removed, and the reaction system was allowed to cool to 50-60 °C. Next, 15 mL of 4 M HCl (aq) was added dropwise into the reaction system to quench the reaction (the temperature would raise to 100 °C). After that, 600 mL of 4 M NaOH aqueous solution was added dropwise to adjust the pH to 10. Aqueous phase obtained was extracted with ethyl acetate (50 mL x 3), and organic phases then obtained were pooled and washed with saturated sodium chloride solution (50 mL) once, then dried over anhydrous sodium sulfate, and concentrated under reduced pressure, and 3.75 g of yellow solid (the intermediate A1-f) was obtained with a yield of 80.5%. The solid was directly used in the next reaction without purification. LC-MS (ESI): [M+1]+ = 261.

The starting material intermediate A1-f (3.75 g) was suspended in 19% hydrochloric acid (30 mL) in a 250 mL three-neck reaction flask. The reaction system was heated to 90 °C (the inner temperature) for reaction for 3 hours. When the reaction completed as confirmed with TLC detection, the flask was placed in an ice-salt bath, cooling to below 5 °C. 4 M NaOH solution (45 mL, 30 eq) was added dropwise to adjust the pH to 10. Aqueous phase obtained was extracted with ethyl acetate (80 mL x 5), and organic phases then obtained were pooled and washed with saturated sodium chloride solution (50 mL) once, then dried over anhydrous sodium sulfate, concentrated under reduced pressure, and 2.45 g of a crude product was obtained. The crude product was purified by column chromatography using DCM as an eluent, and then

1.93 g of yellow solid (Compound A1) was obtained with a yield of 76%. The total yield of the two steps was found to be 61.2%.

LC-MS (ESI): [M+1]+ = 177.

1H NMR (400 MHz, DMSO) δ 6.76 (d, J = 8.7 Hz, 1H), 6.68 (s, 2H), 6.42 (d, J = 8.7 Hz, 1H), 4.17 (s, 2H), 2.55 (t, J = 5.9 Hz, 2H), 2.46 (t, J = 6.2 Hz, 2H), 2.00 - 1.80 (m, 2H)

Method 3: Compound A1 was synthesized by lactam hydrolysis, and synthetic route is shown as follows:

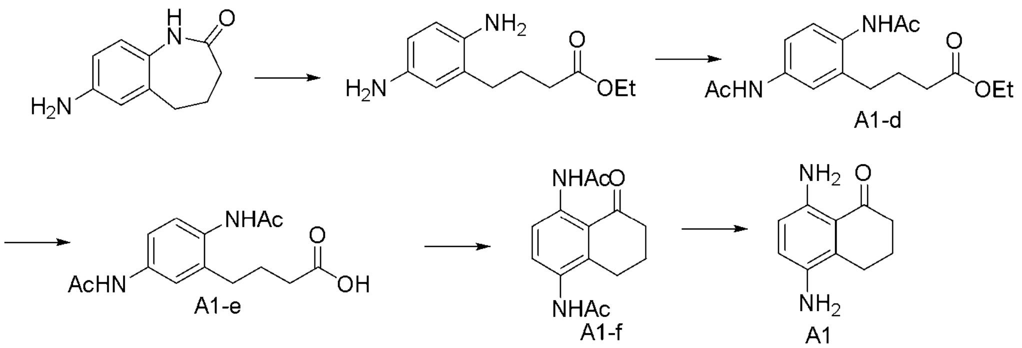

A1-d

A1-e

A1-f

A1

An aminolactam compound was prepared according to the method described in the patent publication CN106349233A.

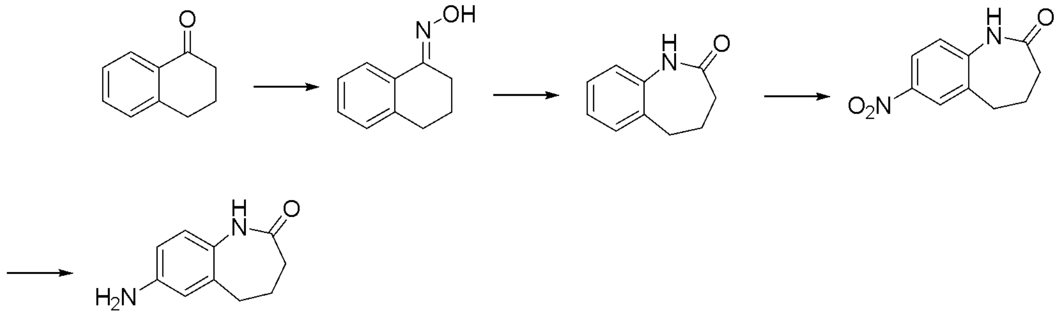

The aminolactam compound (17.6 g, 0.1 mmol), ethanol (250 mL), and 98% sulfuric acid (5 mL) were mixed in a 500 mL three-neck flask, and heated under reflux and reacted for 24 hours. Reaction liquid was sampled and detected to see whether the reaction completed. When the reaction completed, the reaction liquid was concentrated under reduced pressure to dryness. Dichloromethane (200 mL) and water (100 mL) were added into residue obtained, and the mixture obtained was placed in an ice bath, cooling to below 10 °C. 1 N sodium hydroxide aqueous solution was added to adjust the pH to 7-8, and then the mixture was stirred to obtain separated phases. Aqueous phase obtained was extracted with dichloromethane, and organic phases then obtained were pooled and washed with saturated saline water, then dried over anhydrous sodium sulfate, suctioned, and distilled under reduced pressure to remove the organic solvent and obtain 25 g of diaminoethyl ester intermediate crude product, which was directly used in the next reaction.

The diaminoethyl ester intermediate was dissolved in dichloromethane (200 mL), into which triethylamine (20.2 g, 0.2 mol, 2 eq) was added. The mixture was placed in an ice bath, cooling to below 10 °C, and acetic anhydride (25.5 g, 0.25 mol, 2.5 eq) was added dropwise. After that, the temperature was maintained for reaction for 1 hour. Reaction liquid was sampled and detected to see whether the reaction completed. When the reaction completed, the reaction liquid was poured into 1 N ice-cold hydrochloric acid, and stirred for 15 min. Separated phases were obtained, and aqueous phase obtained was extracted with dichloromethane (50 mL × 3); and organic phases then obtained were pooled and dried over anhydrous sodium sulfate, suctioned, and distilled under reduced pressure to remove the organic solvent and obtain a crude product. The crude product was slurried with 400 mL of MTBE, then filtered, dried, and 26.9 g of the intermediate A1-e was obtained as light yellow solid. The total yield of the two steps was found to be 87.8%. LC-MS (ESI): m/z 307 $(M+H)^+$.

Compound A1 was then prepared from intermediate A1-e according to Method 2.

## Example 5 Synthesis of Compound A2

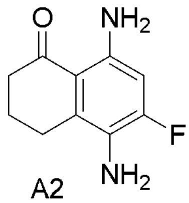

A2

Compound A2 was synthesized according to the method described in the patent publication WO2021148501, and synthetic route is shown as follows:

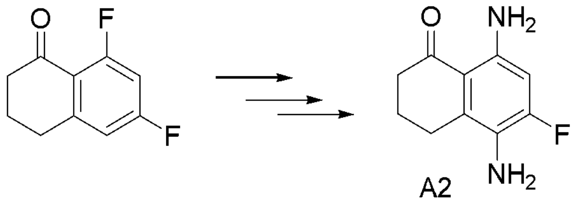

A2

**Example** 6 Synthesis of Compound A3

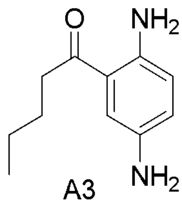

A3

Compound A3 was synthesized according to the method described in the patent publication US2004266803A.

**Example 7 Synthesis of Compound** A4

Compound A4 was synthesized according to a method similar to that described in Journal of Medicinal chemistry, 1998, 41(13), 2308-2318, and synthetic route is shown as follows:

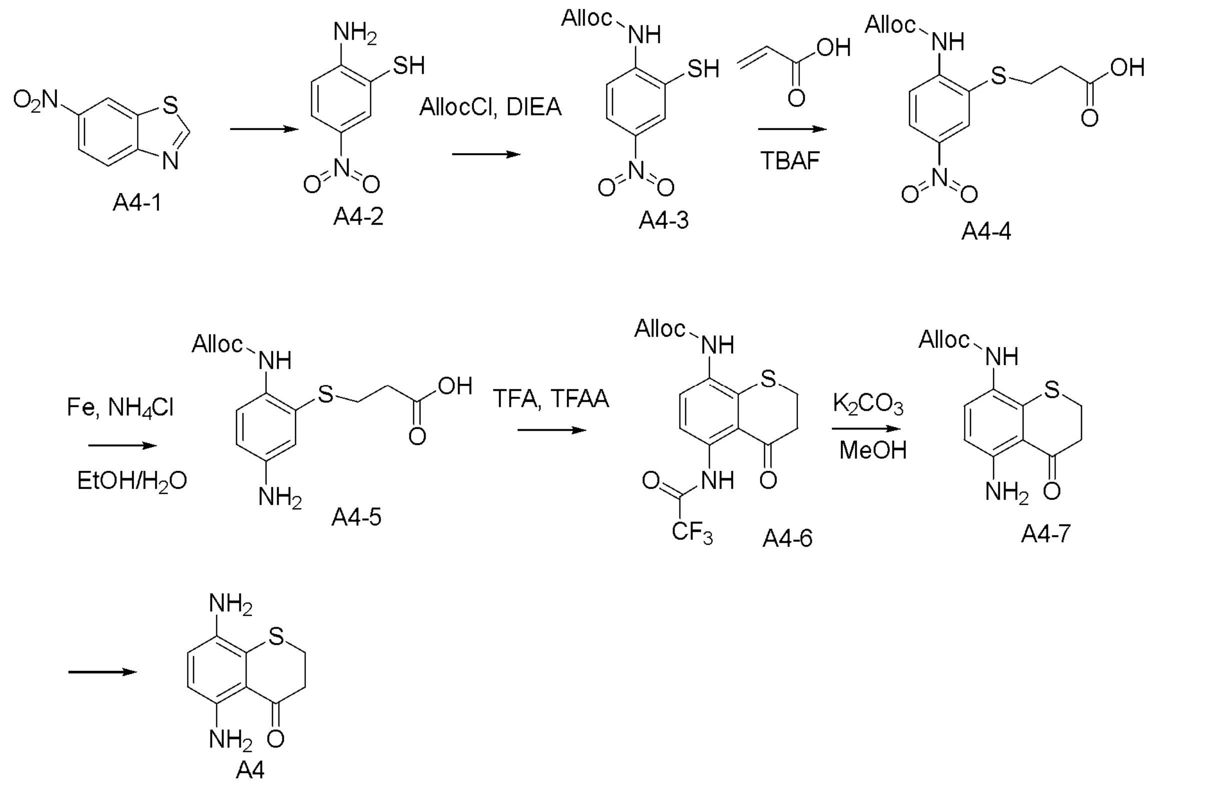


Step 1:

The intermediate A4-2 was prepared from the compound A4-1 according to the method described in the literature.

Step 2:

The intermediate A4-2 (3.4 g) was dissolved in dichloromethane at -5-5 °C, into which triethylamine (3.0 g) was added and AllocCl (2.8 g) was then added dropwise slowly. When the addition was finished, mixture obtained was stirred and reacted for 1-2 hours, and then the reaction was quenched by adding water. Reaction product obtained was washed with water once, with saturated saline once, dried over anhydrous sodium sulfate, concentrated to dryness, and 5.5 g of the intermediate A4-3 crude product was obtained. LC-MS (ESI): [M+1]+ =255.7.

Step 3:

The intermediate A4-3 (5.5 g) was added into TBAF (55 ml) and acrylic acid (110 ml) at normal temperature, and mixture obtained was heated to 50-55 °C, and stirred and reacted for 24 hours. Reaction liquid was directly concentrated to dryness, and purified by column chromatography using petroleum ether to a methanol/dichloromethane (1:50) mixture as eluents, and about 5.2 g of the intermediate A4-4 crude product was obtained. LC-MS (ESI): [M+1]+ = 327.4.

Step 4:

The intermediate A4-4 (5.0 g) was added into 100 ml of a mixed solution of ethanol/water (3: 1) at normal temperature, into which ammonium chloride (5.5 g) and iron powder (5.5 g) were added, and mixture obtained was heated under reflux and reacted for 1-2 hours. Reaction liquid was cooled to room temperature, filtered, and the solid obtained was washed with ethanol and concentrated to dryness, and about 3.8 g of the intermediate A4-5 crude product was obtained. LC-MS (ESI): [M+1]+ = 297.4.

Step 5:

The intermediate A4-5 (3.8 g) was added into trifluoroacetic acid (38 ml) at 20-30 °C, into which trifluoroacetic anhydride (38 ml) was added. Mixture obtained was stirred and reacted for 18-24 hours, and then the reaction product

obtained was subjected to the following post-treatment: it was directly concentrated to dryness, and purified by column chromatography using a petroleum ether/ethyl acetate (1:0-4:1) mixture as an eluent, and 1.0 g of the intermediate A4-6 was obtained. LC-MS (ESI): [M+1]+ = 375.3.

Step 6:

The intermediate A4-6 (1.0 g) was added into methanol (20 ml) at normal temperature, and then potassium carbonate (2.0 g) and water (5 ml) were added. Mixture obtained was stirred and reacted for 1-2 hours. Water was added to dilute reaction liquid, which then was extracted with ethyl acetate. Organic phase obtained was washed with saturated saline, dried over anhydrous sodium sulfate, and concentrated to dryness, and a crude product was obtained. The crude product was purified by column chromatography using a petroleum ether/ethyl acetate (1:0-2:1) mixture as an eluent, and 0.65 g of the intermediate A4-7 was obtained. LC-MS (ESI): [M+1]+ = 279.3.

Step 7:

The intermediate A4-7 (500 mg) was dissolved in tetrahydrofuran (20 ml) at normal temperature, into which pyrrole (120 mg) and Tetrakis(triphenylphosphine)palladium (160 mg) were added under the protection of nitrogen. When the addition was finished, mixture obtained was stirred and reacted for 1-2 hours. Reaction liquid was directly concentrated to dryness, and purified by column chromatography using a dichloromethane/methanol (30:1) mixture as an eluent, and 30 mg of Compound A4 was obtained as brown solid. LC-MS (ESI): [M+1]+ = 195.4.

**Example 8 Synthesis of Compound A5**

Compound A5 was synthesized according to a method similar to that described in Journal of Medicinal chemistry, 1998, 41(13), 2308-2318, and synthetic route is shown as follows:

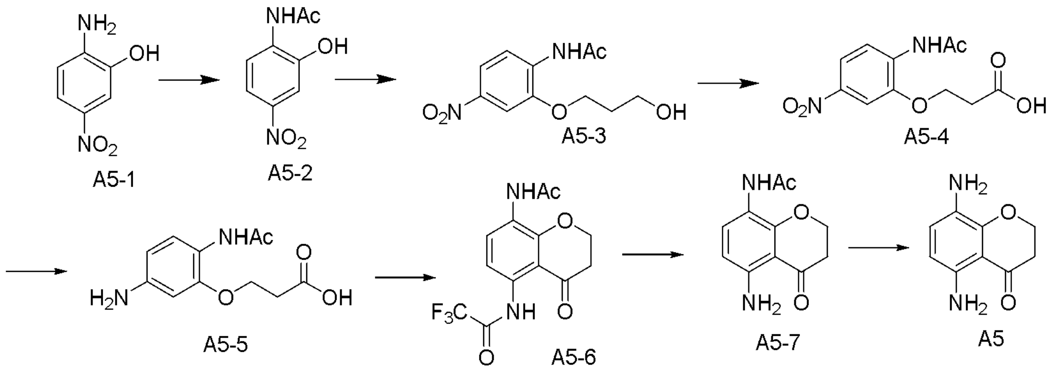

Step 1:

Compound A5-1 (25 g) was added into acetic acid (100 ml) at 20-30 °C, into which acetic anhydride (24.9 g) was added slowly. When the addition was finished, mixture obtained was stirred for 3-4 hours. Then reaction liquid was slowly placed into ice water, and stirred. Solid precipitated, and was filtered and collected, which was then washed with water, and dried in vacuum, and 31.0 g of the intermediate A5-2 was obtained as yellow solid. LC-MS (ESI): [M+1]+ = 197.2.

Step 2:

The intermediate A5-2 (29 g), potassium carbonate (40 g), potassium iodide (5 g) and bromopropanol (25 g) were mixed in DMF (300 ml) at 20-30 °C, and mixture obtained was heated to 100-110 °C and stirred for 3-4 hours. Then reaction liquid was cooled to room temperature, and placed in ice water for quenching, and then was extracted with 2 L of ethyl acetate for three times. Organic phases obtained were pooled, washed with saturated saline, dried over anhydrous sodium sulfate, and concentrated to dryness, and then was slurried with petroleum ether to obtain a solid. The solid was dried in vacuum, and 33.0 g of the intermediate A5-3 was obtained as yellow solid. LC-MS (ESI): [M+1]+ = 255.3.

Step 3:

The intermediate A5-3 (23 g) was dissolved in acetonitrile (1 L) at 20-25 °C, into which a solution of sodium

dihydrogen phosphate (0.67 mol, pH 6.7, 900 ml), TEMPO (5 g), a solution of sodium chlorite (52.0 g dissolved in 60 ml of water), and a solution of sodium hypochlorite (38 ml mixed with 38 ml of water) were added sequentially. When the addition was finished, reaction liquid obtained appeared dark brown, and was stirred for 30 minutes. Next, the reaction liquid was cooled to room temperature, adjusted to pH 2-3 with the addition of 2 N hydrochloric acid, and was extracted with ethyl acetate (1000 ml × 3). Ethyl acetate phases obtained were pooled, washed with saturated saline, dried over anhydrous sodium sulfate, filtered and concentrated to dryness, and then was slurried with petroleum ether, and 24.0 g of the intermediate A5-4 crude product was obtained. LC-MS (ESI): [M+1]+ = 269.2.

Step 4:

The intermediate A5-4 (25 g) and 5% palladium-on-charcoal (5 g) were mixed in methanol (500 ml) at normal temperature, and pressurized hydrogenation was carried out for 3-4 hours. The palladium-on-charcoal was filtered out, and solid obtained was washed with methanol; and the filtrate was concentrated and residue obtained was slurried with petroleum ether to obtain a crude product. The crude product was dried in vacuum, and 18.0 g of the intermediate A5-5 as yellow solid was obtained. LC-MS (ESI): [M+1]+ = 239.5.

Next, starting from the intermediate A5-5, the intermediate A5-7 was synthesized according to procedures similar to those described in step 5 and step 6 in the method for synthesizing Compound A4, and was then deprotected to remove acetyl group with 6 N hydrochloric acid, and Compound A5 was obtained as yellow solid. LC-MS (ESI): [M+1]+=179.2.

**Example 9 Synthesis of Compound A6**

Compound A6 was synthesized according to a method described in Journal of Medicinal chemistry, 1998, 41(13), 2308-2318, and synthetic route is shown as follows:

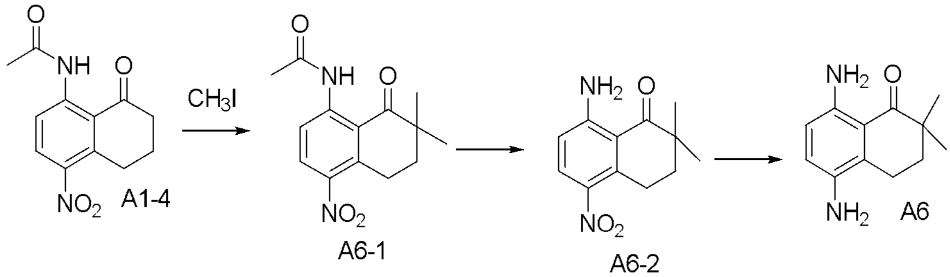


Compound A6 is yellow solid. LC-MS (ESI): [M+1]+ = 248.9.

$^1$H NMR (400MHz, d6-DMSO): δ 12.289 (1H, s), 8.666-8.648 (1H, d), 8.198-8.1 (1H, d), 3.138-3.108 (2H, t), 2.768-2.734 (2H, t), 2.219 (3H, s), 2.050-1.991 (2H, m)

Step 1:

The intermediate A1-4 (1.25 g, 5 mmol, 1 eq) was dissolved in 150 ml of tetrahydrofuran under the protection of nitrogen in a 250 mL three-neck reaction flask. The reaction system was cooled to -60 °C in a dry ice bath, and then LDA (2M in THF, 7.6 ml, 15.2 mmol, 3.04 eq) was added. When the addition was finished, mixture obtained was stirred at -60 °C for 30 minutes. MeI (1.45 g, 10.21 mmol, 2.04 eq) was added. When the addition was finished, the dry ice bath was removed and the reaction was allowed to warm naturally to room temperature and continued overnight. Next day, the reaction was quenched by the addition of 50 ml of aqueous ammonium chloride solution, and extracted with ethyl acetate (150 ml × 3). Organic phases obtained were pooled, washed with water once, and concentrated to dryness to obtain a crude product. The crude product was purified by column chromatography using a petroleum ether/ethyl acetate (1:0-5:1) mixture as an eluent, and 350 mg of Compound A6-1 was obtained as yellow solid with a yield of 28%.

$^1$H NMR($CDCl_3$): δ 12.49 (1H, s), 8.77 (1H, d), 8.03 (1H, d), 3.21 (2H, m), 2.22 (3H, s), 1.90 (2H, t), 1.20 (6H, s)

Step 2:

Compound A6-1 (280 mg) was mixed with 20 ml of 6 N hydrochloric acid in a 100 ml single-neck flask, and mixture obtained was then heated under reflux and reacted for 2 hours. The heating was stopped, and reaction liquid was allowed to cool to room temperature. Then the reaction liquid was adjusted to pH 7-8 with the addition of sodium bicarbonate, and extracted with dichloromethane (30 ml × 3). Organic phases obtained were pooled, and concentrated to dryness, and 275

mg of Compound A6-2 was obtained as brown gray solid. The crude product was used directly in the next reaction without purification.

Step 3:

Compound A6-2 (220 mg) was dissolved in acetic acid (25 ml) in a 50 ml three-neck flask, into which 1.3 g of iron powder was then added. After the addition was finished, mixture obtained was stirred for 1-2 hours at 80-85 °C, and when the reaction completed, reaction liquid was distilled under reduced pressure to remove acetic acid, and water (30 ml) was added into residue obtained. Mixture obtained was adjusted to pH 7-8 with the addition of sodium bicarbonate, and extracted with dichloromethane (30 ml × 3). Organic phases obtained were pooled, and concentrated to dryness, to obtain a crude product. The crude product was purified by column chromatography using a petroleum ether/ethyl acetate (1:0-2:1) mixture as an eluent, and 122 mg of Compound A6 was obtained as brown solid with a yield of 75%. LC-MS (ESI): [M+1]+ = 205.0.

**Example 10 Synthesis of Compound A7**

Synthetic route is shown as follows:

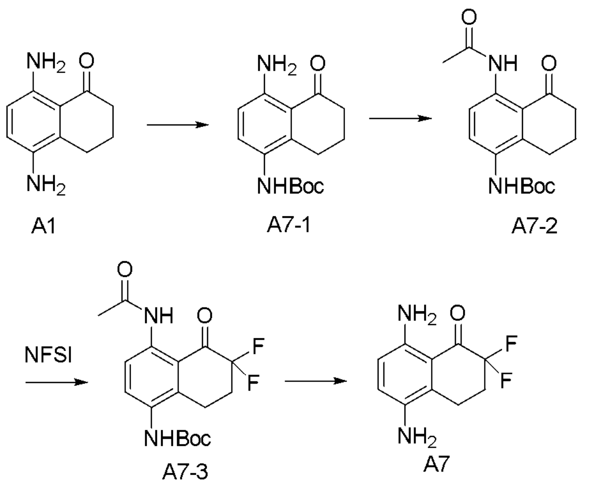


A1 A7-1 A7-2 A7-3 A7

Compound A1 (2.18 g, 124 mmol, 1 eq) was dissolved in tetrahydrofuran (100 ml) in a 250ml three-neck flask, into which Boc anhydride (8.2 g, 376mmol, 3 eq) was then added. Mixture obtained was stirred at 40-45 °C for 5 hours, and then reaction liquid was directly concentrated to dryness, and residue obtained was purified by column chromatography using a petroleum ether/ethyl acetate (1:0-1:3) mixture as an eluent, and 3.0 g of Compound A7-1 was obtained as yellow solid with a yield of 90%.

1H NMR (400 MHz, CDCl3) δ 7.25 (s, 1H), 6.41 (t, J = 10.2 Hz, 3H), 5.90 (s, 1H), 2.73 (t, J = 6.2 Hz, 2H), 2.61 - 2.47 (m, 2H), 2.02 - 1.86 (m, 3H), 1.49 - 1.36 (m, 9H)

Compound A7-1 (3.0 g, 10.8 mmol, 1 eq) and DIPEA (3.5 g, 27.1 mmol, 1.5 eq) were mixed in dichloromethane (125 ml) in a 250 ml three-neck flask. Reaction liquid was cooled in an ice-salt bath to -5-0 °C, and acetyl chloride (1.3 g, 16.5mmol, 2 eq) was added dropwise. When the addition was finished, the ice-salt bath was removed, and the reaction liquid was allowed to warm naturally and then was stirred at room temperature and reacted for 4 hours. Then the reaction liquid was concentrated to dryness, and residue obtained was purified by column chromatography using a petroleum ether/ethyl acetate (1:0-1:3) mixture as an eluent, and 3.48 g of Compound A7-2 was obtained as light yellow solid with a yield of 100%.

1H NMR (400 MHz, CDCl3) δ 12.01 (s, 1H), 8.55 (d, J = 9.1 Hz, 1H), 7.57 (t, J = 36.2 Hz, 1H), 6.09 (s, 1H), 2.80 (t, J = 6.2 Hz, 2H), 2.65 - 2.58 (m, 2H), 2.15 (s, 3H), 2.07 - 1.98 (m, 2H), 1.44 (s, 9H)

Compound A7-2 (2.45 g, 7.7 mmol, 1 eq) was dissolved in tetrahydrofuran (200 ml) in a 500 ml three-neck flask under the protection of nitrogen. Reaction liquid was cooled in a dry ice-acetone bath to -70 °C - -60 °C, and KHMDS (1 M, 31 ml, 31.0 mmol, 4 eq) was added dropwise slowly. When the addition was finished, the reaction liquid was stirred at -70 °C - -60 °C for 10 minutes. Next, a solution of NFSI in tetrahydrofuran (NFSI (7.35 g, 23 mmol, 3 eq) was dissolved in tetrahydrofuran (70 ml)) was added dropwise, and when the addition was finished, mixture obtained was stirred at -70 °C - -60 °C for 10 minutes. Then reaction liquid was allowed to warm naturally to 20-30 °C and stirred for 3 hours. Saturated

ammonium chloride aqueous solution (80 ml) was added to quench the reaction and reaction liquid was extracted with ethyl acetate (150 ml × 3). Organic phases obtained were pooled, washed with saturated saline, and concentrated to dryness to obtain a crude product. The crude product obtained was purified by column chromatography using a petroleum ether/ethyl acetate (1:0-1:3) mixture as an eluent, and 0.85 g of Compound A7-3 was obtained as yellow solid with a yield of 31.2%. LC-MS (ESI): [M+1]+ = 355.8

1H NMR (400 MHz, CDCl3) δ 11.49 (s, 1H), 8.64 (d, J = 9.2 Hz, 1H), 7.74 (d, J = 8.5 Hz, 1H), 6.06 (s, 1H), 3.01 (t, J = 6.4 Hz, 2H), 2.55 - 2.39 (m, 2H), 2.19 (s, 3H), 1.44 (s, 9H)

Compound A7-3 (0.85 g, 2.4 mmol) was mixed with 6 N hydrochloric acid (30 ml) in a 100 ml single-neck flask, and mixture obtained was heated under reflux for 4 hours. Then the heating was stopped, and when reaction liquid was allowed to cool to room temperature, it was adjusted to pH 7-8 with sodium bicarbonate, and extracted with dichloromethane (30 ml × 3). Organic phases obtained were pooled, washed with saturated saline, and concentrated to dryness, to obtain a crude product. The crude product obtained was purified by column chromatography using a petroleum ether/ethyl acetate (1:0-1:3) mixture as an eluent, and 0.25 g of Compound A7 was obtained as brown solid with a yield of 49.1%. LC-MS (ESI): [M+1]+ = 213.9.

**Example 11 Synthesis of Compound A8**

Synthetic route is shown as follows:

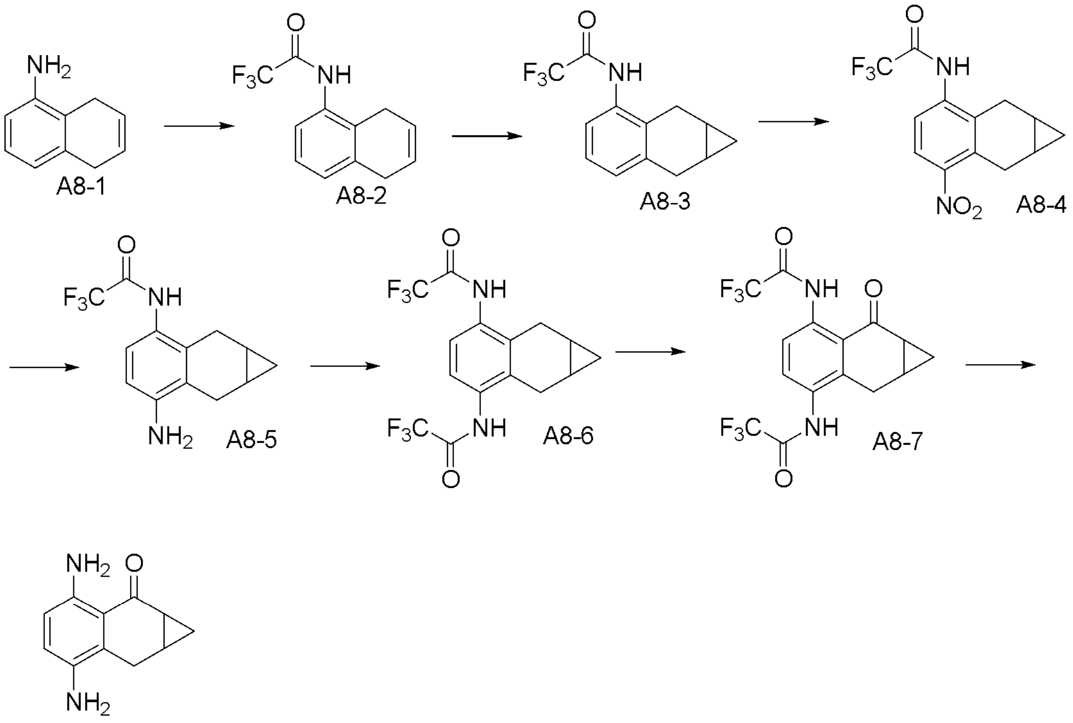
A8-1
A8-2
A8-3
A8-4
A8-5
A8-6
A8-7
A8

Compound A8-1 was synthesized according to the method described in Journal of Medicinal Chemistry, 1989, vol. 32, # 6, p.1217-1230. LC-MS: (ESI): [M+1]+ = 146.3.

Compound A8-1 (1.8 g) and DIEA (4.8 g) were added into DCM (50 ml), and cooled to 0 °C. Trifluoroacetic anhydride (4.8 g) was added thereto, and mixture obtained was warmed to room temperature and reacted for 1 hour. Water (20 ml) was added to separate phases, and organic phase was spun to dryness. Residue obtained was purified by column chromatography (EA: PE = 0-30%), and 2.7 g of the intermediate A8-2 was obtained as white solid. LC-MS: (ESI): [M+1]+ = 242.2.

$Zn(Et)_2$ (33.6 mL) was added into DCM (70 mL) and cooled to 0 °C. TFA (3.8 g) and $CH_2I_2$ (9.0 g) were added thereto, and mixture obtained was reacted for 15 minutes. A solution of Compound A8-2 (2.7 g) in DCM (30 ml) was added dropwise. When the addition was finished, mixture obtained was stirred at room temperature overnight. Water (30 ml) was added to separate phases, and organic phase was spun to dryness, and 2.7 g of the intermediate A8-3 was obtained as off white solid. LC-MS (ESI): [M+1]+ = 256.1.

The intermediate A8-3 (1.5 g) was added into DCM (100 ml) and acetic anhydride (30 ml), and cooled to 0 °C. 65% nitric acid (2.8 g) was added thereto, and mixture obtained was reacted overnight at room temperature. Water (50 ml) and DCM (100 ml) were added to separate phases, and organic phase was spun in an oil pump to dryness, and 1.6 g of the intermediate A8-4 crude product was obtained. The crude product was directly used in the next step. LC-MS (ESI): [M+1]+ = 301.2.

The intermediate A8-4 (1.6 g crude), iron powder (3.2 g), and ammonium chloride (6.4 g) were added to anhydrous ethanol (100 ml), and mixture obtained was warmed under reflux overnight. Reaction liquid was cooled, filtered, spun-dried, and residue obtained was purified by column chromatography (EA: PE = 0-50%), and 0.25 g of the intermediate A8-5 was obtained as brown oil. LC-MS (ESI): [M+1]+ = 271.3.

The intermediate A8-5 (250 mg) and DIEA (400 mg) were added into DCM (10 ml), and cooled to 0 °C. Trifluoroacetic anhydride (320 mg) was added thereto, and mixture obtained was reacted at room temperature for 1 hour. Reaction liquid was washed with water (5 ml), and organic phase was obtained, dried, filtered, and spun to dryness, and 340 mg of the intermediate A8-6 was obtained as yellow solid. LC-MS (ESI): [M+1]+ = 367.3.

The intermediate A8-6 (150 mg) and $CrO_3$ (400 mg) were added to acetic acid (8 ml) and acetic anhydride (4 ml), and mixture obtained was reacted at 30 to 40 °C for 3 hours. Reaction liquid was spun to dry the solvents, and then water (5 ml) was added and extracted with DCM (20 ml). Organic phase was obtained, dried, filtered, and spun to dryness, and 160 mg of the intermediate A8-7 crude product was obtained. The crude product was directly used in the next step. LC-MS (ESI): [M+1]+ = 381.3.

The intermediate A8-7 was deprotected with 6 N hydrochloric acid to remove the two trifluoroacetyl groups and then Compound A8 was obtained upon being subjected to purification by column chromatography. LC-MS (ESI): [M+1]+ = 189.3.

Following compounds can be synthesized according to methods similar to those described above.

| | Structure | Mass spectrum | Synthetic Method |
|---|---|---|---|
| A9 | 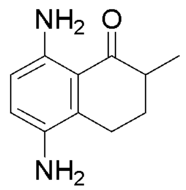 | $[M+1]^+$=191.2 | Synthesized according to a method similar to the method for synthesizing Compound A6, in which 1.5 eq of LDA and 1.2 eq of MeI were used. |
| A10 | 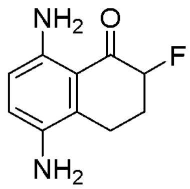 | $[M+1]^+$=195.2 | Synthesized according to a method similar to the method for synthesizing Compound A7, in which 1.5 eq of LDA and 1.2 eq of NFSI were used. |
| A11 | 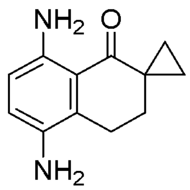 | $[M+1]^+$=203.2 | Synthesized according to a method similar to the method for synthesizing Compound A6, in which 1.2 eq of 1,2-diiodoethane was used, instead of MeI. |
| A12 | 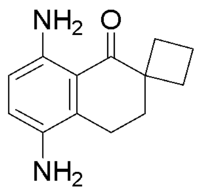 | $[M+1]^+$=217.2 | Synthesized according to a method similar to the method for synthesizing Compound A6, in which 1.2 eq of 1,3-dibromopropane, instead of MeI. |
| A13 | 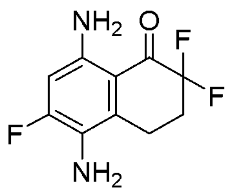 | $[M+1]^+$=231.2 | Synthesized according to a method similar to the method for synthesizing Compound A7, in which Compound A2 was used, instead of Compound A1. |

(continued)

| | Structure | Mass spectrum | Synthetic Method |
|---|---|---|---|
| A14 | 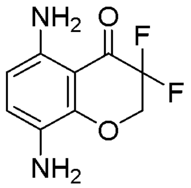 | $[M+1]^+$=215.2 | Synthesized according to a method similar to the method for synthesizing Compound A7, in which Compound A5 was used, instead of Compound A1. |
| A15 | 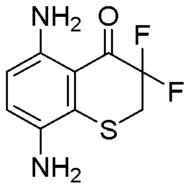 | $[M+1]^+$=231.2 | Synthesized according to a method similar to the method for synthesizing Compound A7, in which Compound A4 was used, instead of Compound A1. |
| A16 | 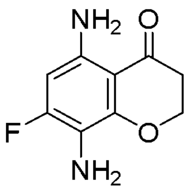 | $[M+1]^+$=197.2 | Synthesized according to a method similar to the method for synthesizing Compound A5, in which 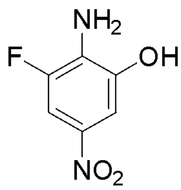 was used. |
| A17 | 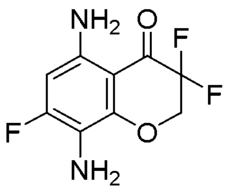 | $[M+1]^+$=233.2 | Synthesized according to a method similar to the method for synthesizing Compound A7, in which Compound A16 was used, instead of Compound A1. |
| A18 | 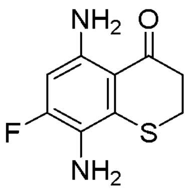 | $[M+1]^+$=213.2 | Synthesized according to a method similar to the method for synthesizing Compound A6, in 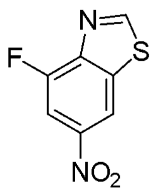 which was used. |
| A19 | 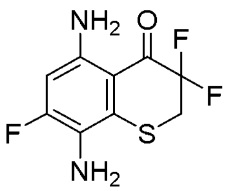 | $[M+1]^+$=249.3 | Synthesized according to a method similar to the method for synthesizing Compound A7, in which Compound A18 was used, instead of Compound A1. |

(continued)

| | Structure | Mass spectrum | Synthetic Method |
|---|---|---|---|
| A20 | 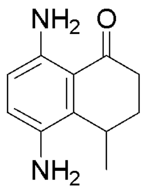 | $[M+1]^{+}$=191.2 | Synthesized according to Method 3 for synthesizing Compound A1, in which 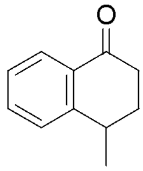 was used. |
| A21 | 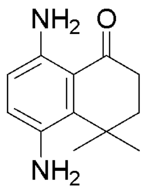 | $[M+1]^{+}$ =205.3 | Synthesized according to Method 3 for synthesizing Compound A1, in which 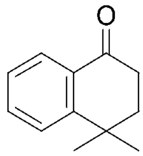 was used. |
| A22 | 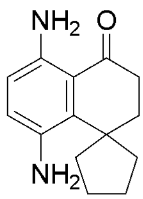 | $[M+1]^{+}$ =231.4 | Synthesized according to Method 3 for synthesizing Compound A1, in which 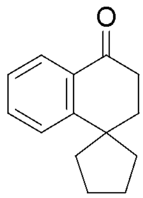 was used. |
| A23 | 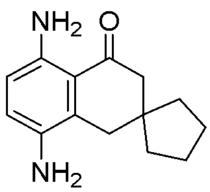 | $[M+1]^{+}$ =231.3 | Synthesized according to Method 3 for synthesizing Compound A1, in which 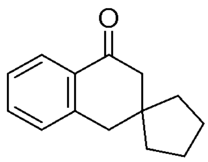 was used. |
| | | | |
| A24 | 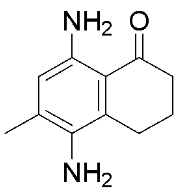 | $[M+1]^{+}$ =191.2 | Synthesized according to Method 2 for synthesizing Compound A1, in which 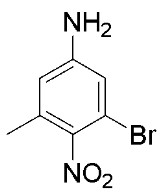 was used. |

(continued)

| | Structure | Mass spectrum | Synthetic Method |
|---|---|---|---|
| A25 | 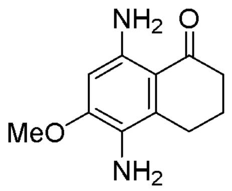 | $[M+1]^+$ =207.3 | Synthesized according to Method 2 for synthesizing Compound A1, in which 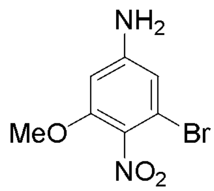 was used. |
| A26 | 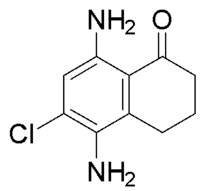 | $[M+1]^+$ =211.6 | Synthesized according to a method similar to the method for synthesizing Compound A2, in 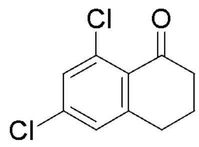 which was used. |
| A27 | 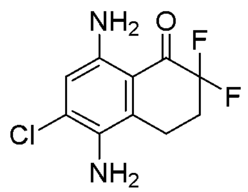 | $[M+1]^+$ =247.6 | Synthesized according to a method similar to the method for synthesizing Compound A7, in which Compound A26 was used, instead of Compound A1. |
| A28 | 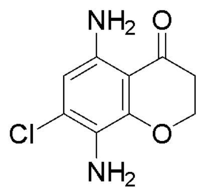 | $[M+1]^+$ =213.6 | Synthesized according to a method similar to the method for synthesizing Compound A5, in which 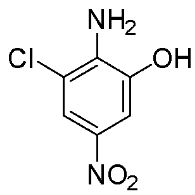 was used. |
| A29 | 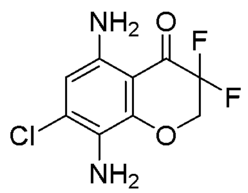 | $[M+1]^+$ =249.6 | Synthesized according to a method similar to the method for synthesizing Compound A7, in which Compound A28 was used, instead of Compound A1. |
| A30 | 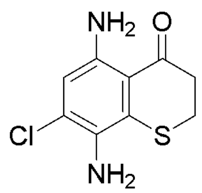 | $[M+1]^+$ =229.7 | Synthesized according to a method similar to the method for synthesizing Compound A6, in 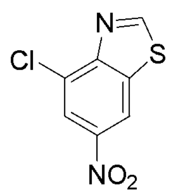 which was used. |

(continued)

| | Structure | Mass spectrum | Synthetic Method |
|---|---|---|---|
| A31 | 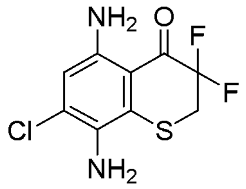 | $[M+1]^+$ =265.7 | Synthesized according to a method similar to the method for synthesizing Compound A7, in which Compound A30 was used, instead of Compound A1. |
| A32 | 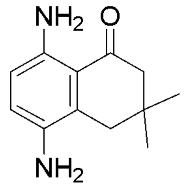 | $[M+1]^+$ =205.3 | Synthesized according to Method 3 for synthesizing Compound A1, in which 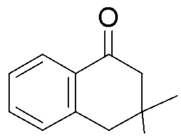 was used. |

**2.** Synthesis of camptothecins (Friedlander reaction)

Control compound of camptothecins MWC-1 was synthesized as follows:

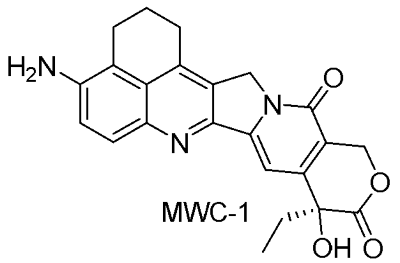

MWC-1

Compound A1 (176 mg, 1 eq), cyclic compound CDE (315 mg, 1.2 eq), and PPTS (301 mg, 1.2 eq) were mixed in toluene (50 ml) at normal temperature, and mixture obtained was reacted under reflux and water separation for 3-4 hours. Then reaction liquid was directly concentrated to dryness, and residue obtained was purified by column chromatography using petroleum ether to a methanol/dichloromethane (1:20) mixture as eluents, and 260 mg of compound MWC-1 was obtained as yellow solid. LC-MS (ESI): [M+1]+ = 404.

**Example** 12 Synthesis of Camptothecin Compound 1

Synthetic route is shown as follows:

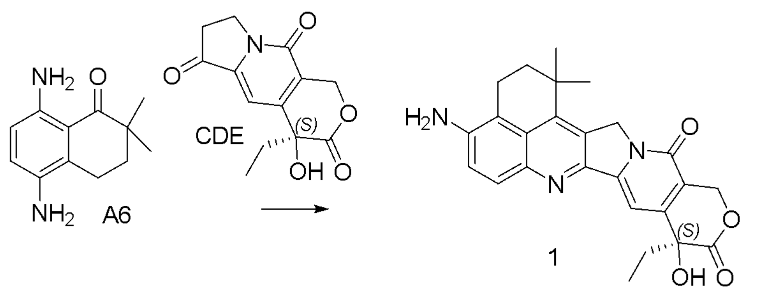

A6 CDE 1

Compound A6 (120 mg, 0.59 mmol, 1 eq), tricyclic compound CDE (180 mg, 0.68 mmol, 1.2 eq), and PPTS (35 mg, 0.14 mmol, 0.25 eq) were mixed in 15 ml in a 50 ml single-neck reaction flask under the protection of nitrogen, and then heated under reflux and reacted for 3-4 hours. Reaction liquid was distilled under reduced pressure to distill off a majority of acetic acid, and residue obtained was purified by column chromatography using a dichloromethane/methanol (15:1) mixture as an eluent, and 80 mg of a crude product was obtained. The crude product was further separated and purified by

preparative liquid phase chromatography, and 3 mg of Camptothecin Compound 1 was obtained as yellow solid.

LC-MS (ESI): [M+1]+ =432.4.

**Example 13 Synthesis of Camptothecin Compound 2 (2A and 2B)**

Synthetic route is shown as follows:

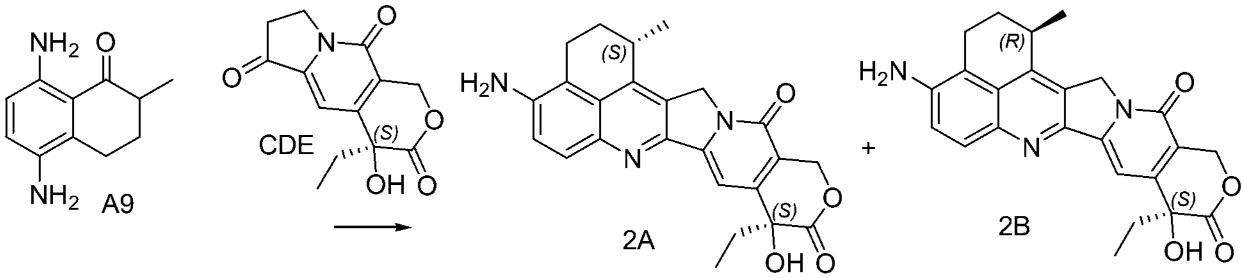

A9 CDE 2A + 2B

According to a method similar to that described in Example 12, in which Compound A9 was used instead of Compound A6, Camptothecin Compound 2A (having a short retention time) and Camptothecin Compound 2B (having a long retention time) were obtained upon separation and purification by preparative liquid phase chromatography. Camptothecin Compound 2A was obtained as yellow solid. LC-MS (ESI): [M+1]+ =418.3. Camptothecin Compound 2B was obtained as yellow solid. LC-MS (ESI): [M+1]+ =418.4.

**Example 14 Synthesis of Camptothecin Compound 3**

Synthetic route is shown as follows:

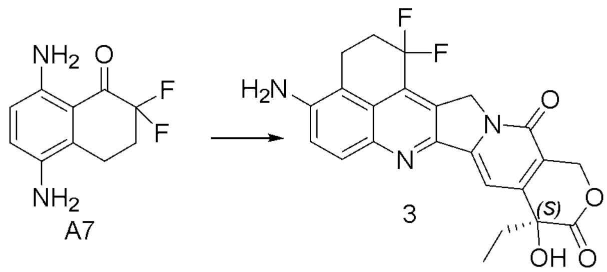

A7 3

According to a method similar to that described in Example 12 in which Compound A7 was used instead of Compound A6 and toluene was used as the solvent instead of acetic acid, Camptothecin Compound 3 was prepared and obtained as earthy yellow solid. LC-MS (ESI): [M+1]+ =440.8.

1H NMR (400 MHz, Acetone) δ 7.89 (d, J = 9.1 Hz, 1H), 7.48 (d, J = 9.1 Hz, 1H), 7.37 (s, 1H), 7.31 (d, J = 8.9 Hz, 0H), 5.59 - 5.26 (m, 5H), 3.11 - 3.05 (m, 4H), 2.73 - 2.59 (m, 3H), 1.98 (dt, J = 14.0, 7.0 Hz, 1H), 1.05 - 0.98 (m, 3H)
1H NMR (400 MHz, DMSO) δ 7.87 (d, J = 9.1 Hz, 0H), 7.41 (d, J = 9.1 Hz, 0H), 7.21 (s, 0H), 6.50 (s, 0H), 6.15 (s, 1H), 5.36 (d, J = 46.6 Hz, 1H), 2.93 (t, J = 6.5 Hz, 1H), 2.66 - 2.54 (m, 1H), 1.97 - 1.68 (m, 3H), 1.24 (s, 1H), 0.88 (t, J = 7.3 Hz, 3H)

**Example 15 Synthesis of Camptothecin Compound 4 (4A and 4B)**

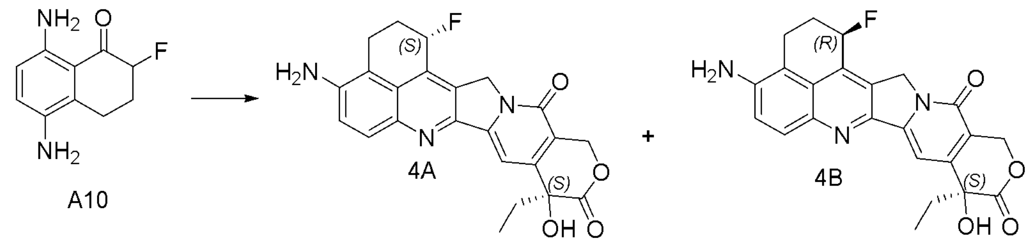

A10 4A + 4B

According to a method similar to that described in Example 12, in which Compound A10 was used instead of Compound A6, Camptothecin Compound 4A (having a short retention time) and Camptothecin Compound 4B (having a long retention time) were obtained upon separation and purification by preparative liquid phase chromatography. Compound 4A was obtained as earthy yellow solid. LC-MS (ESI): [M+1]+ =422.3. Camptothecin Compound 4B was obtained as earthy yellow solid. LC-MS (ESI): [M+1]+=422.3.

**Example 16 Synthesis of Camptothecin Compound 5**

Synthetic route is shown as follows:

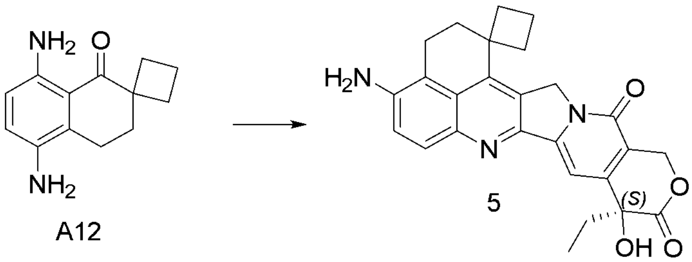

A12 5

According to a method similar to that described in Example 12, in which Compound A12 was used instead of Compound A6, Camptothecin Compound 5 was prepared and obtained as yellow solid. LC-MS (ESI): [M+1]+=444.3.

**Example 17 Synthesis of Camptothecin Compound** 6

Synthetic route is shown as follows:

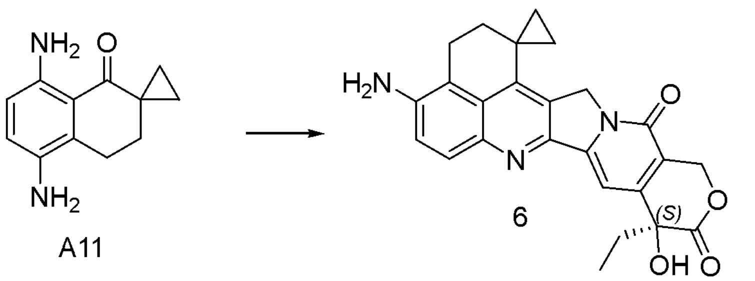

A11 6

According to a method similar to that described in Example 12, in which Compound A11 was used instead of Compound A6, Camptothecin Compound 6 was prepared and obtained as yellow solid. LC-MS (ESI): [M+1]+ =430.5.

**Example 18 Synthesis of Camptothecin Compound 7**

Synthetic route is shown as follows:

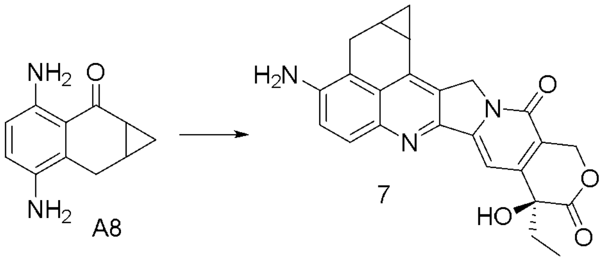

A8 7

According to a method similar to that described in Example 12, in which Compound A8 was used instead of Compound A6, 0.7 mg of Camptothecin Compound 7 was prepared and obtained as yellow solid. LC-MS (ESI): [M+1]+ =416.3.

**<u>Example</u> 19 Synthesis of Camptothecin Compound 9**

Synthetic route is shown as follows:

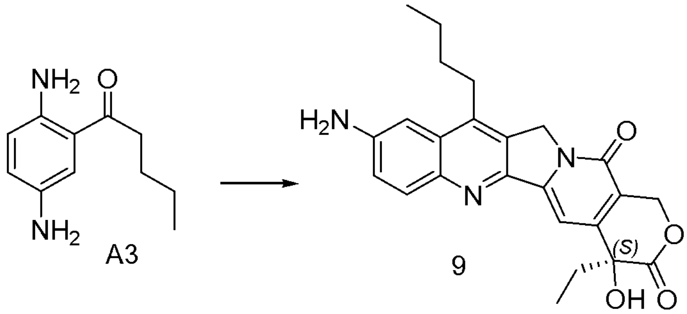

A3 9

According to a method similar to that described in Example 12, in which Compound A3 was used instead of Compound A6, Camptothecin Compound 9 was prepared and obtained as brownish red solid. LC-MS (ESI): [M+1]+ =420.3.

**<u>Example</u> 20 Synthesis of Camptothecin Compound 12**

Compound 12 was synthesized according to a method similar to that described in Journal of Medicinal chemistry, 1998, 41(13), 2308-2318, and synthetic route is shown as follows:

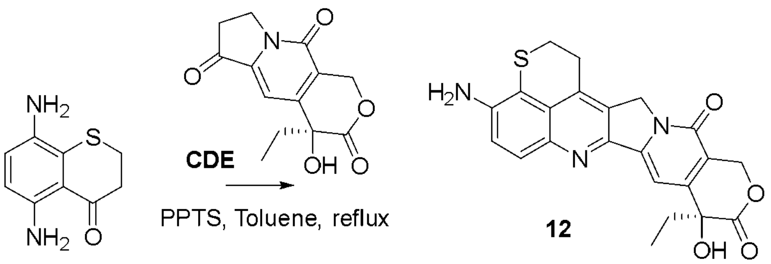


**12**

**A4**

According to a method similar to that described in Example 12, in which Compound A4 was used instead of Compound A6, and toluene was used as the solvent instead of acetic acid, 30 mg of Camptothecin Compound 12 was synthesized and obtained as brown solid. LC-MS (ESI): [M+1]+ =422.5.

**<u>Example</u> 21 Synthesis of Camptothecin Compound 13**

Synthetic route is shown as follows:

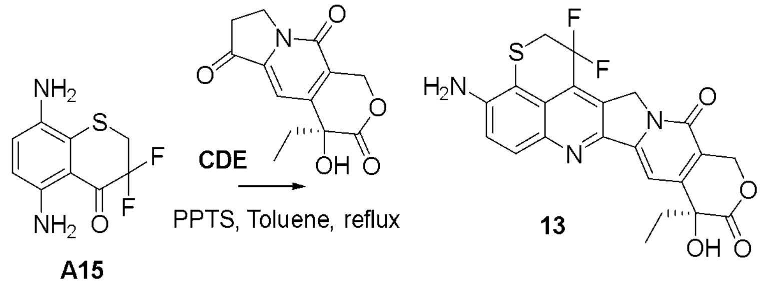


A15

13

According to a method similar to that described in Example 12, in which Compound A15 was used instead of Compound A6 and toluene was used as the solvent, Compound 13 was synthesized and obtained as brown solid. LC-MS (ESI): [M+1]+=458.4.

## Example 22 Synthesis of Camptothecin Compound 14

Synthetic route is shown as follows:

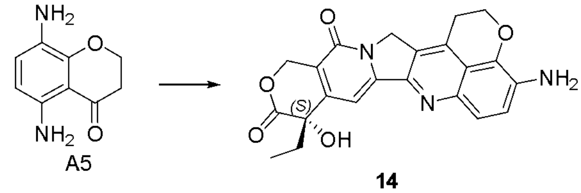

A5

**14**

According to a method similar to that described in Example 12, in which Compound A5 was used instead of Compound A6 and toluene was used as the solvent, Compound 14 was synthesized and obtained as yellow solid. LC-MS (ESI): [M+1]+=406.1.

## Example 23 Synthesis of Camptothecin Compound 14-P

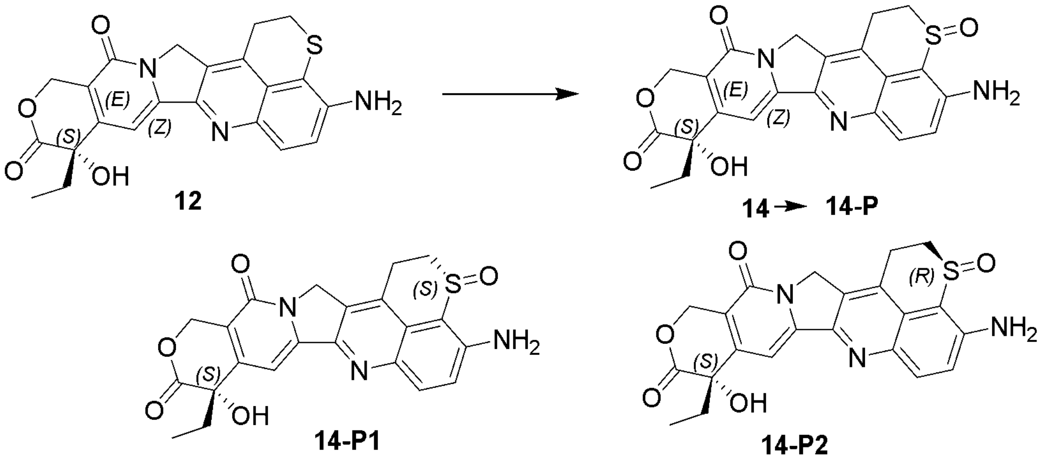

**12**

**14 → 14-P**

**14-P1**

**14-P2**

Compound 12 (200 mg) was dissolved in a mixed solvent of acetic acid (24 ml) and water (3 ml) at normal temperature, and mixture obtained was cooled to 5 °C under the protection of nitrogen, into which 30% $H_2O_2$ (580 mg) was then added. Reaction liquid was stirred for 2 hours, concentrated to dryness, and residue obtained was subjected to preparative HPLC, and Compounds 14-P1 and 14-P2 were separated and obtained. Being lyophilized, 42 mg of Compound 14-P1 was obtained as brown solid; and 25 mg of Compound 14-P2 was obtained as brown solid. LC-MS (ESI): [M+1]+ =438.5.

**Example 24 Synthesis of Camptothecin Compound 15**

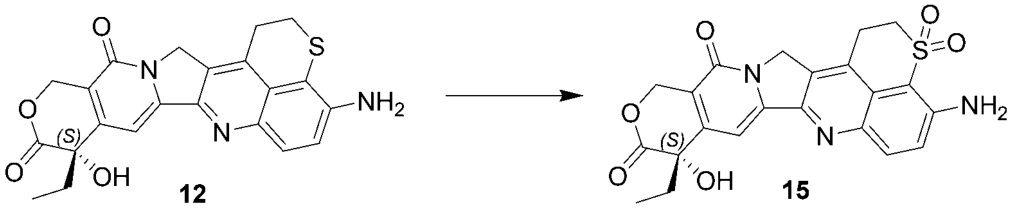

12 15

Compound 12 (100 mg) was dissolved in a mixed solvent of acetic acid (24 ml) and water (3 ml) at normal temperature, and mixture obtained was cooled to 5 °C under the protection of nitrogen, into which 30% $H_2O_2$ (750 mg) was then added. Reaction liquid was stirred for 2 hours, allowed to warm to room temperature 25 °C, and stirred overnight. After the reaction completed as confirmed with LCMS analysis, the reaction liquid was concentrated to dryness, and residue obtained was subjected to preparative HPLC, and Compound 15 was separated and collected. Being lyophilized, 8 mg of Compound 15 was obtained as brown solid. LC-MS (ESI): [M+1]+=454.4.

Example 25 Synthesis of Camptothecin Compound 16

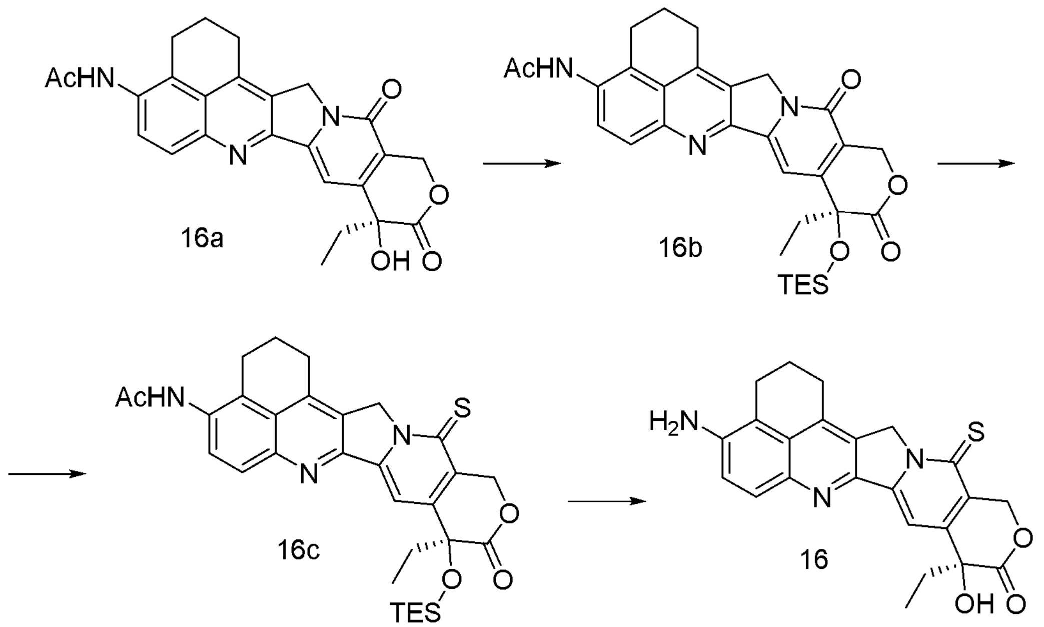

16a 16b 16c 16

The intermediate 16a was synthesized according to the method described in WO2020200880A1, and then the intermediate 16c having a structure of 16a-thiocamptothecin was prepared according to the method described in a literature (J. Med. Chem. 2008, 51, 3040-3044), and finally was de-protected to remove the acetyl group, and Compound 16 was prepared and obtained. LC-MS (ESI): [M+1]+=420.3.

**Example 26 Synthesis of Camptothecin Compound** 17

Synthetic route is shown as follows:

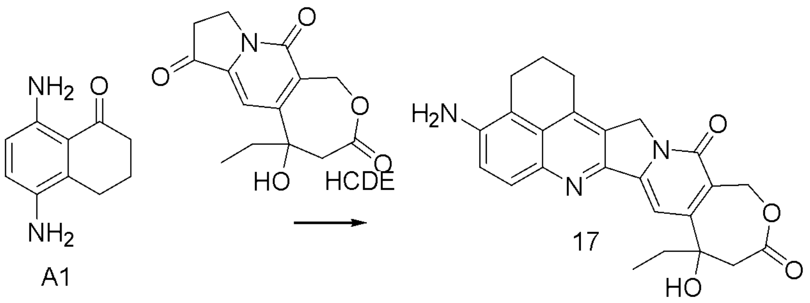


A1

17

According to a method similar to that described in Example 12, in which Compound A1 was used instead of Compound A6, HCDE was used instead of CDE, and toluene was used as the solvent, homocamptothecin Compound 17 (a mixture of S, and R configurations) was obtained.

LC-MS (ESI): [M+1]+=418.5.

**Example 27 Synthesis of Camptothecin Compound 18**

Synthetic **route is shown as follows:**

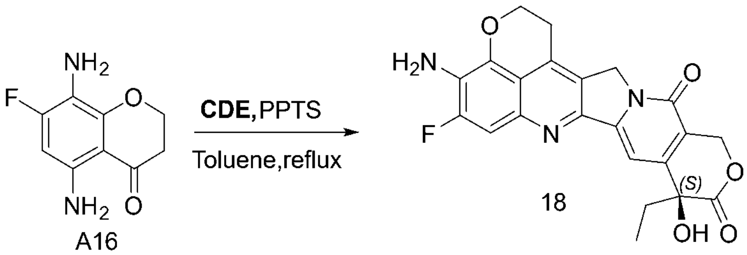


A16

18

According to a method similar to that described in Example 12, in which Compound A16 was used instead of Compound A6 and toluene was used as the solvent, Compound 18 was synthesized as light yellow solid. LC-MS (ESI): [M+1]+ =424.2.

**Example 28 Synthesis of Camptothecin Compound 19**

Synthetic route is shown as follows:

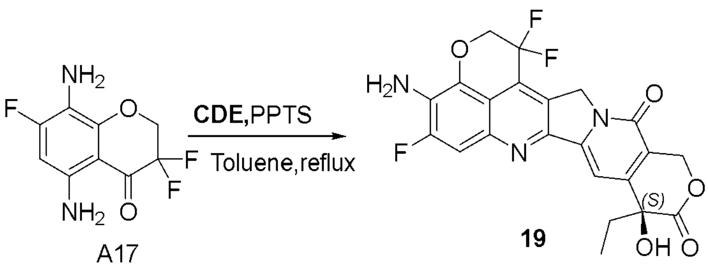


A17

**19**

According to a method similar to that described in Example 12, in which Compound A17 was used instead of Compound A6 and toluene was used as the solvent, Compound 19 was synthesized as yellow solid. LC-MS (ESI): [M+1]+ =460.5.

**Example 29 Synthesis of Camptothecin Compound** 20

Synthetic route is shown as follows:

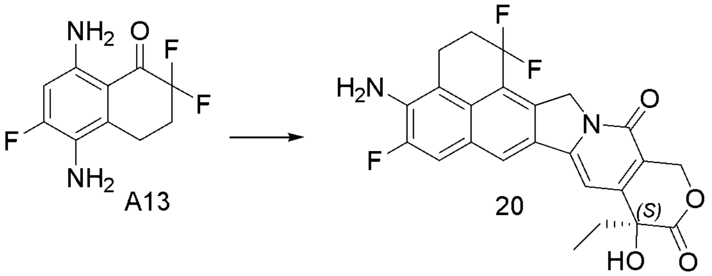

A13

20

According to a method similar to that described in Example 12, in which Compound A13 was used instead of Compound A6 and toluene was used as the solvent, Compound 20 was synthesized as yellow solid. LC-MS (ESI): [M+1]+ =458.2.

**Example 30 Synthesis of Camptothecin Compound** 21

Synthetic route is shown as follows:

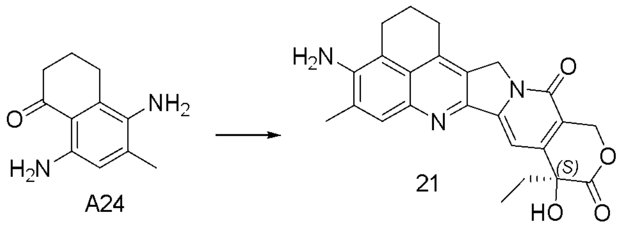

A24

21

According to a method similar to that described in Example 12, in which Compound A24 was used instead of Compound A6 and toluene was used as the solvent, Compound 21 was obtained as brownish red solid. LC-MS (ESI): [M+1]+ = 418.4.

**Example 31 Synthesis of Camptothecin Compound** 22

Synthetic route is shown as follows:

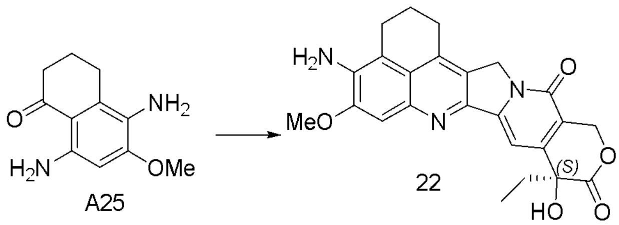

A25

22

According to a method similar to that described in Example 12, in which Compound A25 was used instead of Compound A6 and toluene was used as the solvent, Compound 22 was obtained as brownish red solid. LC-MS (ESI): [M+1]+= 434.4.

**Example** 32 Synthesis of Camptothecin Compound 23

Synthetic route is shown as follows:

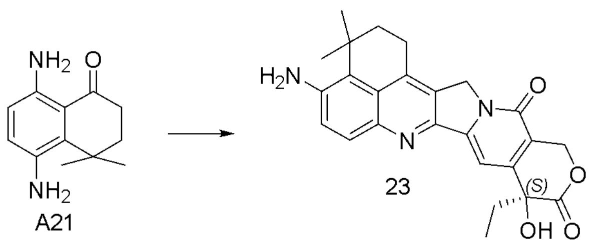

A21

23

According to a method similar to that described in Example 12, in which Compound A21 was used instead of Compound A6 and toluene was used as the solvent, Compound 23 was obtained as brownish red solid. LC-MS (ESI): [M+1]+ = 432.4.

**Example 33 Synthesis of Camptothecin Compound** 24

Synthetic route is shown as follows:

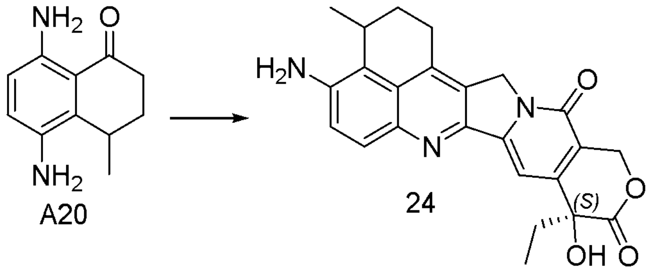

A20 24

According to a method similar to that described in Example 12, in which Compound A20 was used instead of Compound A6 and toluene was used as the solvent, Compound 24 (a pair of isomers) was obtained as brownish red solid. LC-MS (ESI): [M+1]+ = 418.4.

**Example 34 Synthesis of Camptothecin Compound** 25

Synthetic route is shown as follows:

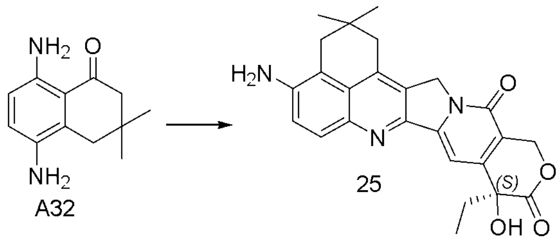

A32 25

According to a method similar to that described in Example 12, in which Compound A32 was used instead of Compound A6 and toluene was used as the solvent, Compound 25 was obtained as brownish red solid. LC-MS (ESI): [M+1]+ = 432.4.

**Example 35 Synthesis of Camptothecin Compound 26**

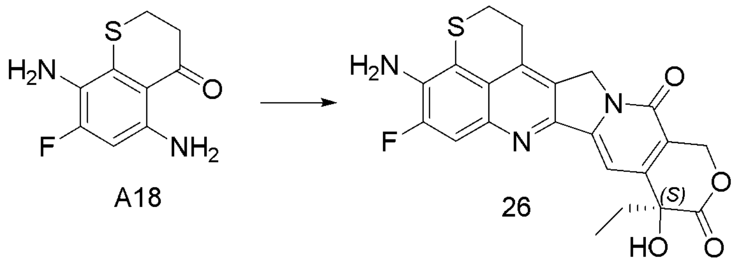

A18 26

According to a method similar to that described in Example 12, in which Compound A18 was used instead of Compound A6 and toluene was used as the solvent, Compound 26 was obtained as brownish red solid. LC-MS (ESI): [M+1]+= 440.4.

**Example 36 Synthesis of Camptothecin Compound 27**

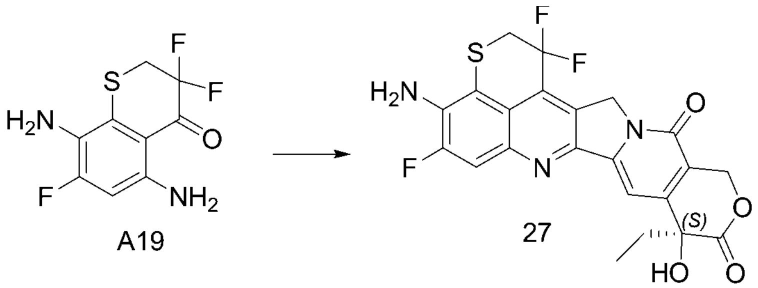

A19 27

According to a method similar to that described in Example 12, in which Compound A19 was used instead of Compound A6 and toluene was used as the solvent, Compound 27 was obtained as brownish red solid. LC-MS (ESI): [M+1]+= 476.4.

**Example 37 Synthesis of Camptothecin Compound 31**

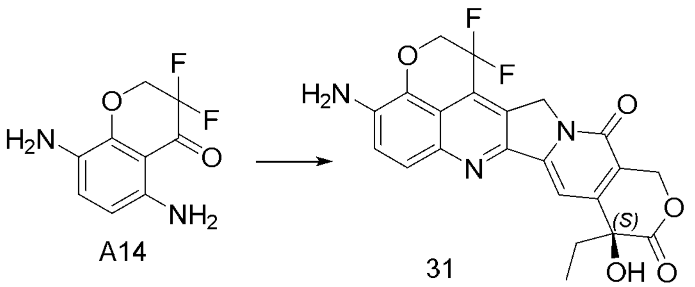

A14 31

According to a method similar to that described in Example 12, in which Compound A14 was used instead of Compound A6 and toluene was used as the solvent, Compound 31 was obtained as yellow solid. LC-MS (ESI): [M+1]+= 442.4.

**Example 38 Synthesis of Camptothecin Compound 32**

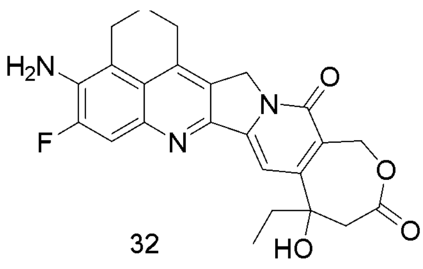

32

According to a method similar to that described in Example 12, in which Compound A2 was used instead of Compound A6, HCDE was used instead of CDE, and toluene was used as the solvent, Compound 32 was synthesized and obtained as yellow solid. LC-MS (ESI): [M+1]+ =436.4.

**Example 39 Synthesis of Camptothecin Compound 33**

Synthetic route is shown as follows:

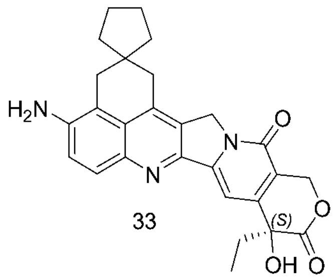

33

According to a method similar to that described in Example 12, in which Compound A23 was used instead of Compound A6, and toluene was used as the solvent, Compound 33 was synthesized as yellow solid. LC-MS (ESI): [M+1] += 458.5.

**<u>Example</u> 40 Synthesis of Camptothecin Compound 34**

Synthetic route is shown as follows:

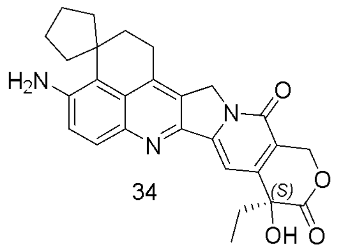

34

According to a method similar to that described in Example 12, in which Compound A22 was used instead of Compound A6, and toluene was used as the solvent, Compound 34 was synthesized as yellow solid. LC-MS (ESI): [M+1] += 458.5.

**<u>Example</u>** 41 **Synthesis of Camptothecin Compound 35 and Compound** 38

Synthetic route is shown as follows:

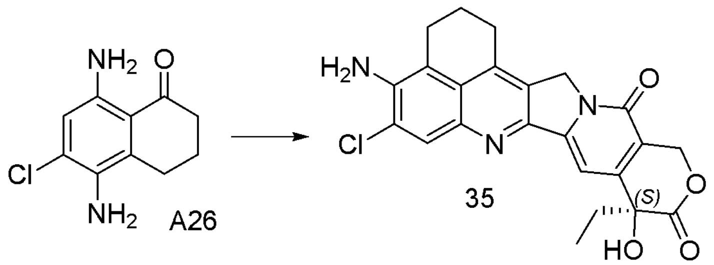

A26 35

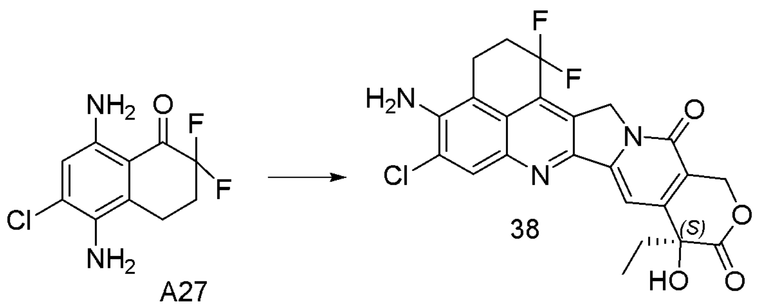

A27 38

According to a method similar to that described in Example 12, in which Compound A26 was used instead of Compound A6, and toluene was used as the solvent, Compound 35 was synthesized as brownish red solid. LC-MS (ESI): [M+1]+ = 438.7.

According to a method similar to that described in Example 12, in which Compound A27 was used instead of Compound A6, and toluene was used as the solvent, Compound 38 was synthesized and obtained as brownish red solid. LC-MS (ESI): [M+1]+= 474.7.

**Example 42 Synthesis of Camptothecin Compound 36 and Compound** 39

Synthetic route is shown as follows:

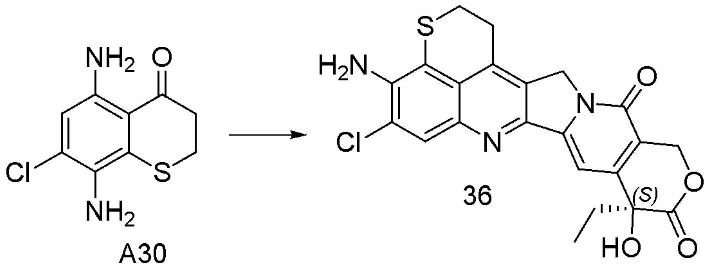

A30 36

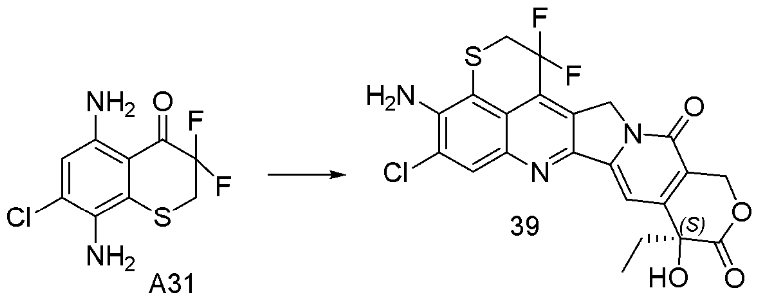

A31 39

According to a method similar to that described in Example 12, in which Compound A30 was used instead of Compound A6, and toluene was used as the solvent, Compound 36 was synthesized as brownish red solid. LC-MS (ESI): [M+1]+= 456.7.

According to a method similar to that described in Example 12, in which Compound A31 was used instead of Compound A6, and toluene was used as the solvent, Compound 39 was synthesized and obtained as brownish red solid. LC-MS (ESI): [M+1]+= 492.7.

**Example 43 Synthesis of Camptothecin Compound 37 and Compound 40**

Synthetic route is shown as follows:

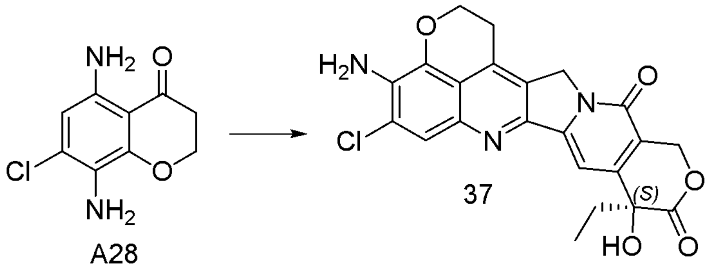

A28

37

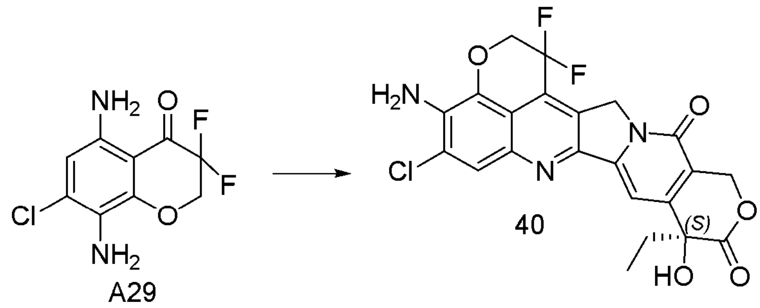

A29

40

According to a method similar to that described in Example 12, in which Compound A28 was used instead of Compound A6, and toluene was used as the solvent, Compound 37 was synthesized as brownish red solid. LC-MS (ESI): [M+1]+= 440.8.

According to a method similar to that described in Example 12, in which Compound A29 was used instead of Compound A6, and toluene was used as the solvent, Compound 40 was synthesized and obtained as brownish red solid. LC-MS (ESI): [M+1]+ = 476.7.

## Group 3 of Examples Synthesis of Linker-payloads

**Example 44** Synthesis of Linker-payload MWD-L1

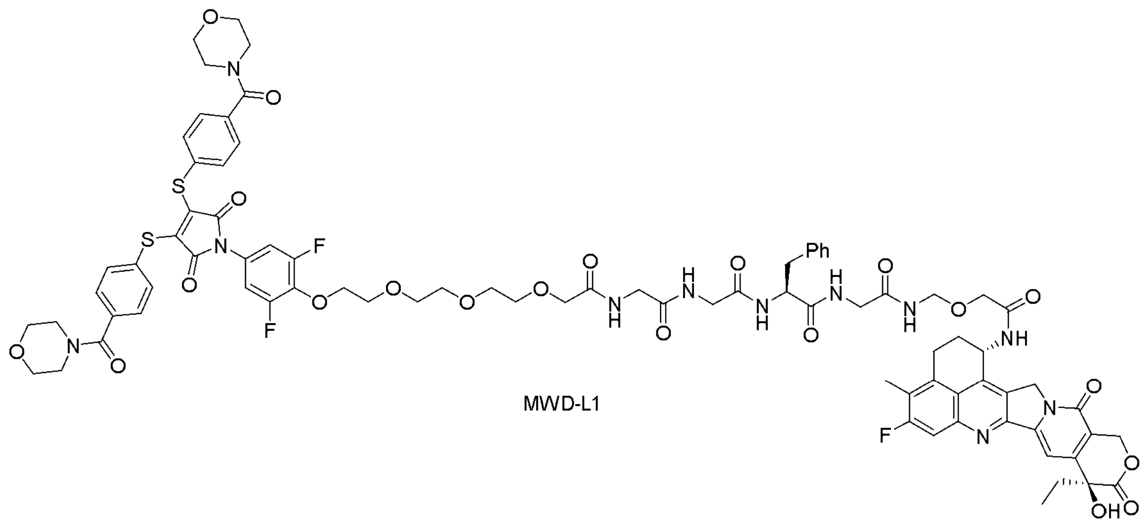

MWD-L1

Synthetic route is shown as follows:

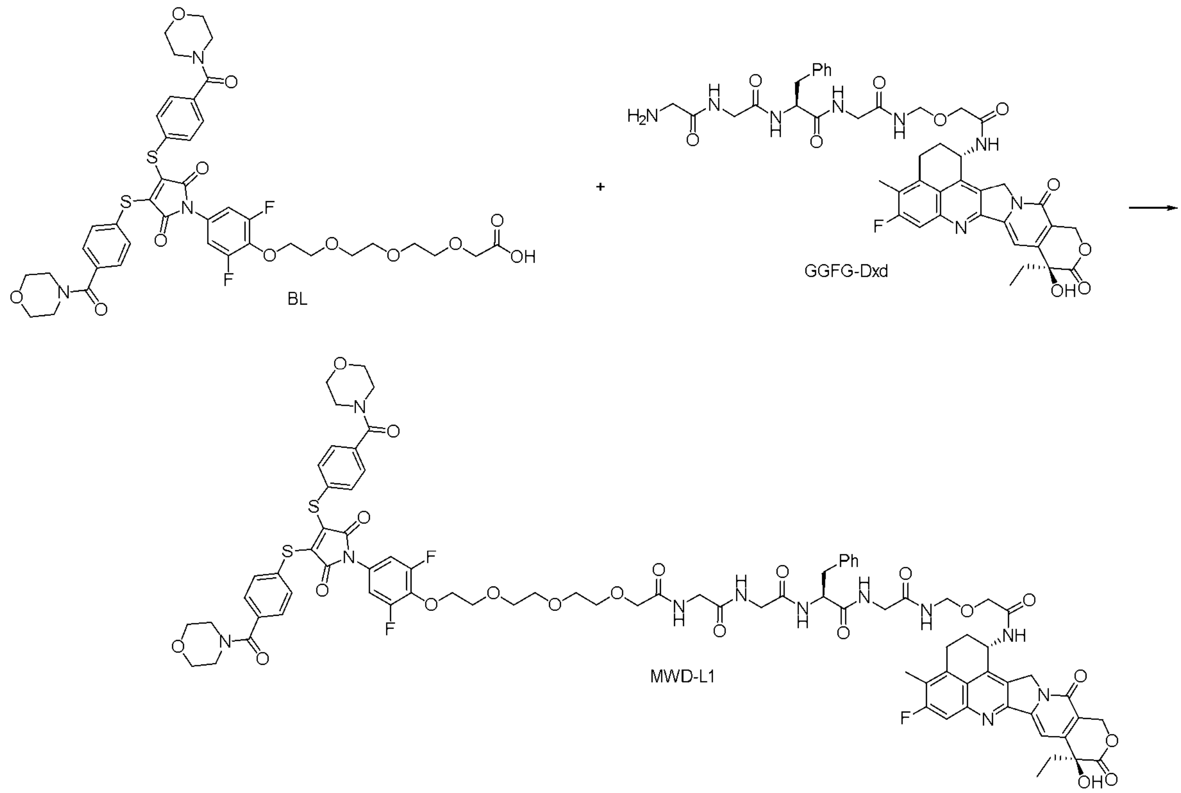

BL GGFG-Dxd MWD-L1

GGFG-Dxd was synthesized according to the method described in the patent publication US20190151328A1:

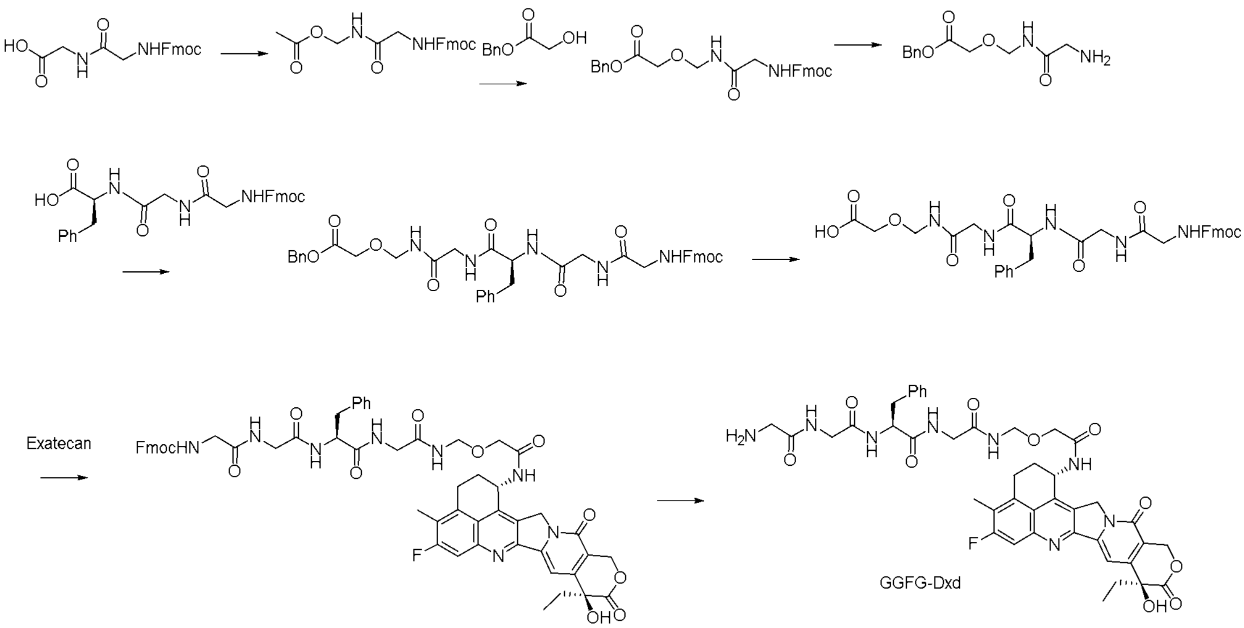


GGFG-Dxd

GGFG-Dxd was obtained as a light yellow solid. LC-MS (ESI): M+1= 841.

Linker compound BL was synthesized according to the method described in the patent publication WO2018/095422A1:

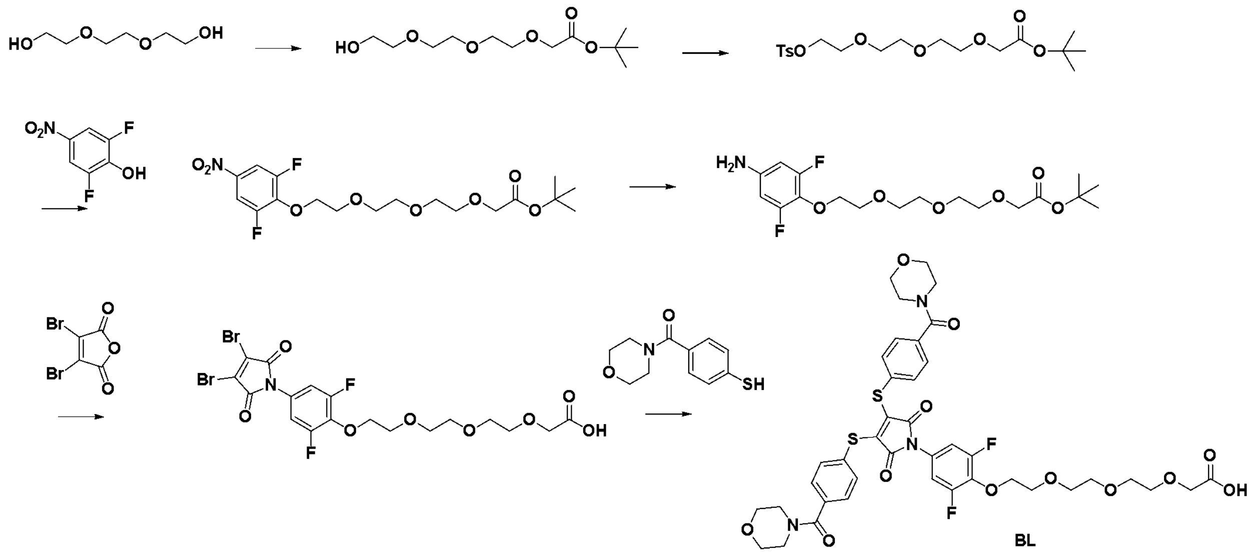

BL

Linker compound BL was obtained as yellow solid. LC-MS (ESI): M+1=858.

Linker compound BL (857 mg, 1 mmol), GGFG-Dxd (840 mg, 1mmol, 1 eq), DIPEA (323 mg, 2.5 mmol, 2.5 eq), and HATU (570 mg, 1.5 mmol, 1.5 eq) were dissolved in 30 ml of DCM, and stirred and reacted for 2 h. Reaction liquid was cooled to 5-10 °C, in which 1 N hydrochloric acid (20 ml) was added, and then was stirred for 0.5 h. Separated phases were obtained, and aqueous phase was extracted with DCM (30 ml × 2). Organic phases then obtained were pooled, washed with saturated brine, dried over anhydrous sodium sulfate, suctioned, and spun to dryness. Residue obtained was purified by column chromatography using a DCM/MeOH (50:1-10:1) mixture as an eluent, and 500 mg of Linker-payload MWD-L1 was obtained as yellow solid with a yield of 29.8%. LC-MS (ESI): M+1 = 1681, (M+1)/2=840.9.

**Example 45** Synthesis of Linker-payload MWC-L2

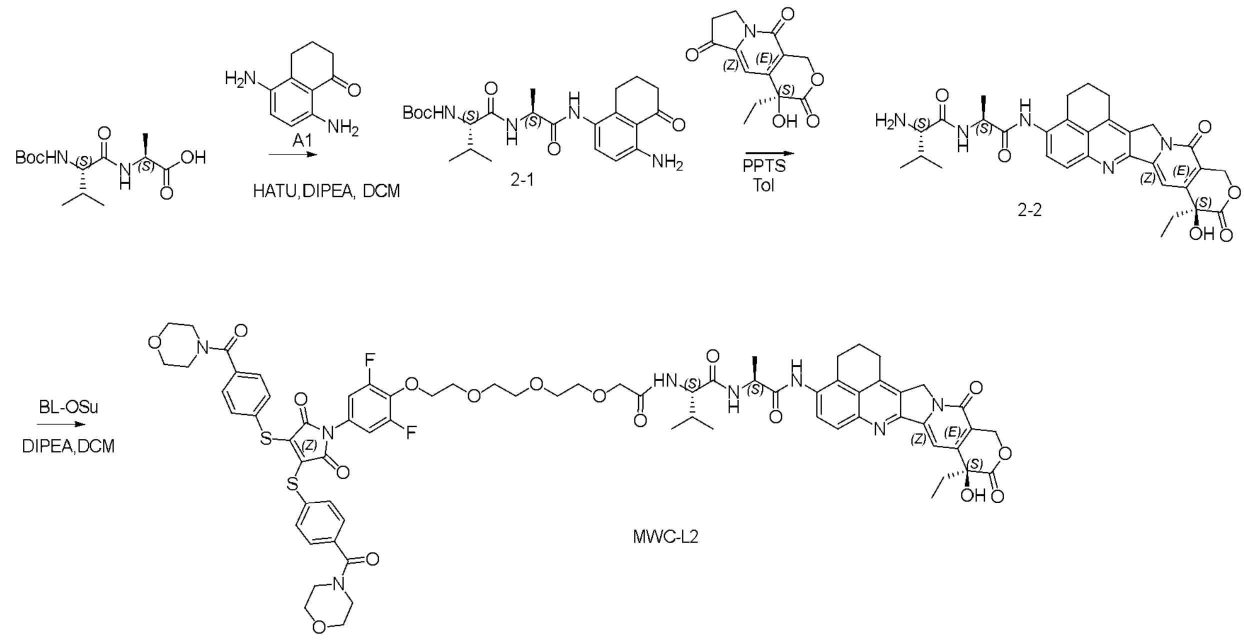


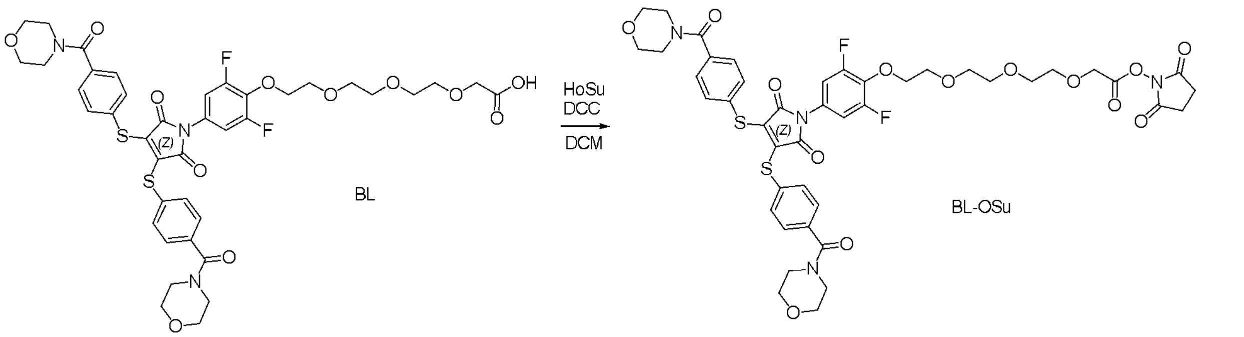


Step 1:

Boc-Val-Ala-OH (288 mg, 1 mmol), Compound A1 (176 mg, 1 mmol, 1 eq), DIPEA (322 mg, 2.5 mmol, 2.5 eq), and HATU (456 mg, 1.2 mmol, 1.2 eq) were dissolved in 30 ml of DCM, and stirred and reacted for 2 h. Reaction liquid was spun to dryness, and residue obtained was purified by column chromatography using a PE/EA (10:1-5:1) mixture as an eluent, and quantitatively 450 mg of the intermediate 2-1 was obtained as gray green solid.

Step 2:

The intermediate 2-1 (450 mg, 1 mmol), tricyclic compound CDE (263 mg, 1 mmol, 1 eq), and pyridinium p-toluenesulfonate (PPTS) (251 mg, 1 mmol, 1 eq) were suspended in 30 ml of toluene, and heated under reflux and reacted for 2 hours. The heating was stopped, and reaction liquid was allowed to cool and solid precipitates were collected, and 750 mg of the intermediate 2-2 crude product was obtained as brown solid. LC-MS (ESI): M+1 =574. The crude product was used directly in the next step without purification.

Step 3:

Linker compound BL (857 mg, 1 mmol), HoSu (138 mg, 1.2 mmol, 1.2 eq), and DCC (310 mg, 1.5 mmol, 1.5 eq) were dissolved in 30 ml of DCM, and stirred at room temperature for 3 h. Reaction liquid was suctioned, and filtrate obtained was a solution of A-Osu in DCM. The filtrate was added to a mixed solution of the crude intermediate 2-2, DIPEA (323 mg, 2.5 mmol, 2.5 eq), and DCM (30 ml), which was then stirred and reacted for 3 h. Then reaction liquid was cooled to below 10 °C and 1 N hydrochloric acid (20 ml) was added thereto, followed by stirring for 0.5 h. Separated phases were obtained, and aqueous phase was extracted with DCM (30 ml × 2). Organic phases then obtained were pooled, washed with saturated

brine, dried over anhydrous sodium sulfate, suctioned, and spun to dryness. Residue obtained was purified by column chromatography using a DCM/MeOH (50:1-10:1) mixture as an eluent, and 325 mg of Linker-payload MWC-L2 was obtained as orange solid with a yield of 23.0%. LC-MS (ESI): M+1 = 1414, (M+1)/2=707.

**Example 46** Synthesis of Linker-payload MWC-L3

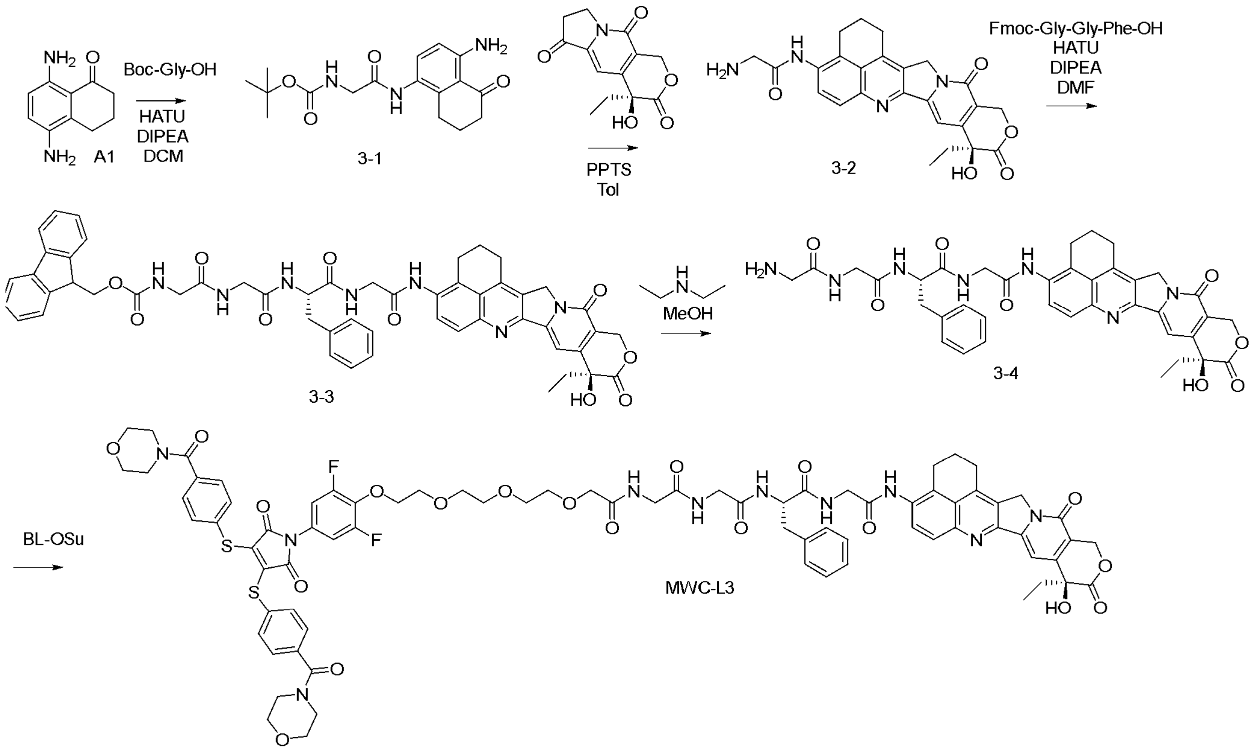


Step 1:

Boc-Gly-OH (175 mg, 1 mmol), Compound A1 (176 mg, 1 mmol, 1 eq), DIPEA (322 mg, 2.5 mmol, 2.5 eq), and HATU (456 mg, 1.2 mmol, 1.2 eq) were dissolved in 30 ml of DCM, and stirred and reacted for 2 h. Reaction liquid was spun to dryness, and residue obtained was purified by column chromatography using a PE/EA (10:1-5:1) mixture as an eluent, and quantitatively 335 mg of the intermediate 3-1 was obtained as light green solid.

Step 2:

The intermediate 3-1 (335 mg, 1 mmol), tricyclic compound CDE (263 mg, 1 mmol, 1 eq), and PPTS (251 mg, 1 mmol, 1 eq) were suspended in 30 ml of toluene, and heated under reflux and reacted for 2 hours. The heating was stopped, and reaction liquid was allowed to cool and solid precipitates were collected, and 560 mg of the intermediate 3-2 crude product was obtained as brown solid. LC-MS (ESI): M+1= 461. The crude product was used directly in the next step without purification.

Step 3:

Fmoc-Gly-Gly-Phe-OH (501 mg, 1 mmol), the crude intermediate 3-2 (560 mg), DIPEA (322 mg, 2.5 mmol, 2.5 eq), and HATU (456 mg, 1.2 mmol, 1.2 eq) were dissolved in 20 ml of DCM, and stirred and reacted for 3 h. 1 N hydrochloric acid (30 ml) was added thereto, followed by stirring for 0.5 h. Reaction liquid was extracted with DCM (30 ml × 2). Organic phases then obtained were pooled, washed with saturated brine, dried over anhydrous sodium sulfate, suctioned, and spun to dryness. Residue obtained was purified by column chromatography using a DCM/MeOH (50:1-10:1) mixture as an eluent, and 350 mg of the intermediate 3-3 was obtained as brown solid. LC-MS (ESI): M+1=945.

Step 4:

The intermediate 3-3 (350 mg, 0.37 mmol) was dissolved in methanol (20 ml), into which diethylamine (2 ml) was then added, and mixture obtained was stirred and reacted for 3 hours. Reaction liquid was spun to dryness, and the intermediate 3-4 crude product was obtained as brown sticky matter. LC-M (ESI): M+1=722. The crude product was used directly in the next step without purification.

Step 5:

Linker compound BL (318 mg, 0.37 mmol), HoSu (51 mg, 0.44 mmol, 1.2 eq), and DCC (114 mg, 0.56 mmol, 1.5 eq) were dissolved in 30 ml of DCM, and stirred at room temperature for 3 h. Reaction liquid was suctioned, and filtrate obtained was added to a mixed solution of the crude intermediate 3-4, DIPEA (120 mg, 0.93mmol, 2.5 eq), and DCM (30 ml), which was then stirred and reacted for 3 h. Then reaction liquid was cooled to below 10 °C and 1 N hydrochloric acid (20 ml) was added thereto, followed by stirring for 0.5 h. Separated phases were obtained, and aqueous phase was extracted with DCM (30 ml × 2). Organic phases then obtained were pooled, washed with saturated brine, dried over anhydrous sodium sulfate, suctioned, and spun to dryness. Residue obtained was purified by column chromatography using a DCM/MeOH (50:1-10:1) mixture as an eluent, and 120 mg of Linker-payload MWC-L3 was obtained as orange solid with a yield of 20.8%. LC-MS (ESI): M+1 = 1562, (M+1)/2=781.

**Example 47** Synthesis of Linker-payload MWE-L4

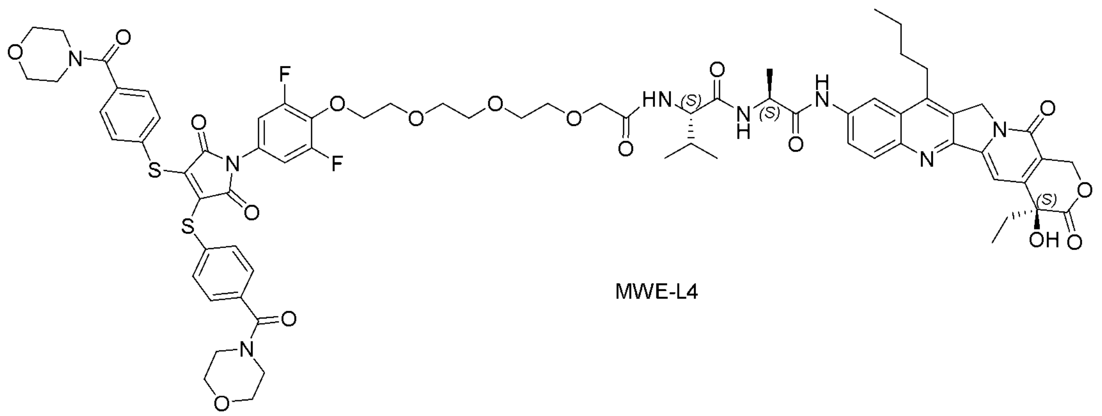

MWE-L4

According to a method similar to that described in Example 45, in which Compound A3 was used instead of Compound A1, MWE-L4 was synthesized and obtained as orange solid. LC-MS (ESI): M+1 =1430.

**Example** 48 Synthesis of Linker-payload MWG-L5

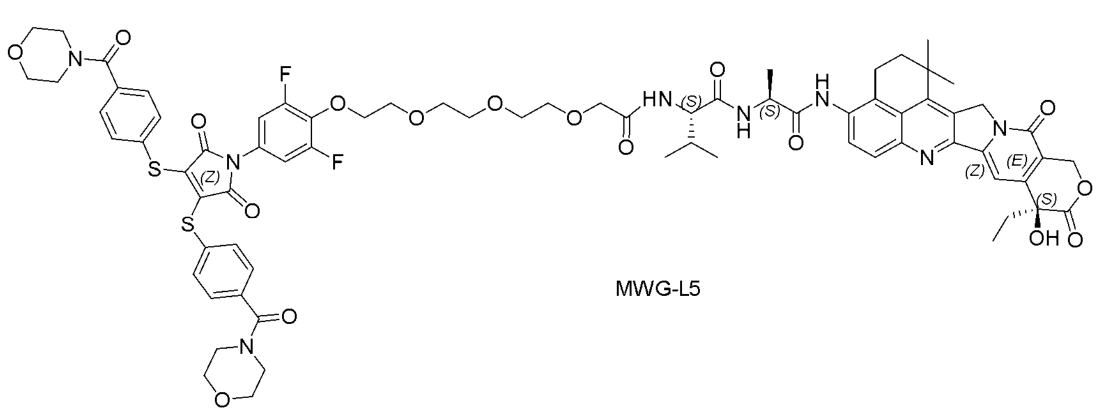

MWG-L5

According to a method similar to that described in Example45, in which Compound A6 was used instead of Compound A1, MWG-L5 was synthesized and obtained as orange solid. LC-MS (ESI): M+1 =1442.

**Example** 49 Synthesis of Linker-payload MWF-L6

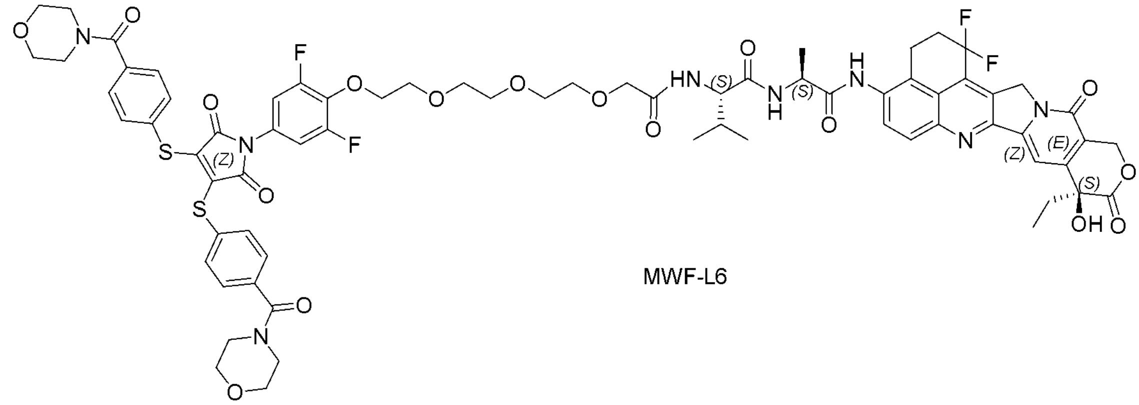

MWF-L6

According to a method similar to that described in Example 45, in which Compound A7 was used instead of Compound A1, MWF-L6 was synthesized and obtained as orange solid. LC-MS (ESI): M+1 =1450.

**Example** 50 Synthesis of Linker-payload MWF-L7

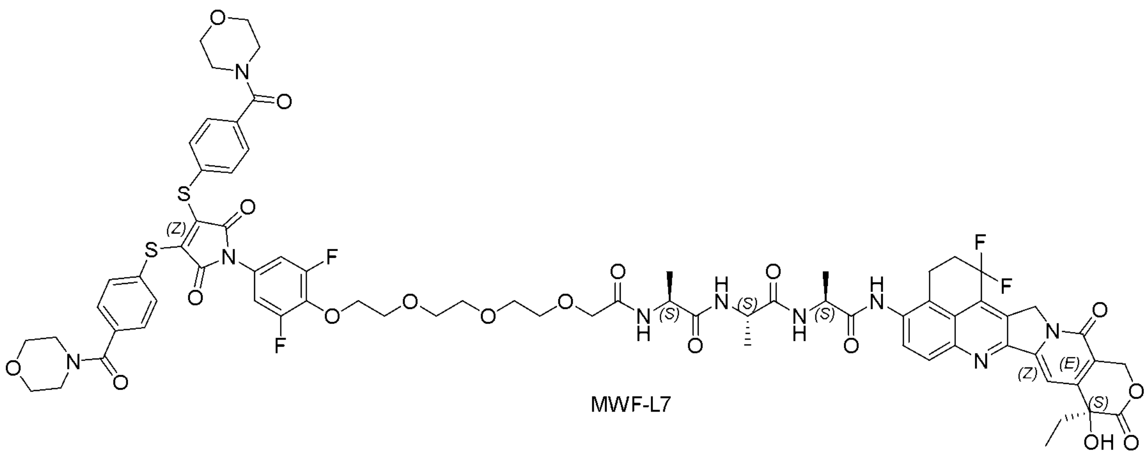

MWF-L7

According to a method similar to that described in Example 45, in which Compound A7 was used instead of Compound A1 and Alloc-Ala-Ala-Ala-OH was used instead of Alloc-Val-Ala-OH, MWF-L7 was synthesized and obtained as orange solid. LC-MS (ESI): M+1=1493.5.

Example 51 Synthesis of Linker-payload MWF-L8

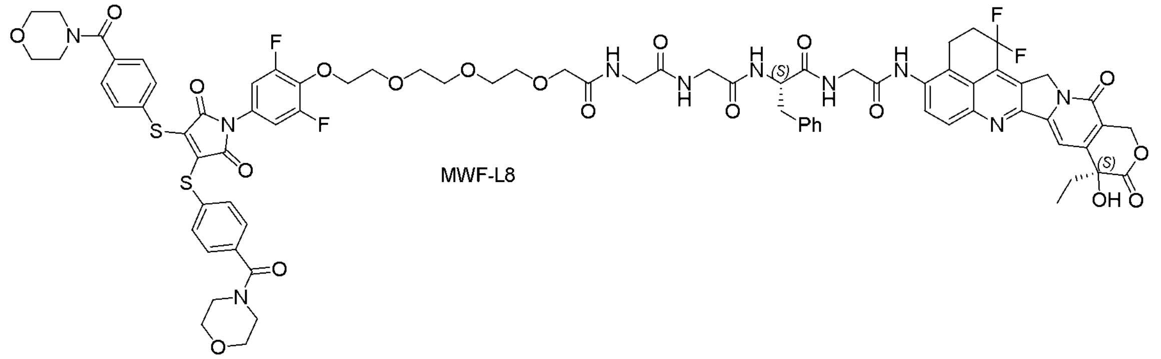

MWF-L8

According to a method similar to that described in Example 46, in which Compound A7 was used instead of Compound A1, MWF-L8 was synthesized and obtained as orange solid. LC-MS (ESI): M+1 =1598.4.

**Example52** Synthesis of Linker-payload MWF-L9

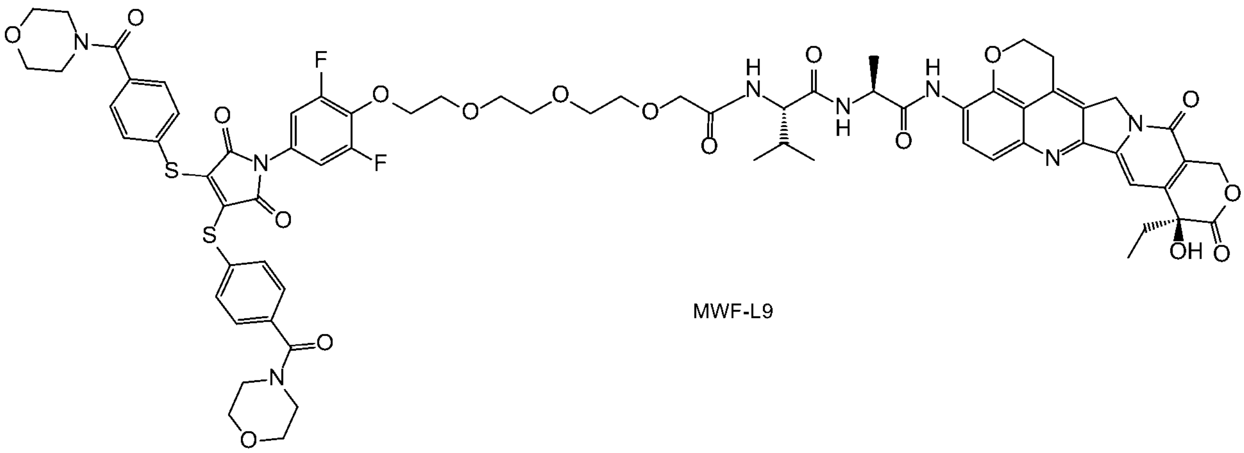

MWF-L9

According to a method similar to that described in Example 45, in which Compound A5 was used instead of Compound A1, MWF-L9 was synthesized and obtained as yellow solid.

LC-MS (ESI): M+1 =1416.4

**Example 53** Synthesis of Linker-payload MWF-L10

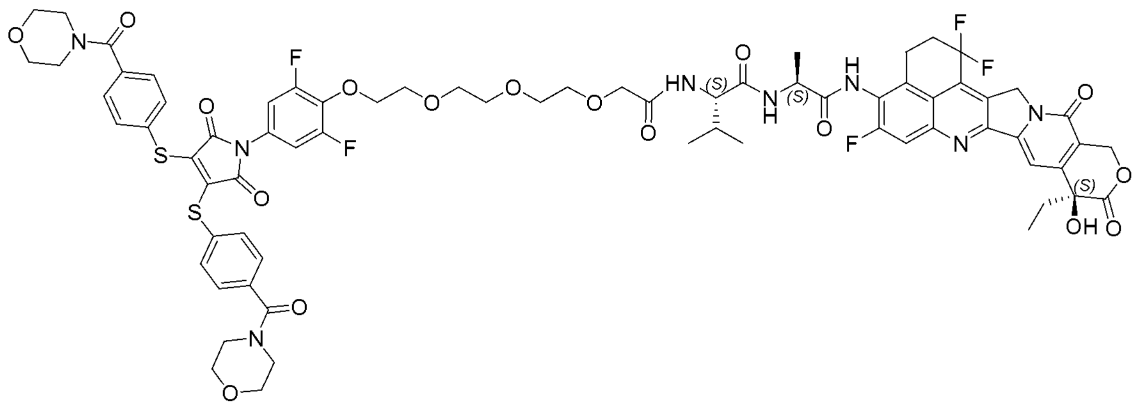

MWF-L10

According to a method similar to that described in Example 45, in which Compound A13 was used instead of Compound A1, MWF-L10 was synthesized and obtained as yellow solid. LC-MS (ESI): M+1 =1468.4.

**Example 54** Synthesis of Linker-payload MWF-L11

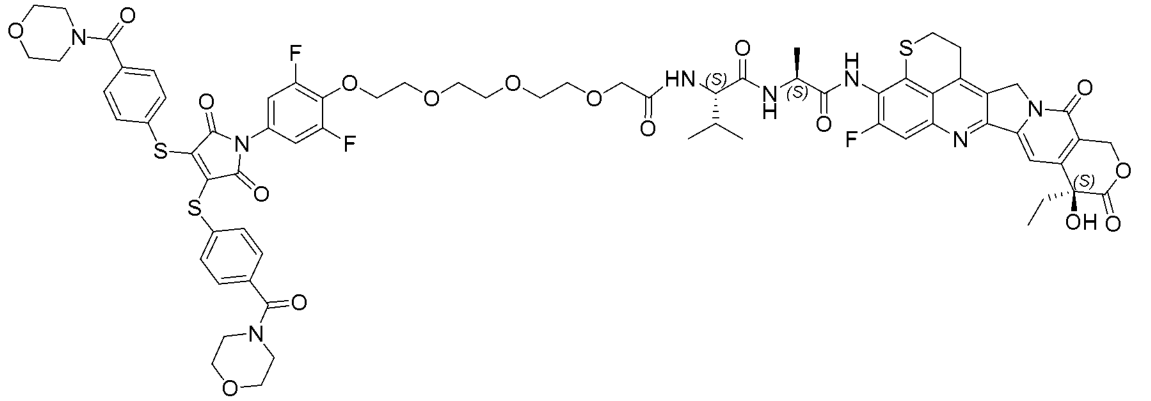

MWF-L11

According to a method similar to that described in Example 45, in which Compound A18 was used instead of Compound A1, MWF-L11 was synthesized and obtained as yellow solid. LC-MS (ESI): M+1 =1450.4.

**Example 55** Synthesis of Linker-payload MWF-L12

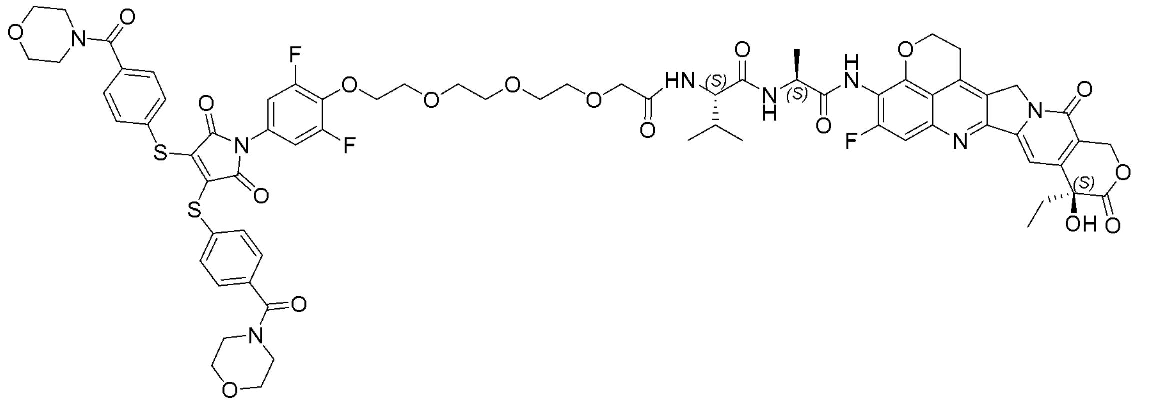

MWF-L12

According to a method similar to that described in Example 45, in which Compound A16 was used instead of Compound A1, MWF-L12 was synthesized and obtained as yellow solid. LC-MS (ESI): M+1 =1434.4.

**Example 56** Synthesis of Linker-payload MWF-L13

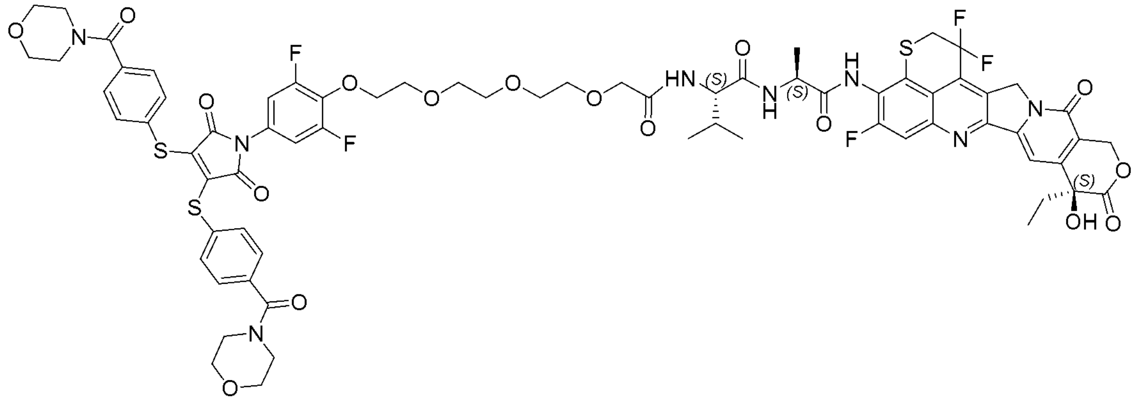

MWF-L13

According to a method similar to that described in Example 45, in which Compound A19 was used instead of Compound A1, MWF-L13 was synthesized and obtained as yellow solid. LC-MS (ESI): M+1 =1486.4.

**Example 57** Synthesis of Linker-payload MWF-L14

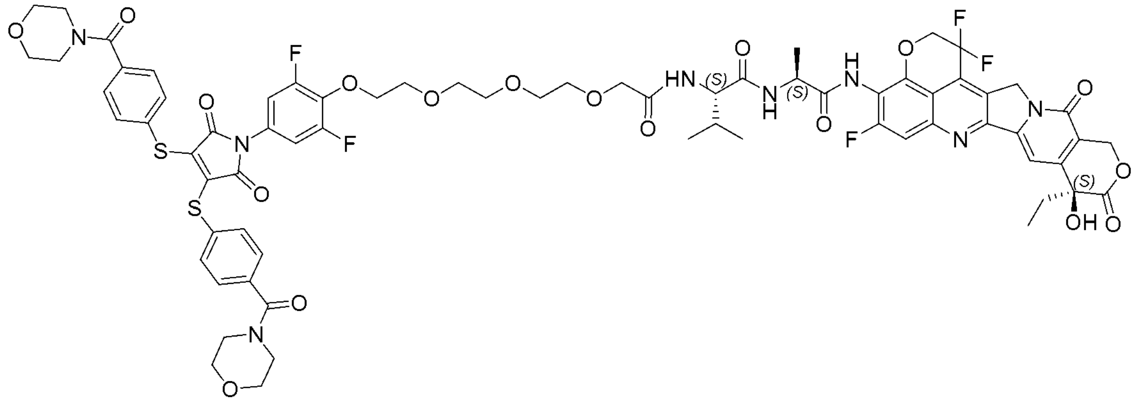

MWF-L14

According to a method similar to that described in Example 45, in which Compound A17 was used instead of Compound A1, MWF-L14 was synthesized and obtained as yellow solid. LC-MS (ESI): M+1 =1470.4.

**Example 58** Synthesis of Linker-payload MWF-L15

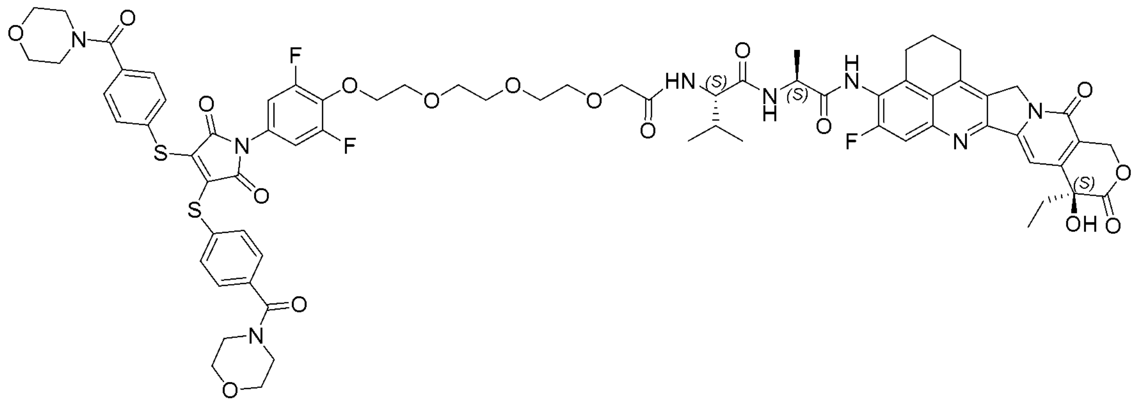

MWF-L15

According to a method similar to that described in Example 45, in which Compound A2 was used instead of Compound A1, MWF-L15 was synthesized and obtained as yellow solid. LC-MS (ESI): M+1 = 1432.4.

**Example 59** Synthesis of Linker-payload MWD-L7

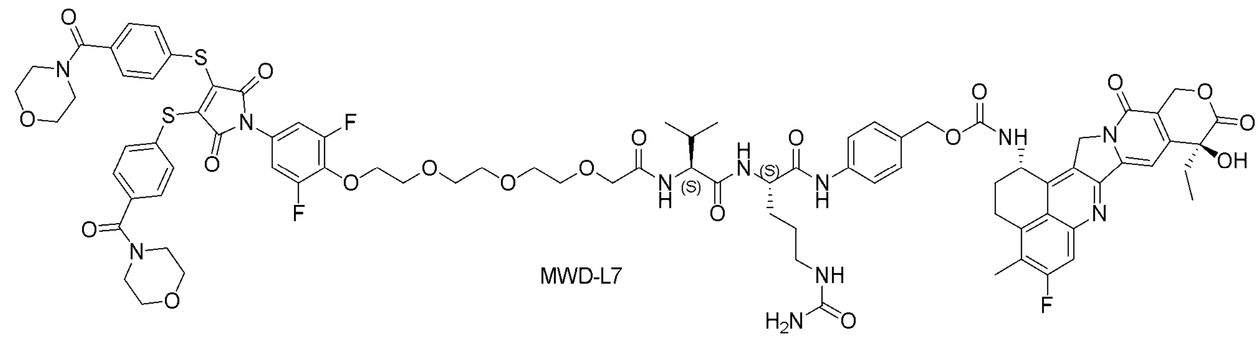

MWD-L7

Synthetic route is as follows:

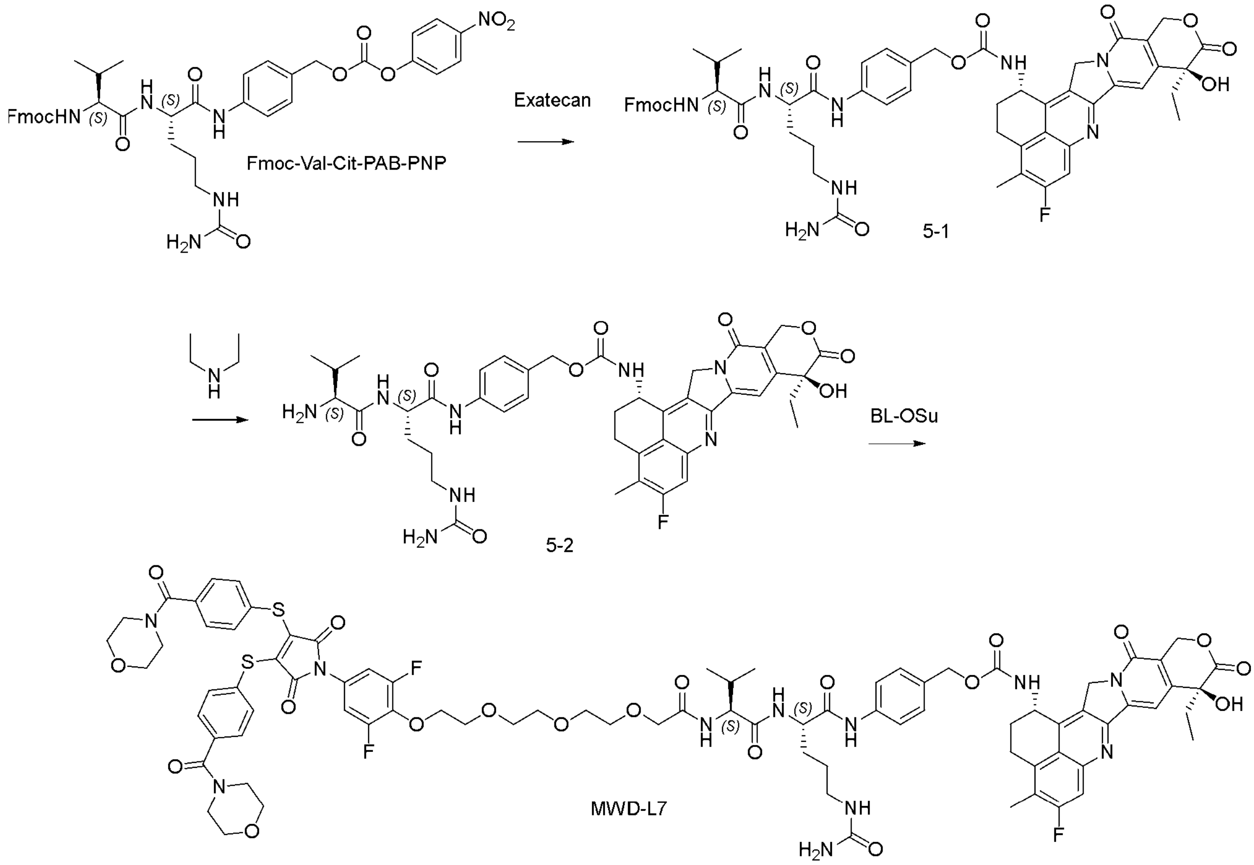


Step 1:

Fmoc-Val-Cit-PAB-PNP (153 mg, 0.2 mmol), Exatecan mesylate (106 mg, 0.2 mmol, 1 eq), and DIPEA (65 mg, 0.5mmol, 2.5 eq) were dissolved in 30 ml of DCM, and stirred and reacted for 2 h. Reaction liquid was spun to dryness, and residue obtained was purified by column chromatography using a DCM/MeOH (50:1-10:1) mixture as an eluent, and 180 mg of the intermediate 5-1 was obtained as yellow solid. LC-MS (ESI): M+1 =1064.

Step 2:

The intermediate 5-1 (180 mg, 0.17 mmol) was dissolved in methanol (20 ml), into which diethylamine (2 ml) was then added, and mixture obtained was stirred and reacted for 3 h. Reaction liquid was spun to dryness, and the intermediate 5-2 crude product was obtained as light yellow sticky matter. LC-M (ESI): M+1 =842. The crude product was used directly in the next step without purification.

Step 3:

Linker compound BL (146 mg, 0.17 mmol), HoSu (25 mg, 0.21 mmol, 1.2 eq), and DCC (54 mg, 0.26 mmol, 1.5 eq) were dissolved in 20 ml of DCM, and stirred at room temperature for 3 h. Reaction liquid was suctioned, and filtrate obtained was added to a mixed solution of the crude intermediate 5-1, DIPEA (56 mg, 0.43 mmol, 2.5 eq), and DCM (20 ml), which was then stirred and reacted for 3 h. Then reaction liquid was cooled to 5-10 °C and 1 N hydrochloric acid (20 ml) was added thereto, followed by stirring for 0.5 h. Separated phases were obtained, and aqueous phase was extracted with DCM (30 ml × 2). Organic phases then obtained were pooled, washed with saturated brine, dried over anhydrous sodium sulfate, suctioned, and spun to dryness. Residue obtained was purified by column chromatography using a DCM/MeOH (50:1-10:1) mixture as an eluent, and 67 mg of Linker-payload MWD-L7 was obtained as yellow solid with a yield of 23.1%. LC-MS (ESI): M+1 =1682, (M+1)/2=841.

<u>**Example 60**</u> Synthesis of Linker-payload MWD-L8

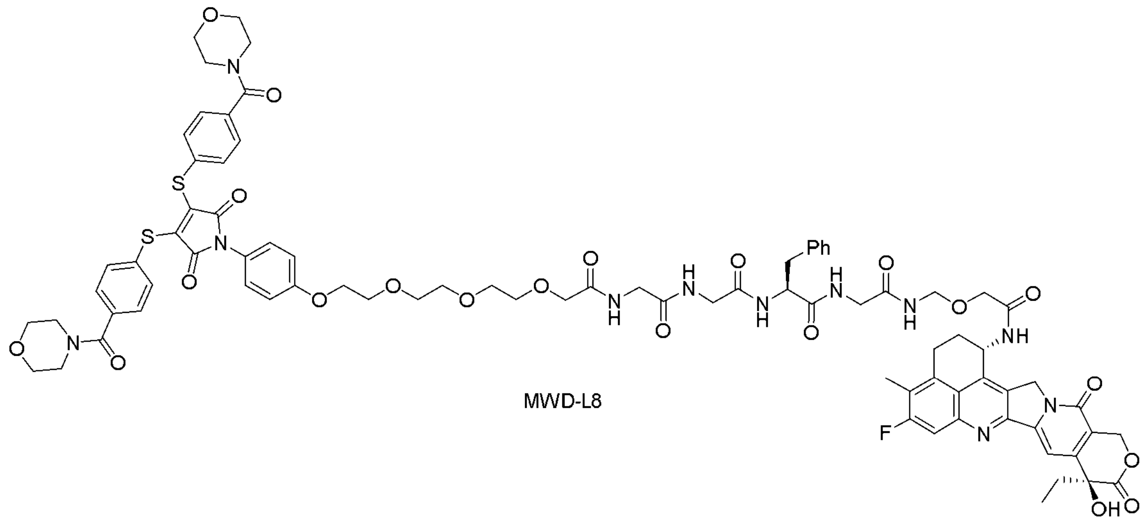

MWD-L8

According to a method similar to that described in Example 44, in which

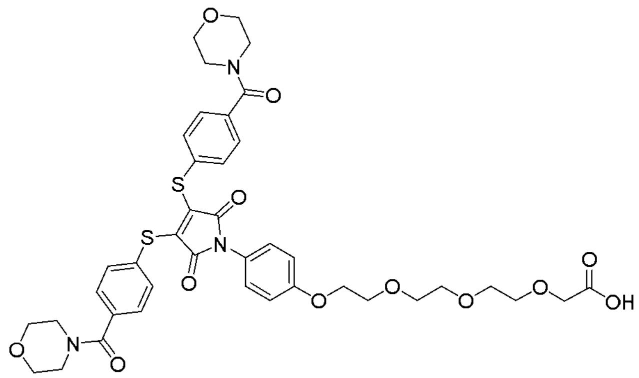

was used instead of Compound BL, MWD-L8 was synthesized and obtained as light yellow solid. LC-MS (ESI): M+1 =1645.7.

**Example 61** Synthesis of Linker-payload MWD-L9

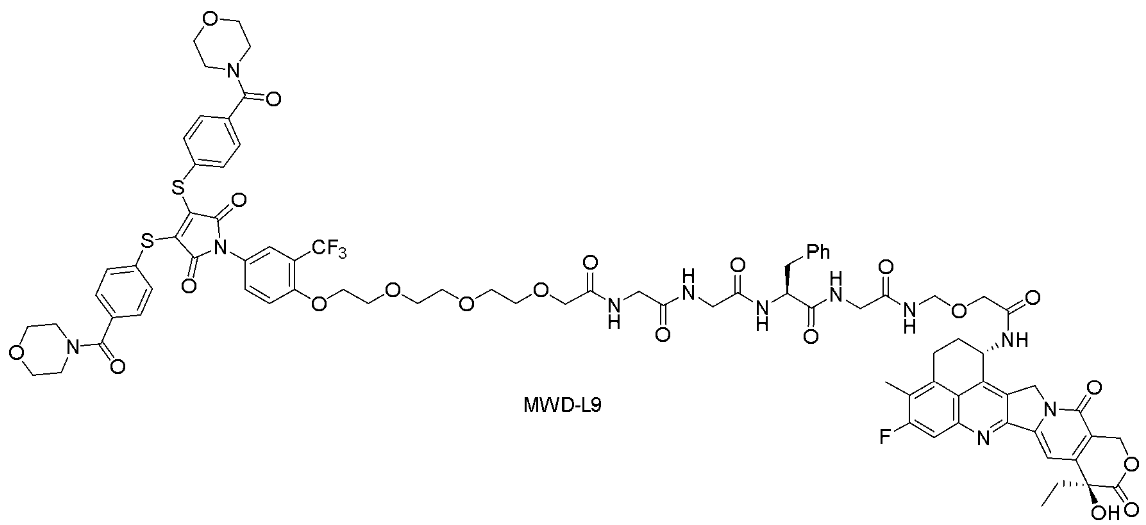

MWD-L9

According to a method similar to that described in Example 44, in which

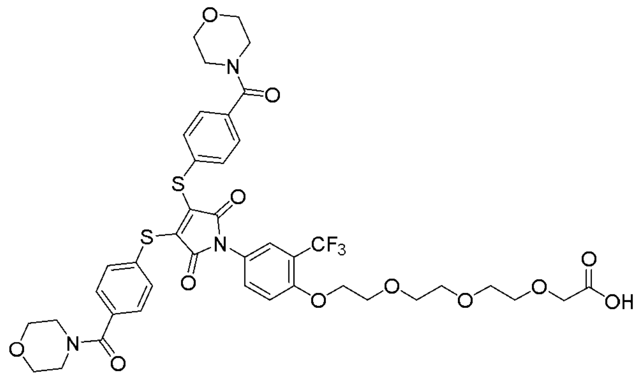

was used instead of Compound BL, MWD-L9 was synthesized and obtained as light yellow solid. LC-MS (ESI): M+1 =1713.7.

**Example 62** Synthesis of Linker-payload L-D

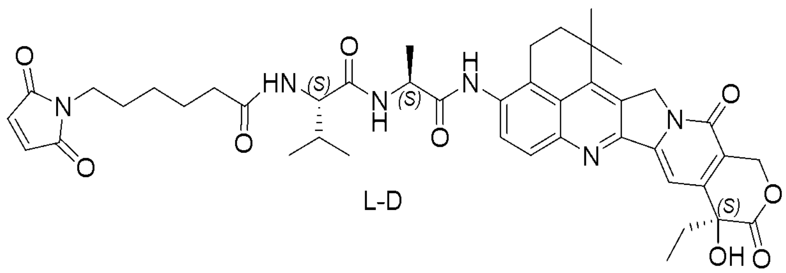

L-D

According to a method similar to that described in Example 48, in which MC-OSU was used instead of BL-OSU, L-D was synthesized and obtained as light yellow solid. LC-MS (ESI): M+1 =795.9.

**Example 63** Synthesis of Linker-payload L-E

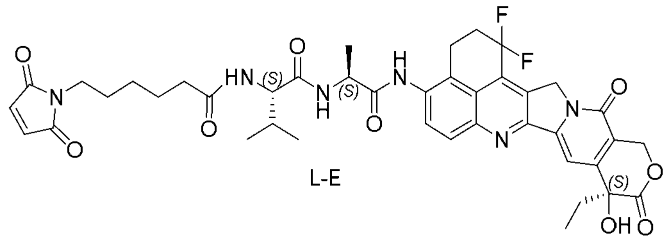

L-E

According to a method similar to that described in Example 45 in which MC-OSU was used instead of BL-OSU, L-E was synthesized and obtained as light yellow solid. LC-MS (ESI): M+1 =803.8.

Example 64 Synthesis of Linker-payload L-F

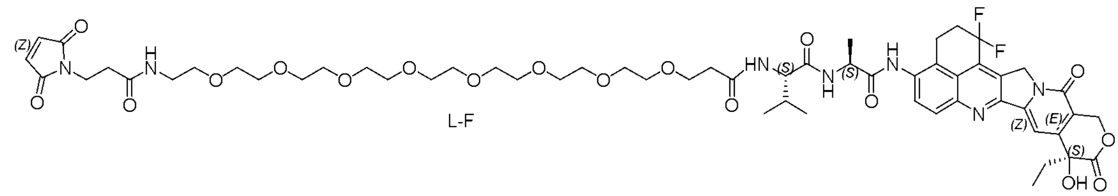

L-F

According to a method similar to that described in Example 45, in which A7 was used instead of A1 and MaL-PEG8-COOH was used instead of Compound BL, L-F was prepared and obtained as yellow solid. LC-MS (ESI): M+1= 1185.

**Example 65** Synthesis of Linker-payload L-G

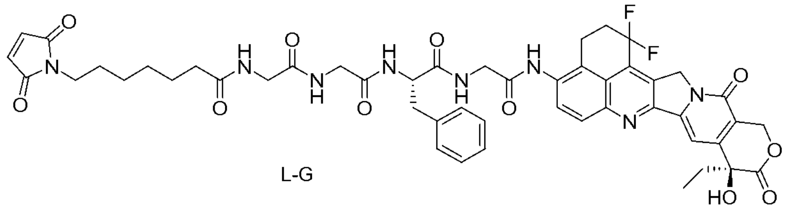

L-G

According to a method similar to that described in Example 46, in which compound MC-OSu was used instead of compound BL-OSu, L-G was prepared and obtained as yellow solid. LC-MS (ESI): M+1=965.98.

Example 66 Synthesis of Linker-payload L-H

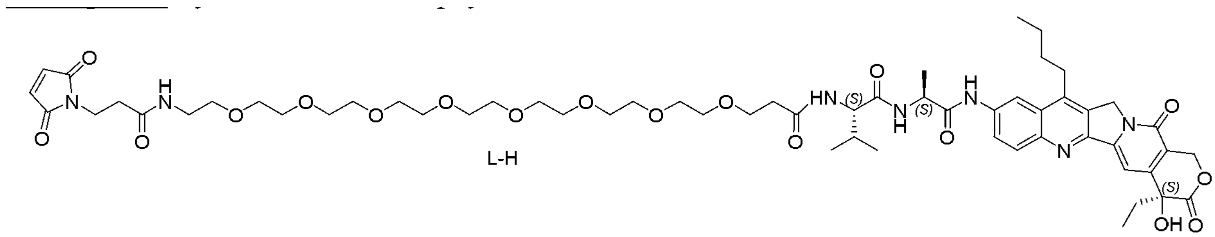

L-H

According to a method similar to that described in Example 47, in which MaL-PEG8-COOH was used instead of Compound BL, L-H was prepared and obtained as light yellow solid. LC-MS (ESI): M+1=1165.3.

**Example 67** Synthesis of Linker-payload L-I

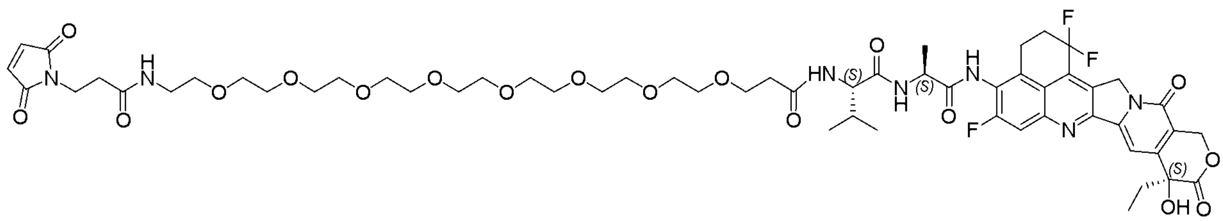

L-I

According to a method similar to that described in Example 45, in which A13 was used instead of A1, L-I was prepared and obtained as yellow solid. LC-MS (ESI): M+1 =1203.4.

**Example 68**Synthesis of Linker-payload L-J

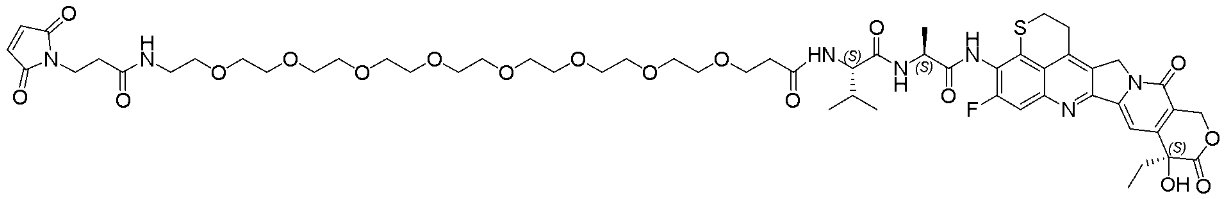

L-J

According to a method similar to that described in Example 45, in which A18 was used instead of A1, L-J was prepared and obtained as yellow solid. LC-MS (ESI): M+1 =1185.4.

**Example 69** Synthesis of Linker-payload L-K

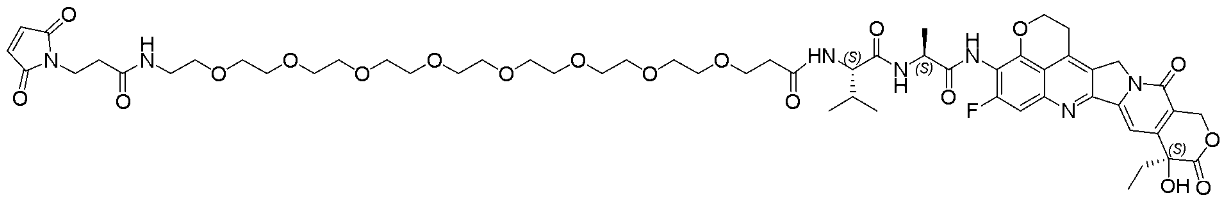

L-K

According to a method similar to that described in Example 45, in which A16 was used instead of A1, L-K was prepared and obtained as yellow solid. LC-MS (ESI): M+1 =1169.3.

Example 70 Synthesis of Linker-payload L-L

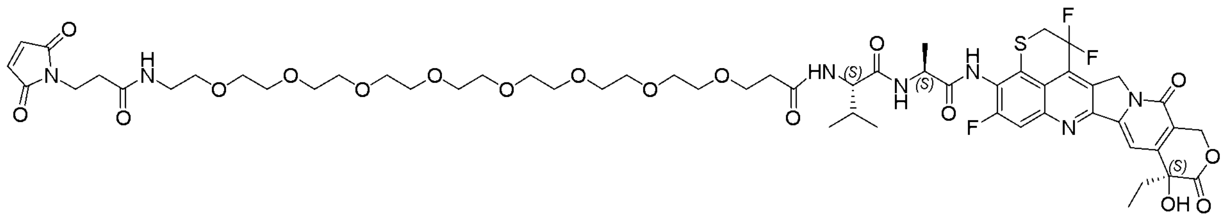

L-L

According to a method similar to that described in Example 45 in which A19 was used instead of A1, L-L was prepared and obtained as yellow solid. LC-MS (ESI): M+1 =1221.4.

**Example 71** Synthesis of Linker-payload L-M

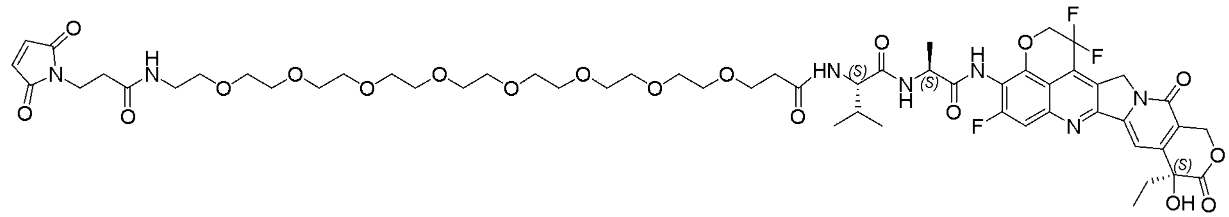

L-M

According toa method similar to that described in Example 45, in which A17 was used instead of A1, L-M was prepared and obtained as yellow solid. LC-MS (ESI): M+1 =1205.4.

**Example 72** Synthesis of Linker-payload L-N

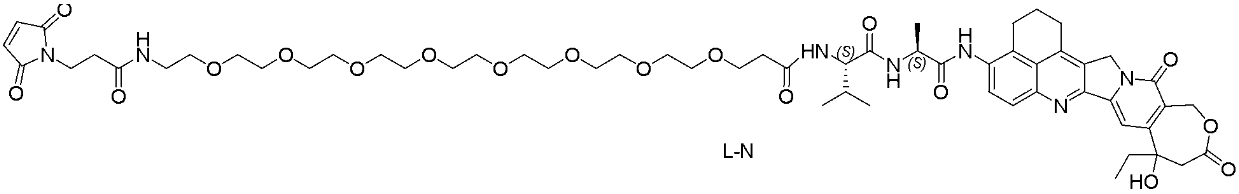

L-N

According to a method similar to that described in Example 45, in which the intermediate

HCDE was used instead of the intermediate CDE and MaL-PEG8-COOH was used instead of Compound BL, L-N was prepared and obtained as yellow solid. LC-MS (ESI): M+1= 1163.2 (mixture of a pair of diastereoisomers).

**Example 73** Synthesis of Linker-payload MWS-L1

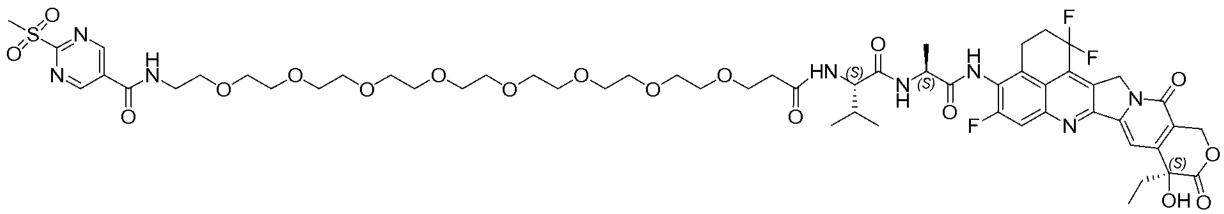

MWS-L1

According to a method similar to that described in Example 45, in which A13 was used instead of A1 and

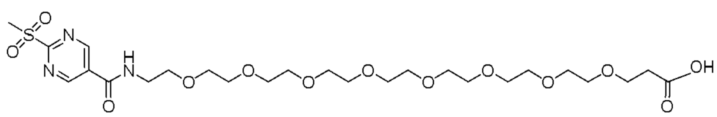

was used instead of Linker compound BL, MWS-L1 was prepared and obtained as yellow solid. LC-MS (ESI): M+1= 1236.3.

**Example 74** Synthesis of Linker-payload MWS-L2

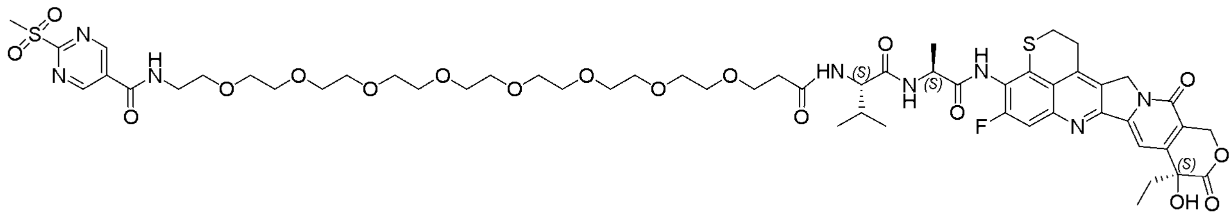

MWS-L2

According to a method similar to that described in Example 45, in which A18 was used instead of A1, MWS-L2 was prepared and obtained as yellow solid. LC-MS (ESI): M+1 =1218.4.

**Example 75** Synthesis of Linker-payload MWS-L3

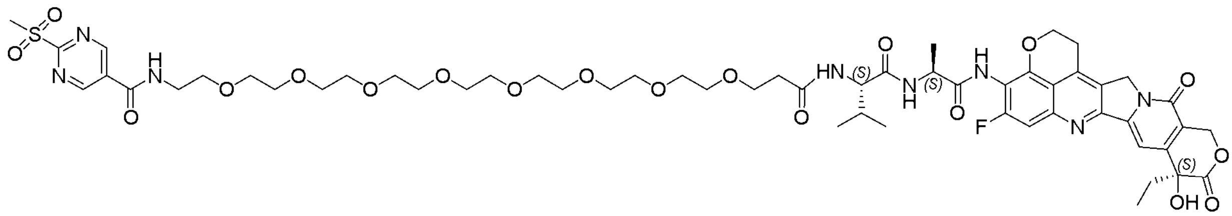

MWS-L3

According to a method similar to that described in Example 45, in which A16 was used instead of A1, MWS-L3 was prepared and obtained as yellow solid. LC-MS (ESI): M+1 =1202.4.

**Example 76** Synthesis of Linker-payload MWS-L4

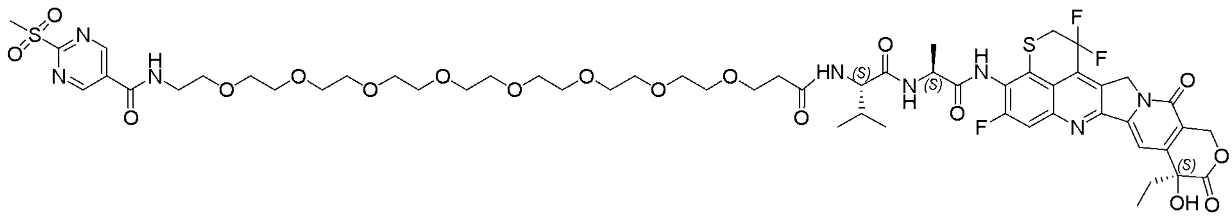

MWS-L4

According to a method similar to that described in Example 45, in which A19 was used instead of A1, MWS-L4 was prepared and obtained as yellow solid. LC-MS (ESI): M+1 =1254.4.

**Example 77** Synthesis of Linker-payload MWS-L5

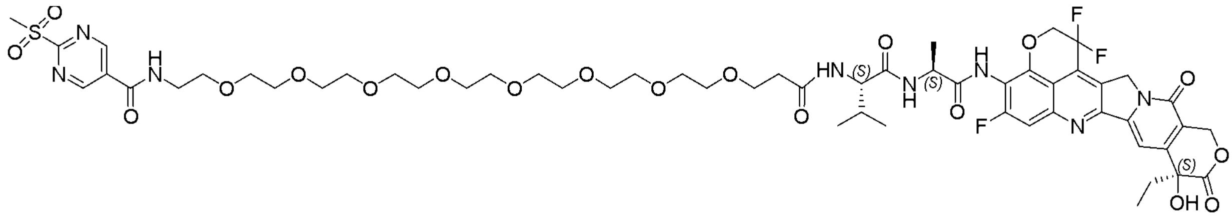

MWS-L5

According to a method similar to that described in Example 45, in which A17 was used instead of A1, MWS-L5 was prepared and obtained as yellow solid. LC-MS (ESI): M+1 =1238.4.

Example 78 Synthesis of Linker-payload MWS-L6

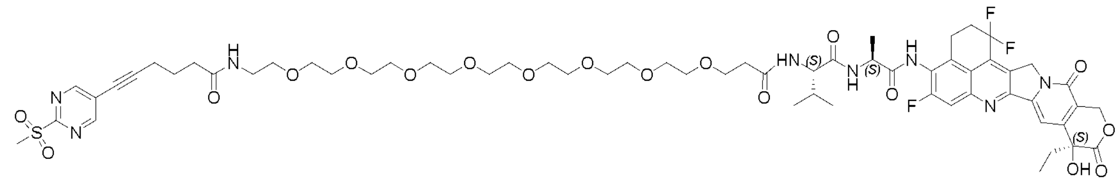

MWS-L6

According to a method similar to that described in Example 54, in which

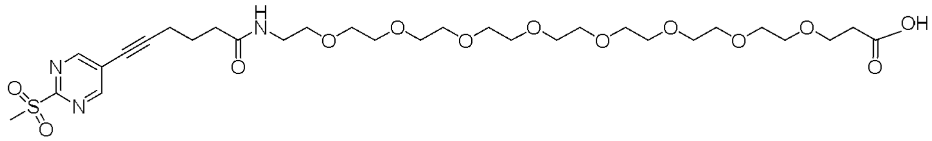

was used instead of Mal-PEG8-COOH, MWS-L6 was prepared and obtained as yellow solid. LC-MS (ESI): M+1= 1302.4.

**Example 79** Synthesis of Linker-payload MWS-L7

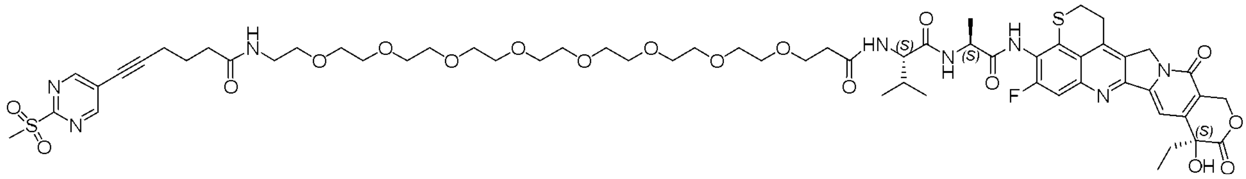

MWS-L7

According to a method similar to that described in Example 45, in which A18 was used instead of A1, MWS-L7 was prepared and obtained as yellow solid. LC-MS (ESI): M+1 =1284.4.

**Example 80** Synthesis of Linker-payload MWS-L8

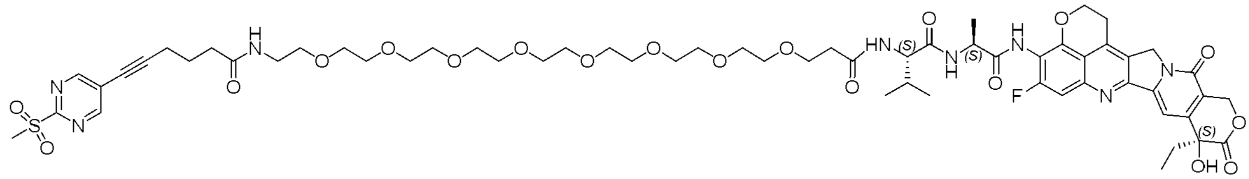

MWS-L8

According to a method similar to that described in Example 45, in which A16 was used instead of A1, MWS-L8 was prepared and obtained as yellow solid. LC-MS (ESI): M+1 =1268.4.

**Example 81** Synthesis of Linker-payload MWS-L9

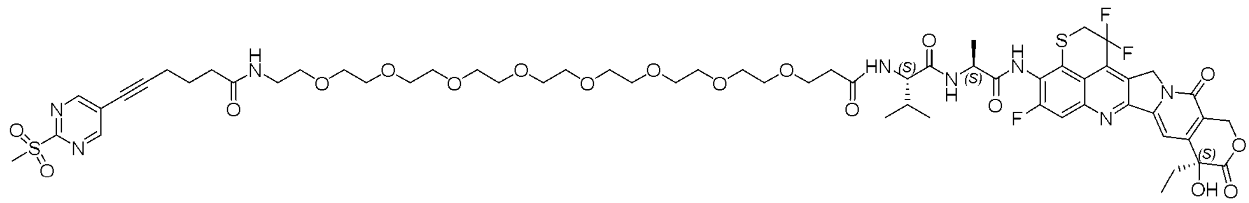

MWS-L9

According to a method similar to that described in Example 45, in which A19 was used instead of A1, MWS-L9 was prepared and obtained as yellow solid. LC-MS (ESI): M+1 =1320.4.

**Example 82** Synthesis of Linker-payload MWS-L10

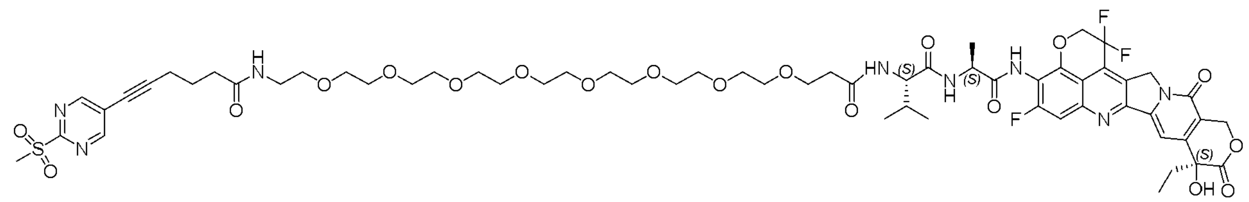

MWS-L10

According to a method similar to that described in Example 45 in which A17 was used instead of A1, MWS-L10 was prepared and obtained as yellow solid. LC-MS (ESI): M+1 =1304.4.

**Group 4 of Examples Synthesis of comparative Linker-payloads**

**Example 83** Synthesis of Linker-payloads L-A, L-B, and L-C

1. Synthesis of Linker-payloads for stochastic conjugating

Linker-payload MC-GGFG-Dxd was synthesized according to the method described in the patent publication US20190151328A1, and MC-GGFG-Dxd was obtained as light yellow solid. LC-MS (ESI): M+1 =1034, $^1$H NMR ($CDCl_3$).

Linker-payloads L-A and L-B were synthesized according to the method described in the patent publication WO2020200880, and L-A was obtained as yellow solid, LC-MS (ESI): M+1 =767; and L-B was obtained as brown sticky matter, LC-MS (ESI): M+1 =1149.

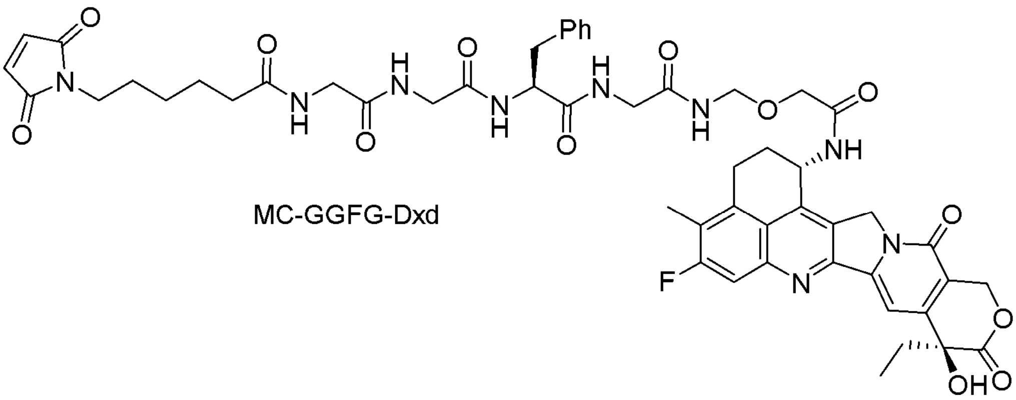

MC-GGFG-Dxd

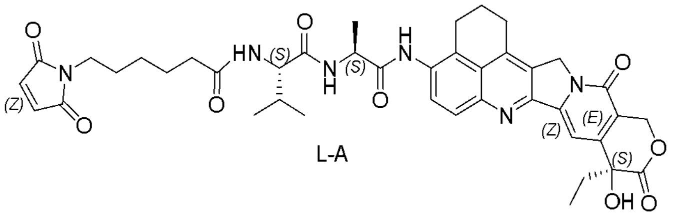

L-A

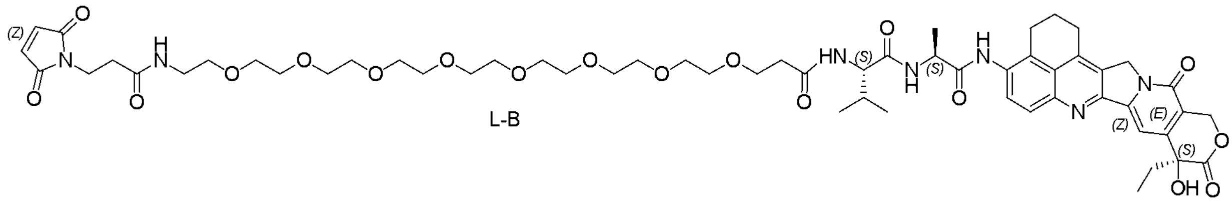

L-B

2. Synthesis of other Linker-payload L-C for ring bridging

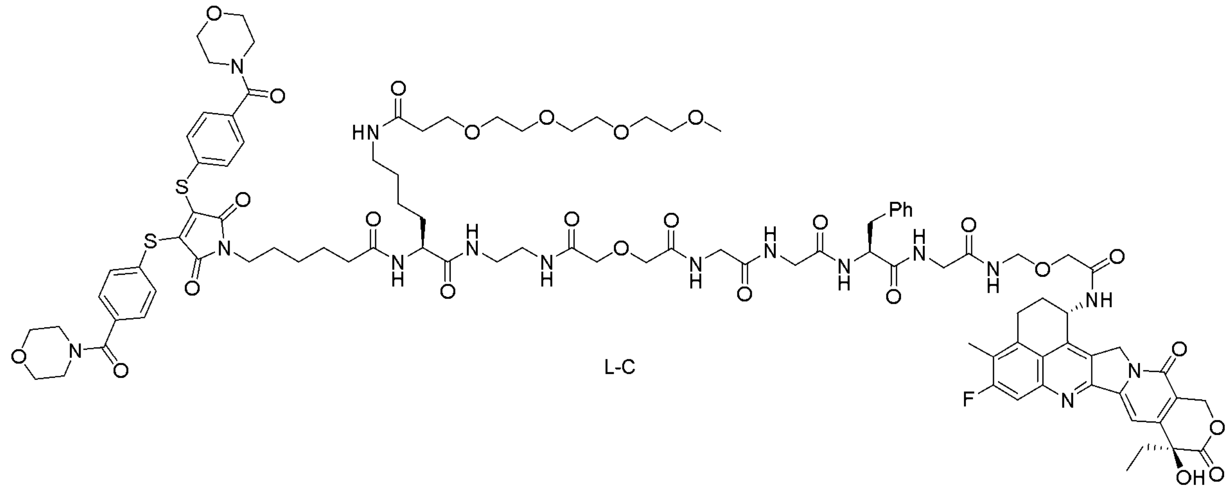

L-C

Synthetic route is as follows:

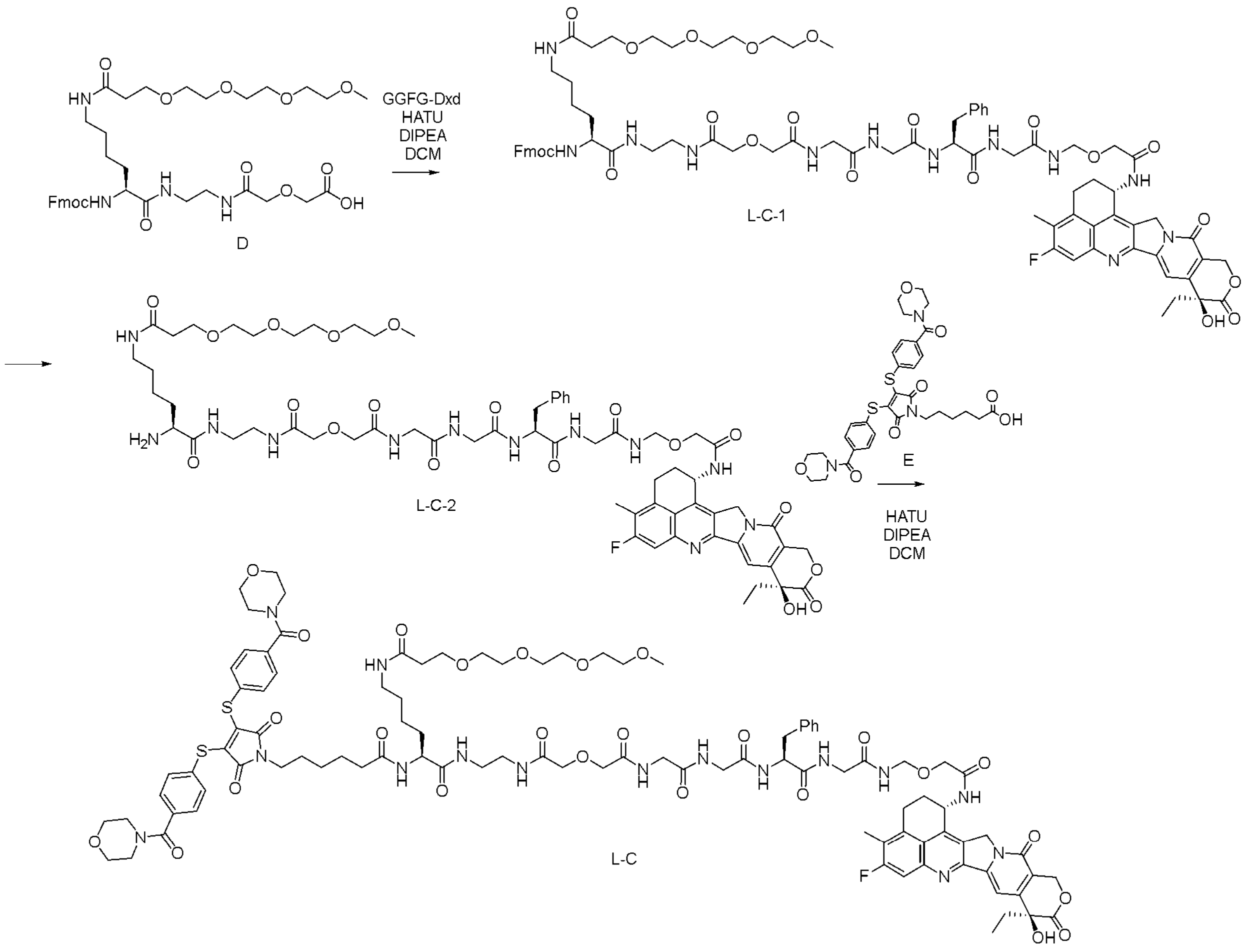


D

L-C-1

L-C-2

L-C

According to a method similar to that for Linker-payload L-1, linker L-C was synthesized as light yellow solid. LC-MS (ESI): M+1 =1982, (M+1)/2=991.

## Group 5 of Examples Preparation and physicochemical characterization of antibody-drug conjugates

### 1. Universal methods for preparing antibody-drug conjugates

**a.** Universal preparation method for site-specific conjugation

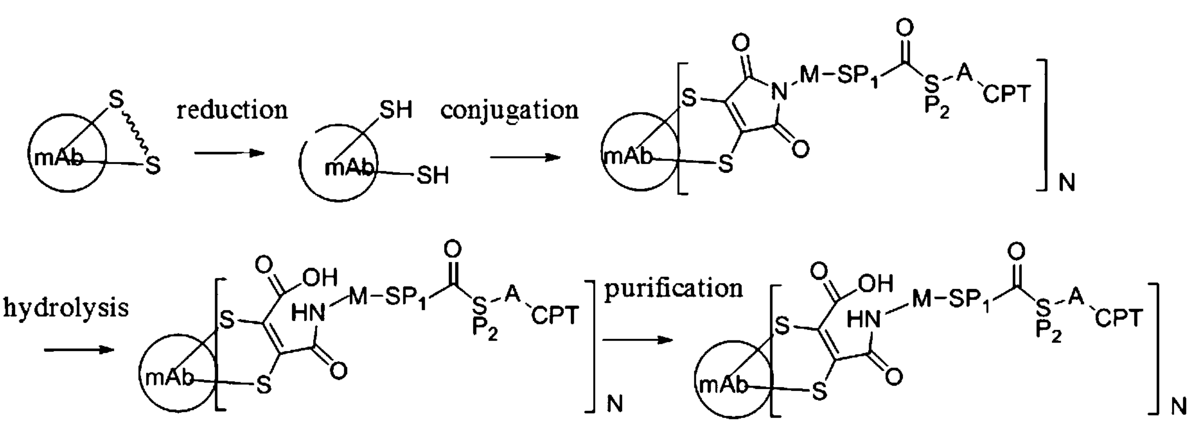


N=1~5

An antibody is reduced to break disulfide bonds, and the reduced antibody is conjugated with a linker to form a bridged antibody-drug conjugate, in which the maleimide ring is then opened via hydrolysis, and the obtained product is purified to provide an antibody-drug conjugate with DAR of 4. The method in detail is as follows:

Antibody reduction: A sample containing 120 mg of an antibody is displaced into a buffer comprising 50 mM sodium chloride and 50 mM sodium dihydrogen phosphate-disodium hydrogen phosphate, pH 7.0, with a NAP-25 chromatographic column packed with Sephadex G-25, in which buffer the antibody concentration is diluted to 10 mg/mL. 2.1 ml of an aqueous solution of TCEP (Sigma-Aldrich) at 10 mg/mL is added into 10 mL of the diluted antibody sample (100 mg in total) at an equivalent molar ratio of 1: 10 (antibody: TCEP). After incubation for 2 hours, the reaction solution is subjected to buffer exchange with a Sephadex G-25 chromatographic column, into a buffer comprising 50 mM NaCl and 50 mM sodium dihydrogen phosphate-disodium hydrogen phosphate, pH 6.5.

Conjugation of antibody and linker-payload and hydrolysis: the reduced antibody as above is diluted to 5 mg/mL, into which 0.38 mL of N,N-Dimethylacetamide (DMA) which accounts for 2% of the total reaction volume as a pre-solvent and a solution of a linker-payload in DMA at 10 mg/mL at an equivalent molar ratio of 1:5.5 (antibody: linker-payload) as a reaction solution are added sequentially. Mixture obtained is stirred at room temperature for 30 minutes, and then is subjected to buffer exchange into a buffer consisting of disodium hydrogen phosphate-sodium dihydrogen phosphate, pH 8.0, with a NAP-25 chromatographic column packed with Sephadex G-25, to remove excessive linker-payload. Solution obtained is heated in water bath at 37 °C for 3 hours.

Purification of antibody-drug conjugate: a sample of the solution as above is concentrated using an AMICOM ultrafiltration centrifuge tube, to a concentration of about 15 mg/mL. A buffer consisting of 50 mM disodium hydrogen phosphate-sodium dihydrogen phosphate and 3 M ammonium phosphate is added to achieve a conductivity of 100 ms/cm. Then the solution is applied to a hydrophobic column packed with TOYOPEAL Butyl-650M (purchased from TOSOH), using phase A: a buffer consisting of 50 mM disodium hydrogen phosphate-sodium dihydrogen phosphate and 0.6 M ammonium sulfate; phase B: a buffer consisting of 50 mM disodium hydrogen phosphate-sodium dihydrogen phosphate. 8 column-volume gradient elution with phase B from 0% to 100% is conducted and main peak is collected.

The final sample obtained is subjected to buffer exchange into a buffer consisting of 50 mM disodium hydrogen phosphate-sodium dihydrogen phosphate, pH 7.4 using an AMICOM ultrafiltration centrifuge tube, which is then filtered through a 0.22 μm filter (Sartorius stedim Ministart).

**b.** Universal preparation method for stochastic conjugation

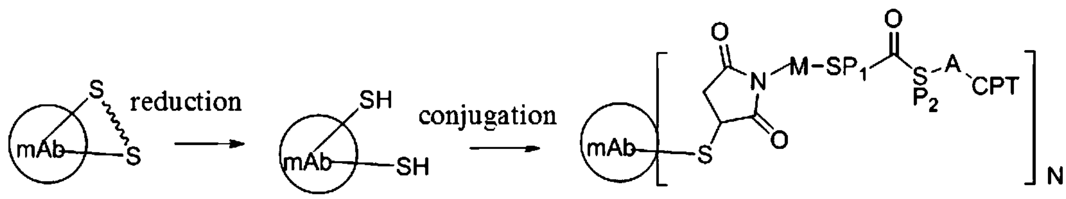


N=1~10

An antibody is reduced to break disulfide bonds, and then is conjugated with a linker to form a bridged antibody-drug conjugate.

Specifically, the antibody-drug conjugates are prepared and obtained according to the preparation method described in the patent publication CN104755494B.

**2. Universal methods for analyzing antibody-drug conjugates**

**a.** Ultraviolet spectrophotometry used to determine drug-to-antibody ratio (UV-DAR method) and concentration

The concentration of an antibody-drug conjugate can be obtained by measuring the UV absorbance at 280 nm and the absorption wavelengths characteristic of small molecules and calculating as follows.

**a1**. Determination of the drug-to-antibody ratio (DAR) values of antibody-drug conjugates

It is known from a literature [Clin Cancer Res. 2004 Oct 15; 10(20): 7063-70] that, $\mathrm{DAR\ (drug\text{-}to\text{-}antibody\ ratio)} = (\varepsilon_{280}^{Ab} - A_{280} / A_z \times \varepsilon_z^{Ab}) / (A_{280} / A_z \times \varepsilon_z^{D} - \varepsilon_{280}^{D})$ , in which $\varepsilon_{280}^{Ab}$

is the molar absorption coefficient of an antibody at 280 nm, $A_{280}$ is the UV absorbance of an antibody-drug conjugate at 280 nm, $A_z$ is the UV absorbance of the antibody-drug conjugate at Z nm which is an absorption wavelength characteristic of the linker-payload in the antibody-drug conjugate, $\varepsilon_z^{Ab}$ is the molar absorption coefficient of the antibody at Z nm which is an absorption wavelength characteristic of the linker-payload, $\varepsilon_z^{D}$ is the molar absorption coefficient of the linker-payload at Z nm, and $\varepsilon_{280}^{D}$ is the molar absorption coefficient of the linker-payload at 280 nm, and in which:

| Linker-payload | $\varepsilon_Z^D$ | $\varepsilon_{280}^D$ |
|---|---|---|
| MWD-L1 | 20146(Z=370nm) | 16171 |
| MWD-L7 | 20146(Z=370nm) | 16171 |
| MWD-L8 | 20146(Z=370nm) | 16171 |
| MWD-L9 | 20146(Z=370nm) | 16171 |
| MWC-L2 | 20196(Z=370nm) | 18643 |
| MWC-L3 | 23501 (Z=360nm) | 21039 |
| MWF-L6 | 16901(Z=385nm) | 18108 |
| MWF-L7 | 16901(Z=385nm) | 18108 |
| MWF-L8 | 16901(Z=385nm) | 18108 |
| MC-GGFG-DXD | 20217(Z=370nm) | 5178 |
| L-A | 21430(Z=370nm) | 7670 |
| L-B | 9635(Z=255nm) | 5534 |
| MWS-L1 | 20822(Z=373nm) | 17178 |
| MWS-L7 | 27723(Z=368nm) | 19508 |
| L-J | 13738(Z=326nm) | 18001 |

**a2.** Determination of the concentration of antibody-drug conjugates

Since the total absorbance at a certain wavelength of a system is equal to the sum of the absorbances of all the absorbent chemical species present in the system (additive nature of the absorbance), it is assumed that if the molar absorption coefficients of the antibody and the linker-payload contained do not change prior to and after the conjugation of the antibody with the linker-payload, the concentration of the antibody-drug conjugate follows the relationship: $A_{280} = \varepsilon_{280}^{ADC} \times C_{ADC} \times L = (\varepsilon_{280}^{D} \times DAR + \varepsilon_{280}^{Ab})\ C_{ADC} \times L$..

Thus, the molar concentration (mol/L) of the antibody-drug conjugate $C_{ADC} = A_{280}\ /\ (\varepsilon_{280}^{Ab} + \varepsilon_{280}^{D} \times DAR)$. Therefore, the concentration (g/L) of the antibody-drug conjugate $C_{ADC} = A_{280}\ /\ (\varepsilon_{280}^{Ab} + \varepsilon_{280}^{D} \times DAR) \times MW_{ADC} = A_{280}\ /\ (\varepsilon_{280}^{Ab} + \varepsilon_{280}^{D} \times DAR) \times (MW_{Ab} + MW_{D} \times DAR)$, in which $MW_{ADC}$ is the molecular weight of the antibody-drug conjugate, $MW_{Ab}$ is the molecular weight of the antibody, and $MW_D$ is the molecular weight of the linker-payload; and the protein concentration can be obtained by putting the DAR value into the equation.

**b.** Hydrophobic chromatography

**b1**. Hydrophobic chromatography (HIC-HPLC) used to determine the DAR values of antibody-drug conjugates

Sample preparation: a sample is diluted to 2.0 mg/mL with mobile phase B, and then is centrifuged at 12000 rpm for 10 min; and supernatant obtained is taken for HPLC analysis.

Chromatographic column: Sepax Proteomix HIC Butyl-NP5, 5 μm, 4.6 mm × 35 mm;

Mobile phase A: 1.5 M $(NH4)_2SO_4$ + 25 mM PB, pH 7.0;
Mobile phase B: 25 mM PB + 20% IPA, pH 7.0;
Flow rate: 0.6 mL/min;
Detection wavelength: 280 nm;
Column temperature: 30 °C;
Loading volume: 10 μL;

Chromatographic gradient used for HIC analysis:

| Time (min) | Mobile phase A (%) | Mobile phase B (%) |
|---|---|---|
| 0 | 100 | 0 |
| 10 | 80 | 20 |
| 20 | 40 | 60 |
| 25 | 0 | 100 |
| 25.1 | 100 | 0 |
| 35 | 100 | 0 |

Equation for DAR calculation:

DAR = Σ (weighted peak area) / 100, i.e., DAR = (D0 peak area ratio × 0 + D1 peak area ratio × 1 + D2 peak area ratio × 2 + D3 peak area ratio × 3 + D4 peak area ratio × 4 + D5 peak area ratio × 5 + D6 peak area ratio × 6 + D7 peak area ratio × 7 + D8 peak area ratio × 8) / 100.

**b2.** Hydrophobic chromatography (HIC-HPLC) used to determine the DAR values of antibody-drug conjugates

Sample preparation: a sample is diluted to 2.0 mg/mL with mobile phase B, and then is centrifuged at 12000 rpm for 10 min; and supernatant obtained is taken for HPLC analysis.

Chromatographic column: TSKgel Butyl-NPR, 2.5 μm, 4.6 mm × 100 mm;
Mobile phase A: 1.2 M $(NH4)_2SO_4$ + 25 mM PB, pH 7.0;
Mobile phase B: 25 mM PB + 20% IPA, pH 7.0;
Flow rate: 0.6 mL/min;
Detection wavelength: 280 nm;
Column temperature: 30 °C;
Loading volume: 10 μL;

Chromatographic gradient used for HIC analysis:

| Time (min) | Mobile phase A (%) | Mobile phase B (%) |
|---|---|---|
| 0 | 100 | 0 |
| 1 | 100 | 0 |
| 20 | 0 | 100 |
| 20.1 | 100 | 0 |
| 30 | 100 | 0 |

Equation for DAR calculation: the same as in **b1**.

**b3.** Hydrophobic chromatography (HIC-HPLC) used to determine the DAR values of antibody-drug conjugates

Sample preparation: a sample is diluted to 2.0 mg/mL with mobile phase B, and then is centrifuged at 12000 rpm for 10 min; and supernatant obtained is taken for HPLC analysis.

Chromatographic column: Sepax Proteomix HIC Butyl-NP5, 5 μm, 4.6 mm × 35 mm;
Mobile phase A: 2.5 M $(NH4)_2SO_4$ + 125 mM PB, pH 7.0;
Mobile phase B: 125 mM PB, pH 7.0;
Mobile phase C: IPA;
Mobile phase D: $H_2O$;
Flow rate: 0.5 mL/min;
Detection wavelength: 280 nm;
Column temperature: 30 °C;
Loading volume: 10 μL;

Chromatographic gradient used for HIC analysis:

| Time (min) | Mobile phase A (%) | Mobile phase B (%) | Mobile phase C (%) | Mobile phase D (%) |
|---|---|---|---|---|
| 0 | 80 | 0 | 0 | 20 |
| 20 | 5 | 45 | 3 | 47 |
| 22 | 0 | 50 | 3 | 47 |
| 22.1 | 80 | 0 | 0 | 20 |
| 30 | 80 | 0 | 0 | 20 |

Equation for DAR calculation: the same as in **b1**.

c. Mass spectrometry (LC-MS) used to determine the DAR values

Sample treatment: an appropriate amount of a sample is placed in an ultrafiltration tube, and is subjected to buffer exchange into a buffer consisting of 50 mM $NH_4HCO_3$ (pH 7.1). Upon supplementing the buffer, the obtained sample is subjected to ultrafiltration centrifugation (13000 g × 5 min). 8 μL of PNGase F enzyme is added into the sample after buffer exchange, which is in turn incubated at 37 °C for 5 h for desugarization. After the incubation, the sample is centrifuged at 12000 rpm for 5 min, and supernatant obtained is added into a sample vial as a test sample for later testing.

Chromatographic column: PolyLC ® PolyHYDROXYETHYLA Column, 300 Å, 5 μm, 2.1 mm × 200 mm;
Mobile phase: 50 mM ammonium acetate, pH 7.0;
Running time: 10 min;
Flow rate: 0.1 mL/min;
Loading volume: 2 μL;
Column temperature: 25 °C;
Detection wavelength: 280 nm;
Ionization mode: ESI positive;
Drying gas temperature: 325 °C;
Drying gas flow rate: 8 L/min;
Atomizer pressure: 20 psig;
Sheath gas temperature: 325 °C;
Sheath gas flow rate: 12 L/min;
Scanning setting: 900-8000 m/z.

d. Size exclusion chromatography (SEC-HPLC) used to determine the molecular size heterogeneity

Sample treatment: a sample is diluted to 1.0 mg/mL with a mobile phase and then is centrifuged at 12000 rpm for 10 min; and supernatant obtained is taken for analysis.

Chromatographic column: TOSOH, TSKgel G3000SWXL, 5 μm, 7.8 mm × 300 mm;

Mobile phase: 100 mM PB +200 mM arginine hydrochloride, 5% isopropanol (pH 6.8);
Flow rate: 0.6 mL/min;
Detection wavelength: 280 nm;
Column temperature: 30 °C;
Loading volume: 20 uL;
Elution time: 20 min;
Elution gradient: isocratic elution.

e. Non-Reducing Capillary Electrophoresis-Sodium Dodecyl Sulfate (NR-CE-SDS) to determine the purity

The determination is conducted according to the method "Monoclonal antibodies - Determination of molecular size variants (CE-SDS)" described in Part IV, General rule 3127 of Chinese Pharmacopoeia.

f. Reverse Phase High Performance Liquid Chromatography (RP-HPLC) used to determine the drug-antibody ratios (DAR values) of antibody-drug conjugates

Analysis is conducted according to the method described in the patent publication US10227417B2.
Equation for DAR calculation:

DAR = 2 × (Σ (weighted peak area of light chain) +Σ (weighted peak area of heavy chain)) / 100, i.e., DAR = 2× (L0 peak area ratio ×0 + L1 peak area ratio X 1 + H0 peak area ratio ×0 + H1 peak area ratio ×1 + H2 peak area ratio ×2 + H3 peak area ratio ×3) / 100.

Example 84 Preparation and characterization of antibody-drug conjugates targeting B7-H3

Anti-B7-H3 antibodies hz10B4m1, hz10B4m2, and hz10B4 (each 100 mg) were conjugated with the Linker-payloads MWD-L1, MWC-L2, MWC-L3, MWF-L6, MWF-L8, and MWF-L11 respectively, according to the method described in this section, and bridged ADCs were obtained.
Anti-B7-H3 antibodies hum30 and hz10B4m1 (each 100 mg) were conjugated with the Linker-payloads MC-GGFG-Dxd, vcMMAE, MWS-L1, MWS-L7, L-J, and L-I respectively, according to the method described in the patent publication CN104755494B, and stochastically conjugated ADCs were obtained.
Concentration, DAR values, and purity of the bridged ADCs were determined using the ultraviolet spectrophotometry described in the section "a. Ultraviolet spectrophotometry used to determine drug-to-antibody ratio (UV-DAR method) and concentration", hydrophobic chromatography described in the section "b. Hydrophobic chromatography", and size exclusion chromatography described in the section "d. Size exclusion chromatography (SEC-HPLC) used to determine the molecular size heterogeneity".
DAR values of ADCs in which Linker-payload MC-GGFG-Dxd was conjugated were determined using the Reverse Phase High Performance Liquid Chromatography described in the section "f. Reverse Phase High Performance Liquid Chromatography (RP-HPLC) used to determine the drug-antibody ratios (DAR values) of antibody-drug conjugates"; DAR values of ADCs in which vcMMAE was conjugated were determined using the hydrophobic chromatography described in the section "b. Hydrophobic chromatography"; and concentration and purity of the non-bridged ADCs were determined using the ultraviolet spectrophotometry described in the section "a. Ultraviolet spectrophotometry used to determine drug-to-antibody ratio (UV-DAR method) and concentration" and size exclusion chromatography described in the section "d. Size exclusion chromatography (SEC-HPLC) used to determine the molecular size heterogeneity".
The results are shown in Table 5.

**Table** 5. Characterization results of antibody-drug conjugates

| ADC # | Name | Antibody | Linker-payload | UV-DAR | Concentration (mg/mL) | SEC |
|---|---|---|---|---|---|---|
| 1a | B7H3-ADC-1 | hz10B4m1 | MWD-L1 | 3.7 | 7.8 | 99.54% |
| 1b | B7H3-ADC-2 | hz10B4m1 | vc-MMAE | 4.8 | 7.4 | 100% |
| 1c | B7H3-ADC-3 | hz10B4m1 | MWF-L8 | 4.0 | 6.1 | 95.05% |
| 1d | B7H3-ADC-4 | hz10B4m1 | MC-GGFG-Dxd | 3.7 | 6.4 | 99.70% |
| 1e* | B7H3-ADC-5 | hz10B4m1 | MC-GGFG-Dxd | 6.9 | 6.5 | 99.58% |
| 1f | B7H3-ADC-6 | hz10B4m1 | MWF-L6 | 3.9 | 10.2 | 98.83% |
| 1g | B7H3-ADC-7 | hz10B4m2 | MWC-L2 | 4.1 | 7.9 | 99.58% |

(continued)

| ADC # | Name | Antibody | Linker-payload | UV-DAR | Concentration (mg/mL) | SEC |
|---|---|---|---|---|---|---|
| 1h | B7H3-ADC-8 | hz10B4 | MWF-L6 | 4.2 | 9.5 | 97.2% |
| 1i | B7H3-ADC-9 (DS-7300) | hum30 | MC-GGFG-Dxd | 3.6 | 6.7 | 98.58% |
| 1j | B7H3-ADC-10 | hz10B4m1 | MWS-L1 | 4.1 | 4.3 | 99.10% |
| 1k | B7H3-ADC-11 | hz10B4m1 | MWS-L7 | 3.7 | 5.4 | 98.91% |
| 11 | B7H3-ADC-12 | hz10B4m1 | MWF-L11 | 3.6 | 6.7 | 99.28% |
| 1m | B7H3-ADC-13 | hz10B4m1 | L-J | 3.7 | 5.5 | 98.98% |
| 1n | B7H3-ADC-14 | hz10B4m1 | L-I | 4.0 | 4.9 | 98.87% |

The results demonstrated that as measured by UV spectroscopy, the DAR values of the prepared low-drug-load ADCs ranged from 3.6 to 4.8, while the DAR value of the high-drug-load ADC (B7H3-ADC-5) was 6.9. SEC results demonstrated that, with the exception of the B7H3-ADC-3 sample (95.05% purity), all other ADC samples exhibited monomer contents above 98%, indicating the high purity of the prepared ADC samples.

## Group 6 of Examples Evaluation of activities

### Example 85 Comparative study on activity of camptothecins

KB oral epithelial cancer cells (purchased from: ATCC) were cultured in DMEM medium supplemented with 10% FBS; NCI-N87 human gastric cancer cells (purchased from: ATCC) were cultured in RPMI1640 medium supplemented with 10% FBS; HT29 human colon cancer cells (purchased from: ATCC) were cultured in RPMI1640 medium supplemented with 10% FBS; and BxPC-3 human pancreatic adenocarcinoma cells (purchased from: ATCC) were cultured in RPMI1640 medium supplemented with 10% FBS.

Using corresponding media described above and in cell plates, the density of KB cells was adjusted to $2\times10^4$/mL; the density of NCI-N87 cells was adjusted to $5\times10^4$/mL; the density of HT29 cells was adjusted to $5\times10^4$/mL, and the cells were plated onto 96-well transparent flat bottom plates, at 100 μL/well, and cultured overnight; and the density of BxPC-3 cells was adjusted to $5\times10^4$/mL.

Samples to be detected were diluted to 20 μM with corresponding media, then 5-fold gradient dilution was performed, and 9 concentrations were obtained. A well having cells but with no any sample added was set as the well showing maximum cell growth; and a well having no cells was set as the well of medium background. The diluted samples were added into the cell plates, at 100 μL/well, which were then incubated in a $CO_2$ incubator for 96 hours. After the incubation, CCK-8 (purchased from: DOJINDO LABORATORIES) prepared beforehand was added into the plates, at 20 μL/well, and after further 3 hours of incubation in the $CO_2$ incubator, OD values at 450 nm were read on a microplate reader.

Data obtained was fitted to a four-parameter curve using software Prism7, and equation for calculating maximum killing is: maximum killing (%) = 1 - (OD450 value of the well showing maximum killing - OD450 value of the well of medium background) / (OD450 value of the well showing maximum cell growth - OD450 value of the cell of medium background) × 100%. The results are shown in Table 6.

**Table 6.** Results of comparative study on the activity of camptothecins

| Cell strain | Sample name | EC50 (nM) | Maximum Killing (%) | Plate no. | Killing days |
|---|---|---|---|---|---|
| HT29 | MWC-1 | 9.894 | 89 | P1 | 4 days |
| | Compound 1 | 43.43 | 91.9 | | |
| | Dxd | 132.8 | 85.4 | | |
| | MWC-1 | 8.678 | 89.9 | P2 | |
| | Compound 3 | 6.045 | 90.6 | | |
| | Dxd | 126.6 | 87.4 | | |

(continued)

| Cell strain | Sample name | EC50 (nM) | Maximum Killing (%) | Plate no. | Killing days |
|---|---|---|---|---|---|
| KB | MWC-1 | 14.83 | 98.6 | P1 | 4 days |
| | Compound 1 | 52.31 | 97.6 | | |
| | Dxd | 135.8 | 94.7 | | |
| | MWC-1 | 15.67 | 98.6 | P2 | |
| | Compound 3 | 7.467 | 95.6 | | |
| | Dxd | 138.5 | 94.9 | | |
| NCI-N87 | MWC-1 | 4.444 | 93.4 | P1 | 4 days |
| | Compound 1 | 21.93 | 92.1 | | |
| | Dxd | 45.33 | 92.8 | | |
| | MWC-1 | 5.217 | 93.3 | P2 | |
| | Compound 3 | 3.371 | 92.1 | | |
| | Dxd | 43.71 | 92.7 | | |
| | Compound 3 | 0.706 | 90.9 | P3 | |
| | Compound 7 | 3.312 | 87.9 | | |
| | Compound 3 | 10.750 | 92.9 | P4 | |
| | Compound 12 | 8.426 | 94.9 | | |
| | Compound 3 | 6.219 | 87.7 | P5 | |
| | Compound 14-P1 | 503.40 | 42.5 | | |
| | Compound 27 | 1.746 | 90.6 | | |
| | Compound 3 | 7.849 | 88.4 | P6 | |
| | Compound 14-P2 | 2001.00 | 68.4 | | |
| | Compound 3 | 10.31 | 92.0 | P7 | |
| | Compound 15 | 178.4 | 83.6 | | |
| | Dxd | 11.930 | 94.6 | P8 | |
| | Compound 17 | 7.429 | 87.6 | | |
| | Compound 3 | 14.510 | 90.2 | P9 | |
| | Compound 18 | 1.574 | 92.9 | | |
| | Compound 26 | 1.021 | 93.4 | | |
| | Dxd | 12.710 | 93.3 | P10 | |
| | Compound 19 | 0.864 | 93.5 | | |
| | Compound 3 | 0.940 | 93.3 | | |
| | Compound 3 | 2.340 | 93.1 | P11 | |
| | Compound 20 | 0.059 | 93.2 | | |
| | Compound 3 | 4.503 | 88.3 | P12 | |
| | Compound 31 | 8.593 | 90.7 | | |

(continued)

| Cell strain | Sample name | EC50 (nM) | Maximum Killing (%) | Plate no. | Killing days |
|---|---|---|---|---|---|
| BxPC-3 | Compound 3 | 2.809 | 98.0 | P1 | 4 days |
| | Compound 7 | 11.36 | 94.4 | | |
| | Compound 3 | 3.531 | 96.6 | P2 | |
| | Compound 12 | 2.148 | 98.5 | | |
| | Compound 3 | 4.197 | 94.7 | P3 | |
| | Compound 14-P1 | 2.17E+19 | 38.8 | | |
| | Compound 27 | 1.524 | 97.7 | | |
| | Compound 3 | 2.39 | 93.1 | P4 | |
| | Compound 14-P2 | 1.96E+32 | 43.3 | | |
| | Compound 3 | 3.234 | 97.7 | P5 | |
| | Compound 15 | 79.640 | 87.9 | | |
| | Dxd | 33.720 | 93.1 | P6 | |
| | Compound 17 | 13.250 | 82.0 | | |
| | Compound 3 | 5.950 | 93.9 | P7 | |
| | Compound 18 | 1.451 | 99.6 | | |
| | Compound 26 | 1.111 | 99.1 | | |
| | Dxd | 24.310 | 91.8 | P8 | |
| | Compound 19 | 1.402 | 97.4 | | |
| | Compound 3 | 2.388 | 95.9 | | |
| | Compound 3 | 3.004 | 99.3 | P9 | |
| | Compound 20 | 0.683 | 99.2 | | |
| | Compound 3 | 5.815 | 93.7 | P10 | |
| | Compound 31 | 4.423 | 93.7 | | |

From Table 6, clearly the novel camptothecins provided by the present disclosure exhibited significantly stronger cell killing activity than the control molecule DXD.

**Example 86 Comparative study on in vitro activity of antibody-drug conjugates targeting B7-H3**

Calu-6 human non-small cell lung cancer cell line with high B7H3 expression (Calu-6-B7H3; generated in-house) was taken from liquid nitrogen, and recovered. Then the density of the cells was adjusted to $5\times10^4$/mL with complete medium, and the cells were added to cell culture plates at 100 μL/well, and cultured overnight.

The ADCs targeting B7-H3 prepared above were diluted respectively to 5 μg/mL using complete medium, followed by 4-fold or 2-fold gradient dilution, and 9 concentrations were obtained. The diluted samples were added into the cell culture plates, and two duplicate wells were set for each of the diluted samples; and a negative control well (cell + medium) was set. Next, the plates were placed in a cell incubator and the cells were incubated for 168 h (7 days). After the incubation, MTS was added at 40 μL/well to the plates which were then placed back into the 37 °C incubator and allowed for reacting for 2-4 h. Then the cell plates were taken out, and OD values at 490 nm were read.

The results are shown in Table 7.

Table 7. Results of the cell killing effect in vitro of ADCs targeting B7-H3

| Plate no. | Sample name | Antibody | Linker-payload | EC50 (ng/mL) | Maximum cell killing effect (%) |
|---|---|---|---|---|---|
| Plate1 (168h) (Calu-6-B7H3:5000 cells/-well) | B7H3-ADC-9 (DS-7300) | hum30 | MC-GGFG-DXD | 137.5 | 102.9 |
| | B7H3-ADC-6 | hz10B4m1 | MWF-L6 | 102.3 | 102 |
| | B7H3-ADC-7 | hz10B4m2 | MWC-L2 | 139.3 | 100.3 |
| Plate2 (168h) (Calu-6-B7H3:5000 cells/-well) | B7H3-ADC-8 | hz10B4 | MWF-L6 | 122.4 | 91.3 |
| | B7H3-ADC-9 (DS-7300) | hum30 | MC-GGFG-DXD | 117.5 | 91.4 |
| Plate3 (168h) (Calu-6-B7H3:5000 cells/-well) | B7H3-ADC-4 | hz10B4m1 | MC-GGFG-Dxd | 96.42 | 98.5 |
| | B7H3-ADC-5 | hz10B4m1 | MC-GGFG-Dxd | 63.06 | 98.4 |
| | B7H3-ADC-6 | hz10B4m1 | MWF-L6 | 97.68 | 96.2 |
| Plate4 (168h) (Calu-6-B7H3:5000 cells/-well) | B7H3-ADC-4 | hz10B4m1 | MC-GGFG-Dxd | 87.13 | 98.3 |
| | B7H3-ADC-1 | hz10B4m1 | MWD-L2 | 117.7 | 97.8 |
| | B7H3-ADC-3 | hz10B4m1 | MWF-L8 | 115.7 | 96.7 |
| Plate5 (168h) (Calu-6-B7H3:5000 | B7H3-ADC-9 (DS-7300) | hum30 | MC-GGFG-DXD | 107.5 | 92.3 |
| cells/well) | B7H3-ADC-11 | hz10B4m1 | MWS-L7 | 82.1 | 82.1 |
| | B7H3-ADC-13 | hz10B4m1 | L-J | 59.7 | 78.9 |
| Plate6 (168h) (Calu-6-B7H3:5000 cells/-well) | B7H3-ADC-9 (DS-7300) | hum30 | MC-GGFG-DXD | 116.2 | 89.9 |
| | B7H3-ADC-10 | hz10B4m1 | MWS-L1 | 150.2 | 89.2 |
| | B7H3-ADC-14 | hz10B4m1 | L-I | 87.1 | 92.4 |

The results of cell killing in vitro of the ADCs targeting B7H3 demonstrated that, B7H3-ADC-7 exhibited 7-day cell-killing activity in vitro comparable to the control ADC (B7H3-ADC-9, i.e., DS-7300) in Calu-6-B7H3 cells; meanwhile, B7H3-ADC-6, B7H3-ADC-11, B7H3-ADC-13, and B7H3-ADC-14 all showed superior 7-day cell-killing activity in vitro compared to the control ADC (DS-7300) in Calu-6-B7H3 cells. Additionally, the high-drug-load ADC (B7H3-ADC-5) demonstrated stronger cell-killing activity in vitro than the low-drug-load ADC (B7H3-ADC-4).

**Example 87 Comparative study on drug potency in vivo of antibody-drug conjugates targeting B7-H3**

Calu-6 human lung cancer cells (purchased from ATCC) were used. Calu-6 cells were cultured in 10-cm culture dishes for adherent culture, in MEM medium containing 10% Fetal Bovine Serum with penicillin and streptomycin supplemented, at 37 °C in an incubator containing 5% $CO_2$. The cells were passaged 2-3 times one week, and when in exponential growth phase, they were digested using trypsin, harvested, counted and inoculated.

A comparison study was performed between groups of different doses of antibody-drug conjugate B7H3-ADC-6. Nude mice subcutaneously transplanted with the Calu-6 human lung cancer cells were administered with the ADC via intravenous injection (IV), once weekly for two doses; the mice in the group of vehicle were administered with the same volume of vehicle (saline). Tumor volumes were measured 2 times per week, and the mice were weighed and the data were recorded.

When the experiment was completed, the experiment achieved the end point (D21), or the tumor volume achieved 2000 $mm^3$, the animals were sacrificed under $CO_2$ anesthesia and the tumors were dissected and photographed. The results are shown in Table 8 and Figure 2.

**Table 8.** Therapeutic effect of B7H3-ADC-6 on subcutaneous tumor in nude mice transplanted with Calu-6 human lung cancer cells (TGI (%) was calculated according to the tumor volumes)

| Group | Mean tumor volume ($mm^3$) | | | Mean tumor volume ($mm^3$) | | | TGI (%) |
|---|---|---|---|---|---|---|---|
| | D0 | | SEM | D21 | | SEM | D21 |
| saline | 115.63 | ± | 1.26 | 2792.41 | ± | 303.45 | - |
| B7H3-ADC-6 1mg/kg | 114.28 | ± | 1.29 | 748.16 | ± | 163.69 | 76.32 |
| B7H3-ADC-6 3mg/kg | 115.61 | ± | 1.79 | 73.65 | ± | 5.83 | 136.29 |
| B7H3-ADC-6 10mg/kg | 115.77 | ± | 0.73 | 67.27 | ± | 1.04 | 141.89 |
| DS-7300 3mg/kg | 116.16 | ± | 0.97 | 246.46 | ± | 54.84 | 95.13 |
| DS-7300 10mg/kg | 115.33 | ± | 1.13 | 73.40 | ± | 1.61 | 136.36 |

The results above show that compared with the group of vehicle, B7H3-ADC-6 exhibited an obvious tumor-inhibiting activity in the transplantation tumor model of Calu-6 human lung cancer; and the tumor-inhibiting activity of B7H3-ADC-6 was superior to that of DS-7300 at the same dose; and further, the tumor-inhibiting activity of B7H3-ADC-6 at 3 mg/kg was comparable to that of DS-7300 at 10 mg/kg.

**Example 88 Comparative study on drug potency in vivo of antibody-drug conjugates (incorporating camptothecins) targeting B7-H3**

Pan 05.04 human pancreatic cancer cells (purchased from ATCC) were used. Pan 05.04 cells were cultured in 10-cm culture dishes for adherent culture, in RPMI 1640 medium containing 10% Fetal Bovine Serum with penicillin and streptomycin supplemented, at 37 °C in an incubator containing 5% $CO_2$. The cells were passaged 2-3 times one week, and when in exponential growth phase, they were digested using trypsin, harvested, counted and inoculated.

A comparison study was performed between groups of different doses of antibody-drug conjugate B7H3-ADC-6. Nude mice subcutaneously transplanted with the Pan 05.04 human pancreatic cancer cells were administered with the ADC via intravenous injection (IV), once weekly for two doses; the mice in the group of vehicle were administered with the same volume of vehicle (saline). Tumor volumes were measured 2 times per week, and the mice were weighed and the data were recorded.

When the experiment was completed, the experiment achieved the end point (D18), or the tumor volume achieved 2000 $mm^3$, the animals were sacrificed under $CO_2$ anesthesia and the tumors were dissected and photographed. The results are shown in Table 9 and Figure 3.

**Table** 9. Therapeutic effect of B7H3-ADC-6 on subcutaneous tumor in nude mice (TGI (%) was calculated according to tumor volumes)

| Group | Mean tumor volume ($mm^3$) | | | Mean tumor volume ($mm^3$) | | | TGI (%) |
|---|---|---|---|---|---|---|---|
| | D0 | | SEM | D18 | | SEM | D18 |
| saline | 123.66 | ± | 3.38 | 2132.10 | ± | 267.63 | - |
| B7H3-ADC-6 1mg/kg | 124.07 | ± | 3.39 | 385.49 | ± | 59.21 | 87 |
| B7H3-ADC-6 3mg/kg | 126.03 | ± | 3.17 | 210.87 | ± | 60.10 | 96 |
| B7H3-ADC-6 10mg/kg | 127.00 | ± | 2.71 | 94.28 | ± | 8.18 | 126 |
| hz10B4m1 10mg/kg + Compound 3 0.11mg/kg | 126.58 | ± | 3.15 | 1280.54 | ± | 274.08 | 43 |
| DS-7300 3mg/kg | 127.30 | ± | 2.89 | 514.33 | ± | 98.28 | 81 |
| DS-7300 10mg/kg | 126.95 | ± | 3.14 | 131.73 | ± | 13.18 | 100 |

The results above show that compared with the group of vehicle, B7H3-ADC-6 exhibited an obvious tumor-inhibiting activity in the transplantation tumor model of Pan 05.04 human pancreatic cancer; and the tumor-inhibiting activity of B7H3-ADC-6 was superior to that of DS-7300 at the same dose; and further, at all three dose levels (1, 3, and 10 mg/kg), B7H3-ADC-6 monotherapy demonstrated stronger tumor-inhibiting activity than the combination treatment of anti-B7-H3 antibody hz10B4m1 and Compound 3 (hz10B4m1 at 10 mg/kg + Compound 3 at 0.11 mg/kg).

**Example 89 Study on DNA damage induced by ADCs targeting B7-H3**

Calu-6 cells were seeded in 6-well plates and treated for 72 hours with the following: B7H3-ADC-6 (0.6572, 6.572, 65.72, 657.2, and 1971.7 nM), DS-7300 (0.6649, 6.649, 66.49, and 664.9 nM), the naked antibody hz10B4m1 (675.4, and 2026.3 nM), and Compound 3 (0.1, and 1 nM) respectively. Subsequently, the cells were collected and lysed using a lysis buffer (0.1 M MES, pH 6.6, 1 mM $MgCl_2$, 1 mM EGTA, 0.5% Triton X-100, 10% glycerol). The lysates were centrifuged at 12,000 rpm for 5 minutes, and then the supernatant was removed, and the cell pellets were resuspended in 1× SDS sample buffer (50 mM Tris, pH 6.8, 100 mM DTT, 2% SDS, 0.1% bromophenol blue, 10% glycerol). The suspensions obtained were denatured in a boiling water bath for 10 minutes and then subjected to Western blot analysis to detect γ-H2AX levels in the cells.

The results indicated that B7H3-ADC-6, DS-7300, and Compound 3 all induced DNA damage in the Calu-6 cells, whereas hz10B4m1 showed no significant DNA damage-inducing effect. The results are shown in Figure 4.

**Example 90** Study on the proliferation-inhibiting effect of ADCs targeting B7-H3 on different tumor cells

For cells growing by adherence, SRB assay was used to detect effect of antibody-drug conjugates, antibodies, or camptothecins on the proliferation of tumor cells in vitro cultured. A certain number of cells in exponential growth phase were inoculated in 96-well cell culture plates, and when the cells grew by adherence overnight, antibody drug conjugates, antibodies, or camptothecins of different concentrations were added into the cells. 144 hours of incubation later, the cells were fixed with trichloroacetic acid, and then stained with SRB (prepared in 1% glacial acetic acid at a concentration of 4 mg/mL). 10 mM Tris solution was added into each well to solubilize the bound SRB. OD values at 510 nm were read on a microplate reader.

Inhibition (%) = (OD value of control well - OD value of dosed well)/OD value of control well ×100%

For cells growing in suspension, MTT assay was used to detect effect of antibody-drug conjugates, antibodies, or camptothecins on the proliferation of tumor cells in vitro cultured. A certain number of cells in exponential growth phase were inoculated in 96-well cell culture plates, and when the cells grew in suspension overnight, antibody-drug conjugates, antibodies, or camptothecins of different concentrations were added into the cells. 144 hours of incubation later, MTT was added to each well of the plates, and the incubation was continued for 4 hours at 37 °C in a 5% $CO_2$ saturated humidity incubator. Next, 100 μL of SDS-isobutanol-HCl solution was added into each well of the plates, and OD values at 570 nm and 690 nm were read on a microplate reader.

Half maximal inhibitory concentration ($IC_{50}$) was calculated from the inhibition (%) at those different concentrations using the software Graphpad Prism 8.0. The experiment was repeated once and the data was expressed as mean ± SD, and a plot of log(concentration) versus inhibition was generated. The results are shown in Table 10.

**Table 10.** Results of the proliferation-inhibiting effect of ADCs targeting B7-H3 on different tumor cells

| Cells | $IC_{50}$ (nM, mean ± SD) | | |
|---|---|---|---|
| | B7H3-ADC-6 | hz10B4m1 | Compound 3 |
| BxPC-3 | 783.7 ± 69.6 | > 2026.3 | 0.26 ± 0.09 |
| Calu-3 | 1125.5 ± 161.9 | > 2026.3 | 0.44 ± 0.10 |
| ES-2 | 483.8 ± 84 | > 2026.3 | 0.21 ± 0.08 |
| HT-29 | > 1971.7 | > 2026.3 | 0.62 ± 0.00 |
| HCT-116 | 1135.5 ± 41.7 | > 2026.3 | 0.30 ± 0.00 |
| MDA-MB-231 | > 1971.7 | > 2026.3 | 0.59 ± 0.03 |
| MDA-MB-468 | 1300 ± 0 | > 2026.3 | 0.21 ± 0.11 |
| NCI-N87 | > 1971.7 | > 2026.3 | 1.26 ± 0.13 |
| PANC 05.04 | 654.5 ± 163.2 | > 2026.3 | 0.35 ± **0.19** |
| SK-OV-3 | > 1971.7 | > 2026.3 | 1.05 ± 0.48 |
| Calu-6 | 501.5 ± 74.2 | > 2026.3 | 0.25 ± 0.06 |
| PANC-1 | 1441.5 ± 30.4 | > 2026.3 | 0.82 ± 0.16 |

(continued)

| Cells | $IC_{50}$ (nM, mean ± SD) | | |
|---|---|---|---|
| | B7H3-ADC-6 | hz10B4m1 | Compound 3 |
| U-87 MG | > 1971.7 | > 2026.3 | 3.72 ± 0.01 |
| MC38 | > 1971.7 | > 2026.3 | 3.27 ± 0.75 |
| MC38/B7H3 | > 1971.7 | > 2026.3 | 4.67 ± 1.98 |
| SW626 | > 1971.7 | > 2026.3 | 3.08 ± 0.63 |

As demonstrated by the above study, B7H3-ADC-6 exhibited significant proliferation-inhibiting effects on BxPC-3, ES-2, PANC05.04, Calu-6, Calu-3, HCT-116, MDA-MB-468, and PANC-1 tumor cells, which were superior to that of the anti-B7-H3 antibody hz10B4m1.

**Example 91 Study on the bystander killing effect of ADCs targeting B7-H3**

B7-H3-positive Calu-6 cells (purchased from ATCC) and B7-H3-negative MV-4-11/luc cells (MV-4-11 cells transfected with luciferase, purchased from Nanjing Cobrier Bioscience Co., Ltd.) were co-seeded at a 1:1 ratio in 96-well plates. The B7-H3-negative MV-4-11/luc cells were seeded alone in 96-well plates as a control. Subsequently, B7H3-ADC-6 (1000, 3000, 10000, 30000, and 100000 ng/mL) was added to the wells and incubated for 144 hours. After incubation, the plates were centrifuged, the supernatant was removed, and the cells were lysed with a lysis buffer. Following another centrifugation step, the supernatant was collected, mixed with the substrate luciferin, and the luminescence intensity was measured using a microplate reader.

Inhibition (%) = (OD value of control well - OD value of dosed well) / OD value of control well × 100%

As demonstrated by the above study, B7H3-ADC-6 exhibited a potent bystander effect: it killed B7-H3-negative MV-4-11/luc cells while simultaneously killing B7-H3-positive Calu-6 cells; and in contrast, it showed no obvious proliferation-inhibiting effect on MV-4-11/luc cells cultured alone. The results are shown in Figure 5.

**Example 92 Study on the cell cycle arrest induced by ADCs targeting B7-H3**

Calu-6 cells (purchased from ATCC) were seeded in 6-well plates and treated for 48 hours with the following: B7H3-ADC-6 (0.6752, 6.752, 67.52, 675.2, and 1971.7 nM), the naked antibody hz10B4m1 (675.4, and 2026.3 nM), and Compound 3 (0.01, 0.1, and 1 nM) respectively. Subsequently, the cells were collected and fixed with ethanol overnight. Next day, RNase and propidium iodide (PI) were added to the cells, which were mixed thoroughly and stained in the dark at 37 °C for 30 minutes. The DNA content of the cells was then analyzed using a flow cytometer (BD FACS CALIBUR 4), with a sample size of $1 \times 10^4$ cells per group. Untreated Calu-6 cells cultured alone served as the control. Experimental data were analyzed using ModFit LT for Mac V3.0.

As demonstrated by the above study, both B7H3-ADC-6 and Compound 3 obviously induced G2/M phase arrest in Calu-6 cells, whereas the naked anti-B7-H3 antibody hz10B4m1 showed no notable cell cycle arrest effect. Furthermore, the results indicated that the cell cycle arrest induced by B7H3-ADC-6 occurred specifically at the G2/M phase, which was consistent with the effect of Compound 3. The results are shown in Figure 6.

The above description of the embodiments of the invention is not intended to limit the present invention, and those skilled in the art may make various changes and modifications to the invention without departing from the spirit of the invention, which should fall within the scope of the appended claims.

## Claims

1. An antibody-drug conjugate or a salt thereof having a structure represented by structural formulae Ia and/or Ib:

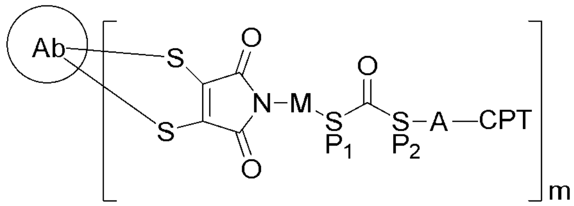

Ia

and/or

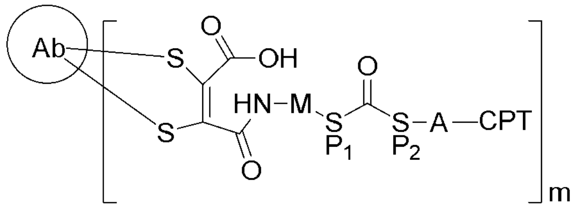

Ib,

wherein,

Ab is an anti-B7-H3 antibody or fragment thereof;
group M is phenylene or phenylene substituted with one or more substituents, or a chemical bond; and in the substituted phenylene, the phenylene is substituted with substituent(s) selected from the group consisting of alkyl (e.g., C1-6 alkyl, preferably C1-4 alkyl), haloalkyl (e.g., C1-6 haloalkyl, preferably C1-4 haloalkyl, e.g., trifluoromethyl), alkoxy (e.g., C1-6 alkoxy, preferably C1-4 alkoxy, preferably methoxy), halogen, ester, amide, and cyano;
group $SP_1$ is selected from the group consisting of C1-8 alkylene, C1-8 cycloalkylene, and C1-21 (preferably C1-16, more preferably C1-11, more preferably C5-9) linear heteroalkylene comprising 1-11 (preferably 1-6, more preferably 3-5) heteroatoms selected from the group consisting of N, O, and S, in which the C1-8 alkylene, C1-8 cycloalkylene, and C1-21 linear heteroalkylene independently of one another are optionally substituted with one or more substituents selected from the group consisting of hydroxyl, amino, sulfonic acid group, and cyano;
group $SP_2$ is selected from the group consisting of $-NH(CH_2CH_2O)_aCH_2CH_2CO-$, $-NH(CH_2CH_2O)_aCH_2CO-$, $-S(CH_2)_aCO-$, and a chemical bond, in which a is an integer in the range of 1 to 20;
group A means a peptide group of 2 to 4 amino acids;
m is in the range of 1 to 10, preferably of 1 to 8 (e.g., 1 to 5), more preferably of 3 to 8; and group CPT refers to a compound of camptothecins.

**2.** The antibody-drug conjugate or a salt thereof according to claim 1, **characterized in that** the anti-B7-H3 antibody or fragment thereof comprises a combination of heavy chain CDRs and light chain CDRs (CDR-H1, CDR-H2, and CDR-H3; and CDR-L1, CDR-L2, and CDR-L3) selected from the group consisting of:

(i) The CDR-H1, CDR-H2, and CDR-H3 respectively comprise amino acid sequences having at least 75% identity to the amino acid sequences shown in SEQ ID NO: 8, SEQ ID NO: 9, and SEQ ID NO: 10; and the CDR-L1, CDR-L2, and CDR-L3 respectively comprise amino acid sequences having at least 75% identity to the amino acid sequences shown in SEQ ID NO: 11, SEQ ID NO: 12, and SEQ ID NO: 14;
(ii) The CDR-H1, CDR-H2, and CDR-H3 respectively comprise amino acid sequences having at least 75% identity to the amino acid sequences shown in SEQ ID NO: 8, SEQ ID NO: 9, and SEQ ID NO: 10; and the CDR-L1, CDR-L2, and CDR-L3 respectively comprise amino acid sequences having at least 75% identity to the amino acid sequences shown in SEQ ID NO: 11, SEQ ID NO: 12, and SEQ ID NO: 15; and
(iii) The CDR-H1, CDR-H2, and CDR-H3 respectively comprise amino acid sequences having at least 75% identity to the amino acid sequences shown in SEQ ID NO: 8, SEQ ID NO: 9, and SEQ ID NO: 10; and the CDR-L1, CDR-L2, and CDR-L3 respectively comprise amino acid sequences having at least 75% identity to the amino acid sequences shown in SEQ ID NO: 11, SEQ ID NO: 12, and SEQ ID NO: 16;

preferably, the anti-B7-H3 antibody or fragment thereof comprises at least a heavy chain variable region (VH) and a light chain variable region (VL);
preferably, the heavy chain variable region and the light chain variable region comprise a sequence combination selected from the group consisting of:

(i) The heavy chain variable region comprises an amino acid sequence having at least 75% identity to the amino acid sequence shown in SEQ ID NO: 1; and the light chain variable region comprises an amino acid sequence having at least 75% identity to the amino acid sequence shown in SEQ ID NO: 3;
(ii) The heavy chain variable region comprises an amino acid sequence having at least 75% identity to the amino acid sequence shown in SEQ ID NO: 1; and the light chain variable region comprises an amino acid sequence having at least 75% identity to the amino acid sequence shown in SEQ ID NO: 4; and
(iii) The heavy chain variable region comprises an amino acid sequence having at least 75% identity to the amino acid sequence shown in SEQ ID NO: 1; and the light chain variable region comprises an amino acid sequence having at least 75% identity to the amino acid sequence shown in SEQ ID NO: 5.

**3.** The antibody-drug conjugate or a salt thereof according to claim 1 or 2, **characterized in that** the anti-B7-H3 antibody or fragment thereof further comprises a constant region, such as a heavy chain constant region (CH) and/or a light chain constant region (CL), or any one or more domains of the constant regions;
alternatively, the anti-B7-H3 antibody or fragment thereof comprises a heavy chain and/or a light chain, wherein for example, the heavy chain comprises a heavy chain constant region of IgG, IgA, IgM, IgD, or IgE, and the light chain comprises a light chain constant region of kappa or lambda type.

**4.** The antibody-drug conjugate or a salt thereof according to any one of claims 1 to 3, **characterized in that** in structural formulae Ia and/or Ib, group M is halogen-substituted phenylene, preferably fluorine-substituted phenylene;

preferably, group $SP_1$ is C1-11, preferably C5-9, more preferably C7 linear heteroalkylene containing 1-6, preferably 3-5, more preferably 4 heteroatoms selected from the group consisting of N, O, and S; and
preferably, group $SP_2$ is preferably a chemical bond.

**5.** The antibody-drug conjugate or a salt thereof according to any one of claims 1 to 4, **characterized in that** group CPT has a structure represented by structural formula I:

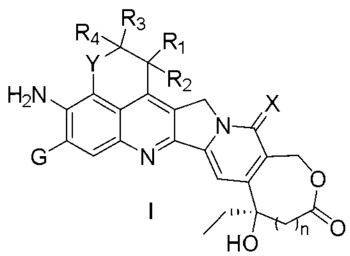

structural formula I,

wherein structural formula I is bonded to the carboxyl of group A in structural formulae Ia and/or Ib via an amide bond; and preferably, the amino adjacent to group G in structural formula I is bonded to the carboxyl of group A in structural formulae Ia and/or Ib via an amide bond;
wherein,
groups $R_1$, $R_2$, $R_3$, and $R_4$ independently of one another are selected from the group consisting of hydrogen, halogen, hydroxyl, C1-6 alkoxy, amino or substituted amino, and C1-7 alkyl or substituted C1-7 alkyl, or any two of groups $R_1$, $R_2$, $R_3$, and $R_4$, together with the carbon atoms to which they are bonded, form a C3-6 (preferably C3-5) cycloalkyl;
group G is hydrogen, halogen, methyl or methoxy;
group Y is oxygen, sulfur, sulfone, sulfoxide, methylene, or substituted methylene;
group X is oxygen or sulfur; and
n = 0 or 1.

**6.** The antibody-drug conjugate or a salt thereof according to any one of claims 1 to 5, **characterized in that** group CPT

has a structure represented by structural formula IA:

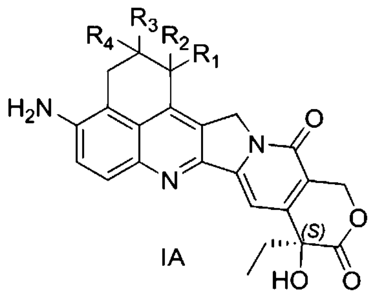

structural formula IA,

wherein structural formula IA is bonded to the carboxyl of group A in structural formulae Ia and/or Ib via an amide bond; and preferably, the amino on the left-side benzene ring in structural formula IA is bonded to the carboxyl of group A in structural formulae Ia and/or Ib via an amide bond;
wherein groups $R_1$, $R_2$, $R_3$, and $R_4$ have the same meaning as defined in claim 5 for the groups $R_1$, $R_2$, $R_3$, and $R_4$ in structural formula I, but groups $R_1$, $R_2$, $R_3$, and $R_4$ are not hydrogen simultaneously.

7. The antibody-drug conjugate or a salt thereof according to any one of claims 1 to 4, **characterized in that** group CPT has a structure represented by structural formula II:

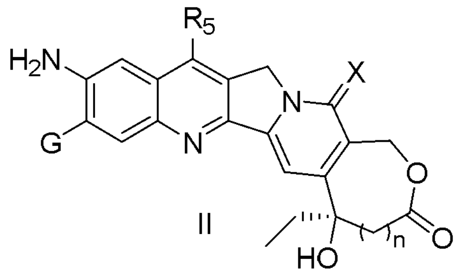

structural formula II,

wherein structural formula II is bonded to the carboxyl of group A in structural formulae Ia and/or Ib via an amide bond; and preferably, the amino adjacent to group G in structural formula II is bonded to the carboxyl of group A in structural formulae Ia and/or Ib via an amide bond;
wherein, group $R_5$ is C1-5 alkyl or C1-5 alkyl substituted with one or more substituents, C3-6 cycloalkyl or C3-6 cycloalkyl substituted with one or more substituents, phenyl or substituted phenyl;
group G is hydrogen, halogen (e.g., fluorine), methyl, or methoxy;
group X is oxygen or sulfur; and
n = 0 or 1.

8. The antibody-drug conjugate or a salt thereof according to claim 7, **characterized in that** group CPT has a structure represented by structural formula IIA:

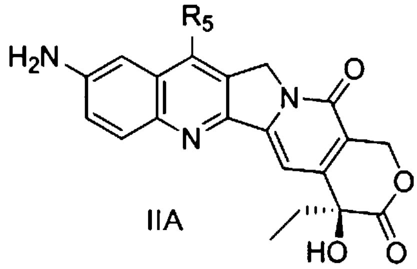

IIA

structural formula IIA,

wherein structural formula IIA is bonded to the carboxyl of group A in structural formulae Ia and/or Ib via an amide bond; and preferably, the amino on the left-side benzene ring in structural formula IIA is bonded to the carboxyl of group A in structural formulae Ia and/or Ib via an amide bond;
wherein group $R_5$ has the same meaning as defined in claim 7 for the group $R_5$ in structural formula II, but group $R_5$ is not n-butyl.

**9.** The antibody-drug conjugate or a salt thereof according to any one of claims 1 to 8, **characterized in that** group CPT has a structure shown below:

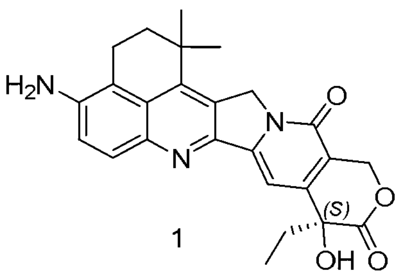

1

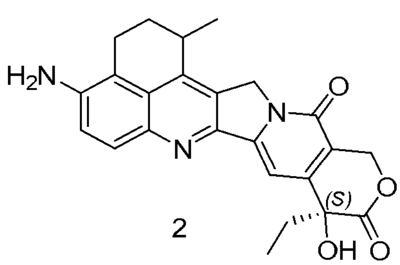

2

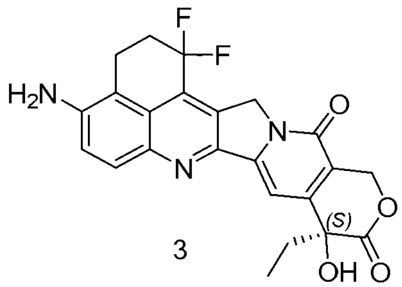

3

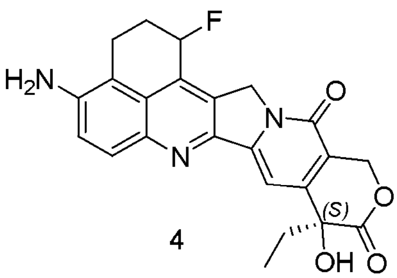

4

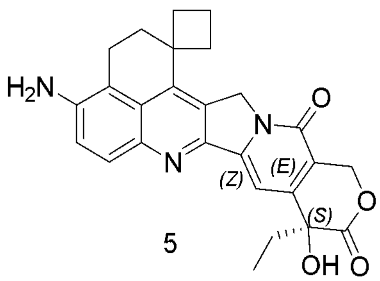

5

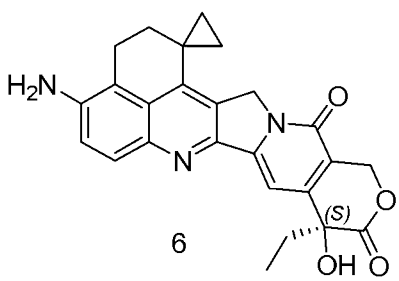

6

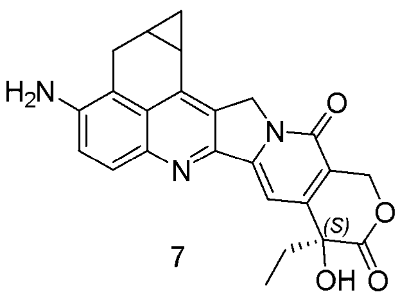

7

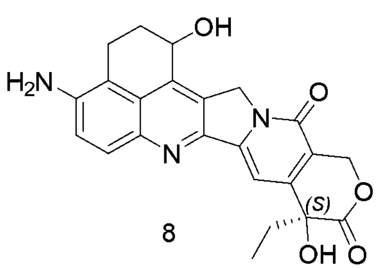

8

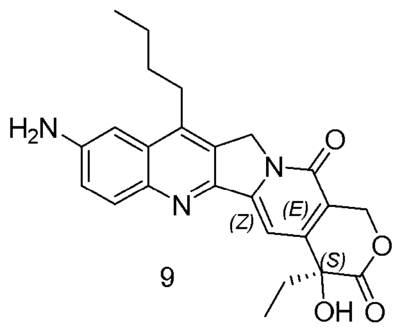

9

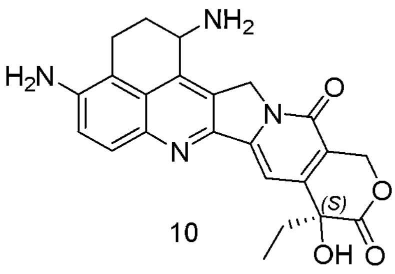
10

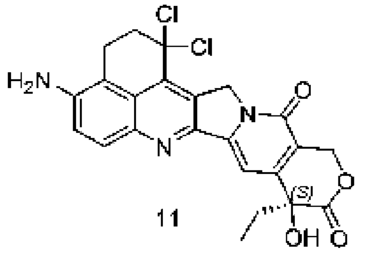
11

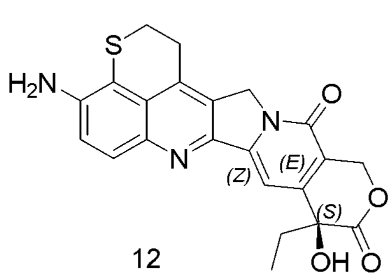
12

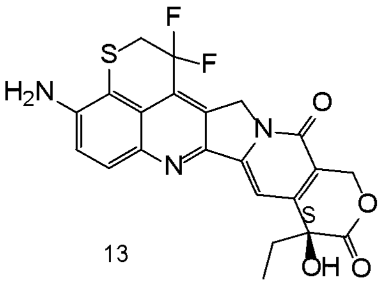
13

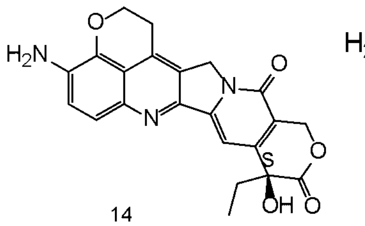
14

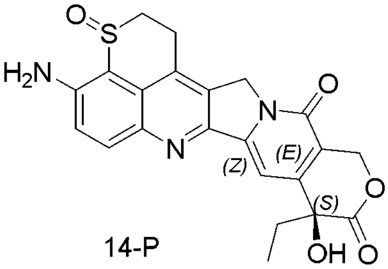
14-P

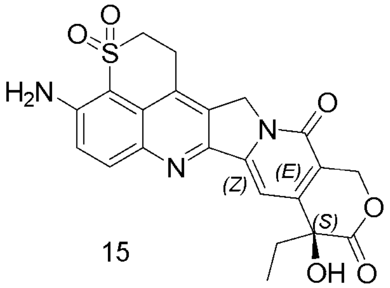
15

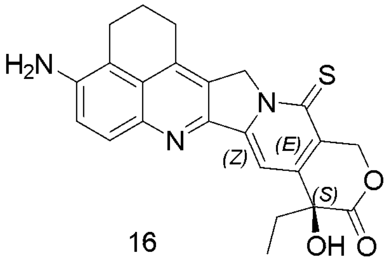
16

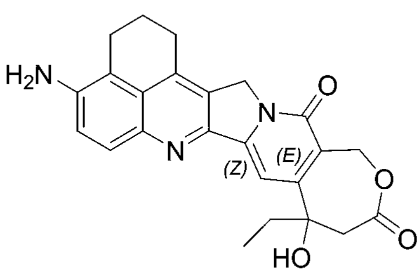
17

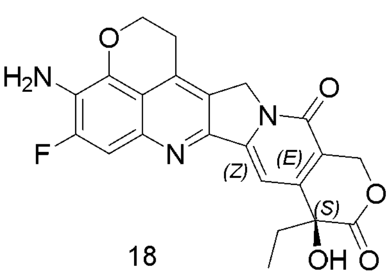
18

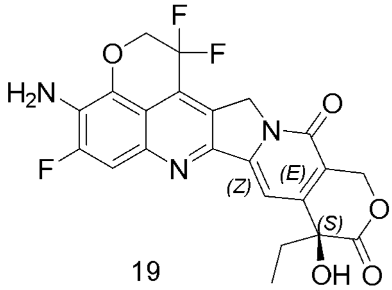
19

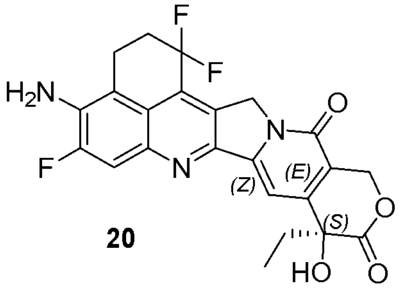
**20**

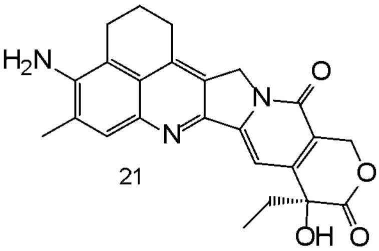
21

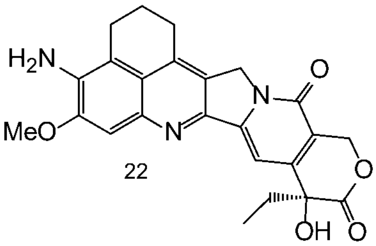
22

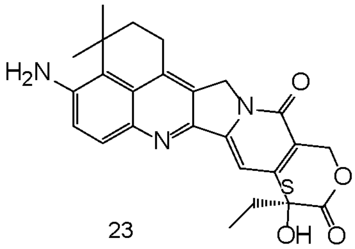
23

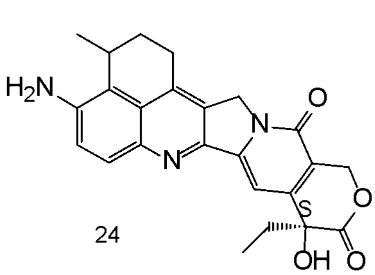
24

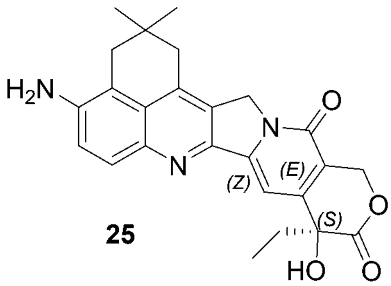
**25**

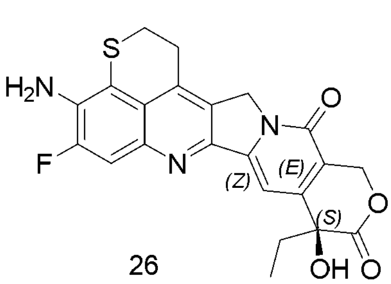
26

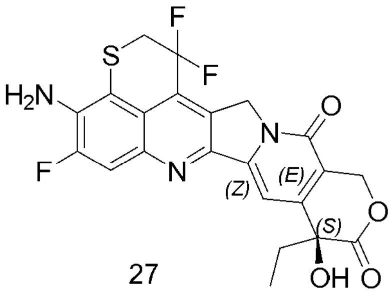
27

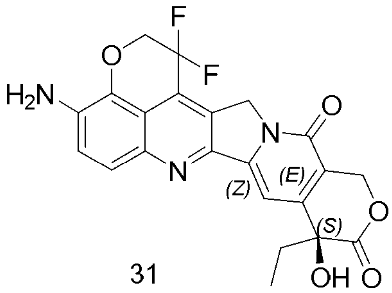
31

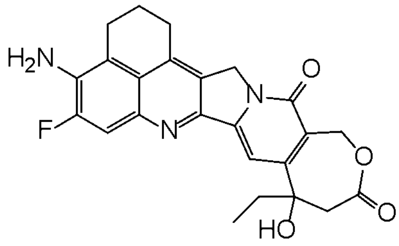
32

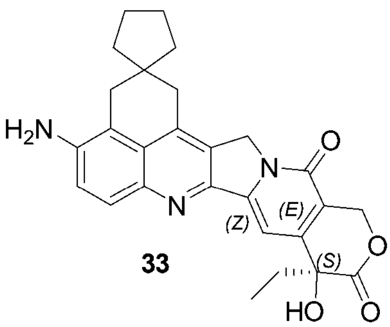
**33**

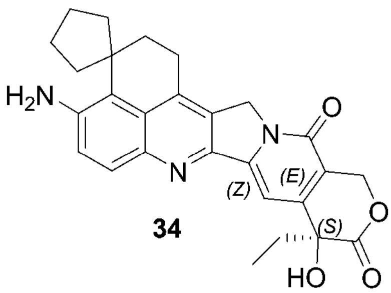
**34**

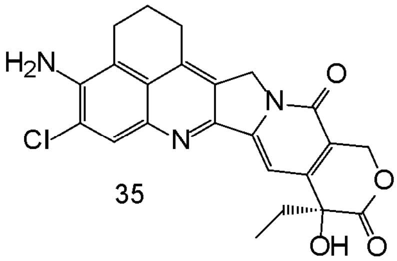
35

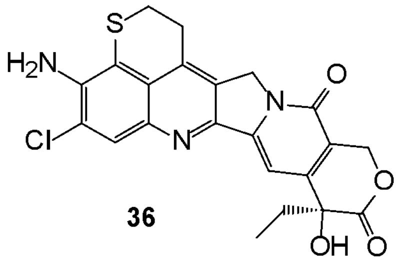
**36**

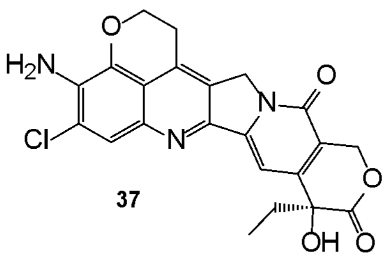
**37**

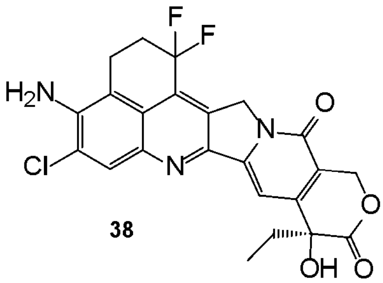
**38**

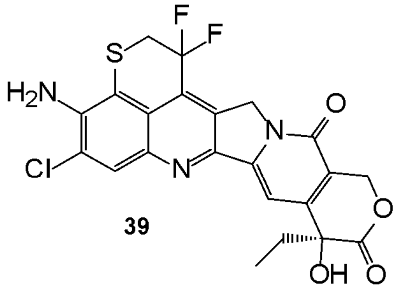
**39**

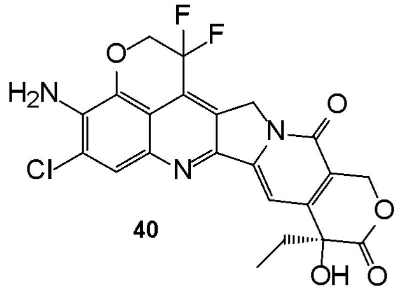

40

,

wherein each structure is bonded to the carboxyl of group A in structural formulae Ia and/or Ib via an amide bond; and preferably, the amino on the left-side benzene ring in each structure is bonded to the carboxyl of group A in structural formulae Ia and/or Ib via an amide bond.

**10.** The antibody-drug conjugate or a salt thereof according to any one of claims 1 to 4, **characterized in that** group CPT has a structure represented by structural formula IV:

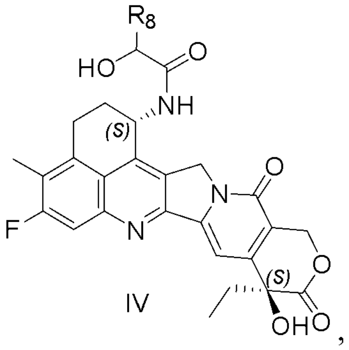

IV ,

structural formula IV,

wherein the hydroxyl bonded to the same carbon as group R8 in structural formula IV is bonded to the carboxyl of group A in structural formulae Ia and/or Ib via a self-immolative structure; and the self-immolative structure is, e.g.

( 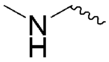 or 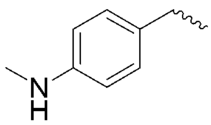 ),

in which the solid line "-" means bonding to the carboxyl of group A in structural formulae Ia and/or Ib, and the wavy line "~~~" means bonding to the hydroxyl in the structural formula IV;
wherein, group $R_8$ is hydrogen, trifluoromethyl, C1-5 alkyl or C1-5 alkyl substituted with one or more substituents, C3-6 cycloalkyl, or C3-6 cycloalkyl substituted with one or more substituents, or halogen.

**11.** An antibody-drug conjugate or a salt thereof, having a structure represented by general formula

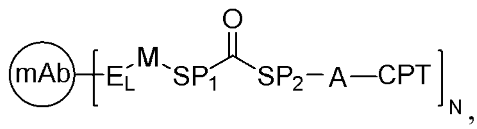

,

in the general formula, mAb is an anti-B7-H3 antibody or fragment thereof;
groups M, $SP_1$, $SP_2$, A, and CPT have the same meaning as defined in any one of claims 1 to 10 for the groups M, $SP_1$, $SP_2$, A, and CPT;
N is in the range of 1 to 10, preferable of 1 to 8 (e.g., 1 to 5), more preferably of 3 to 8; and

$E_L$ is selected from the group consisting of following groups:
$E_L$-1a and/or $E_L$-1b:

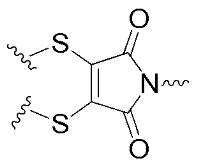 and/or 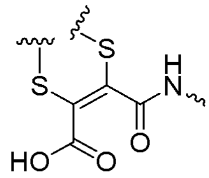 ; $E_L$-2: 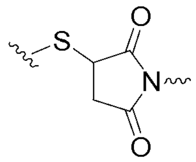 ; $E_L$-3:

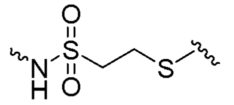 ; $E_L$-4: 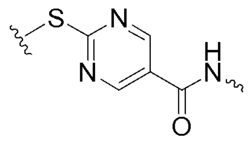 ; $E_L$-5: 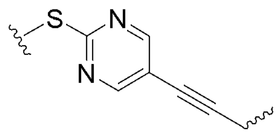 ; $E_L$-6:

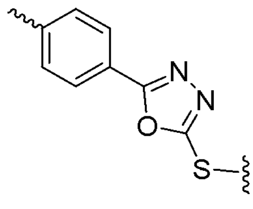,

wherein,

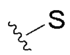

means bonding to the cysteine in mAb, and the wavy line

“ 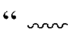 ”

means bonding to group M.

**12.** A method for preparing the antibody-drug conjugate or a salt thereof according to any one of claims 1 to 11, comprising the following steps:

a. Antibody reduction: adding a reducing agent to a phosphate buffer containing an antibody in a concentration of 5-30 mg/mL at an equivalent molar ratio of ≥ 5.5: 1 (the reducing agent: the antibody), and reacting the reducing agent and the antibody for 1.5-2 hours, wherein the reducing agent is one or more selected from the group consisting of TCEP, DTT, 2-MEA, and DTBA;
b. Antibody conjugation and hydrolysis: displacing the reduced antibody obtained in step a into a phosphate buffer at pH 6.5-7.8, thereby diluting the antibody to a concentration of 3.5-15 mg/mL in the buffer to obtain a diluted antibody solution; adding a linker-payload dissolved in an organic co-solvent to the diluted antibody solution at an equivalent molar ratio of 4.5-6.5: 1 (the linker-payload: the antibody), and then reacting the reaction system under stirring at 15-35 °C for ≥ 0.5 hours, wherein the organic co-solvent is one or more selected from the group consisting of DMA, DMSO, DMF, and ACN; and then, displacing the antibody conjugation product into a phosphate buffer at pH 7.4-9.0, and heating the buffer at 35+10 °C for 2-24 hours, to obtain a hydrolysis product;
c. Hydrophobic chromatography: purifying the antibody conjugation product obtained through hydrophobic chromatography using hydrophobic filler.

**13.** The method according to claim 12, **characterized by** that in step a, the antibody is an antibody against tumor-associated antigen which is, e.g., HER2, B7-H3, HER3, CD19, CD20, CD22, CD30, CD33, CD37, CD45, CD56, CD66e, CD70, CD74, CD73, CD79b, CD138, CD147, CD223, EpCAM, Mucin 1, STEAP1, GPNMB, FGF2, FOLR1, EGFR, EGFRvIII, Tissue factor, c-MET, FGFR, Nectin 4, AGS-16, Guanylyl cyclase C, Mesothelin, SLC44A4, PSMA, EphA2, AGS-5, GPC-3, c-KIT, RoR1, PD-L1, CD27L, 5T4, Mucin16, NaPi2b, STEAP, SLITRK6, ETBR, BCMA, Trop-2, CEACAM5, SC-16, SLC39A6, Delta-like protein 3, or Claudin 18.2.

**14.** A composition comprising the antibody-drug conjugate or a salt thereof according to any one of claims 1 to 11.

**15.** Use of the antibody-drug conjugate or a salt thereof according to any one of claims 1 to 11 and/or the composition according to claim 14 in the manufacture of a medicament for the treatment of a tumor.

**16.** The use according to claim 15, **characterized by** that the tumor is associated with the positive or high expression of a tumor-associated antigen, which is, e.g., HER2, B7-H3, HER3, CD19, CD20, CD22, CD30, CD33, CD37, CD45, CD56, CD66e, CD70, CD74, CD73, CD79b, CD138, CD147, CD223, EpCAM, Mucin 1, STEAP1, GPNMB, FGF2, FOLR1, EGFR, EGFRvIII, Tissue factor, c-MET, FGFR, Nectin 4, AGS-16, Guanylyl cyclase C, Mesothelin, SLC44A4, PSMA, EphA2, AGS-5, GPC-3, c-KIT, RoR1, PD-L1, CD27L, 5T4, Mucin16, NaPi2b, STEAP, SLITRK6, ETBR, BCMA, Trop-2, CEACAM5, SC-16, SLC39A6, Delta-like protein 3, or Claudin 18.2.

**17.** A method for treating a tumor, comprising administering to a subject in need thereof the antibody-drug conjugate or a salt thereof according to any one of claims 1 to 11 and/or the composition according to claim 14.

Figure 1

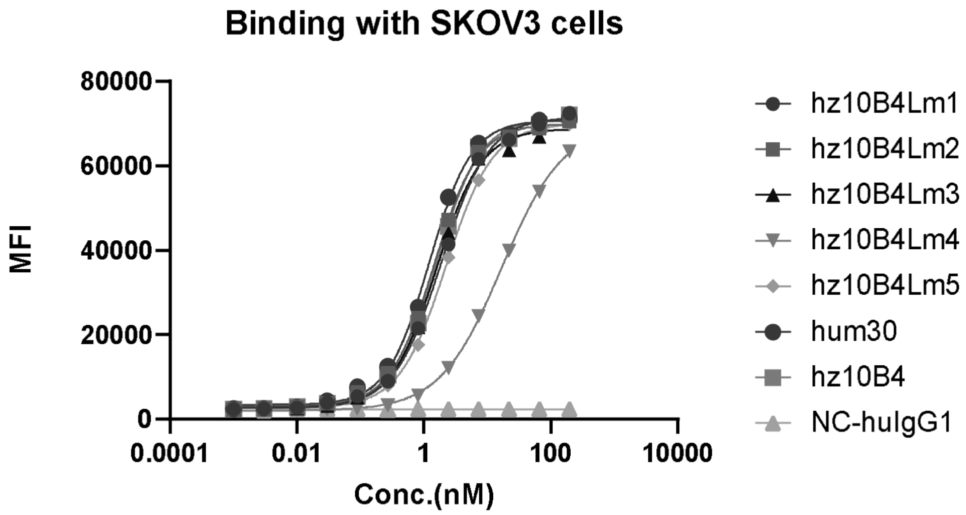


Figure 2

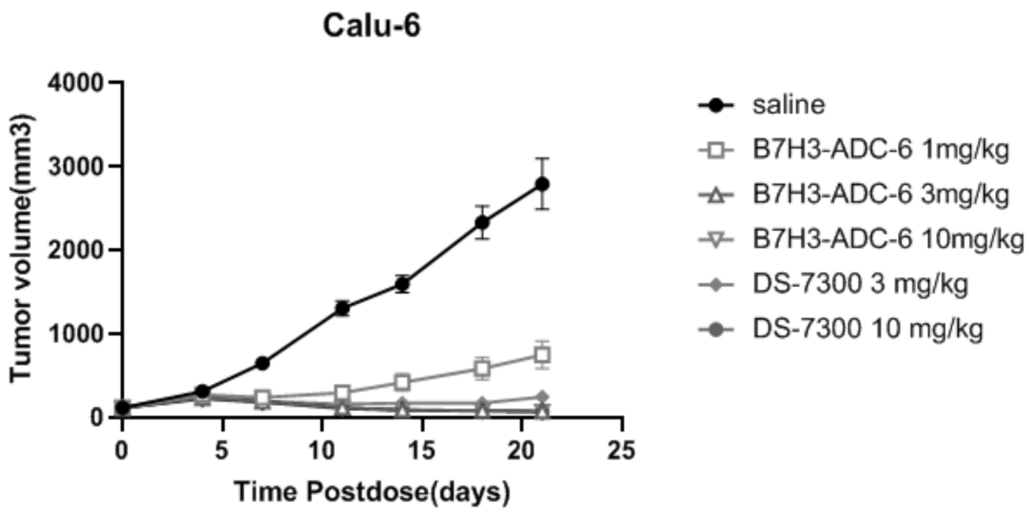

Figure 3

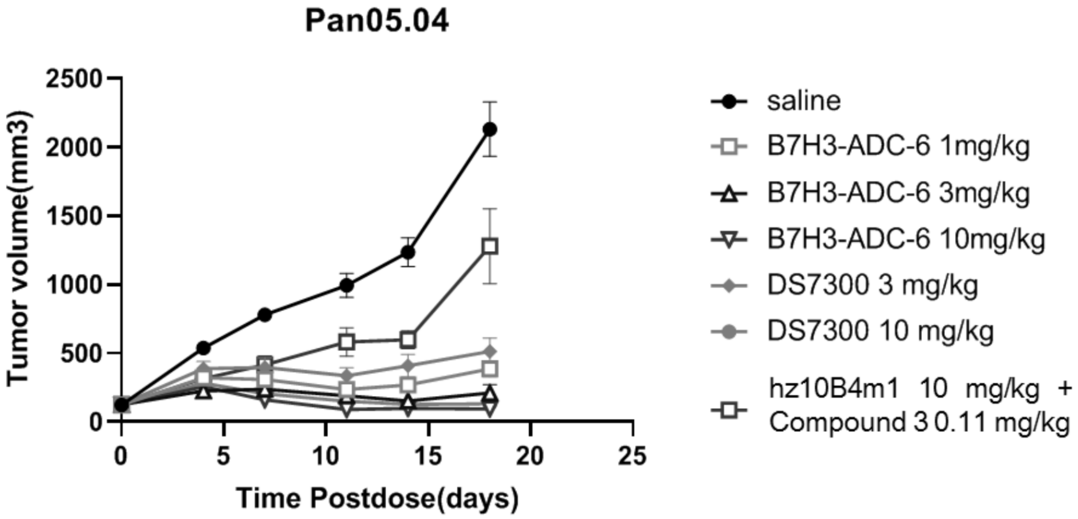


Figure 4

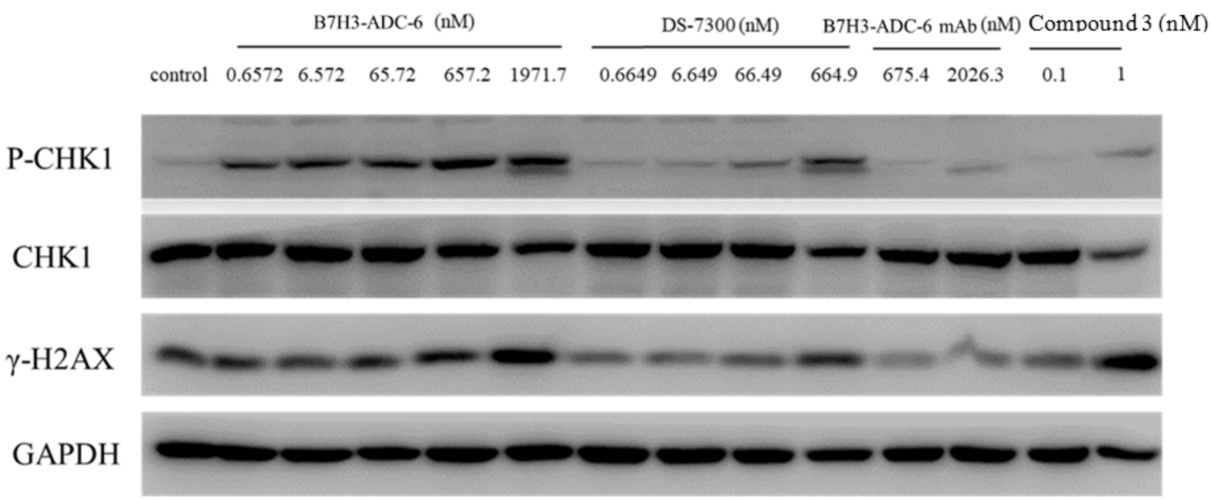

Figure 5

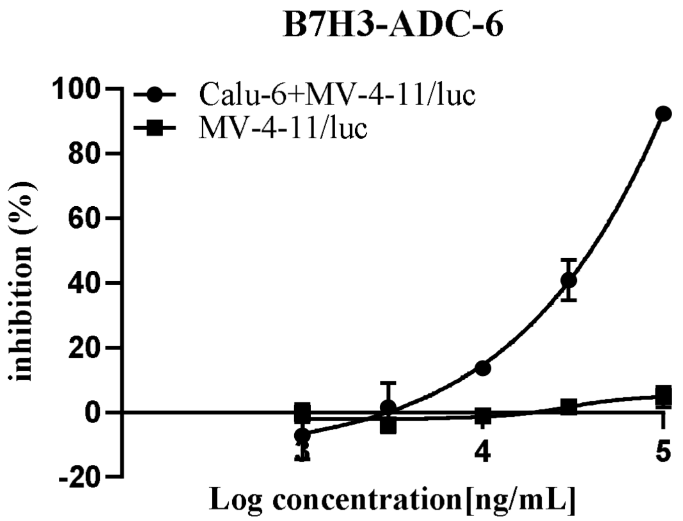


Figure 6

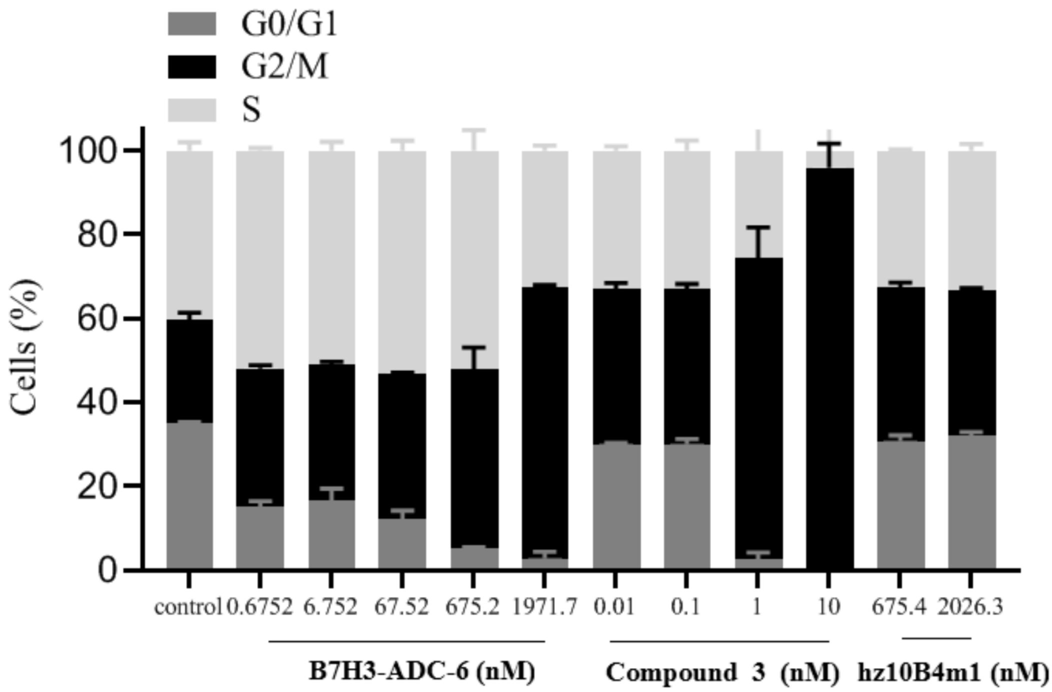

**INTERNATIONAL SEARCH REPORT**

International application No.
**PCT/CN2024/097511**

**A. CLASSIFICATION OF SUBJECT MATTER**

A61K47/68(2017.01)i; C07D491/22(2006.01)i; A61P35/00(2006.01)i

According to International Patent Classification (IPC) or to both national classification and IPC

**B. FIELDS SEARCHED**

Minimum documentation searched (classification system followed by classification symbols)

A61K, C07D, A61P

Documentation searched other than minimum documentation to the extent that such documents are included in the fields searched

Electronic data base consulted during the international search (name of data base and, where practicable, search terms used)

CNABS, CNTXT, DWPI, VEN, ENTXTC, ENTXT, CNKI, PUBMED, ISI_Web of Science, Science Direct, STNext: 江苏迈威康新药研发有限公司, 迈威(上海)生物科技股份有限公司, 喜树碱, 偶联物, 肿瘤, 癌, camptothecin, analog+, tumor, cancer, conjugate, B7H3, B7-H3, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| PX | WO 2023109965 A1 (MABWELL (SHANGHAI) BIOSCIENCE CO., LTD. et al.) 22 June 2023 (2023-06-22)<br>abstract, and claims 1-20 | 1, 3-17 |
| Y | WO 2018095422 A1 (SHANGHAI QINGRUN PHARMACEUTICAL TECHNOLOGY CO., LTD.) 31 May 2018 (2018-05-31)<br>abstract, and claims 1-27 | 1, 3-17 |
| Y | WO 2020063673 A1 (JIANGSU HENGRUI MEDICINE CO., LTD. et al.) 02 April 2020 (2020-04-02)<br>abstract, and claims 1-27 | 1, 3-17 |
| Y | EP 0495432 A1 (DAIICHI SEIYAKU CO., LTD. et al.) 22 July 1992 (1992-07-22)<br>abstract, claims 1-9, and embodiment 31 | 5-10, 12-17 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **12 September 2024** | **19 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/097511**

**C. DOCUMENTS CONSIDERED TO BE RELEVANT**

| Category* | Citation of document, with indication, where appropriate, of the relevant passages | Relevant to claim No. |
|---|---|---|
| Y | ADAMS, D.J. et al. "Camptothecin Analogs with Enhanced Activity against Human Breast Cancer Cells. I. Correlation of Potency with Lipophilicity and Persistence in the Cleavage Complex" *Cancer Chemotherapy and Pharmacology,* Vol. vol. 57, 20 August 2005 (2005-08-20), pp. 135-144 | 5-10, 12-17 |
| A | CN 114573695 A (MABWELL (SHANGHAI) BIOSCIENCE CO., LTD.) 03 June 2022 (2022-06-03) abstract, and claims 1-22 | 1-17 |
| A | WO 2019170131 A1 (FUDAN UNIVERSITY) 12 September 2019 (2019-09-12) abstract, and claims 1-18 | 1-17 |
| A | WO 2022228563 A1 (JIANGSU MABWELL HEALTH PHARMACEUTICAL R&D CO., LTD. et al.) 03 November 2022 (2022-11-03) abstract, and claims 1-21 | 1-17 |

Form PCT/ISA/210 (second sheet) (July 2022)

**INTERNATIONAL SEARCH REPORT**

International application No.
**PCT/CN2024/097511**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

**INTERNATIONAL SEARCH REPORT**

International application No.
**PCT/CN2024/097511**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **17**
because they relate to subject matter not required to be searched by this Authority, namely:

Claim 17 relates to a method for treating tumors, which falls within subject matter for which no search is required by the International Searching Authority as defined in PCT Rule 39.1(iv). A search is performed on the basis that the designation of the subject matter thereof is a pharmaceutical use.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/097511**

| Patent document cited in search report | Publication date (day/month/year) | Patent family member(s) | Publication date (day/month/year) |
|---|---|---|---|
| WO 2023109965 A1 | 22 June 2023 | KR 20240120744 A | 07 August 2024 |
| | | CA 3241157 A1 | 22 June 2023 |
| | | AU 2022408887 A1 | 18 July 2024 |
| WO 2018095422 A1 | 31 May 2018 | EP 3546448 A1 | 02 October 2019 |
| | | EP 3546448 A4 | 01 July 2020 |
| | | EP 3546448 B1 | 06 April 2022 |
| | | DK 3546448 T3 | 27 June 2022 |
| | | PL 3546448 T3 | 05 September 2022 |
| | | JP 2020510677 A | 09 April 2020 |
| | | JP 7058666 B2 | 22 April 2022 |
| | | CA 3044898 A1 | 31 May 2018 |
| | | CA 3044898 C | 05 April 2022 |
| | | KR 20190085539 A | 18 July 2019 |
| | | KR 102562760 B1 | 04 August 2023 |
| | | US 2019388555 A1 | 26 December 2019 |
| | | US 10987430 B2 | 27 April 2021 |
| | | ES 2921236 T3 | 22 August 2022 |
| WO 2020063673 A1 | 02 April 2020 | MX 2021003446 A | 15 June 2021 |
| | | JP 2022512568 A | 07 February 2022 |
| | | JP 7408646 B2 | 05 January 2024 |
| | | EP 3854816 A1 | 28 July 2021 |
| | | EP 3854816 A4 | 07 September 2022 |
| | | TW 202028242 A | 01 August 2020 |
| | | ZA 202102696 B | 25 October 2023 |
| | | CA 3114474 A1 | 02 April 2020 |
| | | AU 2019351427 A1 | 15 April 2021 |
| | | US 2021347894 A1 | 11 November 2021 |
| | | KR 20210068457 A | 09 June 2021 |
| | | BR 112021004829 A2 | 08 June 2021 |
| EP 0495432 A1 | 22 July 1992 | DE 69211023 D1 | 04 July 1996 |
| | | DE 69211023 T2 | 09 January 1997 |
| | | NO 920201 D0 | 15 January 1992 |
| | | NO 920201 L | 17 July 1992 |
| | | NO 180448 B | 13 January 1997 |
| | | NO 180448 C | 23 April 1997 |
| | | CA 2059305 A1 | 17 July 1992 |
| | | CA 2059305 C | 31 July 2001 |
| | | EP 0495432 B1 | 29 May 1996 |
| | | ES 2090366 T3 | 16 October 1996 |
| | | HK 1001561 A1 | 26 June 1998 |
| | | AU 1018592 A | 23 July 1992 |
| | | AU 640549 B2 | 26 August 1993 |
| | | GR 3020395 T3 | 30 September 1996 |
| | | KR 920014816 A | 25 August 1992 |
| | | KR 100191193 B1 | 15 June 1999 |
| | | ATE 138661 T1 | 15 June 1996 |
| | | FI 920194 A0 | 16 January 1992 |
| | | FI 920194 A | 17 July 1992 |
| | | FI 103047 B | 15 April 1999 |
| | | IE 920079 A1 | 29 July 1992 |

Form PCT/ISA/210 (patent family annex) (July 2022)

**INTERNATIONAL SEARCH REPORT**
**Information on patent family members**

International application No.
**PCT/CN2024/097511**

| Patent document cited in search report | | | Publication date (day/month/year) | Patent family member(s) | | | Publication date (day/month/year) |
|---|---|---|---|---|---|---|---|
| | | | | JPH | 0559061 | A | 09 March 1993 |
| | | | | JP | 3008226 | B2 | 14 February 2000 |
| | | | | RU | 2071476 | C1 | 10 January 1997 |
| | | | | DK | 0495432 | T3 | 21 October 1996 |
| CN | 114573695 | A | 03 June 2022 | EP | 4289862 | A1 | 13 December 2023 |
| | | | | JP | 2023551721 | A | 12 December 2023 |
| | | | | CA | 3201064 | A1 | 09 June 2022 |
| | | | | KR | 20230117189 | A | 07 August 2023 |
| | | | | AU | 2021390121 | A1 | 29 June 2023 |
| | | | | WO | 2022117040 | A1 | 09 June 2022 |
| WO | 2019170131 | A1 | 12 September 2019 | None | | | |
| WO | 2022228563 | A1 | 03 November 2022 | US | 2024226318 | A1 | 11 July 2024 |
| | | | | JP | 2024517776 | A | 23 April 2024 |
| | | | | KR | 20240004708 | A | 11 January 2024 |
| | | | | EP | 4331615 | A1 | 06 March 2024 |
| | | | | CA | 3217112 | A1 | 03 November 2022 |
| | | | | AU | 2022267613 | A1 | 23 November 2023 |
| | | | | MX | 2023012861 | A | 08 December 2023 |
| | | | | BR | 112023022549 | A2 | 02 January 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202310655433X
- CN 202111459992
- CN 114573695 A
- US 20160368990 A1
- WO 2020200880 A1
- CN 106349233 A
- WO 2021148501 A
- US 2004266803 A
- US 20190151328 A1
- WO 2018095422 A1
- WO 2020200880 A
- CN 104755494 B
- US 10227417 B2

### Non-patent literature cited in the description

- *Bioorganic & Medicinal Chemistry*, 2010, vol. 18 (9), 3140-3146
- *Journal of Medicinal chemistry*, 1998, vol. 41 (13), 2308-2318
- *Journal of Medicinal Chemistry*, 1989, vol. 32 (6), 1217-1230
- *J. Med. Chem.*, 2008, vol. 51, 3040-3044
- *Clin Cancer Res.*, 15 October 2004, vol. 10 (20), 7063-70